# EUROPEAN PATENT APPLICATION

(11) **EP 1 464 653 A1**
(43) Date of publication of application: **06.10.2004**
(21) Application number: 04006387.7
(22) Date of filing: 04.11.1998
(51) Int. Cl.: C07K 14/47, G01N 33/68, G01N 33/53, C07K 16/00, C12N 15/11, C12N 15/12, C12N 15/00, C12N 15/63, A61K 38/17, A61K 38/16, C12P 21/02

(54) **Human secreted proteinteins**

(30) Priority: 07.11.1997 US 64911 P; 07.11.1997 US 64912 P; 07.11.1997 US 64983 P; 07.11.1997 US 64900 P; 07.11.1997 US 64988 P; 07.11.1997 US 64987 P; 07.11.1997 US 64908 P; 07.11.1997 US 64984 P; 07.11.1997 US 64985 P; 17.11.1997 US 66094 P; 17.11.1997 US 66100 P; 17.11.1997 US 66089 P; 17.11.1997 US 66095 P; 17.11.1997 US 66090 P
(62) Divisional of application: 98956533.8
(71) Applicant: Human Genome Sciences, Inc., Rockville, MD 20850 (US)
(72) Inventor: Feng, Ping, Germantown, Maryland 20874 (US); Carter, Kenneth, North Potomac, Maryland 20878 (US); Endress, Gregory A., Florence, MA 01062 (US); Rosen, Craig A., Laytonsville, Maryland 20882 (US); Ruben, Steven M., Brookeville, Maryland 20833 (US); Janat, Fouad, Westerly, Rhode Island 02891 (US); Ni, Jian, Germantown, Maryland 20874 (US); Wei, Ying-Fei, Berkeley, CA 94704 (US); Moore, Paul A., Germantown, Maryland 20874 (US); Soppet, Daniel R., Centreville, Virginia 22020 (US); Kyaw, Hia, Frederick, Maryland 21703 (US); LaFleur, David W., N.W., Washington, D.C. 20015 (US); Olsen, Henrik S., Gaithersburg, Maryland 20878 (US); Yanggu, Shi, Gaithersburg, MD 20878 (US); Ebner, Reinhard, Gaithersburg, Maryland 20878 (US)
(74) Representative: VOSSIUS & PARTNER

(57) **Abstract**

The present invention relates to a human secreted protein and isolated nucleic acids containing the coding regions of the gene encoding such a protein. Also provided are vectors, host cells, antibodies, and recombinant methods for producing the said human secreted protein. The invention further relates to diagnostic and therapeutic methods useful for diagnosing and treating disorders related to this novel human secreted protein.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein , a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York; pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

The translation product of this gene shares sequence homology with transcytosis-associated protein (TAP), which is thought to be important in the docking of transport vesicles with their target membrane. The gene encoding the disclosed cDNA is thought to reside on chromosome 4. Accordingly, polynucleotides related to this invention are useful as a marker in linkage analysis for chromosome 4.

This gene is expressed primarily in developing brain, other embryonic tissue and placenta.

Therefore, polynucleotides and polypeptides of the invention are useful as reagents for differential identification of the tissue(s) or cell type(s) present in a biological sample and for diagnosis of diseases and conditions which include, but are not limited to, developmental and neurodegenerative diseases of the brain as well as other developmental anomalies or fetal deficiencies. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g. embryonic, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:136 as residues: Pro-51 to Arg-56, Lys-89 to Gln-94, Glu-144 to Gln-151, Gln-178 to Gln-183, Leu-224 to Gln-229, Tyr-284 to Pro-298, Lys-324 to Lys-334.

The tissue distribution in developing brain and placental tissues and the homology to transcytosis-associated protein (TAP) indicates that polynucleotides and polypeptides corresponding to this gene are useful for a host of conditions which arise as a result of a failure of, or deficiency in, the secretory or endocytic pathway. In addition, the expression in brain would suggest a role in the detection and treatment of brain tumors, developmental and behavioral disorders such as mania, depression, paranoia, addictive behavior and sleep disorders. Furthermore, expression within embryonic tissue and other cellular sources marked by proliferating cells indicates that this protein may play a role in the regulation of cellular division. Similarly, embryonic development also involves decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:11 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 996 of SEQ ID NO:11, b is an integer of 15 to 1010, where both a and b correspond to the positions of nucleotide residues shown in SEQ ID NO:11, and where b is greater than or equal to a + 14.

### FEATURES OF PROTEIN ENCODED BY GENE NO: 2

This gene is expressed primarily in human adrenal gland tumor.

Therefore, polynucleotides and polypeptides of the invention are useful as reagents for differential identification of the tissue(s) or cell type(s) present in a biological sample and for diagnosis of diseases and conditions which include, but are not limited to, endocrine disorders, particularly adrenal gland tumors. Similarly, polypeptides and antibodies directed to these polypeptides are useful in providing immunological probes for differential identification of the tissue(s) or cell type(s). For a number of disorders of the above tissues or cells, particularly of the adrenal gland, expression of this gene at significantly higher or lower levels may be routinely detected in certain tissues or cell types (e.g. endocrine, adrenal gland, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis or treatment of adrenal gland tumors. Furthermore, polynucleotides and polypeptides corresponding to this gene are useful for the detection, treatment, and/or prevention of various endocrine disorders and cancers, particularly Addisoni's disease, Cushingi's Syndrome, and disorders and/or cancers of the pancrease (e.g. diabetes mellitus), adrenal cortex, ovaries, pituitary (e.g., hyper-, hypopituitarism), thyroid (e.g. hyper-, hypothyroidism), parathyroid (e.g. hyper-,hypoparathyroidism) , hypothallamus, and testes. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 12 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1545 of SEQ ID NO: 12, b is an integer of 15 to 1559, where both a and b correspond to the positions of nucleotide residues shown in SEQ ID NO: 12, and where b is greater than or equal to a + 14.

### FEATURES OF PROTEIN ENCODED BY GENE NO: 3

When tested against U937 Myeloid cell lines, supernatants removed from cells containing this gene activated the GAS assay. Thus, it is likely that this gene activates myeloid cells, including their progenitors, through the Jak-STAT signal transduction pathway. The gamma activating sequence (GAS) is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells.

This gene is expressed primarily in small intestine.

Therefore, polynucleotides and polypeptides of the invention are useful as reagents for differential identification of the tissue(s) or cell type(s) present in a biological sample and for diagnosis of diseases and conditions which include, but are not limited to, a variety of gastrointestinal disorders including duodenal uclers. Similarly, polypeptides and antibodies directed to these polypeptides are useful in providing immunological probes for differential identification of the tissue(s) or cell type(s). For a number of disorders of the above tissues or cells, particularly of the gastrointestinal system, expression of this gene at significantly higher or lower levels may be routinely detected in certain tissues or cell types (e.g. gastrointestinal, smooth muscle, endothelial, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 138 as residues: Gln-77 to Pro-86.

The tissue distribution in small intestine indicates that the translation product of this gene is useful for the diagnosis and/or treatment of a number of disorders having to do with the gastrointestinal system, and specifically the small intestine, such as obstructions of the ileum, meckel's diverticulum, Crohn's disease, celiac sprue, tropical sprue, and lymphoma. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO:13 and may have been publicly available prior to conception of the present invention. Preferably; such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1575 of SEQ ID NO: 13, b is an integer of 15 to 1589, where both a and b correspond to the positions of nucleotide residues shown in SEQ ID NO: 13, and where b is greater than or equal to a + 14.

### FEATURES OF PROTEIN ENCODED BY GENE NO: 4

The translation product of this gene shares sequence homology with the mouse astrotactin protein, which is thought to be important in supporting neuronal migration along glial fibers. Additionally, astrotactin is thought to act as a ligand for neuron-glial binding during neuronal migration. The gene encoding the disclosed cDNA is thought to reside on chromosome 1. Accordingly, polynucleotides related to this invention are useful as a marker in linkage analysis for chromosome 1.

This gene is expressed primarily in brain tissue from a patient with Alzheimer's disease.

Therefore, polynucleotides and polypeptides of the invention are useful as reagents for differential identification of the tissue(s) or cell type(s) present in a biological sample and for diagnosis of diseases and conditions which include, but are not limited to, neural or CNS disorders, particularly neurodegenerative disorders such as Alzheimer's disease. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:139 as residues: Gln-43 to Trp-53, Arg-69 to Ser-76.

The tissue distribution in brain combined with the homology to mouse astrotactin indicates that polynucleotides and polypeptides corresponding to this gene are useful for the diagnosis and/or treatment of CNS diseases, such as Alzheimer's disease. Moreover, polynucleotides and polypeptides corresponding to this gene are useful for the detection/treatment of neurodegenerative disease states, behavioural disorders, or inflamatory conditions such as Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:14 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1241 of SEQ ID NO: 14, b is an integer of 15 to 1255, where both a and b correspond to the positions of nucleotide residues shown in SEQ ID NO:14, and where b is greater than or equal to a + 14.

### FEATURES OF PROTEIN ENCODED BY GENE NO: 5

The translation product of this gene shares sequence homology with transporter protein, which is thought to be important in metabolic and respiratory functions.

This gene is expressed primarily in T-cell lymphoma and dendritic cells, and to a lesser extent in placenta.

Therefore, polynucleotides and polypeptides of the invention are useful as reagents for differential identification of the tissue(s) or cell type(s) present in a biological sample and for diagnosis of diseases and conditions which include, but are not limited to, haemopoietic disorders, particularly cancer including T-cell lymphoma and disorders associated with embryogenesis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 140 as residues: Thr-87 to Trp-94.

The tissue distribution in T-cell lymphoma and dendritic cells and the homology to transporter protein indicates that polynucleotides and polypeptides corresponding to this gene are useful for treatment and diagnosis of haemopoietic disorders such as cancer, particularly T-cell lymphoma and disorders associated with embryogenesis. Furthermore, this gene product may play a role in the survival, proliferation, and/or differentiation of hematopoieitic lineages. Expression of this gene product in T cells and primary dendritic cells also strongly indicates a role for this protein in immune function and immune surveillance.

Many polynucleotide sequences, such as EST sequences, are publicly available and accessible through sequence databases. Some of these sequences are related to SEQ ID NO: 15 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1177 of SEQ ID NO:15, b is an integer of 15 to 1191, where both a and b correspond to the positions of nucleotide residues shown in SEQ ID NO: 15, and where b is greater than or equal to a + 14.

### FEATURES OF PROTEIN ENCODED BY GENE NO: 6

This gene is expressed primarily in the liver, and to a lesser extent, in testis.

Therefore, polynucleotides and polypeptides of the invention are useful as reagents for differential identification of the tissue(s) or cell type(s) present in a biological sample and for diagnosis of diseases and conditions which include, but are not limited to, hepatic, reproductive, or endocrine disorders, particularly hepatoma or male infertility. Similarly, polypeptides and antibodies directed to these polypeptides are useful in providing immunological probes for differential identification of the tissue(s) or cell type(s). For a number of disorders of the above tissues or cells, particularly of the immune and hematopoetic systems, expression of this gene at significantly higher or lower levels may be routinely detected in certain tissues or cell types (e.g. hepatic, reproductive, endocrine, testical, immune, and cancerous and wounded tissues) or bodily fluids (e.g. lymph, serum, serminal fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:141 as residues: Ser-21 to Trp-34, Cys-68 to Gly-89, Cys-122 to Phe-133.

The tissue distribution in liver tissue indicates that polynucleotides and polypeptides corresponding to this gene are useful for the diagnosis and treatment of liver disorders, particularly those affecting the immune and hematopoetic systems such as hepatomas. Furthermore, the protein product of this gene would also be useful for the detection and treatment hepatoblastoma, jaundice, hepatitis, or liver metabolic diseases and conditions that are attributable to the differentiation of hepatocyte progenitor cells. Furthermore, the expression within testis indicates that the protein may show utility in the treatment and/or detection of a variety of reproductive disorders such as male infertility, impotence, and may even be useful as a contraceptive. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 16 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1172 of SEQ ID NO:16, b is an integer of 15 to 1186, where both a and b correspond to the positions of nucleotide residues shown in SEQ ID NO: 16, and where b is greater than or equal to a + 14.

### FEATURES OF PROTEIN ENCODED BY GENE NO: 7

The translation product of this gene shares sequence homology with urokinase receptor which is thought to be important in cell matrix remodeling and cell movement. In specific embodiments, polypeptides of the invention comprise the following amino acid sequence:
FYIADHSFTARPTLRMFRISAVVATDKMTFTSGGTLFGDGCASSVA GEVMNC QTVLCILWTPFVFCPSIAVIIIPCVFFSKALEAIWKWCRVERRPHIIEVDVLGKCP AF (SEQ ID NO:261), RPTLRMFRISAVVATDKMTFTSGGT (SEQ ID NO:262), PSIAVIIIPCVFTSKALEAIWKWCRVER (SEQ ID NO:263), TSVSFHHRYKSS DRPAHKVS (SEQ ID NO:264). Polynucleotides encoding these polypeptides are also encompassed by the invention.

This gene is expressed primarily in fetal lung, breast, and Hodgkin's Lymphoma II.

Therefore, polynucleotides and polypeptides of the invention are useful as reagents for differential identification of the tissue(s) or cell type(s) present in a biological sample and for diagnosis of diseases and conditions which include, but are not limited to, pulmonary, reproductive, immune, or hematopoietic disorders, particularly cell growth and differentiation conditions. Similarly, polypeptides and antibodies directed to these polypeptides are useful in providing immunological probes for differential identification of the tissue(s) or cell type(s). For a number of disorders of the above tissues or cells, particularly of the fetal lung, breast, and tissues involved in Hodgkin's Lymphoma II expression of this gene at significantly higher or lower levels may be routinely detected in certain tissues and cell types (e.g. Pulmonary, immune, reproductive, and cancerous and wounded tissues) or bodily fluids (e.g.lymph, amniotic fluid, pulmonary surfactant or sputum, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 142 as residues: Asn-32 to Asp-38, Thr-40 to Phe-46, Asn-53 to Gln-74, Ser-84 to Ile-91, Cys-95 to Glu-100, Ser-109 to Cys-121.

The tissue distribution in proliferating and differentiating tissues, combined with the homology to a urokinase receptor indicates that polynucleotides and polypeptides corresponding to this gene are useful for the diagnosis and treatment of cell growth and differentiation disorders, particularly of the lung, renal, breast, immune and endothelial tissues. Moreover, the expression within fetal tissue and other cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 17 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1168 of SEQ ID NO: 17, b is an integer of 15 to 1182, where both a and b correspond to the positions of nucleotide residues shown in SEQ ID NO:17, and where b is greater than or equal to a + 14.

### FEATURES OF PROTEIN ENCODED BY GENE NO: 8

The translation product of this gene shares sequence homology with cell adhesion molecules, which are implicated in cell migration, axonal guidance and fasiculation, and growth and tumorogenesis. When tested against U937 cell lines, supernatants removed from cells containing this gene activated the GAS (gamma activating sequence) promoter element. Thus, it is likely that this gene activates myeloid cells, including their progenitors, through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
RHNDFNKLSYTECNNMNKRMAKPEKKKGSVKSSLGIFLGPNCHLISSLFLFS VSLYPFATQF PFHYVLIFIIQAFGLCLPLTERQEAKSGLGGLCPDYTWPC PCLLVSCLSLLRL (SEQ ID NO:265), CEVFSWHFPWSKLSPHLFLVSFLCIPL SLCHTV SFSLCSNIYNPGLRTMLAPHRETGGQVWAGWALSRLHVALPMSLG VLSLPAPTVTVVRMEGGDWKVCEQL GQCTYSHRMTK (SEQ ID NO:266), KRMAKPEKKKGSVKSSLGIFLGP (SEQ ID NO:267), and/or YNPGLRTMLA PHRETGGQVWAGWALSRLHVA (SEQ ID NO:268). Polynucleotides encoding these polypeptides are also encompassed by the invention.

This gene is expressed primarily in the meningima, melanocytes, and to a lesser extent, in breast.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disease states and behavioral disorders, in addition to integumentary or reproductive disorders, particularly of the breast. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g.neural, integumentary, breast, reproductive, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 143 as residues: Asn-71 to Asp-79.

The tissue distribution in menigima combined with the homology to cell adhesion molecules and the detected GAS biological activity indicates that polynucleotides and polypeptides corresponding to this gene are useful for the treatment and/or detection of neurodegenerative disease states and behavioural disorders such as Alzheimer's Disease, Parkinson's Disease, Huntington's Disease, schizophrenia, mania, dementia, paranoia, obsessive compulsive disorder and panic disorder. Moreover, the expression within melanocytes and breast tissue indicates polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Boweni's disease, basal cell carcinoma, squamous cell carcinoma, malignant melanoma, Pageti's disease, mycosis fungoides, and Kaposii's sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. In addition, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Moreover, the protein product of this gene may also be useful for the treatment or diagnosis of various connective tissue disorders such as arthritis, trauma, tendonitis, chrondomalacia and inflammation, autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Furthermore, This protein may show utility in modulating the immune systems response to various degenerative neural conditions based upon the detected GAS biological activity. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 18 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1157 of SEQ ID NO:18, b is an integer of 15 to 1171, where both a and b correspond to the positions of nucleotide residues shown in SEQ ID NO: 18, and where b is greater than or equal to a + 14.

### FEATURES OF PROTEIN ENCODED BY GENE NO: 9

This gene is expressed primarily in fetal liver and spleen, and infant brain.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, neural, and developmental disorders. Similarly, polypeptides and antibodies directed to these polypeptides are useful in providing immunological probes for differential identification of the tissue(s) or cell type(s). For a number of disorders of the above tissues or cells, particularly of the immune and developmental systems, expression of this gene at significantly higher or lower levels may be routinely detected in certain tissues and cell types (e.g. Immune, hematopoietic, neural, developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 144 as residues: Thr-187 to Lys-192, Asn-255 to Leu-262.

The tissue distribution of this gene in fetal liver spleen indicates a key role in the development of the immune system. Thus this gene could be used in the treatment and/or detection of immune disorders including arthritis, asthma, immunodeficiency diseases and leukemia. Expression in infant brain also indicates a role in the treatment and/or detection of neurodegenerative disease states and behavioural disorders such as Alzheimers Disease, Parkinsons Disease, Huntintons Disease, schizophrenia, mania, dementia, paranoia, obsessive compulsive disorder and panic disorder. Moreover, expression within fetal tissue and other cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 19 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1323 of SEQ ID NO: 19, b is an integer of 15 to 1337, where both a and b correspond to the positions of nucleotide residues shown in SEQ ID NO: 19, and where b is greater than or equal to a + 14.

### FEATURES OF PROTEIN ENCODED BY GENE NO: 10

This gene is expressed primarily in breast, and to a lesser extent in liver.

Therefore, polynucleotides and polypeptides of the invention are useful as reagents for differential identification of the tissue(s) or cell type(s) present in a biological sample and for diagnosis of diseases and conditions which include, but are not limited to, breast cancer, hepatoblastoma, hepatitis, liver metabolic diseases and conditions that are attributable to the differentiation of hepatocyte progenitor cells. Similarly, polypeptides and antibodies directed to these polypeptides are useful in providing immunological probes for differential identification of the tissue(s) or cell type(s). For a number of disorders of the above tissues or cells, particularly of the breast and liver, expression of this gene at significantly higher or lower levels may be routinely detected in certain tissues or cell types (e.g. breast, liver, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:145 as residues: Gln-29 to Gly-38, Lys-57 to Asp-62.

The tissue distribution indicates that polynucleotides and polypeptides corresponding to this gene are useful for the detection and treatment of liver disorders and cancers (e.g. hepatoblastoma, jaundice, hepatitis, liver metabolic diseases), and conditions that are attributable to the differentiation of hepatocyte progenitor cells. In addition, the expression in breast would suggest a possible role in the detection and treatment of breast cancer.

Many polynucleotide sequences, such as EST sequences, are publicly available and accessible through sequence databases. Some of these sequences are related to SEQ ID NO:20 and may have been publicly available prior to conception of the present invention. Preferably, such related polynucleotides are specifically excluded from the scope of the present invention. To list every related sequence is cumbersome: Accordingly, preferably excluded from the present invention are one or more polynucleotides comprising a nucleotide sequence described by the general formula of a-b, where a is any integer between 1 to 1148 of SEQ ID NO:20, b is an integer of 15 to 1162, where both a and b correspond to the positions of nucleotide residues shown in SEQ ID NO:20, and where b is greater than or equal to a + 14.

### FEATURES OF PROTEIN ENCODED BY GENE NO: 11

This gene is expressed primarily in brain, and to a lesser extent in retina.

Therefore, polynucleotides and polypeptides of the invention are useful as reagents for differential identification of the tissue(s) or cell type(s) present in a biological sample and for diagnosis of diseases and conditions which include, but are not limited to, developmental, degenerative and behavioral diseases of the brain such as depression, schizophrenia, Alzheimer's disease, Parkinson's disease; Huntington's disease, specific brain tumors, aphasia, mania, depression, dementia, paranoia, addictive behavior and sleep disorders as well as conditions that affect vision and function of the eye such as retinoblastoma and cataracts. Similarly, polypeptides and antibodies directed to these polypeptides are useful in providing immunological probes for differential identification of the tissue(s) or cell type(s). For a number of disorders of the above tissues or cells, particularly of the brain and eye, expression of this gene at significantly higher or lower levels may be routinely detected in certain tissues or cell types (e.g., brain, retina, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:146 as residues: Pro-46 to Gln-60, Pro-68 to Gly-75, Leu-78 to Ala-86, Gln-93 to Asp-98.

The tissue distribution indicates that polynucleotides and polypeptides corresponding to this gene are useful for the treatment and diagnosis of developmental, degenerative and behavioral diseases, and conditions of the brain such as aphasia, depression, schizophrenia, Alzheimer's disease, Parkinson's disease, Huntington's disease, specific brain tumors, mania, depression, dementia, paranoia, addictive behavior and sleep disorders. In addition, the expression in retina would also suggest a role for this gene product in the diagnosis and treatment of conditions that affect vision and function of the eye such as retinoblastoma, myopia, hyperopia and cataracts.

Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1823 of SEQ ID NO:21, b is an integer of 15 to 1837, where both a and b correspond to the positions of nucleotide residues shown in SEQ ID NO:21, and where b is greater than or equal to a + 14.

### FEATURES OF PROTEIN ENCODED BY GENE NO: 12

The gene encoding the disclosed cDNA is thought to reside on chromosome 8. Accordingly, polynucleotides related to this invention are useful as a marker in linkage analysis for chromosome 8. One embodiment of this gene comprises polypeptides of the following amino acid sequence:
MSPYASQGFPFLPPYPPQEANRSITSLSVADTVSSSTTSHTTAKPAAPSFGVLSN LPLPIPTVDASIPTSQNGFGYKMPDVPDAFPELSELSVSQLTDMNEQEEVLLEQF LTLPQLKQLKIITDKDDLVKSIEELARKNLLLEPSLEAKRQTVLDKYELLTQMKSTF EKKMQRQHELSESCSASALQARLKVAAHEAEEESDNIAEDFLEGKMEIDDFLSS FMEKRTICHCRRAKEEKLQQAIAMHSQFHAPL (SEQ ID NO:269), LPPYPPQE ANRSITSLSVADTVS (SEQ ID NO:270), TAKPAAPSFGVLSNLPLPIPTVDASIP (SEQ ID NO:271), PDVPDAFPELSELSVSQLTDMNEQE (SEQ ID NO:272), QFLTL PQLKQIITDKDDLVKSIEELARKN (SEQ ID NO:273), RQTVLDKYELLTQ MKS TFEKKMQRQ (SEQ ID NO:274), ASALQARLKVAAHEAEEESDNIAEDFLE (SEQ ID NO:275), and/or MEKRTICHCRRAKEEKLQQAIAMHSQF )SEQ ID NO: 276). An additional embodiment is the polynucleotides encoding these polypeptides.

This gene is expressed primarily in breast and placenta, and to a lesser extent in a variety of other tissues and cell types.

Therefore, polynucleotides and polypeptides of the invention are useful as reagents for differential identification of the tissue(s) or cell type(s) present in a biological sample and for diagnosis of diseases and conditions which include, but are not limited to, breast and endometrial cancers as well as prenatal disorders and deficiencies. Similarly, polypeptides and antibodies directed to these polypeptides are useful in providing immunological probes for differential identification of the tissue(s) or cell type(s). For a number of disorders of the above tissues or cells, particularly of the breast and reproductive system, expression of this gene at significantly higher or lower levels may be routinely detected in certain tissues or cell types (e.g. breast, placental, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection and treatment of breast cancer, ovarian and other endometrial cancers, infertility and pre-natal disorders. Furthermore, the tissue distribution indicates that polynucleotides and polypeptides corresponding to this gene are useful for treating female infertility. The protein product is likely involved in preparation of the endometrium of implantation and could be administered either topically or orally. Alternatively, this gene could be transfected in gene-replacement treatments into the cells of the endometrium and the protein products could be produced. Similarly, these treatments could be performed during artificial insemination for the purpose of increasing the likelyhood of implantation and development of a healthy embryo. In both cases this gene or its gene product could be administered at later stages of pregnancy to promote heathy development of the endometrium.

Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1040 of SEQ ID NO:22, b is an integer of 15 to 1054, where both a and b correspond to the positions of nucleotide residues shown in SEQ ID NO:22, and where b is greater than or equal to a + 14.

### FEATURES OF PROTEIN ENCODED BY GENE NO: 13

This gene is expressed primarily in anergic T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly autoimmune disorders such as lupus. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:148 as residues: Lys-49 to Gln-57, Arg-63 to Ala-69.

The tissue distribution in T-cells indicates that the polypeptides or polynucleotides are useful for treatment, prophylaxis, and diagnosis of immune and autoimmune diseases, such as lupus, transplant rejection, allergic reactions, arthritis, asthma, immunodeficiency diseases, leukemia, and AIDS. The expression observed predominatly in hematopoietic cells also indicates that the polynucleotides or polypeptides are important in treating and/or detecting hematopoietic disorders, such as graft versus host reaction, graft versus host disease, transplant rejection, myelogenous leukemia, bone marrow fibrosis, and myeloproliferative disease. The polypeptides or polynucleotides are also useful to enhance or protect proliferation, differentiation, and functional activation of hematopoietic progenitor cells (e.g., bone marrow cells), useful in treating cancer patients undergoing chemotherapy or patients undergoing bone marrow transplantation. The polypeptides or polynucleotides are also useful to increase the proliferation of peripheral blood leukocytes, which can be used in the combat of a range of hematopoietic disorders, including immmunodeficiency diseases, leukemia, and septicemia. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1052 of SEQ ID NO:23, b is an integer of 15 to 1066, where both a and b correspond to the positions of nucleotide residues shown in SEQ ID NO:23, and where b is greater than or equal to a + 14.

### FEATURES OF PROTEIN ENCODED BY GENE NO: 14

The translation product of this gene shares sequence homology with a drought-induced protease inhibitor from soybean. As a result, the protein product of this gene may show utility in the treatment and/or prevention of a variety of proliferative disorders (e.g. for inhibition of key proteolytic events during cellular metabolism of the tumor which may lead to cessation of mitosis) or for the treatment of degenerative conditions where the inhibition of aberrant proteolysis may lead to cessation of degeneration and ultimately in immune protection.

This gene is expressed primarily in the kidney.

Therefore, polynucleotides and polypeptides of the invention are useful as reagents for differential identification of the tissue(s) or cell type(s) present in a biological sample and for diagnosis of diseases and conditions which include, but are not limited to, disorders of the kidney. Similarly, polypeptides and antibodies directed to these polypeptides are useful in providing immunological probes for differential identification of the tissue(s) or cell type(s). For a number of disorders of the above tissues or cells, particularly of the urogenital system, expression of this gene at significantly higher or lower levels may be routinely detected in certain tissues or cell types (e.g. kidney, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 149 as residues: Glu-48 to Arg-56, Ser-61 to Gly-66.

The tissue distribution in kidney tissue combined with the homology to a protease inhibitor indicates that polynucleotides and polypeptides corresponding to this gene are useful for the diagnosis and treatment of disorders affecting the kidney. Furthermore,this gene or gene product could be used in the treatment and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 914 of SEQ ID NO:24, b is an integer of 15 to 928, where both a and b correspond to the positions of nucleotide residues shown in SEQ ID NO:24, and where b is greater than or equal to a + 14.

### FEATURES OF PROTEIN ENCODED BY GENE NO: 15

When tested against U937 cell lines, supernatants removed from cells containing this gene activated the GAS (gamma activating sequence) promoter element. Thus, it is likely that this gene activates myeloid cells, including their progenitors, through the JAK-STAT signal transduction pathway. GAS is a promoter element found upstream of many genes which'are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence: TRPVFLSMTPLKGIKSVILPQVFLCAYMAAFNSINGNRSYTCKPLERSLLMAGA VASSTFLGVIPQFVQ (SEQ ID NO:277), PLKGIKSVILPQVFLCAYMAA (SEQ ID NO:278), and/or AFNSINGNRSYTCKPLERSLL (SEQ ID NO:279). Polynucleotides encoding these polypeptides are also encompassed by the invention. The gene encoding the disclosed cDNA is believed to reside on chromosome 10. Accordingly, polynucleotides related to this invention are useful as a marker in linkage analysis for chromosome 10.

This gene is expressed primarily in B cell and T cell lymphomas.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly B cell and T cell lymphomas, infections, multiple myeloma, immunodeficiencies, and inflammatory conditions. Similarly, polypeptides and antibodies directed to these polypeptides are useful in providing immunological probes for differential identification of the tissue(s) or cell type(s). For a number of disorders of the above tissues or cells, particularly immune or hematopoietic disorders, such as B- and T-cell lymphomas, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:150 as residues: Phe-85 to Gly-96, Glu-133 to Thr-143.

The tissue distribution in B- and T-cell lymphomas, combined with the detected GAS biological activity indicates that polynucleotides and polypeptides corresponding to this gene are useful for the diagnosis and treatment of a variety of immune disorders, particularly proliferative conditions such as cancer and leukemias. In addition, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 952 of SEQ ID NO:25, b is an integer of 15 to 966, where both a and b correspond to the positions of nucleotide residues shown in SEQ ID NO:25, and where b is greater than or equal to a + 14.

### FEATURES OF PROTEIN ENCODED BY GENE NO: 16

The protein product of this gene was found to have homology to the Poly(A) polymerase of Bos taurus, which is known to be important in the creation of the 3' poly(A) tail of mRNA's. The gene encoding the disclosed cDNA is believed to reside on chromosome 14. Accordingly, polynucleotides related to this invention are useful as a marker in linkage analysis for chromosome 14.

This gene is expressed primarily in brain, and to a lesser extent, in prostate.

Therefore, polynucleotides and polypeptides of the invention are useful as reagents for differential identification of the tissue(s) or cell type(s) present in a biological sample and for diagnosis of diseases and conditions which include, but are not limited to, neural disorders, such as neurodegenerative disease states and behavioral conditions, in addition to reproductive disorders, particularly of the prostate. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues and cell types (e.g.neural, reproductive, and cancerous and wounded tissues) or bodily fluids (e.g.lymph, serum, seminal fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:151 as residues: Glu-47 to Ser-52.

The tissue distribution in brain indicates that polynucleotides and polypeptides corresponding to this gene are useful for the detection/treatment of neurodegenerative disease states and behavioural disorders such as Alzheimers Disease, Parkinsons Disease, Huntingtons Disease, schizophrenia, mania, dementia, paranoia, obsessive compulsive disorder and panic disorder. Moreover, expression of the gene in prostate indicates that polynucleotides and polypeptides corresponding to this gene are useful for the detection or treatment of prostate disorders including benign prostate hyperplasia, prostate cancer, and metabolic disorders. The homology to the PAP polyA polymerase indicates that the protein product of this gene, antibodies directed to this protein, or the gene encoding this protein via a gene therapy approach, may show utility as a preventative therapy for proliferative conditions. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:26 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1132 of SEQ ID NO:26, b is an integer of 15 to 1146, where both a and b correspond to the positions of nucleotide residues shown in SEQ ID NO:26, and where b is greater than or equal to a + 14.

### FEATURES OF PROTEIN ENCODED BY GENE NO: 17

This gene is expressed primarily in epididymus.

Therefore; polynucleotides and polypeptides of the invention are useful as reagents for differential identification of the tissue(s) or cell type(s) present in a biological sample and for diagnosis of diseases and conditions which include, but are not limited to, diseases of the reproductive organs. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g. reproductive, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:152 as residues: Met-1 to Pro-6, Glu-58 to Cys-63, Glu-65 to Gly-72, Thr-74 to Val-87.

The tissue distribution in epididymus indicates that polynucleotides and polypeptides corresponding to this gene are useful for the diagnosis and treatment of disorders of the epididymus and reproductive organs. Furthermore, the tissue distribution indicates that the protein product of this gene is useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents as such agents (antagonists) are useful as male contraceptive agents. Similarly, the protein is believed to by useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications.

Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 788 of SEQ ID NO:27, b is an integer of 15 to 802, where both a and b correspond to the positions of nucleotide residues shown in SEQ ID NO:27, and where b is greater than or equal to a + 14.

### FEATURES OF PROTEIN ENCODED BY GENE NO: 18

This gene is expressed primarily in synovium and rhabdomyosarcoma.

Therefore, polynucleotides and polypeptides of the invention are useful as reagents for differential identification of the tissue(s) or cell type(s) present in a biological sample and for diagnosis of diseases and conditions which include, but are not limited to, muscular skeletal system and cancer. Similarly, polypeptides and antibodies directed to these polypeptides are useful in providing immunological probes for differential identification of the tissue(s) or cell type(s). For a number of disorders of the above tissues or cells, particularly of the muscular skeletal system, expression of this gene at significantly higher or lower levels may be routinely detected in certain tissues or cell types (e.g. musculo-skeletal, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:153 as residues: Trp-30 to Val-35, Lys-44 to Arg-49.

The tissue distribution indicates that polynucleotides and polypeptides corresponding to this gene are useful for the treatment and diagnosis of disorders of the muscular skeletal system and cancer. Furthermore, the expression of this gene product in synovium would suggest a role in the detection and treatment of disorders and conditions affecting the skeletal system, in particular osteoporosis as well as disorders afflicting connective tissues (e.g. arthritis, trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1155 of SEQ ID NO:28, b is an integer of 15 to 1169, where both a and b correspond to the positions of nucleotide residues shown in SEQ ID NO:28, and where b is greater than or equal to a + 14.

### FEATURES OF PROTEIN ENCODED BY GENE NO: 19

The gene encoding the disclosed cDNA is thought to reside on chromosome 5. Accordingly, polynucleotides related to this invention are useful as a marker in linkage analysis for chromosome 5.

This gene is expressed primarily in fetal liver/spleen, and to a lesser extent, in tonsils.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, or hepatic disorders, particularly mutiple myeloma, immunodeficiencies, and cancers. Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues or cell types (e.g. immune, cancerous and wounded tissues) or bodily fluids (e.g. lymph, serum, bile, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 154 as residues: Asp-27 to Ser-36.

Expression of this gene at either the RNA or protein level could be used as a diagnostic indicator of hepatic cancer. Furthermore, the tissue distribution in fetal liver and tonsil tissue indicates that polynucleotides and polypeptides corresponding to this gene are useful for the diagnosis and treatment of a variety of immune system disorders. Moreover, the protein product of this gene may play a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1452 of SEQ ID NO:29, b is an integer of 15 to 1466, where both a and b correspond to the positions of nucleotide residues shown in SEQ ID NO:29, and where b is greater than or equal to a + 14.

### FEATURES OF PROTEIN ENCODED BY GENE NO: 20

This gene is expressed primarily in human brain.

Therefore, polynucleotides and polypeptides of the invention are useful as reagents for differential identification of the tissue(s) or cell type(s) present in a biological sample and for diagnosis of diseases and conditions which include, but are not limited to, disorders or diseases of the central nervous system. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates that polynucleotides and polypeptides corresponding to this gene are useful for the diagnosis and the treatment of CNS disorders. Furthermore, polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1212 of SEQ ID NO:30, b is an integer of 15 to 1226, where both a and b correspond to the positions of nucleotide residues shown in SEQ ID NO:30, and where b is greater than or equal to a + 14.

### FEATURES OF PROTEIN ENCODED BY GENE NO: 21

In specific embodiments, polypeptides of the invention comprise the following amino acid sequence:
MSDFEKVDISVHQHIHVGPLLLMTTESWGPSCAPSPALLSGHTAASFTHTLGG VLGCPPYHKFYSS AHTSDHRKETNKVEEGRWVDVTRSLGNFNFRRKFFC VSELLICGIFLDSSWKLQINSNDCKVL (SEQ ID NO:280), VGPLLLMTTESW GPSCAPSPALLSGHTAAS (SEQ ID NO:281), and/or ETNKVEEGRWVDVTRS LGNFNFRRKFF (SEQ ID NO:282). Polynucleotides encoding these polypeptides are also encompassed by the invention.

This gene is expressed primarily in fetal spleen or liver, adult spleen, and to a lesser extent, in activated T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly abnormal proliferation or activation of hematopoeitic cells, particularly of T-cells and their progenitors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:156 as residues: Arg-19 to Phe-24, Ala-44 to Asp-51, Glu-60 to Ile-66.

The tissue distribution in spleen tissues and T-cells indicates that polynucleotides and polypeptides corresponding to this gene are useful for modulating or detecting the abnormal proliferation or activation of T-cells and immune cell precursor cells. Moreover, expression within fetal spleen indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Similarly, This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1080 of SEQ ID NO:31, b is an integer of 15 to 1094, where both a and b correspond to the positions of nucleotide residues shown in SEQ ID NO:31, and where b is greater than or equal to a + 14.

### FEATURES OF PROTEIN ENCODED BY GENE NO: 22

This gene is expressed primarily in the amygdala.

Therefore, polynucleotides and polypeptides of the invention are useful as reagents for differential identification of the tissue(s) or cell type(s) present in a biological sample and for diagnosis of diseases and conditions which include, but are not limited to, developmental, degenerative and behavioral diseases of the brain such as depression, schizophrenia, Alzheimer's disease, Parkinson's disease, Huntington's disease, specific brain tumors, aphasia, mania, depression, dementia, paranoia, addictive behavior and sleep disorders. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:157 as residues: Pro-94 to Ala-107.

The tissue distribution indicates that polynucleotides and polypeptides corresponding to this gene are useful for the treatment and diagnosis of developmental, degenerative and behavioral diseases and conditions of the brain such as aphasia, depression, schizophrenia, Alzheimer's disease, Parkinson's disease, Huntington's disease, specific brain tumors, mania, depression, dementia, paranoia, addictive behavior and sleep disorders.

Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1023 of SEQ ID NO:32, b is an integer of 15 to 1037, where both a and b correspond to the positions of nucleotide residues shown in SEQ ID NO:32, and where b is greater than or equal to a + 14.

### FEATURES OF PROTEIN ENCODED BY GENE NO: 23

The translation product of this gene shares sequence homology with octaprenyltransferase, which is thought to be important in cellular respiration and metabolism. When tested against fibroblast cell lines, supernatants removed from cells containing this gene activated the EGR1 assay. Thus, it is likely that this gene activates fibroblast cells through a signal transduction pathway. Early growth response 1 (EGR1) is a promoter associated with certain genes that induces various tissues and cell types upon activation, leading the cells to undergo differentiation and proliferation. The gene encoding the disclosed cDNA is thought to reside on chromosome 4. Accordingly, polynucleotides related to this invention are useful as a marker in linkage analysis for chromosome 4.

This gene is expressed primarily in synovium, liver cells, dendritic cells and stromal cells.

Therefore, polynucleotides and polypeptides of the invention are useful as reagents for differential identification of the tissue(s) or cell type(s) present in a biological sample and for diagnosis of diseases and conditions which include, but are not limited to, metabolic and respiratory disorders, immune disorders. Similarly, polypeptides and antibodies directed to these polypeptides are useful in providing immunological probes for differential identification of the tissue(s) or cell type(s). For a number of disorders of the above tissues or cells, particularly of the metabolic processes and immune system, expression of this gene at significantly higher or lower levels may be routinely detected in certain tissues or cell types (e.g. immune, liver, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:158 as residues: Asp-54 to Asn-69, His-176 to Asp-181, Phe-194 to Trp-201, Ser-220 to Pro-225, Arg-248 to Trp-253, Trp-276 to Ile-288.

The tissue distribution and homology to octaprenyltransferase indicates that polynucleotides and polypeptides corresponding to this gene are useful for the treatment and diagnosis of metabolic and respiratory disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1362 of SEQ ID NO:33, b is an integer of 15 to 1376, where both a and b correspond to the positions of nucleotide residues shown in SEQ ID NO:33, and where b is greater than or equal to a + 14.

### FEATURES OF PROTEIN ENCODED BY GENE NO: 24

This gene is expressed primarily in activated T cells and in the spleen from a patient suffering from lymphocytic leukemia.

Therefore, polynucleotides and polypeptides of the invention are useful as reagents for differential identification of the tissue(s) or cell type(s) present in a biological sample and for diagnosis of diseases and conditions which include, but are not limited to, immune or hematopoetic disorders, particularly immunodeficiencies, multiple myeloma, and leukemias. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cells and spleen tissue indicates that polynucleotides and polypeptides corresponding to this gene are useful for the diagnosis or treatment of leukemia. Furthermore, the tissue distribution indicates that the polypeptides or polynucleotides are useful for treatment, prophylaxis, and diagnosis of immune and autoimmune diseases, such as lupus, transplant rejection, allergic reactions, arthritis, asthma, immunodeficiency diseases, leukemia, and AIDS. The expression observed predominantly in hematopoietic cells also indicates that the polynucleotides or polypeptides are important in treating and/or detecting hematopoietic disorders, such as graft versus host reaction, graft versus host disease, transplant rejection, myelogenous leukemia, bone marrow fibrosis, and myeloproliferative disease. The polypeptides or polynucleotides are also useful to enhance or protect proliferation, differentiation, and functional activation of hematopoietic progenitor cells (e.g., bone marrow cells), useful in treating cancer patients undergoing chemotherapy or patients undergoing bone marrow transplantation. The polypeptides or polynucleotides are also useful to increase the proliferation of peripheral blood leukocytes, which can be used in the combat of a range of hematopoietic disorders, including immmunodeficiency diseases, leukemia, and septicemia. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1206 of SEQ ID NO:34, b is an integer of 15 to 1220, where both a and b correspond to the positions of nucleotide residues shown in SEQ ID NO:34, and where b is greater than or equal to a + 14.

### FEATURES OF PROTEIN ENCODED BY GENE NO: 25

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, immune or hematopoeitic disorders, particularly disorders afflicting stem cell or myeloid progenitors, and in particular multiple myeloma, immunodeficiencies, or SCID. Similarly, polypeptides and antibodies directed to these polypeptides are useful in providing immunological probes for differential identification of the tissue(s) or cell type(s). For a number of disorders of the above tissues or cells, particularly of the immune and hematopoetic systems, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in bone marrow indicates that polynucleotides and polypeptides corresponding to this gene are useful for the diagnosis and treatment of disorders affecting the immune and hematopoetic systems. In addition, The protein product of this gene is useful for the diagnosis and/or treatment of hematopoietic disorders. Furthermore, this gene product is primarily expressed in hematopoietic cells and tissues, suggesting that it plays a role in the survival, proliferation, and/or differentiation of hematopoieitic lineages. This is particularly supported by the expression of this gene product in bone marrow, which is a primary sites of definitive hematopoiesis. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:35 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1332 of SEQ ID NO:35, b is an integer of 15 to 1346, where both a and b correspond to the positions of nucleotide residues shown in SEQ ID NO:35, and where b is greater than or equal to a + 14.

### FEATURES OF PROTEIN ENCODED BY GENE NO: 26

This gene is expressed primarily in the cells of the immune system.

Therefore, polynucleotides and polypeptides of the invention are useful as reagents for differential identification of the tissue(s) or cell type(s) present in a biological sample and for diagnosis of diseases and conditions which include, but are not limited to, disorders of the immune systems, such as AIDS. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:161 as residues: His-17 to Ser-24, Glu-53 to Asn-58, Glu-66 to Lys-72.

The tissue distribution in immune cells indicates that polynucleotides and polypeptides corresponding to this gene are useful for the diagnosis and treatment of a variety of immune system disorders. Further, the expression of this gene product indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1012 of SEQ ID NO:36, b is an integer of 15 to 1026, where both a and b correspond to the positions of nucleotide residues shown in SEQ ID NO:36, and where b is greater than or equal to a + 14.

### FEATURES OF PROTEIN ENCODED BY GENE NO: 27

The translation product of this gene shares sequence homology with glucan synthetase which is thought to be important in modifying carbohydrate moieties on extracellular molecules.

This gene is expressed primarily in T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly autoimmune diseases and inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:162 as residues: Gly-33 to Leu-39, Thr-69 to Ser-77, Arg-102 to Thr-109.

The tissue distribution in T-cells combined with the homology to glucan synthetase indicates that polynucleotides and polypeptides corresponding to this gene are useful for modifying the response and production of active cytokines by T cells, in modulating cell-cell interactions, or cell-tissue interactions, and in inflammatory conditions. Alternatively, this gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues; such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:37 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 818 of SEQ ID NO:37, b is an integer of 15 to 832, where both a and b correspond to the positions of nucleotide residues shown in SEQ ID NO:37, and where b is greater than or equal to a + 14.

### FEATURES OF PROTEIN ENCODED BY GENE NO: 28

In specific embodiments, polypeptides of the invention comprise the following amino acid sequence:
GRGDKPRQDRPASLRLKGPPSCQAPASHSSTLSSHCPCSLFACGSVWPGSLGS GIFARLSQLLPSPASWG WDFLTLRQAQQMLGPSLCPGHSTSAHQHYGAYVLP RDLCSFLLTSTVQGTAPLKNSRVTCLIGSQQVPLC (SEQ ID NO:283), AEVTSPA KTDLQVFVSRDLPHARPLPLTAAPFPLIVPVPFLPVDLFGQGPWGQEYLQDSAS SFPAQPLGA GTFSPCGRHNRCWDPVSAQVTAQVHISTMGPMSCPETSAPSC SHPQFRARRPSRTPESPVSSAPSKCLFV YDVPLL (SEQ ID NO:284), SLRLKGP PSCQAPASHSSTLSSHCPCSLFA (SEQ ID NO:285), QQMLGPSLCPGHS TSAH QHYGAYVLPRDLC (SEQ ID NO:286), DLQVFVSRDLPHARPLPLTAAPF PLIV PVPF (SEQ ID NO:287), AQVHISTMGPMSCPETSAPSCSHPQFRARRP SRTPE SPV (SEQ ID NO:288), and/or QAPPRQTCKSSSQGTSL (SEQ ID NO:289). Polynucleotides encoding these polypeptides are also encompassed by the invention.

This gene is expressed primarily in endometrial tumors, fetal spleen, and to a lesser extent, in activated monocytes and T-cells.

Therefore, polynucleotides and polypeptides of the invention are useful as reagents for differential identification of the tissue(s) or cell type(s) present in a biological sample and for diagnosis of diseases and conditions which include, but are not limited to, reproductive, immune, hematopoietic disorders, particularly pregnancy defects. Similarly, polypeptides and antibodies directed to these polypeptides are useful in providing immunological probes for differential identification of the tissue(s) or cell type(s). For a number of disorders of the above tissues or cells, particularly of the reproductive and immune systems, expression of this gene at significantly higher or lower levels may be routinely detected in certain tissues and cell types (e.g. reproductive, endometrial, immune, hematopoietic, and cancerous and wounded tissues) or bodily fluids (e.g.lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:163 as residues: Ser-66 to Thr-75.

The tissue distribution in endometrial tissue indicates that the protein product of this gene could be used in the teatment and/or detection of pregnancy associated disorders including miscarriage, and endometriosis. Alternativelym expression in hematopoietic cells indicates that polynucleotides and polypeptides corresponding to this gene are useful for the treatment and/or detection of immune system related diseases including arthritis, asthma, immunodeficiency diseases and leukemia. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 692 of SEQ ID NO:38, b is an integer of 15 to 706, where both a and b correspond to the positions of nucleotide residues shown in SEQ ID NO:38, and where b is greater than or equal to a + 14.

### FEATURES OF PROTEIN ENCODED BY GENE NO: 29

In specific embodiments, polypeptides of the invention comprise the following amino acid sequence: AALRPSGSLAGPEWPWQHWCGCWREHXVKPQQVDLHSARLWAAPAAVGPA HAGGSPGMPPGGTAPHARRH SLPSPTAQSHLWHVHGLRQRGPKAVPLDLAQ LVTTTTPLFXLALSALLLGRRHHPLQLAAMGPLCLGAAC SLAGEFRTPPT GCGFLLAATCLRGLKSVQQSALLQEERLDAVTLLYATSLPSFCLLAGAALVLEA GVAPP PTAGDSRLWACILLSCLLSVLYNLASFSLLALTSALTVHVLGNLTVV GNLILSRLLFGSRLSALSYVGIA LTLSGMFLYHNCEFVASWAARRGLW RRDQPSKGL (SEQ ID NO:290), GQPSGPPAAWPGPSGHGSTGVAAGGSTXSSL NKWIFTVHGFGRPLLLSALHMLVAALACHRGARRP (SEQ ID NO:291), WPGPS GHGSTGVAAGGSTXSS (SEQ ID NO:292), EWPWQHWCGCWREHXVKPQQVD LHSA (SEQ ID NO:293), QQSALLQEERLDAVTLLYATSLPSFCLL (SEQ ID NO:294), ACILLSCLLSVLYNLASFSLLALTSAL (SEQ ID NO:295), and/or SLNKWIFTVHGFGRPLLLSAL (SEQ ID NO:296). Polynucleotides encoding these polypeptides are also encompassed by the invention.

This gene is expressed primarily in brain tissue from a patient suffering from Alzheimer's disease (spongy change), and to a lesser extent, in human umbilical vein and human pancreas tumor tissue.

Therefore, polynucleotides and polypeptides of the invention are useful as reagents for differential identification of the tissue(s) or cell type(s) present in a biological sample and for diagnosis of diseases and conditions which include, but are not limited to, developmental, immune, metabolic, digestive or neural disorders, such as Alzheimer's disease, in addition to cancers and tumors. Similarly, polypeptides and antibodies directed to these polypeptides are useful in providing immunological probes for differential identification of the tissue(s) or cell type(s). For a number of disorders of the above tissues or cells, particularly of the immune and secretory systems, expression of this gene at significantly higher or lower levels may be routinely detected in certain tissues and cell types (e.g.developmental, immune, metabolic, digestive, cancerous and wounded tissues) or bodily fluids (e.g.lymph, bile, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates that polynucleotides and polypeptides corresponding to this gene are useful for diagnosis and treatment of Alzheimer's disease, and immune and secretory system disorders such as cancers. Moreover, polynucleotides and polypeptides corresponding to this gene are useful for the detection/treatment of neurodegenerative disease states, behavioural disorders, or inflamatory conditions such as Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses, autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:39 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1333 of SEQ ID NO:39, b is an integer of 15 to 1347, where both a and b correspond to the positions of nucleotide residues shown in SEQ ID NO:39, and where b is greater than or equal to a + 14.

### FEATURES OF PROTEIN ENCODED BY GENE NO: 30

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, infection and inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:165 as residues: Asn-43 to Ala-49.

The tissue distribution in neutrophils indicates that polynucleotides and polypeptides corresponding to this gene are useful for the diagnosis and treatment of infection and inflammation related immune diseases. Furthermore, the gene product may also be involved in lymphopoiesis, therefore, it can be used in immune disorders such as infection, inflammation, allergy, immunodeficiency, etc. In addition, this gene product may have commercial utility in the expansion of stem cells and committed progenitors of various blood lineages, and in the differentiation and/or proliferation of various cell types. Additionally, expression of this gene product in neutrophils also strongly indicates a role for this protein in immune function and immune surveillance.

Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1453 of SEQ ID NO:40, b is an integer of 15 to 1467, where both a and b correspond to the positions of nucleotide residues shown in SEQ ID NO:40, and where b is greater than or equal to a + 14.

### FEATURES OF PROTEIN ENCODED BY GENE NO: 31

The translation product of this gene shares sequence homology with Ly6C antigen, which is thought to be important in T-cell activation. In specific embodiments, polypeptides of the invention comprise the following amino acid sequence:
KSTLSAAVVATILRTLA (SEQ ID NO:297), GDHSEQCLIKEMGARERRFCKAR GYRDTG REAQAKAGGRRGSQWNESQCS SQRPRPAKEVRKTRPRAGVGRGP ALLQLSLLQQVVLYVRPSLRLVWLKA S (SEQ ID NO:298), MERGEYGGWG TYGSLDLGSQLCTVRSSGPCGSLHWGQH RSPISGPDPNPSSSR GQQSIGSK VGSPSRSQWRSWKEVGRDPEKGE (SEQ ID NO:299), QAKAGGRRGSQWNESQ CSSQRPR (SEQ ID NO:300), VGRGPALLQL SLLQQVVLYVRPSLRL (SEQ ID NO:301), YGSLDLGSQLCTVRSSGPCGSL (SEQ ID NO:302), and/or KVGSPSR SQWRSWKEVGRDP (SEQ ID NO:303). Polynucleotides encoding these polypeptides are also encompassed by the invention.

This gene is expressed primarily in bone cancer, fetal brain, lung, and adipose tissue.

Therefore, polynucleotides and polypeptides of the invention are useful as reagents for differential identification of the tissue(s) or cell type(s) present in a biological sample and for diagnosis of diseases and conditions which include, but are not limited to, skeletal, developmental, pulmonary, or metabolic disorders, particular disorders in the immune responses to the above conditions, such as in autoimmunities. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. skeletal, developmental, pulmonary, metabolic, and cancerous and wounded tissues) or bodily fluids (e.g. lymph, amniotic fluid, pulmonary surfactant or sputum, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:166 as residues: Gln-37 to Gln-45, Phe-76 to Leu-83, Thr-89 to Thr-105.

The tissue distribution combined with the homology to the Ly6C T-cell activation antigen indicates that polynucleotides and polypeptides corresponding to this gene are useful for the diagnosis and intervention of immune related disorders. The tissue distribution in tissues particularily active in immune reaction, for example bone cancer, indicate that this gene may also be involved in T-cell activation. Thus the gene product can be used either for the development of immune suppressants, or modulators, for immune responses. Moreover, the expression within brain tissue indicates that the protein is useful for the treatment and/or prevention of neurodegenerative disorders, particularly, but not limited to, Alzheimer's or Parkinson's disease. Alternatively, the expression within fetal tissue and other cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 900 of SEQ ID NO:41, b is an integer of 15 to 914, where both a and b correspond to the positions of nucleotide residues shown in SEQ ID NO:41, and where b is greater than or equal to a + 14.

### FEATURES OF PROTEIN ENCODED BY GENE NO: 32

The gene encoding the disclosed cDNA is thought to reside on chromosome 12. Accordingly, polynucleotides related to this invention are useful as a marker in linkage analysis for chromosome 12.

This gene is expressed primarily in brain, keratinocytes and fibroblasts.

Therefore, polynucleotides and polypeptides of the invention are useful as reagents for differential identification of the tissue(s) or cell type(s) present in a biological sample and for diagnosis of diseases and conditions which include, but are not limited to, disorders of the brain and epidermal system. Similarly, polypeptides and antibodies directed to these polypeptides are useful in providing immunological probes for differential identification of the tissue(s) or cell type(s). For a number of disorders of the above tissues or cells, particularly of the epidermal and neural systems, expression of this gene at significantly higher or lower levels may be routinely detected in certain tissues or cell types (e.g. skin, brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of diseases of the neural and epidermal systems. Furthermore, the tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and perception. Protein, as well as, antibodies directed against the protein may show utility as a tumor marker and/or immunotherapy targets for the above listed tissues. Additionally, the tissue distribution indicates that polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Boweni's disease, basal cell carcinoma, squamous cell carcinoma, malignant melanoma, Pageti's disease, mycosis fungoides, and Kaposii's sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. Moreover, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm).

Many polynucleotide sequences, such as EST sequences, are publicly available and accessible through sequence databases. Some of these sequences are related to SEQ ID NO:42 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1117 of SEQ ID NO:42, b is an integer of 15 to 1131, where both a and b correspond to the positions of nucleotide residues shown in SEQ IID NO:42, and where b is greater than or equal to a + 14.

### FEATURES OF PROTEIN ENCODED BY GENE NO: 33

The translation product of this gene shares sequence homology with a sodium dependent sulfate transporter which is thought to be important in sulfate uptake by cells. The gene encoding the disclosed cDNA is thought to reside on chromosome 7. Accordingly, polynucleotides related to this invention are useful as a marker in linkage analysis for chromosome 7. One embodiment of this gene comprises polypeptides of the following amino acid sequence:
MPQSLSSLASSSSSFQRXKPCFGKKNDGENQEHSLGTEPIITWKDFQKTMPWE IVILVGGGYALASGSKSSGLSTWIGNQMLSLSSLPPWAVTLLACILVSIVTEFVS NPATITIFLPILCSLSETLHINPLYTLIPVTMCISFAVMLPVGNPPNAIVFSYGHCQ IKDMVKAGLGVNVIGLVIVMVAINTWGVSLFHLDTYPAWARVSNITDQA (SEQ ID NO:304), NDGENQEHSLGTEPIITWKDFQK (SEQ ID NO:305), IGNQMLSLSS LPPWAVTLLACILV (SEQ ID NO:306), ATITIFLPILCSLSETLHINPLYTLIP (SEQ ID NO:307), LPVGNPPNAIVFSYGHCQIKDMVKAG (SEQ ID NO:308), and/or LVIVMVAINTWGVSLFHLDTYPAWARVSN (SEQ ID NO:309). An additional embodiment is the polynucleotides encoding these polypeptides.

This gene is expressed primarily in placenta, and to a lesser extent, in infant brain and spinal cord.

Therefore, polynucleotides and polypeptides of the invention are useful as reagents for differential identification of the tissue(s) or cell type(s) present in a biological sample and for diagnosis of diseases and conditions which include, but are not limited to, metabolic, reproductive, or central nervous system disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. CNS, reproductive, metabolic, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in placental and neural tissues, combined with the homology to a sodium dependent sulfate transporter indicates that polynucleotides and polypeptides corresponding to this gene are useful for the treatment of metabolic disorders involving sodium and sulfate metabolism and CNS disorders involving neuronal signalling abnormalities. Moreover, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:43 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1319 of SEQ ID NO:43, b is an integer of 15 to 1333, where both a and b correspond to the positions of nucleotide residues shown in SEQ ID NO:43, and where b is greater than or equal to a + 14.

### FEATURES OF PROTEIN ENCODED BY GENE NO: 34

Contact of cells with supernatant expressing the product of this gene increases the permeability of bovine chondrocyte cells to calcium. Thus, it is likely that the product of this gene is involved in a signal transduction pathway that is initiated when the product of this gene binds a receptor on the surface of the chondrocyte cell. Thus, polynucleotides and polypeptides have uses which include, but are not limited to, activating chondrocyte cells.

This gene is expressed primarily in CD34 positive cells.

Therefore, polynucleotides and polypeptides of the invention are useful as reagents for differential identification of the tissue(s) or cell type(s) present in a biological sample and for diagnosis of diseases and conditions which include, but are not limited to, immune or reproductive disorders, particularly diseases related to lymphocytes. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. bone, immune, hematopoietic, reproductive, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:169 as residues: Leu-26 to Arg-32, Asn-40 to Ser-46.

The tissue distribution in CD34 positive cells indicates that polynucleotides and polypeptides corresponding to this gene are useful for the diagnosis or treatment of the diseases of the immune system particularly those related to T lymphocytes. Furthermore, the tissue distribution, as well as the detected calcium flux biological activity data, suggest that polynucleotides and polypeptides corresponding to this gene are useful for the diagnosis and/or treatment of bone and hematopoietic disorders. The ability of the translation product of this gene to induce a calcium flux in chondrocytes indicates that it may play a role in the survival, proliferation, and/or growth of bone. Therefore, it may be useful in influencing bone mass in such conditions as osteoporosis. More generally, as evidenced by expression in CD34 positive cells, this gene may play a role in the survival, proliferation, and/or differentiation of hematopoietic cells, and may be of use in the augmentation of the numbers of stem cells and committed progenitors. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:44 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where is any integer between 1 to 990 of SEQ ID NO:44, b is an integer of 15 to 1004, where both a and b correspond to the positions of nucleotide residues shown in SEQ ID NO:44, and where b is greater than or equal to a + 14.

### FEATURES OF PROTEIN ENCODED BY GENE NO: 35

The gene encoding the disclosed cDNA is thought to reside on chromosome 9. Accordingly, polynucleotides related to this invention are useful as a marker in linkage analysis for chromosome 9.

This gene is expressed primarily in the brain, and to a lesser extent, in liver.

Therefore, polynucleotides and polypeptides of the invention are useful as reagents for differential identification of the tissue(s) or cell type(s) present in a biological sample and for diagnosis of diseases and conditions which include, but are not limited to, disorders affecting the brain, central nervous system, or liver, including cancer. Similarly, polypeptides and antibodies directed to these polypeptides are useful in providing immunological probes for differential identification of the tissue(s) or cell type(s). For a number of disorders of the above tissues or cells, particularly of the immune, hematopoetic, or central nervous systems, expression of this gene at significantly higher or lower levels may be routinely detected in certain tissues or cell types (e.g. brain, liver, immune, hematopoietic, and cancerous and wounded tissues) or bodily fluids (e.g. lymph, serum, plasma, bile, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain and liver tissue indicates that polynucleotides and polypeptides corresponding to this gene are useful for the diagnosis and treatment of disorders affecting the immune, hematopoetic, or central nervous systems. Furthermore, polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, the gene or gene product may also play a role in the treatment and/or detection of developmental disorders associated with the developing embryo. Alternatively, the expression within hepatic tissue indicates polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:45 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1480 of SEQ ID NO:45, b is an integer of 15 to 1494, where both a and b correspond to the positions of nucleotide residues shown in SEQ ID NO:45, and where b is greater than or equal to a + 14.

### FEATURES OF PROTEIN ENCODED BY GENE NO: 36

When tested against U937 and Jurkat cell lines, supernatants removed from cells containing this gene activated the GAS (gamma activating sequence) promoter element. Thus, it is likely that this gene activates myeloid and T-cells through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
ETCPSNGIELRQAPTSLYILLLHIQPTPTHPMLGRSYVLPAFSXNXEHGGLPNQI PKGDRNGNIRHSRIT FPCSSSTLQPESHLGFIRSKLHGLVRPGKDLRGRRSL QLSKHSLSTCYMLRWETYKQVSYTAV (SEQ ID NO:310), QRHQENDKRNVH RFLHTCVHMPMCTHTHTQAVLSTWEGQFSNVASFTSLKRIPLSII YIHSSHSP RRFVKVCQLRQEKALELTEVYVSASLKLQLYHLHCHFHTAV (SEQ ID NO:311), RQAPTSLYILLLHIQPTPTHPMLG (SEQ ID NO:312), SHLGFIRSKLHGLVRPG KDLRGRRS (SEQ ID NO:313), RNVHRFLHTCVHMPMCTHTHTQ (SEQ ID NO:314), and/or QLRQEKALELTEVYVSASLKLQLYH (SEQ ID NO:315). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, particularly neutropenia, cancer, inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoieic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils, combined with the detected GAS biological activity indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy or may be useful in the treatment of immune dysfunction or anti-inflamatory by inhibiting infiltration of neutrophils to the site of injury or distress. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:46 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1152 of SEQ ID NO:46, b is an integer of 15 to 1166, where both a and b correspond to the positions of nucleotide residues shown in SEQ ID NO:46, and where b is greater than or equal to a + 14.

### FEATURES OF PROTEIN ENCODED BY GENE NO: 37

In specific embodiments, polypeptides of the invention comprise the following amino acid sequence: PRVRGRKEPGCLGPGRAGGDSQKEIGSWQQM (SEQ ID NO:316), LSKGNRIMAADDDNGDGTSLFDVFSASPLKNNDEGSLDIYA GLDSAVSDSA SKSCVPSRNCLDLYEEILTEEGTAKEATYNDLQVEYGKCQ LQMKELMKKFKEIQTQNFSLINENQSLKKN ISALIKTARVEINRKDEEI SNLHQKIVLSFHIFEIIIKLQGHLIQLKQKILNLDLHIWMIVQRLITRAKS DVSKD VHHSTSLPNLEKEGKPHSDKRSTSHLPTSVEKHCTNGVWSRSHYQVGEGSSN EDSRRGRKDIRHS QFNRGTERVRKDLSTGCGDGEPRILEASQRLQGTS (SEQ ID NO:317), NRIMAADDDNGDGTSLFDVFSASPLKN (SEQ ID NO:318), CLDLY EEILTEEGTAKEATYNDL (SEQ ID NO:319), DEEISNLHQKIVLSFHIFEIIIKLQG (SEQ ID NO:320), EKEGKPHSDKRSTSHLPTSVEK (SEQ ID NO:321), and/or TERVRKDLSTGCGDGEPRILEASQRL (SEQ ID NO:322). Polynucleotides encoding these polypeptides are also encompassed by the invention.

This gene is expressed primarily in activated T cells.

Therefore, polynucleotides and polypeptides of the invention are useful as reagents for differential identification of the tissue(s) or cell type(s) present in a biological sample and for diagnosis of diseases and conditions which include, but are not limited to, immune and inflammatory disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g., cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cells indicates that polynucleotides and polypeptides corresponding to this gene are useful for the diagnosis and treatment of immune and inflammatory disorders. Furthermore, expression of this gene product in tonsils indicates a role in regulating the proliferation; survival; differentiation; and/or activation of hematopoietic cell lineages, including blood stem cells. This gene product may be involved in the regulation of cytokine production. antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:47 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1522 of SEQ ID NO:47, b is an integer of 15 to 1536, where both a and b correspond to the positions of nucleotide residues shown in SEQ ID NO:47, and where b is greater than or equal to a + 14.

### FEATURES OF PROTEIN ENCODED BY GENE NO: 38

Contact of cells with supernatant expressing the product of this gene has been shown to increase the permeability of the plasma membrane of chondrocytes to calcium. Thus it is likely that the product of this gene is involved in a signal transduction pathway that is initiated when the product binds a receptor on the surface of the plasma membrane of both chondrocytes, in addition to other cell lines or tissue cell types. Thus, polynucleotides and polypeptides have uses which include, but are not limited to, activating chondrocytes. Binding of a ligand to a receptor is known to alter intracellular levels of small molecules, such as calcium, potassium and sodium, as well as alter pH and membrane potential. Alterations in small molecule concentration can be measured to identify supernatants which bind to receptors of a particular cell. In specific embodiments, polypeptides of the invention comprise the following amino acid sequence:
KSYFRTMGGTKRGIKKLVNVCLKHPKNTSLSQQLVFAKINKILISKTTK STNLKGLKCLPPLSVSIHPTFIYYKHNTTLRIVFGTYFDFFPYRKNKDQAFEGE DWESSLNVSDAW (SEQ ID NO:323), TKRGIKKLVNVCLKHPKNTSLS (SEQ ID NO:324), and/or SIHPTHYYKHNTTLRIVFGTYFDFF (SEQ ID NO:325). Polynucleotides encoding these polypeptides are also encompassed by the invention. The gene encoding the disclosed cDNA is believed to reside on chromosome 3. Accordingly, polynucleotides related to this invention are useful as a marker in linkage analysis for chromosome 3.

This gene is expressed primarily in resting T-cells, and to a lesser extent, in retina and placenta.

Therefore, polynucleotides and polypeptides of the invention are useful as reagents for differential identification of the tissue(s) or cell type(s) present in a biological sample and for diagnosis of diseases and conditions which include, but are not limited to, immune, reproductive, or eye disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, eye, reproductive, and cancerous and wounded tissues) or bodily fluids (e.g.lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:173 as residues: Met-1 to Pro-12.

The tissue distribution of this gene predominantly in T-cells and placenta, combined with the detected calcium flux activity indicates that the gene could be important for the treatment or detection of immune or hematopoietic disorders including arthritis, asthma, immunodeficiency diseases and leukemia. Expression of the gene at high levels in the retina indicates a role in the treatment and/or detection of eye disorders including color blindness, blindness, vision defects, and light sensitivity. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:48 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1024 of SEQ ID NO:48, b is an integer of 15 to 1038, where both a and b correspond to the positions of nucleotide residues shown in SEQ ID NO:48, and where b is greater than or equal to a + 14.

### FEATURES OF PROTEIN ENCODED BY GENE NO: 39

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, developmental, degenerative and behavioral diseases of the brain such as depression, schizophrenia, Alzheimer's disease, Parkinson's disease, Huntington's disease, specific brain tumors, aphasia, mania, depression, dementia, paranoia, addictive behavior and sleep disorders. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g., brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 174 as residues: Pro-35 to Met-42.

The tissue distribution indicates that polynucleotides and polypeptides corresponding to this gene are useful for the treatment and diagnosis of developmental, degenerative and behavioral diseases and conditions of the brain such as aphasia, depression, schizophrenia, Alzheimer's disease, Parkinson's disease, Huntington's disease, specific brain tumors, mania, depression, dementia, paranoia, addictive behavior and sleep disorders.

Many polynucleotide sequences, such as EST sequences, are publicly available and accessible through sequence databases. Some of these sequences are related to SEQ ID NO:49 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1162 of SEQ ID NO:49, b is an integer of 15 to 1176, where both a and b correspond to the positions of nucleotide residues shown in SEQ ID NO:49, and where b is greater than or equal to a + 14.

### FEATURES OF PROTEIN ENCODED BY GENE NO: 40

The gene encoding the disclosed cDNA is thought to reside on chromosome 17. Accordingly, polynucleotides related to this invention are useful as a marker in linkage analysis for chromosome 17.

This gene is expressed primarily in synovium.

Therefore, polynucleotides and polypeptides of the invention are useful as reagents for differential identification of the tissue(s) or cell type(s) present in a biological sample and for diagnosis of diseases and conditions which include, but are not limited to, disorders of the muscular-skeletal system. Similarly, polypeptides and antibodies directed to these polypeptides are useful in providing immunological probes for differential identification of the tissue(s) or cell type(s). For a number of disorders of the above tissues or cells, particularly of the musclular skeletal system, expression of this gene at significantly higher or lower levels may be routinely detected in certain tissues or cell types (e.g. synovium, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:175 as residues: Pro-15 to Cys-29, Gly-40 to Tyr-54, Pro-72 to His-79.

The tissue distribution indicates that polynucleotides and polypeptides corresponding to this gene are useful for the treatment and diagnosis of disorders of the muscular skeletal system. Furthermore, the expression of this gene product in synovium would suggest a role in the detection and treatment of disorders and conditions affecting the skeletal system, in particular osteoporosis, as well as disorders afflicting connective tissues (e.g. arthritis, trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:50 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 717 of SEQ ID NO:50, b is an integer of 15 to 731, where both a and b correspond to the positions of nucleotide residues shown in SEQ ID NO:50, and where b is greater than or equal to a + 14.

### FEATURES OF PROTEIN ENCODED BY GENE NO: 41

The translation product of this gene shares sequence homology with Enoyl-CoA hydratase, which is an RNA binding protein with intrinsic enzymatic activity thought to be important in metabolic disorders. The gene encoding the disclosed cDNA is thought to reside on chromosome 1. Accordingly, polynucleotides related to this invention are useful as a marker in linkage analysis for chromosome 1.

This gene is expressed primarily in fetal liver.

Therefore, polynucleotides and polypeptides of the invention are useful as reagents for differential identification of the tissue(s) or cell type(s) present in a biological sample and for diagnosis of diseases and conditions which include, but are not limited to, metabolic disorders, liver disorders and cancer. Similarly, polypeptides and antibodies directed to these polypeptides are useful in providing immunological probes for differential identification of the tissue(s) or cell type(s). For a number of disorders of the above tissues or cells, particularly of the hepatic and metabolic systems, expression of this gene at significantly higher or lower levels may be routinely detected in certain tissues or cell types (e.g. liver, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 176 as residues: Pro-10 to Arg-15, Leu-96 to Ser-103, Gly-172 to Pro-178, Gln-213 to Asp-218, Asn-268 to Leu-275, Arg-282 to Phe-289.

The tissue distribution and homology to Enoyl-CoA hydratase indicates that polynucleotides and polypeptides corresponding to this gene are useful for the treatment and diagnosis of metabolic and liver diseases and cancer. Furthermore, the tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:51 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1423 of SEQ ID NO:51, b is an integer of 15 to 1437, where both a and b correspond to the positions of nucleotide residues shown in SEQ ID NO:51, and where b is greater than or equal to a + 14.

### FEATURES OF PROTEIN ENCODED BY GENE NO: 42

This gene is expressed primarily in rhabdomyosarcoma tissue.

Therefore, polynucleotides and polypeptides of the invention are useful as reagents for differential identification of the tissue(s) or cell type(s) present in a biological sample and for diagnosis of diseases and conditions which include, but are not limited to, disorders of the muscular skeletal system and cancer. Similarly, polypeptides and antibodies directed to these polypeptides are useful in providing immunological probes for differential identification of the tissue(s) or cell type(s). For a number of disorders of the above tissues or cells, particularly of the muscular skeletal system, expression of this gene at significantly higher or lower levels may be routinely detected in certain tissues or cell types (e.g. musculo-skeletal, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of disorders of the muscular skeletal system and cancer. Furthermore, the tissue distribution indicates a role in the detection and treatment of disorders and conditions affecting the musculo-skeletal system, in particular rhabdomyosarcomas as well as related cancers. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:52 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1355 of SEQ ID NO:52, b is an integer of 15 to 1369, where both a and b correspond to the positions of nucleotide residues shown in SEQ ID NO:52, and where b is greater than or equal to a + 14.

### FEATURES OF PROTEIN ENCODED BY GENE NO: 43

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of aberrant immune responses to foreign antigens. Furthermore, expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:53 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1023 of SEQ ID NO:53, b is an integer of 15 to 1037, where both a and b correspond to the positions of nucleotide residues shown in SEQ ID NO:53, and where b is greater than or equal to a + 14.

### FEATURES OF PROTEIN ENCODED BY GENE NO: 44

This gene is expressed primarily in neutrophils induced with IL-1 and LPS.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly in aberrant neutrophil responses to infection. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 179 as residues: Lys-36 to Cys-42.

The tissue distribution in neutrophils indicates that polynucleotides and polypeptides corresponding to this gene are useful for the diagnosis and treatment of a lack of immune response to infection. Furthermore, expression of this gene product in neutrophils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:54 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1359 of SEQ ID NO:54, b is an integer of 15 to 1373, where both a and b correspond to the positions of nucleotide residues shown in SEQ ID NO:54, and where b is greater than or equal to a + 14.

### FEATURES OF PROTEIN ENCODED BY GENE NO: 45

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, CNS disorders. Similarly, polypeptides and antibodies directed to these polypeptides are useful in providing immunological probes for differential identification of the tissue(s) or cell type(s). For a number of disorders of the above tissues or cells, particularly of the central nervous system (CNS), expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of disorders of the central nervous system. Furthermore, elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's.

Many polynucleotide sequences, such as EST sequences, are publicly available and accessible through sequence databases. Some of these sequences are related to SEQ ID NO:55 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1333 of SEQ ID NO:55, b is an integer of 15 to 1347, where both a and b correspond to the positions of nucleotide residues shown in SEQ ID NO:55, and where b is greater than or equal to a + 14.

### FEATURES OF PROTEIN ENCODED BY GENE NO: 46

This gene is expressed primarily in spleen.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly those affecting the spleen, such as in T- and B-cell maturation and their resulting efficacy in the immune response. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, spleen, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:181 as residues: Ser-20 to Ser-34, Thr-40 to Ser-46.

The tissue distribution in spleen indicates that polynucleotides and polypeptides corresponding to this gene are useful for the diagnosis and treatment of disorders affecting the spleen and immune system. Furthermore, this gene may play a role in the survival, proliferation, and/or differentiation of hematopoietic cells in general, and may be of use in the augmentation of the numbers of stem cells and committed progenitors. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:56 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 808 of SEQ ID NO:56, b is an integer of 15 to 822, where both a and b correspond to the positions of nucleotide residues shown in SEQ ID NO:56, and where b is greater than or equal to a + 14.

### FEATURES OF PROTEIN ENCODED BY GENE NO: 47

In specific embodiments, polypeptides of the invention comprise the following amino acid sequence:
QRPHPQPWXPMTLMGTGIPVFAHKMLPFDPPCHLSCTHINPKPXXPQGDEQK SQGTEEWCDREGKKRRSI (SEQ ID NO:326), PMTLMGTGIPVFAHKMLPFDP (SEQ ID NO:327), PPCHLSCTHINPKPXXPQGDE (SEQ ID NO:328), EQKSQGT EEWCDREGKKRRSI (SEQ ID NO:329), DEWGAGRRMEWEDNLPLEFSCPVT KLLSVPSWTPLDAQMLLLFFPSLSHHSSVPWLFCSSPCGXXGLGFI (SEQ ID NO:330), EWEDNLPLEFSCPVTKLLSVP (SEQ ID NO:331), PSWTPLDAQM LLLFFPSLSHH (SEQ ID NO:332), and/or HSSVPWLFCSSPCGXXGLGFI (SEQ ID NO:333). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including neutropenia, cancer, inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy or may be useful in the treatment of immune dysfunction or anti-inflamatory by inhibiting infiltration of neutrophils to the site of injury or distress. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:57 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 522 of SEQ ID NO:57, b is an integer of 15 to 536, where both a and b correspond to the positions of nucleotide residues shown in SEQ ID NO:57, and where b is greater than or equal to a + 14.

### FEATURES OF PROTEIN ENCODED BY GENE NO: 48

This gene is expressed primarily in prostate, brain and T-cells.

Therefore, polynucleotides and polypeptides of the invention are useful as reagents for differential identification of the tissue(s) or cell type(s) present in a biological sample and for diagnosis of diseases and conditions which include, but are not limited to, disorders of the reproductive, CNS and immune system. Similarly, polypeptides and antibodies directed to these polypeptides are useful in providing immunological probes for differential identification of the tissue(s) or cell type(s). For a number of disorders of the above tissues or cells, particularly of the reproductive, CNS and immune system, expression of this gene at significantly higher or lower levels may be routinely detected in certain tissues or cell types (e.g. immune, brain, prostate, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:183 as residues: Asp-26 to Gly-32, Ile-37 to Trp-44.

The tissue distribution indicates that polynucleotides and polypeptides corresponding to this gene are useful for the treatment and diagnosis of disorders of the reproductive, CNS and immune systems. Furthermore, the tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, mania, dementia, paranoia, obsessive compulsive disorder, panic disorder, learning disabilities, ALS, psychoses , autism, and altered bahaviors, including disorders in feeding, sleep patterns, balance, and perception. Additionally, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in T cells strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:58 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1248 of SEQ ID NO:58, b is an integer of 15 to 1262, where both a and b correspond to the positions of nucleotide residues shown in SEQ ID NO:58, and where b is greater than or equal to a + 14.

### FEATURES OF PROTEIN ENCODED BY GENE NO: 49

This gene is expressed primarily in frontal cortex of schizophrenics.

Therefore, polynucleotides and polypeptides of the invention are useful as reagents for differential identification of the tissue(s) or cell type(s) present in a biological sample and for diagnosis of diseases and conditions which include, but are not limited to, CNS diseases and Schizophrenia. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of disorders of the CNS and schizophrenia. Furthermore, the tissue distribution indicates that polynucleotides and polypeptides corresponding to this gene are useful for the diagnosis and/or treatment of disorders of the brain and nervous system. Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's.

Many polynucleotide sequences, such as EST sequences, are publicly available and accessible through sequence databases. Some of these sequences are related to SEQ ID NO:59 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1255 of SEQ ID NO:59, b is an integer of 15 to 1269, where both a and b correspond to the positions of nucleotide residues shown in SEQ ID NO:59, and where b is greater than or equal to a + 14.

### FEATURES OF PROTEIN ENCODED BY GENE NO: 50

This gene is expressed primarily in the testes.

Therefore, polynucleotides and polypeptides of the invention are useful as reagents for differential identification of the tissue(s) or cell type(s) present in a biological sample and for diagnosis of diseases and conditions which include, but are not limited to, reproductive or endocrine disordes, particularly for male infertility and testicular cancer. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues or cell types (e.g. reproductive, testicular, and cancerous and wounded tissues) or bodily fluids (e.g. lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:185 as residues: His-62 to Ser-74, Leu-99 to Gln-104.

The tissue distribution in testes indicates that polynucleotides and polypeptides corresponding to this gene are useful for treating male infertility. The protein product is likely involved in sperm development and could be administered by injection or related techniques. Alternatively, this gene could be transfected in gene-replacement treatments into the cells of the testes and the protein products could be produced. The presence of expression of this gene at either the RNA or protein level could be used as a diagnostic in testicular cancer. Furthermore, the tissue distribution indicates that the protein product of this gene is useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents as such agents (antagonists) are useful as male contraceptive agents. Similarly, the protein is believed to be useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications.

Many polynucleotide sequences, such as EST sequences, are publicly available and accessible through sequence databases. Some of these sequences are related to SEQ ID NO:60 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1815 of SEQ ID NO:60, b is an integer of 15 to 1829, where both a and b correspond to the positions of nucleotide residues shown in SEQ ID NO:60, and where b is greater than or equal to a + 14.

### FEATURES OF PROTEIN ENCODED BY GENE NO: 51

In specific embodiments, polypeptides of the invention comprise the following amino acid sequence: QGLSHIFWMNEQTLK (SEQ ID NO:334). Polynucleotides encoding these polypeptides are also encompassed by the invention.

This gene is expressed primarily in activated T-cells.

Therefore, polynucleotides and polypeptides of the invention are useful as reagents for differential identification of the tissue(s) or cell type(s) present in a biological sample and for diagnosis of diseases and conditions which include, but are not limited to, immune disorders, particularly acute inflammatory conditions or autoimmune disease. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in activated T-cells indicates that polynucleotides and polypeptides corresponding to this gene are useful for modulating the response of activated T-cells to treat inflammation or autoimmune diseases. The expression of this gene product indicates a role in regulating the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:61 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1098 of SEQ ID NO:61, b is an integer of 15 to 1112, where both a and b correspond to the positions of nucleotide residues shown in SEQ ID NO:61, and where b is greater than or equal to a + 14.

### FEATURES OF PROTEIN ENCODED BY GENE NO: 52

When tested against U937 cell lines, supernatants removed from cells containing this gene activated the GAS (gamma activating sequence) promoter element. Thus, it is likely that this gene activates myeloid cells, including their progenitors, through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
TLVCLGVSSEEGSCPRDVTGPGCCFSLTLTGF (SEQ ID NO:335), ADLIVLWH HHPLWPQHLALPSSGASHDH VELTVYPKTVAASWLLELSRPPIFCLFTXPALT XHGLDRVAALVECTIWXXXGMWYRRRYSCCQFRDRSI-RDVFPEAVMLQQH LRHLAVATYRCRRRSPCKAPTVEEAEGGKPRAVPSGTGFQKHGQEPGGSTSP HWFWG HLQLLVLSVNNRQLFVQGRAGYLEMTGLPCPKLLLTLLRGLT PGVGHGLCAYRRGCLAWRLDXAS (SEQ ID NO:336), ILWRQAPEAPHCSQDSV SSSPRLQEDLAHVTQVTRHPHFRSLPSAWCSHSSLLPVSLPRHALATKSPNMX XSSPILHLIQFTGQISS PLGGXVQPPGQTASPICTQPMSHPRRQASQQCEQ QLWTGQTSBLQIPCPALNKELPVVDTQDKELQMSPE PMWGCGPSRLLPM LLESCA (SEQ ID NO:337), MLQQHLRHLAVATYRCRRRSPCKAPTVEEAEGGK (SEQ ID NO:338), VTQVTRHPHFRSLPSAWCSHSSLLPVSLP (SEQ ID NO:339), and/or GQTASPICTQPMSHPRRQASQQCEQQLW (SEQ ID NO:340). Polynucleotides encoding these polypeptides are also encompassed by the invention.

This gene is expressed primarily in activated T-cells.

Therefore, polynucleotides and polypeptides of the invention are useful as reagents for differential identification of the tissue(s) or cell type(s) present in a biological sample and for diagnosis of diseases and conditions which include, but are not limited to, immune disorders, particularly autoimmune diseases and inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:187 as residues: Ser-25 to Lys-33.

The tissue distribution in neutrophils, combined with the detected GAS biological activity indicates that polynucleotides and polypeptides corresponding to this gene are useful for modulating the response of activated T-cells and other cells of the immune system involved in inflammation and autoimmune diseases. Similarly, this gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:62 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1660 of SEQ ID NO:62, b is an integer of 15 to 1674, where both a and b correspond to the positions of nucleotide residues shown in SEQ ID NO:62, and where b is greater than or equal to a + 14.

### FEATURES OF PROTEIN ENCODED BY GENE NO: 53

In specific embodiments, polypeptides of the invention comprise the following amino acid sequence:
FITLRLGPKNMAGVLWRHSNLQTPHYISWCPLLNYRETGNCLLHVSG FLNSR LLANCSGEASGKVIQTLLWPGEISAVA (SEQ ID NO:341), KIRTFLFSGHRLFST QGQSLTVKAHTAF MLIVKNLRYFIAFKFLMGISDSSEIGLVMQPLQKPHTV ILIRGIEFLSPGGVLP (SEQ ID NO:342), MAGVLWRHSNLQTPHYISWCPLLNYR (SEQ ID NO:343), and/or YFIAFKFLMGISDSSEIGLVMQPLQKPHT (SEQ ID NO:344). Polynucleotides encoding these polypeptides are also encompassed by the invention. The gene encoding the disclosed cDNA is believed to reside on chromosome 12. Accordingly, polynucleotides related to this invention are useful as a marker in linkage analysis for chromosome 12.

This gene is expressed primarily in spleen, and to a lesser extent, in bone marrow and B-cells.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic disorders, particularly mutiple myeloma, immunodeficiencies, and infections. Similarly, polypeptides and antibodies directed to these polypeptides are useful in providing immunological probes for differential identification of the tissue(s) or cell type(s). For a number of disorders of the above tissues or cells, particularly of the immune and hematopoietic disorders, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene predominantly in hematopoietic cell types and immune tissues indicates that the gene could be important for the treatment or detection of immune or hematopoietic disorders including arthritis, asthma, immunodeficiency diseases and leukemia. Moreover, this gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:63 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1031 of SEQ ID NO:63, b is an integer of 15 to 1045, where both a and b correspond to the positions of nucleotide residues shown in SEQ ID NO:63, and where b is greater than or equal to a + 14.

### FEATURES OF PROTEIN ENCODED BY GENE NO: 54

The translation product of this gene shares very weak sequence homology with follicle-stimulating hormone beta subunit, which is thought to be important in hormonal regulation. When tested against K562 leukemia cell lines, supernatants removed from cells containing this gene activated the ISRE assay. Thus, it is likely that this gene activates leukemia cells through the Jak-STAT signal transduction pathway. The interferon-sensitive response element is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the ISRE element, can be used to indicate proteins involved in the proliferation and differentiation of cells. The gene encoding the disclosed cDNA is thought to reside on chromosome 4. Accordingly, polynucleotides related to this invention are useful as a marker in linkage analysis for chromosome 4.

This gene is expressed primarily in adult brain and adipocytes.

Therefore, polynucleotides and polypeptides of the invention are useful as reagents for differential identification of the tissue(s) or cell type(s) present in a biological sample and for diagnosis of diseases and conditions which include, but are not limited to, endocrine diseases. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain and homology to follicle stimulating hormone indicates that polynucleotides and polypeptides corresponding to this gene are useful as a hormone for the diagnosis and treatment of endocrine disorders. The brain is a major site for secreting various hormones that regulate a wide range of body physiology. The secretory molecule encoded by this gene has very weak homology with FSH, and further indicates that it may serves as an endocrine. Endocrines can often be used in hormonal treatment of pathological disorders or change of physiology under certain circumstances such as in the treatment of reproductive disorders.

Many polynucleotide sequences, such as EST sequences, are publicly available and accessible through sequence databases. Some of these sequences are related to SEQ ID NO:64 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1037 of SEQ ID NO:64, b is an integer of 15 to 1051, where both a and b correspond to the positions of nucleotide residues shown in SEQ ID NO:64, and where b is greater than or equal to a + 14.

### FEATURES OF PROTEIN ENCODED BY GENE NO: 55

The translation product of this gene shares homology with a number of a C. elegans proteases, which are thought to be important in programmed cell death.

This gene is expressed primarily in activated T-cells and to a lesser extent in human stomach.

Therefore, polynucleotides and polypeptides of the invention are useful as reagents for differential identification of the tissue(s) or cell type(s) present in a biological sample and for diagnosis of diseases and conditions which include, but are not limited to, immune disorders or stomach diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 190 as residues: Lys-41 to Arg-47, Asp-125 to Lys-139, Ser-177 to Glu-185.

The tissue distribution in activated T-cells and stomach indicates that polynucleotides and polypeptides corresponding to this gene are useful for the diagnosis and treatment of immune disorders, transplantation or stomach disease. Particularily, the expression of the gene by activated T-cells can be used for the development of therapeutic agents as immune suppressants or immune modulators.

Many polynucleotide sequences, such as EST sequences, are publicly available and accessible through sequence databases. Some of these sequences are related to SEQ ID NO:65 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1168 of SEQ ID NO:65, b is an integer of 15 to 1182, where both a and b correspond to the positions of nucleotide residues shown in SEQ ID NO:65, and where b is greater than or equal to a + 14.

### FEATURES OF PROTEIN ENCODED BY GENE NO: 56

The translation product of this gene shares sequence homology with CD53 tetraspan transmembrane molecule which is thought to be important in leukocyte activation. The gene encoding the disclosed cDNA is thought to reside on chromosome 7. Accordingly, polynucleotides related to this invention are useful as a marker in linkage analysis for chromosome 7.

This gene is expressed primarily in KMH2 and activated T-cells, and to a lesser extent in tonsils.

Therefore, polynucleotides and polypeptides of the invention are useful as reagents for differential identification of the tissue(s) or cell type(s) present in a biological sample and for diagnosis of diseases and conditions which include, but are not limited to, infection, inflammation and other immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:191 as residues: Lys-99 to Arg-107.

The tissue distribution and homology to CD53 indicates that polynucleotides and polypeptides corresponding to this gene are useful for diagnosis and development of therapeutic agents for immune disorders including infection, allergy, inflammation, transplantation and immune deficiencies. Furthermore, expression of this gene product in tonsils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Expression of this gene product in T cells strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:66 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 661 of SEQ ID NO:66, b is an integer of 15 to 675, where both a and b correspond to the positions of nucleotide residues shown in SEQ ID NO: 66, and where b is greater than or equal to a + 14.

### FEATURES OF PROTEIN ENCODED BY GENE NO: 57

The gene encoding the disclosed cDNA is thought to reside on chromosome 17. Accordingly, polynucleotides related to this invention are useful as a marker in linkage analysis for chromosome 17.

This gene is expressed primarily in fetal liver and to a lesser extent in neutrophils and keratinocytes.

Therefore, polynucleotides and polypeptides of the invention are useful as reagents for differential identification of the tissue(s) or cell type(s) present in a biological sample and for diagnosis of diseases and conditions which include, but are not limited to, inflammation, autoimmune and skin defects. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. liver, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO: 192 as residues: Pro-41 to Gln-50.

The tissue distribution indicates that polynucleotides and polypeptides corresponding to this gene are useful for the study and treatment of inflammatory, general immune, and skin disorders. Furthermore, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. This is particularly supported by the expression of this gene product in fetal liver, which is a primary site of definitive hematopoiesis. Expression of this gene product in neutrophils also strongly indicates a role for this protein in immune function and immune surveillance.

Many polynucleotide sequences, such as EST sequences, are publicly available and accessible through sequence databases. Some of these sequences are related to SEQ ID NO:67 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1091 of SEQ ID NO:67, b is an integer of 15 to 1105, where both a and b correspond to the positions of nucleotide residues shown in SEQ ID NO:67, and where b is greater than or equal to a + 14.

### FEATURES OF PROTEIN ENCODED BY GENE NO: 58

This gene is expressed primarily in induced neutrophils.

Therefore, polynucleotides and polypeptides of the invention are useful as reagents for differential identification of the tissue(s) or cell type(s) present in a biological sample and for diagnosis of diseases and conditions which include, but are not limited to, immune and haemopoietic disorders. Similarly, polypeptides and antibodies directed to these polypeptides are useful in providing immunological probes for differential identification of the tissue(s) or cell type(s). For a number of disorders of the above tissues or cells, particularly of the haemopoietic and immune systems, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of disorders of the haemopoietic and immune systems. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Expression of this gene product in neutrophils also strongly indicates a role for this protein in immune function and immune surveillance.

Many polynucleotide sequences, such as EST sequences, are publicly available and accessible through sequence databases. Some of these sequences are related to SEQ ID NO:68 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1265 of SEQ ID NO:68, b is an integer of 15 to 1279, where both a and b correspond to the positions of nucleotide residues shown in SEQ ID NO:68, and where b is greater than or equal to a + 14.

### FEATURES OF PROTEIN ENCODED BY GENE NO: 59

This gene is expressed primarily in the endometrium.

Therefore, polynucleotides and polypeptides of the invention are useful as reagents for differential identification of the tissue(s) or cell type(s) present in a biological sample and for diagnosis of female infertility or reproductive disorders. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g. reproductive, endometrium, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in endometrium indicates that polynucleotides and polypeptides corresponding to this gene are useful for treating female infertility. The protein product may show utility in the preparation of the endometrium of implantation and could be administered either topically or orally. Alternatively, this gene could be transfected in gene-replacement treatments into the cells of the endometrium and the protein products could be produced. Similarly, these treatments could be performed during artificial insemination for the purpose of increasing the likelihood of implantation and development of a healthy embryo. In both cases this gene or its gene product could be administered at later stages of pregnancy to promote heathy development of the endometrium. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:69 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1624 of SEQ ID NO:69, b is an integer of 15 to 1638, where both a and b correspond to the positions of nucleotide residues shown in SEQ ID NO:69, and where b is greater than or equal to a + 14.

### FEATURES OF PROTEIN ENCODED BY GENE NO: 60

This gene is expressed primarily in the cells of the immune system, such as eosinophils, T-cells, dendritic cells, and tonsils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, such as AIDS, inflammatory conditions, multiple myeloma, or SCID. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types or cell type (e.g. immune, hemaopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in various immune cells and tissues indicates that polynucleotides and polypeptides corresponding to this gene are useful for the diagnosis and treatment of immune system disorders, such as AIDS. Furthermore, expression of this gene product in tonsils and other immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:70 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 873 of SEQ ID NO:70, b is an integer of 15 to 887, where both a and b correspond to the positions of nucleotide residues shown in SEQ ID NO:70, and where b is greater than or equal to a + 14.

### FEATURES OF PROTEIN ENCODED BY GENE NO: 61

The translation product of this gene shares homology with human stannin, which is thought to play a role in the toxic effects of organotins. Moreover, the protein product of this gene may also show utility in the treament, and/or prevention of a variety of defects in calcium regulation and metabolism.

This gene is expressed primarily in GM-CSF treated macrophages.

Therefore, polynucleotides and polypeptides of the invention are useful as reagents for differential identification of the tissue(s) or cell type(s) present in a biological sample and for diagnosis of diseases and conditions which include, but are not limited to, immune disorders, particularly in the treatment or amelioration of abberant immune response to tumor or foreign antigens, and in phagocytosis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:196 as residues: Gly-43 to Gly-55.

The tissue distribution in macrophages indicates that polynucleotides and polypeptides corresponding to this gene are useful for the treatment and diagnosis of immune disorders. Furthermore, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in macrophage also strongly indicates a role for this protein in immune function and immune surveillance. The protein product may even serve to stimulate the immune response, or may be used to inhibit such a response which may be useful during host versus graft disease or autoimmune disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:71 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 850 of SEQ ID NO:71, b is an integer of 15 to 864, where both a and b correspond to the positions of nucleotide residues shown in SEQ ID NO:71, and where b is greater than or equal to a + 14.

### FEATURES OF PROTEIN ENCODED BY GENE NO: 62

This gene is expressed primarily in activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in monocytes indicates that polynucleotides and polypeptides corresponding to this gene are useful for diagnosing and/or treating immune or hematopoietic disorders. This gene product is primarily expressed in hematopoietic cells and tissues, suggesting that it plays a role in the survival, proliferation, and/or differentiation of hematopoieitic lineages. Expression of this gene product in monocytes also strongly indicates a role for this protein in immune function and immune surveillance. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:72 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1203 of SEQ ID NO:72, b is an integer of 15 to 1217, where both a and b correspond to the positions of nucleotide residues shown in SEQ ID NO:72, and where b is greater than or equal to a + 14.

### FEATURES OF PROTEIN ENCODED BY GENE NO: 63

This gene is expressed primarily in activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:198 as residues: Met-1 to Gly-6.

The tissue distribution in monocytes indicates that polynucleotides and polypeptides corresponding to this gene are useful for diagnosing and/or treating immune or hematopoietic disorders. This gene product is primarily expressed in hematopoietic cells and tissues, suggesting that it plays a role in the survival, proliferation, and/or differentiation of hematopoieitic lineages. Expression of this gene product in monocytes also strongly indicates a role for this protein in immune function and immune surveillance. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:73 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1703 of SEQ ID NO:73, b is an integer of 15 to 1717, where both a and b correspond to the positions of nucleotide residues shown in SEQ ID NO:73, and where b is greater than or equal to a + 14.

### FEATURES OF PROTEIN ENCODED BY GENE NO: 64

When tested against K562 leukemia cell lines, supernatants removed from cells containing this gene activated the ISRE assay. Thus, it is likely that this gene activates leukemia cells through the Jak-STAT signal transduction pathway. The interferon-sensitive response element is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the ISRE element, can be used to indicate proteins involved in the proliferation and differentiation of cells. The gene encoding the disclosed cDNA is thought to reside on chromosome 3. Accordingly, polynucleotides related to this invention are useful as a marker in linkage analysis for chromosome 3.

This gene is expressed primarily in spleen from a chronic lymphocytic leukemia patient,

Therefore, polynucleotides and polypeptides of the invention are useful as reagents for differential identification of the tissue(s) or cell type(s) present in a biological sample and for diagnosis of diseases and conditions which include, but are not limited to, immune or hematopoieitic disorders, particularly leukemias. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. spleen, immune, hematopoeitic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in leukemia cells combined with the detected ISRE biological activity in K562 cell lines indicates that polynucleotides and polypeptides corresponding to this gene are useful for the diagnosis and/or treatment of chronic lymphocytic leukemia. Furthermore, since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:74 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1262 of SEQ ID NO:74, b is an integer of 15 to 1276, where both a and b correspond to the positions of nucleotide residues shown in SEQ ID NO:74, and where b is greater than or equal to a + 14.

### FEATURES OF PROTEIN ENCODED BY GENE NO: 65

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for diagnosis and treatment of neutrophils inactivation and other immune system disorders. Furthermore, polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in neutrophils also strongly indicates a role for this protein in immune function and immune surveillance. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:75 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1130 of SEQ ID NO:75, b is an integer of 15 to 1144, where both a and b correspond to the positions of nucleotide residues shown in SEQ ID NO:75, and where b is greater than or equal to a + 14.

### FEATURES OF PROTEIN ENCODED BY GENE NO: 66

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly neutropenia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for diagnosis and treatment of immune system disorders. Furthermore, expression of this gene product in neutrophils also strongly indicates a role for this protein in immune function and immune surveillance. The protein may also be useful in the inhibition of neutrophil activation which may show utility in host-versus-graft disease and autoimmune disorders. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:76 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 904 of SEQ ID NO:76, b is an integer of 15 to 918, where both a and b correspond to the positions of nucleotide residues shown in SEQ ID NO:76, and where b is greater than or equal to a + 14.

### FEATURES OF PROTEIN ENCODED BY GENE NO: 67

When tested against U937 myeloid cell lines, supernatants removed from cells containing this gene activated the GAS promoter element. Thus, it is likely that this gene activates myeloid cells, and their progenitors, through the Jak-STAT signal transduction pathway. The gamma activating sequence (GAS) is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, such as neutropenia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:202 as residues: Asp-23 to Trp-29.

The tissue distribution in neutrophilsm, combined with the detected GAS biological activity in myeloid cell lines indicates that polynucleotides and polypeptides corresponding to this gene are useful for the diagnosis and treatment of immune system disorders. Furthermore, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in neutrophils also strongly indicates a role for this protein in immune function and immune surveillance. The protein product of this gene may show utility in the inhibition of neutrophil activation which may show utility in host-versus-graft disease and in autoimmune disorders. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:77 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1051 of SEQ ID NO:77, b is an integer of 15 to 1065, where both a and b correspond to the positions of nucleotide residues shown in SEQ ID NO:77, and where b is greater than or equal to a + 14.

### FEATURES OF PROTEIN ENCODED BY GENE NO: 68

This gene is expressed primarily in neutrophils induced with IL-1 and LPS.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, such as neutropenia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of inactive immune response to foreign antigens. Furthermore, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in neutrophils also strongly indicates a role for this protein in immune function and immune surveillance. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. The protein product of this gene may also show utility in the inactivation of neutrophils which may show utility in host-versus-graft disease or in autoimmune disorders, for example. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:78 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1112 of SEQ ID NO:78, b is an integer of 15 to 1126, where both a and b correspond to the positions of nucleotide residues shown in SEQ ID NO:78, and where b is greater than or equal to a + 14.

### FEATURES OF PROTEIN ENCODED BY GENE NO: 69

The translation product of this nucleotide sequence shares homology with a number of cysteine proteinases. Contact of cells with supernatant expressing the product of this gene increases the permeability of TF-1 Myeloid cells to calcium. Thus, it is likely that the product of this gene is involved in a signal transduction pathway that is initiated when the product of this gene binds a receptor on the surface of the myeloid cell. Thus, polynucleotides and polypeptides have uses which include, but are not limited to, activating myeloid cells.

This gene is expressed primarily in tissue from an ovarian tumor.

Therefore, polynucleotides and polypeptides of the invention are useful as reagents for differential identification of the tissue(s) or cell type(s) present in a biological sample and for diagnosis of diseases and conditions which include, but are not limited to, reproductive disorders, particularly ovarian cancer. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g. reproductive, immune, hematopoeitic, ovarian, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The homology to proteins of the cysteine proteinase family, tissue distribution in ovarian tissues, combined with the detected calcium flux activity in myeloid cells indicates that the protein product of this gene may show utility in the treatment, and/or prevention of a variety of reproductive disorders, such as in ovarian cancer, or even in the modulation of the immune response to. Thus, it is useful for diagnosis and treatment of ovarian cancer. Furthermore, the biological activity data, when compared to the tissue distribution, suggest that the translation product of this gene could be useful in activating the immune system to respond to cancerous growths, particularly those involving ovarian cancer. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO:79 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 970 of SEQ ID NO:79, b is an integer of 15 to 984, where both a and b correspond to the positions of nucleotide residues shown in SEQ ID NO:79, and where b is greater than or equal to a + 14.

### FEATURES OF PROTEIN ENCODED BY GENE NO: 70

This gene is expressed primarily in anergic T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, such as autoimmune disorders including lupus. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:205 as residues: Ser-26 to Lys-34.

The tissue distribution in T-cells indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Expression of this gene product in T cells also strongly indicates a role for this protein in immune function and immune surveillance. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:80 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1233 of SEQ ID NO:80, b is an integer of 15 to 1247, where both a and b correspond to the positions of nucleotide residues shown in SEQ ID NO:80, and where b is greater than or equal to a + 14.

### FEATURES OF PROTEIN ENCODED BY GENE NO: 71

This gene shares homology with the human adult heart neutral calponin, which is implicated in the regulation and modulation of smooth muscle contraction. It is capable of binding to actin, calmodulin, troponin C, and tropomyosin. The interaction of calponin with actin inhibits the actomyosin Mg-ATPase activity. Therefore, the protein product of this gene may be beneficial as a vasoconstrictor or vasodilator, a muscle relaxor, treatment for tetanus stimuli, or for the treatment of various cardiovascular disorders. The gene encoding the disclosed cDNA is thought to reside on chromosome 19. Accordingly, polynucleotides related to this invention are useful as a marker in linkage analysis for chromosome 19.

This gene is expressed primarily in adrenal gland tumor and human 12 week embryo. Furthermore, the gene is expressed in cardiomyopathy tissue.

Therefore, polynucleotides and polypeptides of the invention are useful as reagents for differential identification of the tissue(s) or cell type(s) present in a biological sample and for diagnosis of diseases and disorders: endocrine, developmental, cardiovascular disorders, particularly diseases involving abnormal cellular proliferation such as cancers particularly of the adrenal gland, and disorders involving heart muscle, such as cardiomyopathy Similarly, polypeptides and antibodies directed to these polypeptides are useful in providing immunological probes for differential identification of the tissue(s) or cell type(s). For a number of disorders of the above tissues or cells, particularly of the adrenal gland, heart, expression of this gene at significantly higher or lower levels may be routinely detected in certain tissues or cell types (e.g. heart, muscle, endocrine, developmental, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of abnormal cellular proliferation, such as tumors. Alternatively, given the tissue distribution and the homology to human adult heart neutral calponin, it indicates that the translation product of this gene is useful for detecting, identifying, and/or treating disorders involving the degeneration of the regulation and modulation of smooth muscle contraction, such as is seen with cardiomyopathies. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:81 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 932 of SEQ ID NO:81, b is an integer of 15 to 946, where both a and b correspond to the positions of nucleotide residues shown in SEQ ID NO:81, and where b is greater than or equal to a + 14.

### FEATURES OF PROTEIN ENCODED BY GENE NO: 72

This gene is expressed primarily in human bone and 9 week embryo.

Therefore, polynucleotides and polypeptides of the invention are useful as reagents for differential identification of the tissue(s) or cell type(s) present in a biological sample and for diagnosis of diseases and conditions which include, but are not limited to, skeletal, immune, hemopoietic, or developmental disordes. Similarly, polypeptides and antibodies directed to these polypeptides are useful in providing immunological probes for differential identification of the tissue(s) or cell type(s). For a number of disorders of the above tissues or cells, particularly of the hematoplastic tissues, expression of this gene at significantly higher or lower levels may be routinely detected in certain tissues or cell types (e.g. immune, bone, cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:207 as residues: Ala-22 to Lys-36.

The tissue distribution in bone and embryonic tissues indicates that polynucleotides and polypeptides corresponding to this gene are useful for the diagnosis or the treatment of hemopoietic diseases. Furthermore, it may be useful in influencing bone mass in such conditions as osteoporosis. More generally, this gene may play a role in the survival, proliferation, and/or differentiation of hematopoietic cells in general, and may be of use in augmentation of the numbers of stem cells and committed progenitors.

Many polynucleotide sequences, such as EST sequences, are publicly available and accessible through sequence databases. Some of these sequences are related to SEQ ID NO:82 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1378 of SEQ ID NO:82, b is an integer of 15 to 1392, where both a and b correspond to the positions of nucleotide residues shown in SEQ ID NO:82, and where b is greater than or equal to a + 14.

### FEATURES OF PROTEIN ENCODED BY GENE NO: 73

This gene is expressed primarily in T cells.

Therefore, polynucleotides and polypeptides of the invention are useful as reagents for differential identification of the tissue(s) or cell type(s) present in a biological sample and for diagnosis of diseases and conditions which include, but are not limited to, disorder of the immune or hematopoietic systems, particularly immunodeficiencies or inflammatory conditions, such as AIDS, SCID, leukemias, or multiple myeloma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:208 as residues: Asp-26 to Leu-36, Leu-42 to Phe-50.

The tissue distribution in T-cells indicates that polynucleotides and polypeptides corresponding to this gene are useful for treatment of disorders of the immune system such as AIDS. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Expression of this gene product in T cells also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:83 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1141 of SEQ ID NO:83, b is an integer of 15 to 1155, where both a and b correspond to the positions of nucleotide residues shown in SEQ ID NO:83, and where b is greater than or equal to a + 14.

### FEATURES OF PROTEIN ENCODED BY GENE NO: 74

When tested against U937 cell lines, supernatants removed from cells containing this gene activated the GAS (gamma activating sequence) promoter element. Thus, it is likely that this gene activates myeloid cells, including progenitors, through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
DVLLPLLYLLVRKHINRAGIGNTFQGGANCI (SEQ ID NO:345), MCCCLCCT SWSGSTSTERVSGTRFREVPTASCSSSAPAPSELGSSLSVAAAALLSLPPRARLA LPRLPRL PSQENLRNPKGPQGNFQAPGAFVLSSSVA (SEQ ID NO:346), CAAA SAVPPGPEAHQQSGYREHVSGRCQLHHVRPLHPRRPNSALLSLLLLLLFSASH QEPGWHSQGSRAF QARRISGIPRDPRGTSKHLELLSFLVLWHRCCLPGG RXF CESLXQGRSACLLHQKPPLLMLSAPLGEQLP TQLLLPPRSSGSKFXRYQRPGP RVGVHLHKGSSEIREAGGPQLWPQCPHPVDLDVLRTTQHCLQSEGPTS VH LSSV (SEQ ID NO:347), EVEEAELAAALPMEPRASIAGASGAADMHFCPAXGTH RXA YPQEGSTYATELERTKAPGAWKFPWGPLGFLRFSWLGRRGSLGSAS RALGGRLRRAAAATEREEPSSDGA GAEDEHDAVGTSLKRVPDTRS VDVLPD QEVQQRQQHI (SEQ ID NO:348), RRISGIPRDPRGTSKHLELLSFLVLWHRCCL (SEQ ID NO:349), and/or RTKAPGAWKFPWGPLGFLRFSWLGRRGSL (SEQ ID NO:350). Polynucleotides encoding these polypeptides are also encompassed by the invention.

This gene is expressed primarily in smooth muscle.

Therefore, polynucleotides and polypeptides of the invention are useful as reagents for differential identification of the tissue(s) or cell type(s) present in a biological sample and for diagnosis of diseases and conditions which include, but are not limited to, disorders of smooth muscle tissue, particularly vascular disorders, such as vasculositis, microvascular disease, atherosclerosis, stroke, aneurysm, and embolism. Similarly, polypeptides and antibodies directed to these polypeptides are useful in providing immunological probes for differential identification of the tissue(s) or cell type(s). For a number of disorders of the above tissues or cells, particularly of smooth muscle tissue, expression of this gene at significantly higher or lower levels may be routinely detected in certain tissues and cell types (e.g.smooth muscle, vascular, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:209 as residues: Ser-23 to Glu-54.

The tissue distribution in smooth muscle, combined with the detected GAS biological activity indicates that polynucleotides and polypeptides corresponding to this gene are useful for diagnosis and treatment of vascular or cardiopulmonary disorders. In addition, the protein may show utility in the modulation of the immune system in response to various vascular disorders, particularly in the early stages of atherosclerosis, embolism, thrombosis, and stroke. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:84 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1359 of SEQ ID NO:84, b is an integer of 15 to 1373, where both a and b correspond to the positions of nucleotide residues shown in SEQ ID NO:84, and where b is greater than or equal to a + 14.

### FEATURES OF PROTEIN ENCODED BY GENE NO: 75.

In specific embodiments, polypeptides of the invention comprise the following amino acid sequence: PRLAQLRLLSL (SEQ ID NO:351),
QSDFREMNQTNSTSNAAKAREAQQGRGRD REAEFSSSALEHLVCYLQAYKHT, LLFIRSLNEHGLQQLLFQWRDGLFGNWYFRIPILLFFTGFHCYHLSC PHLPC AQRQSSRGTVPYVLCPHPHHHLHHYSWFPFLIPVLHTLPKLQPKFHGRPEQPL NLLQVKPTSGTI ASAEQVWVK (SEQ ID NO:352). VCYLQAYKHTLLFIRSLNEH GLQQLLFQW (SEQ ID NO:353), and/or VPYVLCPHPHHHLHHYSWFPFLIPVLH TLPKL (SEQ ID NO:354). Polynucleotides encoding these polypeptides are also encompassed by the invention. The gene encoding the disclosed cDNA is believed to reside on chromosome 1. Accordingly, polynucleotides related to this invention are useful as a marker in linkage analysis for chromosome 1.

This gene is expressed primarily in brain, ulcerative colitis, pancreas tumor, placenta, and to a lesser extent, in thyroid, bone marrow stromal cells, B-cell lymphoma, and hemangiopericytoma.

Therefore, polynucleotides and polypeptides of the invention are useful as reagents for differential identification of the tissue(s) or cell type(s) present in a biological sample and for diagnosis of diseases and conditions which include, but are not limited to, tumors and degenerative conditions involving infiltration by the immune system, particularly in soft-tissues, in addition to, neural, gastrointestinal, metabolic, reproductive, endocrine, and hematopoietic, or immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.neural, gastrointestinal, metabolic, reproductive, endocrine, hematopoietic, immune disorders, and cancerous and wounded tissues) or bodily fluids (e.g.lymph, serum, bile, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:210 as residues: Lys-33 to Arg-51, Gly-64 to Gly-74.

The tissue distribution in brain tissues indicates that polynucleotides and polypeptides corresponding to this gene are useful for treating the secondary effects of immune system involvement in diseases such as pancreatic tumors, ulcerative colitis, and Alzheimer's disease. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:85 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1244 of SEQ ID NO:85, b is an integer of 15 to 1258, where both a and b correspond to the positions of nucleotide residues shown in SEQ ID NO:85, and where b is greater than or equal to a + 14.

### FEATURES OF PROTEIN ENCODED BY GENE NO: 76

When tested against PC12 cell lines, supernatants removed from cells containing this gene activated the EGR1 (early growth response gene 1) promoter element. Thus, it is likely that this gene activates sensory neuron cells through the EGR1 signal transduction pathway. EGR1 is a separate signal transduction pathway from Jak-STAT, genes containing the EGR1 promoter are induced in various tissues and cell types upon activation, leading the cells to undergo differentiation and proliferation. In specific embodiments, polypeptides of the invention comprise the following amino acid sequence: ESERAVVYLITGALFIVSSCVLCFLPSSRRE (SEQ ID NO:355). Polynucleotides encoding these polypeptides are also encompassed by the invention. The gene encoding the disclosed cDNA is believed to reside on chromosome 12. Accordingly, polynucleotides related to this invention are useful as a marker in linkage analysis for chromosome 12.

This gene is expressed primarily in activated T cells, tonsils, and activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune and inflammatory disorders. Similarly, polypeptides and antibodies directed to these polypeptides are useful in providing immunological probes for differential identification of the tissue(s) or cell type(s). For a number of disorders of the above tissues or cells, particularly of the activated T cells, tonsils and activated monocytes, expression of this gene at significantly higher or lower levels may be routinely detected in certain tissues and cell types (e.g. Immune, hematopoietic, neural, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cells and immune tissues or cell types, combined with the detected EGR biological activity indicates that polynucleotides and polypeptides corresponding to this gene are useful for diagnosis and treatment of immune and inflammatory disorders. Moreover, this gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:86 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1304 of SEQ ID NO:86, b is an integer of 15 to 1318, where both a and b correspond to the positions of nucleotide residues shown in SEQ ID NO:86, and where b is greater than or equal to a + 14.

### FEATURES OF PROTEIN ENCODED BY GENE NO: 77

When tested against fibroblast cell lines, supernatants removed from cells containing this gene activated the EGR1 assay. Thus, it is likely that this gene activates fibroblast cells through a signal transduction pathway. Early growth response 1 (EGR1) is a promoter associated with certain genes that induces various tissues and cell types upon activation, leading the cells to undergo differentiation and proliferation. The gene encoding the disclosed cDNA is thought to reside on chromosome 16. Accordingly, polynucleotides related to this invention are useful as a marker in linkage analysis for chromosome 16.

This gene is expressed primarily in eosinophils and activated T-cells and to a lesser extent in lung and thymus stromal cells.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:212 as residues: Met-1 to Trp-10.

The tissue distribution indicates that polynucleotides and polypeptides corresponding to this gene are useful for the disgnosis and treatment of immune disorders, including infection, allergy, inflammation, graft rejection and immunodeficiency. Furthermore, the tissue distribution indicates that polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in T cells and eosinophils also strongly indicates a role for this protein in immune function and immune surveillance.

Many polynucleotide sequences, such as EST sequences, are publicly available and accessible through sequence databases. Some of these sequences are related to SEQ ID NO:87 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 964 of SEQ ID NO:87, b is an integer of 15 to 978, where both a and b correspond to the positions of nucleotide residues shown in SEQ ID NO:87, and where b is greater than or equal to a + 14.

### FEATURES OF PROTEIN ENCODED BY GENE NO: 78

One embodiment of this gene comprises polypeptides of the following amino acid sequence:
MWVXGEEVLGSHAASPAFLHRCFSEESCVSIPEVEGYVVVLQPDAPQILLSGTA HFARPAVDFEGTNGVPLFPDLQITCSISHQVEAKKDESWQGTVTDTRMSDEIVH NLDGCEISLVGDDLDPERESLLLDTTSLQQRGLELTNTSAYLTIAGVESITVYEEI LRQARYRLRHGAALYTRKFRLSCSEMNGRYSSNEFIVEVNVLHSMNRVAHPS HVLSXQQFLHRGHQPPPEMAGHSLASSHRNSST (SEQ ID NO:356), LGSHAA SPAFLHRCFSEESCVSI (SEQ ID NO:357), GYVVVLQPDAPQILLSGTAHFARP AVDFE (SEQ ID NO:358), ITCSISHQVEAKKDESWQGTVTDTRM (SEQ ID NO:359), NLDGCEISLVGDDLDPERESLLLDTTSLQ (SEQ ID NO:360), SAYLTI AGVESITVYEEILRQAR (SEQ ID NO:361), RLSCSEMNGRYSSNEFIVEVNVLH SM (SEQ ID NO:362), and/or QQFLHRGHQPPPEMAGHSLASSHRN (SEQ ID NO:363). An additional embodiment is the polynucleotides encoding these polypeptides.

This gene is expressed primarily in brain and spleen.

Therefore, polynucleotides and polypeptides of the invention are useful as reagents for differential identification of the tissue(s) or cell type(s) present in a biological sample and for diagnosis of diseases and conditions which include, but are not limited to, brain afflictions such as depression, schizophrenia, Alzheimer's disease, Parkinson's disease, Huntington's disease, specific brain tumors, aphasia, mania, depression, dementia, paranoia, addictive behavior and sleep disorders, as well as immune disorders such as leukemias, lymphomas, AIDS, arthritis and imflammation. Similarly, polypeptides and antibodies directed to these polypeptides are useful in providing immunological probes for differential identification of the tissue(s) or cell type(s). For a number of disorders of the above tissues or cells, particularly of the central nervous and immune systems, expression of this gene at significantly higher or lower levels may be routinely detected in certain tissues or cell types (e.g., brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:213 as residues: Gly-36 to Leu-44.

The tissue distribution indicates that polynucleotides and polypeptides corresponding to this gene are useful for the treatment and diagnosis of developmental, degenerative and behavioral diseases and conditions of the brain such as aphasia, depression, schizophrenia, Alzheimer's disease, Parkinson's disease, Huntington's disease, specific brain tumors, mania, depression, dementia, paranoia, addictive behavior and sleep disorders. In addition, the expression in spleen would suggest a possible role in the detection and treatment of immune disorders including: leukemias, lymphomas, auto-immunities, immunodeficiencies (e.g. AIDS), immuno-supressive conditions (transplantation) and hematopoeitic disorders as well as conditions of general microbial infection, inflammation or cancer.

Many polynucleotide sequences, such as EST sequences, are publicly available and accessible through sequence databases. Some of these sequences are related to SEQ ID NO:88 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1849 of SEQ ID NO:88, b is an integer of 15 to 1863, where both a and b correspond to the positions of nucleotide residues shown in SEQ ID NO:88, and where b is greater than or equal to a + 14.

### FEATURES OF PROTEIN ENCODED BY GENE NO: 79

When tested against K562 leukemia cell lines, supernatants removed from cells containing this gene activated the ISRE assay. Thus, it is likely that this gene activates leukemia cells through the Jak-STAT signal transduction pathway. The interferon-sensitive response element is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the ISRE element, can be used to indicate proteins involved in the proliferation and differentiation of cells. One embodiment of this gene comprises polypeptides of the following amino acid sequence:
MADSETFISLE ECRGHKRARKRTSMETALALEKLFPKQCQVLGIVTPGIVVXPMGSGSNRPQEI EIGESGFALLFPQIEGI KIQPFHFIKDPKNLTLERHQLTEVGLLDNPELRVVLV FGYNCCKVGASNYLQQVVSTFSDMNIILAGGQV DNLSSLTSEKNPLDID AS GVVGLSFSGHRIQSATVLLNEDVSDEKTAEAAMQRLKAANIPEHNTIGFMFA CVGRGFQYYRAKGNVEADAFRKFFPSVPLFGFFGNGEIGCDRIVTGNFILRKC NEVKDDDLFHSYTTIMA LIHLGSSK (SEQ ID NO:364), HKRARKRTSMETAL ALEKLFP (SEQ ID NO:365), MGSGSNRPQEIEIGESGFALLFPQ (SEQ ID NO:366), FHFIKDPKNLTLERHQLTEVGL (SEQ ID NO:367), FGYNCCKVGASN YLQQVVSTFSD (SEQ ID NO:368), TSEKNPLDIDASGVVGLSFS (SEQ ID NO:369), NEDVSDEKTAEAAMQRLKAANIPEHN (SEQ ID NO:370, YYRAKGNV EADAFRKFFPSVPLFGF (SEQ-ID NO:371), and/or IGCDRIVTGNFILRKCNE VKDDDLFH (SEQ ID NO:372). An additional embodiment is the polynucleotides encoding these polypeptides.

This gene is expressed primarily in endothelial cells and to a lesser extent in reproductive and various endocrine organs.

Therefore, polynucleotides and polypeptides of the invention are useful as reagents for differential identification of the tissue(s) or cell type(s) present in a biological sample and for diagnosis of diseases and conditions which include, but are not limited to, cancer, cardiovascular and immune defects. Similarly, polypeptides and antibodies directed to these polypeptides are useful in providing immunological probes for differential identification of the tissue(s) or cell type(s). For a number of disorders of the above tissues or cells, particularly of the immune, cardiovascular, and reproductive systems, expression of this gene at significantly higher or lower levels may be routinely detected in certain tissues or cell types (e.g. endothelial, reproductive, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:214 as residues: Ser-44 to Ala-50.

The tissue distribution indicates that polynucleotides and polypeptides corresponding to this gene are useful for the diagnosis and treatment of cancer, cardiovascular and reproductive disorders.

Many polynucleotide sequences, such as EST sequences, are publicly available and accessible through sequence databases. Some of these sequences are related to SEQ ID NO:89 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2072 of SEQ ID NO:89, b is an integer of 15 to 2086, where both a and b correspond to the positions of nucleotide residues shown in SEQ ID NO:89, and where b is greater than or equal to a + 14.

### FEATURES OF PROTEIN ENCODED BY GENE NO: 80

This gene is expressed primarily in human tongue and TNF-induced epithelium.

Therefore, polynucleotides and polypeptides of the invention are useful as reagents for differential identification of the tissue(s) or cell type(s) present in a biological sample and for diagnosis of diseases and conditions which include, but are not limited to, mucosal, oral, and inflammatory conditons. Similarly, polypeptides and antibodies directed to these polypeptides are useful in providing immunological probes for differential identification of the tissue(s) or cell type(s). For a number of disorders of the above tissues or cells, particularly of mucosal and epidermal tissues, expression of this gene at significantly higher or lower levels may be routinely detected in certain tissues or cell types (e.g. tongue, epithelial, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:215 as residues: Ser-39 to Leu-48, Ala-65 to Pro-75, Pro-81 to Cys-87.

The tissue distribution indicates that polynucleotides and polypeptides corresponding to this gene are useful for the study and treatment of disorders of the oral and intestinal mucosa, inflammation and other epithelial disorders.

Many polynucleotide sequences, such as EST sequences, are publicly available and accessible through sequence databases. Some of these sequences are related to SEQ ID NO:90 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 877 of SEQ ID NO:90, b is an integer of 15 to 891, where both a and b correspond to the positions of nucleotide residues shown in SEQ ID NO:90, and where b is greater than or equal to a + 14.

### FEATURES OF PROTEIN ENCODED BY GENE NO: 81

This gene is expressed primarily in activated neutrophils.

Therefore, polynucleotides and polypeptides of the invention are useful as reagents for differential identification of the tissue(s) or cell type(s) present in a biological sample and for diagnosis of diseases and conditions which include, but are not limited to, immune, autoimmune, and inflammatory conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the study, diagnosis and treatment of immune, autoimmune, and inflammatory disorders. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Expression of this gene product in neutrophils strongly indicates a role for this protein in immune function and immune surveillance.

Many polynucleotide sequences, such as EST sequences, are publicly available and accessible through sequence databases. Some of these sequences are related to SEQ ID NO:91 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1960 of SEQ ID NO:91, b is an integer of 15 to 1974, where both a and b correspond to the positions of nucleotide residues shown in SEQ ID NO:91, and where b is greater than or equal to a + 14.

### FEATURES OF PROTEIN ENCODED BY GENE NO: 82

This gene is expressed primarily in primary dendritic cells, and to a lesser extent in neutrophils, monocytes, and osteoblasts.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic conditions. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues or cell types (e.g. immune, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:217 as residues: Gly-47 to Arg-53.

The tissue distribution indicates that polynucleotides and polypeptides corresponding to this gene are useful for the study and treatment of immune, inflammatory and hematopoietic disorders. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Expression of this gene product in neutrophils and primary dendritic cells also strongly indicates a role for this protein in immune function and immune surveillance.

Many polynucleotide sequences, such as EST sequences, are publicly available and accessible through sequence databases. Some of these sequences are related to SEQ ID NO:92 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1409 of SEQ ID NO:92, b is an integer of 15 to 1423, where both a and b correspond to the positions of nucleotide residues shown in SEQ ID NO:92, and where b is greater than or equal to a + 14.

### FEATURES OF PROTEIN ENCODED BY GENE NO: 83

One embodiment of this gene comprises polypeptides of the following amino acid sequence:
MPKRKVTFQGVGDEEDEDEIIVPKKKLVDPVAGSGGPGSRFKGKHSLDSDEEE DDDDGGSSKYDILASEDVEGQEAATLPSEGGVRITPFNLQEEMEEGHFDADGN YFLNRDAQIRDSWLDNIDWVKIRERPPGQRQASDSEEEDSLGQTSMSAQALLEG LLELLLPRETVAGALRRLGARGGGKGRKGPGQPSSPQRLDRLSGLADQMVAR GNLGVYQETRERLAMRLKGLGCQTLGPHNPTPPPSLDMFAEELAEEELETPTPT QRGEAESRGDGLVDVMWEYKWENTGDAELYGPFTSAQMQTWVSEGYFPDGV YCRKLDPPGGQFYNSKRIDFDLYT (SEQ ID NO:373), TFQGVGDEEDEDEIIVP KKKLVDP (SEQ ID NO:374), PGSRFKGKHSLDSDEEEDDDDGGSSKY (SEQ ID NO:375), EAATLPSEGGVRITPFNLQEEMEEG (SEQ ID NO:376), FLNRDAQIRDS WLDNIDWVKIRERPPGQR (SEQ ID NO:377), SLGQTSMSAQALLEGLLELLL PRETV (SEQ ID NO:378), RGGGKGRKGPGQPSSPQRLDRLSGLADQ (SEQ ID NO:379), QETRERLAMRLKGLGCQTLGPHNP (SEQ ID NO:380, DMFAEELAEEE LETPTPTQRGEAESRGD (SEQ ID NO:381), and/or ELYGPFTSAQMQTW VSEGYFPDGVYCRKLD (SEQ ID NO:382). An additional embodiment is the polynucleotides encoding these polypeptides.

This gene is expressed primarily in fetal lung, stromal cells and lymphoma cells.

Therefore, polynucleotides and polypeptides of the invention are useful as reagents for differential identification of the tissue(s) or cell type(s) present in a biological sample and for diagnosis of diseases and conditions which include, but are not limited to, haemopoietic and respiratory disorders and cancer. Similarly, polypeptides and antibodies directed to these polypeptides are useful in providing immunological probes for differential identification of the tissue(s) or cell type(s). For a number of disorders of the above tissues or cells, particularly of the haemopoietic and respiratory systems, expression of this gene at significantly higher or lower levels may be routinely detected in certain tissues or cell types (e.g. lung, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:218 as residues: Met-1 to Trp-15, Thr-52 to Met-58.

The tissue distribution indicates that polynucleotides and polypeptides corresponding to this gene are useful for the treatment and diagnosis of diseases of the haemopoietic and respiratory systems. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO:93 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1351 of SEQ ID NO:93, b is an integer of 15 to 1365, where both a and b correspond to the positions of nucleotide residues shown in SEQ ID NO:93, and where b is greater than or equal to a + 14.

### FEATURES OF PROTEIN ENCODED BY GENE NO: 84

In specific embodiments, polypeptides of the invention comprise the following amino acid sequence: PHSSRVSFLQSLSF (SEQ ID NO:383), RGQPRPCVSGVCLS PHSRFWECCSFYLQGLPALRCSRTPPGCHFFRVFPSCPFSSSRSPSCFT HICPV VRIQFSRALWVSTCLVLAITPGKWLLPEDRALSLMLLASLQCCPPPFGAWWMQ VLTHKGRQAGLG PGVSSRPL (SEQ ID NO:384, S NIKSLPPTNSLSLLRA QTGTDCAVSPGLAGPCHQRGLEDTPGPRPACLPLCVSTCIHQAPKGGGQHWR EA SSIRDRALSSGRSHFPGVMAKTKHVDTHNARENWIRTTGQMWVKHEG EREEEKGHEGKTLKK (SEQ ID NO:385), VCLSPHSRFWECCSFYLQGLPALRC (SEQ ID NO:386), QFSRALWVSTCLVLAITPGKWLLPEDR (SEQ ID NO:3 87), SLSLLRAQTGTDCAVSPGLAGPCHQRG (SEQ ID NO:388), and/or SGRSHFPG VMAKTKHVDTHNARENWIRT (SEQ ID NO:389). Polynucleotides encoding these polypeptides are also encompassed by the invention. When tested against U937 cell lines, supernatants removed from cells containing this gene activated the GAS (gamma activating sequence) promoter element. Thus, it is likely that this gene activates myeloid cells, including their progenitors, through the Jak-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells.

This gene is expressed primarily in T-cells and lungs.

Therefore, polynucleotides and polypeptides of the invention are useful as reagents for differential identification of the tissue(s) or cell type(s) present in a biological sample and for diagnosis of diseases and conditions which include, but are not limited to, respiratory and immune diseases. Similarly, polypeptides and antibodies directed to these polypeptides are useful in providing immunological probes for differential identification of the tissue(s) or cell type(s). For a number of disorders of the above tissues or cells, particularly of the immune and respiratory systems, expression of this gene at significantly higher or lower levels may be routinely detected in certain tissues or cell types (e.g. pulmonary, immune, hematopoietic, and cancerous and wounded tissues) or bodily fluids (e.g. lymph, pulmponary surfactant or sputum, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:219 as residues: His-38 to Ala-43.

The tissue distribution in T-cells and lung tissue, combined with the detected GAS biological activity indicates that polynucleotides and polypeptides corresponding to this gene are useful for the treatment and diagnosis of disorders of the respiratory and immune systems. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. The protein may show utility in modulating the immune response to various pulmonary disorders or conditions, particularly in emphysema, or ARDS.

Many polynucleotide sequences, such as EST sequences, are publicly available and accessible through sequence databases. Some of these sequences are related to SEQ ID NO:94 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 742 of SEQ ID NO:94, b is an integer of 15 to 756, where both a and b correspond to the positions of nucleotide residues shown in SEQ ID NO:94, and where b is greater than or equal to a + 14.

### FEATURES OF PROTEIN ENCODED BY GENE NO: 85

In specific embodiments, polypeptides of the invention comprise the following amino acid sequence: ARVEVQGQGPGAKVDAGEGQ (SEQ ID NO:390) WVVL SQLQA QGVAGMMCSYPEGQKKGKEATRSHRWVPRSLPGMGSXLAAPHS NPWLAPLALLEIPXPVLCEWKRKLIAL EEVSECRPGVGGGGGFLSXCRR GHLSFLSGAPYPLFPISPLX (SEQ ID NO:391), ELRHGGPRQVKDSFLDYM GYPDEDRAGPPSRWFPRERFLSPPTV VPLCVELRLGFESGMGWGVPGSSHS EGGPEARWPLIAPMYTVTQWFQRPNSGRGPQPPPQXRGEIGKRGY GAPER KLRWPLLXWERXPPPPPTPGRHSETSSSAISFLFHSQRTGWGISSSANGASQGL LWGAARXLPIP GRDLGTHLWDLVASFPFFCPSG (SEQ ID NO:392), PEGQKK GKEATRSHRWVPRSLPGM (SEQ ID NO:393), LRLGFESGMGWGVPGSSHSEG GPEAR (SEQ ID NO:394), and/or HSQRTGWGISSSANGASQGLLWGA (SEQ ID NO:395. Polynucleotides encoding these polypeptides are also encompassed by the invention.

This gene is expressed primarily in eosinophils, dendritic cells, Jurkat cells and tonsils.

Therefore, polynucleotides and polypeptides of the invention are useful as reagents for differential identification of the tissue(s) or cell type(s) present in a biological sample and for diagnosis of diseases and conditions which include, but are not limited to, immune, or hematopoietic disorders, particularly inflammatory, autoimmune, allergy, and hypersensitivity conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in a variety of immune and hematopoietic-specific cells and tissues indicates that polynucleotides and polypeptides corresponding to this gene are useful for modifying the response of the immune system in autoimmune diseases and inflammatory conditions. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); antiinflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:95 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 924 of SEQ ID NO:95, b is an integer of 15 to 938, where both a and b correspond to the positions of nucleotide residues shown in SEQ ID NO:95, and where b is greater than or equal to a + 14.

### FEATURES OF PROTEIN ENCODED BY GENE NO: 86

This gene is expressed primarily in cells from fibrosarcoma tumors.

Therefore, polynucleotides and polypeptides of the invention are useful as reagents for differential identification of the tissue(s) or cell type(s) present in a biological sample and for diagnosis of diseases and conditions which include, but are not limited to, muscle, or endothelial disorders, particularly fibrosarcomas and fibroids. Similarly, polypeptides and antibodies directed to these polypeptides are useful in providing immunological probes for differential identification of the tissue(s) or cell type(s). For a number of disorders of the above tissues or cells, particularly of the skeleto-muscular system, expression of this gene at significantly higher or lower levels may be routinely detected in certain tissues or cell types (e.g. skeleto-muscular, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fibrosarcoma tissue indicates that polynucleotides and polypeptides corresponding to this gene are useful for the detection, treatment, and/or prevention of various muscle disorders, in particular fibrosarcomas. In addition, the expression of this gene product in synovium would suggest a role in the detection and treatment of disorders and conditions affecting the skeletal system, in particular osteoporosis as well as disorders afflicting connective tissues (e.g. arthritis, trauma, tendonitis, chrondomalacia and inflammation). The gene or protein product of tis gene may also show utility in modulating the immune response to proliferative tissues. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:96 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 914 of SEQ ID NO:96, b is an integer of 15 to 928, where both a and b correspond to the positions of nucleotide residues shown in SEQ ID NO:96, and where b is greater than or equal to a + 14.

### FEATURES OF PROTEIN ENCODED BY GENE NO: 87

This gene is expressed primarily in helper T-Cells, cerebellum, and to a lesser extent, in mesangial cells, fetal lung, fetal liver, cortex, and adipose tissue.

Therefore, polynucleotides and polypeptides of the invention are useful as reagents for differential identification of the tissue(s) or cell type(s) present in a biological sample and for diagnosis of diseases and conditions which include, but are not limited to, immune, or neural disorders, particularly, for modulatin of immune responses to viral or bacterial infections, or neurodefeciencies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g.renal, developmental, pulmonary, hepatic, neural, metabolic, immune, and cancerous and wounded tissues) or bodily fluids (e.g.lymph, serum, bile, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in helper T-cells indicates that polynucleotides and polypeptides corresponding to this gene are useful for modifying the immune response to foreign agents such as bacteria or virus. In addition, this gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Moreover, based upon the expression within the cerebellum and cortex, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism; and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:97 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1701 of SEQ ID NO:97, b is an integer of 15 to 1715, where both a and b correspond to the positions of nucleotide residues shown in SEQ ID NO:97, and where b is greater than or equal to a + 14.

### FEATURES OF PROTEIN ENCODED BY GENE NO: 88

When tested against U937 cell lines, supernatants removed from cells containing this gene activated the GAS (gamma activating sequence) promoter element. Thus, it is likely that this gene activates promyelocytic cells through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
FIMKLLYQLLMLTTSSSYSLITHLCYSIFLCSFYFHFPCNVSLFVLISEEFIYD (SEQ ID NO:396), LMLTTSSSYSLITHLCYSIFL (SEQ ID NO:397), LCSFYFH FPCNVSLFVLISEE (SEQ ID NO:398), MRKNIFAILDKMLTCLIINELFRNQYKET NITREVKIKGTEENGIAQMSYKAI (SEQ ID NO:399), DKMLTCLIINELFRNQ YKETN (SEQ ID NO:400), and/or NITREVKIKGTEENGIAQMSY (SEQ ID NO:401). Polynucleotides encoding these polypeptides are also encompassed by the invention.

This gene is expressed primarily in fetal lung.

Therefore, polynucleotides and polypeptides of the invention are useful as reagents for differential identification of the tissue(s) or cell type(s) present in a biological sample and for diagnosis of diseases and conditions which include, but are not limited to, pulmonary and developmental disorders. Similarly, polypeptides and antibodies directed to these polypeptides are useful in providing immunological probes for differential identification of the tissue(s) or cell type(s). For a number of disorders of the above tissues or cells, particularly of the developmental and pulmonary systems, expression of this gene at significantly higher or lower levels may be routinely detected in certain tissues and cell types (e.g. Pulmonary, developmental, and cancerous and wounded tissues) or bodily fluids (e.g.lymph, serum, pulmonary surfactant or sputum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution of this gene only in fetal lung, combined with the detected GAS biological activity indicates that it plays a key role in development of the pulmonary system. This would suggest that misregulation of the expression of this protein product in the adult could lead to lymphoma or sarcoma formation, particularly in the lung. It may also be involved in the predisposition to certain pulmonary defects such as pulmonary edema and embolism, bronchitis and cystic fibrosis. Moreover, the protein product of this gene may be beneficial in the treatment of underdeveloped lung tissue, as exists in premature infants; both through the use of antibodies directed against the protein, through a gene therapy-based regimine, or through the action of the protein itself, either directly or indirectly. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:98 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 664 of SEQ ID NO:98, b is an integer of 15 to 678, where both a and b correspond to the positions of nucleotide residues shown in SEQ ID NO:98, and where b is greater than or equal to a + 14.

### FEATURES OF PROTEIN ENCODED BY GENE NO: 89

When tested against U937 cell lines, supernatants removed from cells containing this gene activated the GAS (gamma activating sequence) promoter element. Thus, it is likely that this gene activates myeloid cells, including their progenitors, through the Jak-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence: GISERKP (SEQ ID NO:402). Polynucleotides encoding these polypeptides are also encompassed by the invention.

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, neural or immune disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g. neural, immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:224 as residues: Ile-40 to Trp-50.

The tissue distribution in brain combined with the detected GAS biological activity indicates that polynucleotides and polypeptides corresponding to this gene are useful for the treatment and diagnosis of central nervous system disorders. Moreover, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Furthermore, the protein may show utility in modulating the immune response to various neurodegenerative conditions. Protein, aswell as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:99 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1527 of SEQ ID NO:99, b is an integer of 15 to 1541, where both a and b correspond to the positions of nucleotide residues shown in SEQ ID NO:99, and where b is greater than or equal to a + 14.

### FEATURES OF PROTEIN ENCODED BY GENE NO: 90

In specific embodiments, polypeptides of the invention comprise the following amino acid sequence: QSPAVSYTVTSQVPWGLGLLAGEKR (SEQ ID NO:403), LPSHPLRPLTFS SAMCMHLPPPLCRRAALSAPFATQHRPWSVAAACLPRIHQN PLDAEYPSGCCRMSFLPAACSNIYSQECH YTLMSHSEASTLQXAQLL (SEQ ID NO:404), MLLQAAGRKLMRQQPDGYSASRGFWWMRGRQAAATLHGRCWVA KGADSAAL RQRGGGRCMHIADEKVRGLSGCDGS (SEQ ID NO:405), LCRRA ALSAPFATQHRPWSVAAACL (SEQ ID NO:406), RGFWWMRGRQAAATLHGR CWVAKG (SEQ ID NO:407), and/or QRGGGRCMHIADEKVRGLSGCDG (SEQ ID NO:408). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, inflammatory and immune conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:225 as residues: Pro-34 to His-39, Pro-44 to His-54.

The tissue distribution in neutrophils indicates that polynucleotides and polypeptides corresponding to this gene are useful for the study, diagnosis, and treatment of inflammatory, general immune, and infectious diseases. Moreover, the expression of this gene indicates a role in regulating the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:100 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 867 of SEQ ID NO: 100, b is an integer of 15 to 881, where both a and b correspond to the positions of nucleotide residues shown in SEQ ID NO: 100, and where b is greater than or equal to a + 14.

### FEATURES OF PROTEIN ENCODED BY GENE NO: 91

When tested against Jurkat cell lines, supernatants removed from cells containing this gene activated the GAS (gamma activating sequence) promoter element. Thus, it is likely that this gene activates myeloid cells through the JAK-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In addition, contact of cells with supernatant expressing the product of this gene has been shown to increase the permeability of the plasma membrane of stromal cells to calcium. Thus, it is likely that the product of this gene is involved in a signal transduction pathway that is initiated when the product binds a receptor on the surface of the plasma membrane of both stromal, in addition to other cell-lines or tissue cell types. Thus, polynucleotides and polypeptides have uses which include, but are not limited to, activating stromal cells. Binding of a ligand to a receptor is known to alter intracellular levels of small molecules, such as calcium, potassium and sodium, as well as alter pH and membrane potential. Alterations in small molecule concentration can be measured to identify supernatants which bind to receptors of a particular cell. In specific embodiments, polypeptides of the invention comprise the following amino acid sequence:
THPSHPSIVIQSTVSLCLTASSRRKKSDCLSLCQVSCSQRPGSHKTNVAWGFLM SRVHFSVRWVSGGRGI TGAICKESSLPCKEIQGKACYFCHHPAQQSTPFSHI (SEQ ID NO:409, VIQSTVSLCLTASSRRKKSDCLSLCQV (SEQ ID NO:410), and/or ICKESSLPCKEIQGKACYFCHHPAQQ (SEQ ID NO:411). Polynucleotides encoding these polypeptides are also encompassed by the invention.

This gene is expressed primarily in neutrophils, and to a lesser extent, in cord blood.

Therefore, polynucleotides and polypeptides of the invention are useful as reagents for differential identification of the tissue(s) or cell type(s) present in a biological sample and for diagnosis of diseases and conditions which include, but are not limited to, immune or developmental disorders, particularly inflammatory conditions. Similarly, polypeptides and antibodies directed to these polypeptides are useful in providing immunological probes for differential identification of the tissue(s) or cell type(s). For a number of disorders of the above tissues or cells, particularly of the immune and hemopoietic systems, expression of this gene at significantly higher or lower levels may be routinely detected in certain tissues or cell types (e.g. immune, developmental, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:226 as residues: Glu-32 to Arg-37.

The tissue distribution in neutrophils, combined with the detected GAS and calcium flux biological activities, indicates that polynucleotides and polypeptides corresponding to this gene are useful for the study and treatment of inflammatory, infectious, and hemopoietic disorders. Similarly, expression within cord blood indicates that this protein may play a role in the regulation of cellular division, and may show utility in the diagnosis and treatment of cancer and other proliferative disorders, particularly of the developing hematopoietic system. Similarly, developmental tissues rely on decisions involving cell differentiation and/or apoptosis in pattern formation. Thus, this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:101 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 933 of SEQ ID NO:101, b is an integer of 15 to 947, where both a and b correspond to the positions of nucleotide residues shown in SEQ ID NO:101, and where b is greater than or equal to a + 14.

### FEATURES OF PROTEIN ENCODED BY GENE NO: 92

The gene encoding the disclosed cDNA is thought to reside on chromosome 15. Accordingly, polynucleotides related to this invention are useful as a marker in linkage analysis for chromosome 15.

This gene is expressed primarily in macrophages, T cells, dendritic cells, testes and pancreas tumors.

Therefore, polynucleotides and polypeptides of the invention are useful as reagents for differential identification of the tissue(s) or cell type(s) present in a biological sample and for diagnosis of diseases and conditions which include, but are not limited to, immune disorders including testis and pancreas tumors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, metabolic, and cancerous and wounded tissues) or bodily fluids (e.g. lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:227 as residues: Gln-85 to Lys-91, Pro-106 to Ser-117, Pro-124 to Ala-130, Trp-154 to Trp-160.

The tissue distribution indicates that polynucleotides and polypeptides corresponding to this gene are useful for the diagnosis and treatment of immune disorders such as testes and pancreas tumors. Furthermore, polynucleotides and polypeptides corresponding to this gene are useful for the diagnosis and/or treatment of hematopoietic disorders. This gene product is primarily expressed in hematopoietic cells and tissues, suggesting that it plays a role in the survival, proliferation, and/or differentiation of hematopoieitic lineages. Expression of this gene product in T cells and primary dendritic cells also strongly indicates a role for this protein in immune function and immune surveillance. Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 102 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1355 of SEQ ID NO: 102, b is an integer of 15 to 1369, where both a and b correspond to the positions of nucleotide residues shown in SEQ ID NO: 102, and where b is greater than or equal to a + 14.

### FEATURES OF PROTEIN ENCODED BY GENE NO: 93

This gene is expressed primarily in brain tissue from a patient suffering from manic depression.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly manic depression. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates that polynucleotides and polypeptides corresponding to this gene are useful for diagnosis of manic depression and other disorders of the CNS. Furthermore, polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 103 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1217 of SEQ ID NO: 103, b is an integer of 15 to 1231, where both a and b correspond to the positions of nucleotide residues shown in SEQ ID NO: 103, and where b is greater than or equal to a + 14.

### FEATURES OF PROTEIN ENCODED BY GENE NO: 94

This gene is expressed primarily in anergic T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly autoimmune disorders such as lupus. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cells indicates that polynucleotides and polypeptides corresponding to this gene are useful for the diagnosis and treatment of a variety of immune system disorders. Moreover, the protein product of this gene may play a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Expression of this gene product in T cells also strongly indicates a role for this protein in immune function and immune surveillance. Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 104 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1228 of SEQ ID NO: 104, b is an integer of 15 to 1242, where both a and b correspond to the positions of nucleotide residues shown in SEQ ID NO:104, and where b is greater than or equal to a + 14.

### FEATURES OF PROTEIN ENCODED BY GENE NO: 95

This gene is expressed primarily in the spinal cord.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly CNS, PNS, and a variety of congenital malformations of the spinal column and injuries of the spinal cord. Similarly, polypeptides and antibodies directed to these polypeptides are useful in providing immunological probes for differential identification of the tissue(s) or cell type(s) present in a biological sample. For a number of disorders of the above tissues or cells, particularly of the central nervous system, expression of this gene at significantly higher or lower levels may be routinely detected in certain tissues or cell types (e.g. CNS, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:230 as residues: Ser-44 to His-52.

The tissue distribution in spinal cord tissue indicates that polynucleotides and polypeptides corresponding to this gene are useful for the diagnosis and/or treatment of disorders of the brain and nervous system. Such involvement may impact many processes, such as learning and cognition. It may also be useful in the treatment of such neurodegenerative disorders as schizophrenia; ALS; or Alzheimer's. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO: 105 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1137 of SEQ ID NO: 105, b is an integer of 15 to 1151, where both a and b correspond to the positions of nucleotide residues shown in SEQ ID NO:105, and where b is greater than or equal to a + 14.

### FEATURES OF PROTEIN ENCODED BY GENE NO: 96

This gene is expressed primarily in smooth muscle.

Therefore, polynucleotides and polypeptides of the invention are useful as reagents for differential identification of the tissue(s) or cell type(s) present in a biological sample and for diagnosis of diseases and conditions which include, but are not limited to, muscular, vascular, or cardiopulmonary disorders, particularly a variety of diseases that include wasting and muscle mass loss including amyotropic lateral sclerosis, embolism, atherosclerosis, stroke, and aneurysm. Similarly, polypeptides and antibodies directed to these polypeptides are useful in providing immunological probes for differential identification of the tissue(s) or cell type(s). For a number of disorders of the above tissues or cells, particularly of the neuromuscular system, expression of this gene at significantly higher or lower levels may be routinely detected in certain tissues or cell types (e.g. muscle, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:231 as residues: Leu-37 to Trp-44.

The tissue distribution in smooth muscle indicates that polynucleotides and polypeptides corresponding to this gene are useful for the detection, treatment, and/or prevention of various muscle disorders, such as muscular dystrophy, cardiomyopathy, fibroids, myomas, vascular disorders, and rhabdomyosarcomas. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 106 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1614 of SEQ ID NO: 106, b is an integer of 15 to 1628, where both a and b correspond to the positions of nucleotide residues shown in SEQ ID NO: 106, and where b is greater than or equal to a + 14.

### FEATURES OF PROTEIN ENCODED BY GENE NO: 97

This gene is expressed primarily in the brain.

Therefore, polynucleotides and polypeptides of the invention are useful as reagents for differential identification of the tissue(s) or cell type(s) present in a biological sample and for diagnosis of diseases and conditions which include, but are not limited to, disorders affecting the brain and central nervous system, such as Alzheimer's disease. Similarly, polypeptides and antibodies directed to these polypeptides are useful in providing immunological probes for differential identification of the tissue(s) or cell type(s). For a number of disorders of the above tissues or cells, particularly of the brain and central nervous system, expression of this gene at significantly higher or lower levels may be routinely detected in certain tissues or cell types (e.g. brain, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, the gene or gene product may also play a role in the treatment and/or detection of developmental disorders associated with the developing embryo. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 107 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1451 of SEQ ID NO: 107, b is an integer of 15 to 1465, where both a and b correspond to the positions of nucleotide residues shown in SEQ ID NO: 107, and where b is greater than or equal to a + 14.

### FEATURES OF PROTEIN ENCODED BY GENE NO: 98

In specific embodiments, polypeptides of the invention comprise the following amino acid sequence: SLQVLRTLGSKCGDFLRSRFCKDVLPKLAGSLVT QAPISARAGPVYSHTLAFKLQLAVLQGLGPLCERLDLGEGDLNKVADACLIYLS VKQPVKLQEAARSVFL HLMKVDPDSTWFLLNELYCPVQFTPPHPSLHPVQLX GASGQQNPXHDQRAPAAQGAAVTLLPHHRGHRSL PYCQPEAGLTPPRP (SEQ ID NO:412), GADGNVSDFDNEEEEQSVPPKVDENDTRPDVEPPLPLQIQIAM DVMERCIHLLSDKNLQIRLKVLDVLDL CVVVLQSHKNQLLPLAHQAWPSL VHRLTRDAPLAVLRAFKFYVPWEASWTFFAAGSAKMSCQSWLAP (SEQ ID NO:413), TLGSKCGDFLRSRFCKDVLPKLAGSL (SEQ ID NO:414), PVYSHTL AFKLQLAVLQGLGPLCERLDLG (SEQ ID NO:415), SVPPKVDENDTRPDV EPPLPLQIQIAM (SEQ ID NO:416), and/or WPSLVHRLTRDAPLAVLRAFK FYVPW (SEQ ID NO:417). Polynucleotides encoding these polypeptides are also encompassed by the invention.

This gene is expressed primarily in kidney cortex, hemangiopericytoma, fetal spleen, infant brain, and to a lesser extent, in pancreas, lymph node, fetal liver, ovarian tumor, T-cells and other tissues.

Therefore, polynucleotides and polypeptides of the invention are useful as reagents for identification of the tissue(s) or cell type(s) present in a biological sample and for diagnosis of diseases and conditions which include, but are not limited to, renal, immune, neural, or developmental disorders, particularly tumors. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues and cell types (e.g.renal, immune, neural, developmental, reproductive, ovarian, hepatic, metabolic, and cancerous and wounded tissues) or bodily fluids (e.g.lymph, serum, bile, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:233 as residues: Pro-24 to Pro-37.

The tissue distribution in proliferating tissues and cells, combined with its distribution in developing tissues indicates that polynucleotides and polypeptides corresponding to this gene are useful for diagnosing and treating tumors. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 108 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1251 of SEQ ID NO:108, b is an integer of 15 to 1265, where both a and b correspond to the positions of nucleotide residues shown in SEQ ID NO:108, and where b is greater than or equal to a + 14.

### FEATURES OF PROTEIN ENCODED BY GENE NO: 99

In specific embodiments, polypeptides of the invention comprise the following amino acid sequence: SLGISTFGIMVFSVYFGGIMISIPYSGISFGNKKELNID SCYNMVNLKNIMFSERSQT (SEQ ID NO:418), HASGNNDPLWFLTYL (SEQ ID NO:419), MVFSVYFGGIMISIPYSGISF (SEQ ID NO:420), and/or FGNKKELNID SCYNMVNLKN (SEQ ID NO:421. Polynucleotides encoding these polypeptides are also encompassed by the invention.

This gene is expressed primarily in T-cells, spleen, and pancreas.

Therefore, polynucleotides and polypeptides of the invention are useful as reagents for differential identification of the tissue(s) or cell type(s) present in a biological sample and for diagnosis of diseases and conditions which include, but are not limited to, immune or endocrine disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, endocrine, pancreatic, cancerous and wounded tissues) or bodily fluids (e.g.lymph, serum, bile, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:234 as residues: Thr-24 to Arg-29.

The tissue distribution of this gene predominantly in cell types or tissues associated with the immune system indicates that the gene could be important for the treatment or detection of immune or hematopoietic disorders including, but not limited to, arthritis, asthma, immunodeficiency diseases and leukemia. Moreover, the expression within pancreatic tissues indicates that the protein product of this gene may be useful in the treatment or prevention of a variety of metabolic disorders, such as diabetes. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 109 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 992 of SEQ ID NO: 109, b is an integer of 15 to 1006, where both a and b correspond to the positions of nucleotide residues shown in SEQ ID NO:109, and where b is greater than or equal to a + 14.

### FEATURES OF PROTEIN ENCODED BY GENE NO: 100

The gene encoding the disclosed cDNA is believed to reside on the X chromosome. Accordingly, polynucleotides related to this invention are useful as a marker in linkage analysis for the X chromosome.

This gene is expressed primarily in urinary bladder carcinoma HSC172 cells, and to a lesser extent, in human adult heart, lung, osteoclastoma, and liver.

Therefore, polynucleotides and polypeptides of the invention are useful as reagents for differential identification of the tissue(s) or cell type(s) present in a biological sample and for diagnosis of diseases and conditions which include, but are not limited to, urogenital, or renal disorders, particularly urinary bladder carcinoma and other cancers. Similarly, polypeptides and antibodies directed to these polypeptides are useful in providing immunological probes for differential identification of the tissue(s) or cell type(s). For a number of disorders of the above tissues or cells, particularly of the bladder, expression of this gene at significantly higher or lower levels may be routinely detected in certain tissues or cell types (e.g. renal, cardiopulmonary, hepatic, and cancerous and wounded tissues) or bodily fluids (e.g.lymph, serum, bile, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:235 as residues: Gly-18 to Lys-23, Pro-31 to Gly-38.

The tissue distribution in urinary bladder carcinoma indicates that polynucleotides and polypeptides corresponding to this gene are useful for diagnosis and therapeutic targeting of urinary bladder carcinoma, osteoclastoma, and other cancers. Additionally, the tissue distribution in heart, lung and osteocarcinoma indicates an indication for the use of this gene and gene product in diagnosis and treatment of disorders in the heart and lung. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 110 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1244 of SEQ ID NO: 110, b is an integer of 15 to 1258, where both a and b correspond to the positions of nucleotide residues shown in SEQ ID NO:110, and where b is greater than or equal to a + 14.

### FEATURES OF PROTEIN ENCODED BY GENE NO: 101

In specific embodiments, polypeptides of the invention comprise the following amino acid sequence:
MNSFSVIASIVVLLPFPGLSVSACLPSHSHQCKTFILLFLPSSEKTLXXXPP SHSSTLGGQGGQIMRSGDRXHXG (SEQ ID NO:422), VVFFXXFFEMESH SVAQAGVQWRNLGSLQAL PPGFMPFSCLSLPGSWDYRRPPPSPANLXCIF SRDGGHHVSQXGLDLLTS (SEQ ID NO:423), IVVLLPFPGLSVSACLPS HSHQCKTFIL (SEQ ID NO:424), and/or PGFMPFSCLSLPGSWDYRRPPPSPAN (SEQ ID NO:425). Polynucleotides encoding these polypeptides are also encompassed by the invention.

This gene is expressed primarily in adipose tissue.

Therefore, polynucleotides and polypeptides of the invention are useful as reagents for differential identification of the tissue(s) or cell type(s) present in a biological sample and for diagnosis of diseases and conditions which include, but are not limited to, obesity and other metabolic disorders. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g. adipose, metabolic, neural, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:236 as residues: Arg-28 to Asn-33.

The tissue distribution in adipose tissue indicates that polynucleotides and polypeptides corresponding to this gene are useful for the treatment of obesity and other metabolic and endocrine conditions or disorders. Furthermore, the protein product of this gene may show utility in ameliorating conditions which occur secondary to aberrant fatty-acid metabolism (e.g. aberrant myelin sheath development), either directly or indirectly. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:111 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1439 of SEQ ID NO: 111, b is an integer of 15 to 1453, where both a and b correspond to the positions of nucleotide residues shown in SEQ ID NO:111, and where b is greater than or equal to a + 14.

### FEATURES OF PROTEIN ENCODED BY GENE NO: 102

In specific embodiments, polypeptides of the invention comprise the following amino acid sequence: YRFKNPKCRLFSVPCR (SEQ ID NO:426), TQNRELLAWK PKGTDDICTSHNTTHIQKMPGE ANSCCPRGAKSYHIDCWPPALFPRCVAYLFL NKPATLRKKYYCKPYHTQLHPAWHREKSAFWIFETVSQS KQSLTSLVYS VNELLVLSNLAQWALG (SEQ ID NO:427), AWKPKGTDDICTSHNTTHIQKMP (SEQ ID NO:428), CPRGAKSYHIDCWPPALFPRCVAYL (SEQ ID NO:429), SYHI DCWPPALFPRCVAYLFLNKPAT (SEQ ID NO:430), and/or RKKYYCKPY HTQLHPAWHREKSAFWIFET (SEQ ID NO:431). Polynucleotides encoding these polypeptides are also encompassed by the invention.

This gene is expressed primarily in dendritic cells and activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly inflammation, immune defects, mutiple myeloma, or immuodeficiecies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in-SEQ ID NO:237 as residues: Thr-27 to Arg-33.

The tissue distribution in dendritic cells and monocytes indicates that polynucleotides and polypeptides corresponding to this gene are useful for the diagnosis and treatment of inflammatory and immune disorders such as cancers, particularly of dendritic cells and monocytes, but also of hematopoietic progenitors. Similarly, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency, etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 112 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1538 of SEQ ID NO: 112, b is an integer of 15 to 1552, where both a and b correspond to the positions of nucleotide residues shown in SEQ ID NO: 112, and where b is greater than or equal to a + 14.

### FEATURES OF PROTEIN ENCODED BY GENE NO: 103

When tested against K562 leukemia cell lines, supernatants removed from cells containing this gene activated the ISRE assay. Thus, it is likely that this gene activates leukemia cells through the Jak-STAT signal transduction pathway. The interferon-sensitive response element is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the ISRE element, can be used to indicate proteins involved in the proliferation and differentiation of cells. The gene encoding the disclosed cDNA is thought to reside on chromosome 5. Accordingly, polynucleotides related to this invention are useful as a marker in linkage analysis for chromosome 5.

This gene is expressed primarily in placenta, adipose tissue and fibroblasts.

Therefore, polynucleotides and polypeptides of the invention are useful as reagents for differential identification of the tissue(s) or cell type(s) present in a biological sample and for diagnosis of diseases and conditions which include, but are not limited to, disorders of the skin, developing organs and metabolic disorders. Similarly, polypeptides and antibodies directed to these polypeptides are useful in providing immunological probes for differential identification of the tissue(s) or cell type(s). For a number of disorders of the above tissues or cells, particularly of the epidermal system metabolic system and embryogenesis, expression of this gene at significantly higher or lower levels may be routinely detected in certain tissues or cell types (e.g. epidermal, cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of disorders of the epidermal system, metabolic system and embryogenesis. Furthermore, the tissue distribution indicates that polynucleotides and polypeptides corresponding to this gene are useful for the diagnosis and/or treatment of disorders of the placenta. Specific expression within the placenta indicates that this gene product may play a role in the proper establishment and maintenance of placental function. Alternately, this gene product may be produced by the placenta and then transported to the embryo, where it may play a crucial role in the development and/or survival of the developing embryo or fetus. Expression of this gene product in a vascular-rich tissue such as the placenta also indicates that this gene product may be produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body.

Many polynucleotide sequences, such as EST sequences, are publicly available and accessible through sequence databases. Some of these sequences are related to SEQ ID NO: 113 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1475 of SEQ ID NO: 113, b is an integer of 15 to 1489, where both a and b correspond to the positions of nucleotide residues shown in SEQ ID NO:113, and where b is greater than or equal to a + 14.

### FEATURES OF PROTEIN ENCODED BY GENE NO: 104

In specific embodiments, polypeptides of the invention comprise the following amino acid sequence: ICLDSCSQVSVTSLWSFLRVHSLVQTLW (SEQ ID NO:432). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including neutropenia, cancer, inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:239 as residues: Ala-35 to Asp-44.

The tissue distribution in neutrophils indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy or may be useful in the treatment of immune dysfunction or anti-inflamatory by inhibiting infiltration of neutrophils to the site of injury or distress. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:114 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 593 of SEQ ID NO:114, b is an integer of 15 to 607, where both a and b correspond to the positions of nucleotide residues shown in SEQ ID NO: 114, and where b is greater than or equal to a + 14.

### FEATURES OF PROTEIN ENCODED BY GENE NO: 105

This gene is expressed primarily in stromal cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in stromal cells indicates that polynucleotides and polypeptides corresponding to this gene are useful for the diagnosis and/or treatment of immune disorders. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 115 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1484 of SEQ ID NO: 115, b is an integer of 15 to 1498, where both a and b correspond to the positions of nucleotide residues shown in SEQ ID NO: 115, and where b is greater than or equal to a + 14.

### FEATURES OF PROTEIN ENCODED BY GENE NO: 106

In specific embodiments, polypeptides of the invention comprise the following amino acid sequence: HYCC DFGTSLLGFYVPFHYYVHMVNIILTTIDFYHYKFC CSQNANKHCFKHFQIMTTVPYLNINKENLRFKNIF K (SEQ ID NO:433), TSL LGFYVPFHYYVHMVNIIL TTIDFY (SEQ ID NO:434), and/or FQIMTTVPYLN INKENLRFKNI (SEQ ID NO:435). Polynucleotides encoding these polypeptides are also encompassed by the invention. The gene encoding the disclosed cDNA is believed to reside on chromosome 5. Accordingly, polynucleotides related to this invention are useful as a marker in linkage analysis for chromosome 5.

This gene is expressed primarily in spleen, breast, placenta, ovarian cancer, and to a lesser extent, in B-cell lymphoma, pancreas tumor, osteoclastoma, thyroid, bone marrow, fetal liver, and stromal cells.

Therefore, polynucleotides and polypeptides of the invention are useful as reagents for differential identification of the tissue(s) or cell type(s) present in a biological sample and for diagnosis of diseases and conditions which include, but are not limited to, diseases characterized by immune cell activation and proliferation, particularly of the reproductive system. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, reproductive, metabolic, skeletal, endocrine, hepatic, placental, ovarian, and cancerous and wounded tissues) or bodily fluids (e.g.lymph, serum, bile, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:241 as residues: Ser-21 to Ser-27.

The tissue distribution in spleen and reproductive tissues indicates that the product of this gene is useful for modifying or detecting the proliferation or activation of cells in the hematopoietic system. The secreted protein can also be used to determine biological activity, to raise antibodies, as tissue markers, to isolate cognate ligands or receptors, to identify agents that modulate their interactions and as nutritional supplements. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); antiinflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 116 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1783 of SEQ ID NO: 116, b is an integer of 15 to 1797, where both a and b correspond to the positions of nucleotide residues shown in SEQ ID NO:116, and where b is greater than or equal to a + 14.

### FEATURES OF PROTEIN ENCODED BY GENE NO: 107

In specific embodiments, polypeptides of the invention comprise the following amino acid sequence:
ISESMSLVRSLQFYRGKNRAERTVISSSSHSCHLIDLEFQPRSDGEVSISFLEKGV ELRWGMGLEDLIGLGLGVSTRRSTVRRKEPTKAGMHTACSEEMEPENREN (SEQ ID NO:436), DGSRSVAQARVQWHHRGSLPPLPPRFKQFPLRHLRVGGITG ACRHTQIIFVVLVQMGFHHVG QAGLELLTSGDPPALASQSAGITGVSHSTRPKL LSWLPSDNLLGMALYSIQWALLANSLYFQVPSPLSML CAFLPLWVPSA (SEQ ID NO:437), RGKNRAERTVISSSSHSCHLIDLEFQP (SEQ ID NO:438), LGLGVST RRSTVRRKEPTKAGMHTACSEEMEP (SEQ ID NO:439), GDPPALASQSAGI TGVSHSTRPKL (SEQ ID NO:440), and/or ALYSIQWALLANSLYFQVPSPLSML (SEQ ID NO:441). Polynucleotides encoding these polypeptides are also encompassed by the invention.

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly bone marrow related diseases such as mutliple myeloma, immunodeficiencies, and hematopoietic disorders. Similarly, polypeptides and antibodies directed to these polypeptides are useful in providing immunological probes for differential identification of the tissue(s) or cell type(s). For a number of disorders of the above tissues or cells, particularly of the bone marrow, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:242 as residues: Gln-46 to Asn-56.

The tissue distribution in bone marrow indicates that polynucleotides and polypeptides corresponding to this gene are useful for the diagnosis and treatment of central nervous system disorders and hemopoietic system developmental disorders. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 117 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 938 of SEQ ID NO: 117, b is an integer of 15 to 952, where both a and b correspond to the positions of nucleotide residues shown in SEQ ID NO:117, and where b is greater than or equal to a + 14.

### FEATURES OF PROTEIN ENCODED BY GENE NO: 108

In specific embodiments, polypeptides of the invention comprise the following amino acid sequence: DRILLFYSRDGQTTSKGPNPACCLFLLKKFYWNTA (SEQ ID NO:442), and/or DGQTTSKGPNPACCLFLLKKF (SEQ ID NO:443). Polynucleotides encoding these polypeptides are also encompassed by the invention.

This gene is expressed primarily in early stage human brain.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly developmental disorders of the brain. Similarly, polypeptides and antibodies directed to these polypeptides are useful in providing immunological probes for differential identification of the tissue(s) or cell type(s). For a number of disorders of the above tissues or cells, particularly of the early stage human brain, expression of this gene at significantly higher or lower levels may be routinely detected in certain tissues and cell types (e.g.neural, developmental, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:243 as residues: Asn-16 to Gln-21.

The tissue distribution in early stage brain indicates that polynucleotides and polypeptides corresponding to this gene are useful for the diagnosis and treatment of brain development disorders. In addition, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Moreover, the expression within embryonic tissue indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 118 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1.171 of SEQ ID NO: 118, b is an integer of 15 to 1185, where both a and b correspond to the positions of nucleotide residues shown in SEQ ID NO: 118, and where b is greater than or equal to a + 14.

### FEATURES OF PROTEIN ENCODED BY GENE NO: 109

In specific embodiments, polypeptides of the invention comprise the following amino acid sequence: DPRVRRTLDLGITLYLFLYIFLSL (SEQ ID NO:444), PALGECCLDAFLFLLGKQLKKSGEKPLLGGSLMEYAILSAIAAMNEPKTCSTTA LKKYV LENHPGTNSNYQMHLLKKTLQKCEKNGWMEQISGKGFSGTFQL CFPYYPSPGVLFPKKEPDDSRDEDEDE DESSEEDSEDEEPPPKRRLQKKTPAKS PGKAASVKQRGSKPAPKVSAAQRGKARPLPKKAPPKAKTPAKK TRPSSTV IKKPSGGSSKKPATSARKEVKLPGKGKSTMKKSFRVKK (SEQ ID NO:445), DFEFHHDTLFSYKIYFFFLKDFFN4VDLPLPGNFFSFLALVAGFF EEPPLGFLM TVDEGLVFLAGVLALGGAFLGKGLAFPRWAAETLGAGLDPLCFTDAAFPGDLA GVFFCNLL LGGGSSSSESSSDDSSSSSSSSLESSGSFFGNRTPGLG (SEQ ID NO:446), CLDAFLFLLGKQLKKSGEKPLLGGSLME (SEQ ID NO:447), YQMHLL KKTLQKCEKNGWMEQISGKGFSGT (SEQ ID NO:448), KTPAKSPGKAAS VKQRGSKPAPKVSAAQ (SEQ ID NO:449), SSKKPATSARKEVKLPGKGKSTM KKSFR (SEQ ID NO:450), VDEGLVFLAGVLALGGAFLGKGL (SEQ ID NO:451), and/or GLDPLCFTDAAFPGDLAGVFFCNLL (SEQ ID NO:452). Polynucleotides encoding these polypeptides are also encompassed by the invention.

This gene is expressed primarily in bone marrow stromal cells, and to a lesser extent, in human osteoblasts and T cells (helper I).

Therefore, polynucleotides and polypeptides of the invention are useful as reagents for differential identification of the tissue(s) or cell type(s) present in a biological sample and for diagnosis of diseases and conditions which include, but are not limited to, connective tissues, haemopoietic, or immune disorders. Similarly, polypeptides and antibodies directed to these polypeptides are useful in providing immunological probes for differential identification of the tissue(s) or cell type(s). For a number of disorders of the above tissues or cells, particularly of the skeletal and immune system, expression of this gene at significantly higher or lower levels may be routinely detected in certain tissues and cell types (e.g.hematopoietic, immune, skeletal, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:244 as residues: Glu-18 to Cys-38.

The tissue distribution in bone marrow stromal cells and T-cells suggest that polynucleotides and polypeptides corresponding to this gene are useful for diagnosis and treatment of defects of stromal development, and immune system disorders. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Moreover, the expression of this gene product in osteoblasts would suggest a role in the detection and treatment of disorders and conditions affecting the skeletal system, in particular osteoporosis, bone cancer, as well as, disorders afflicting connective tissues (e.g. arthritis, trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 119 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1084 of SEQ ID NO: 119, b is an integer of 15 to 1098, where both a and b correspond to the positions of nucleotide residues shown in SEQ ID NO: 119, and where b is greater than or equal to a + 14.

### FEATURES OF PROTEIN ENCODED BY GENE NO: 110

This gene is expressed primarily in rhabdomyosarcoma, CD34 positive cells, breast lymph nodes, neutrophils and endothelial cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, developmental, proliferative, and vascular disorders, particularly fibroids or atherosclerosis. Similarly, polypeptides and antibodies directed to these polypeptides are useful in providing immunological probes for differential identification of the tissue(s) or cell type(s). For a number of disorders of the above tissues or cells, particularly of the immune or hematopoietic systems, expression of this gene at significantly higher or lower levels may be routinely detected in certain tissues or cell types (e.g. immune, hematopoietic, developmental, vascular, endothelial, and cancerous and wounded tissues) or bodily fluids (e.g. lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils and lymph nodes indicates that polynucleotides and polypeptides corresponding to this gene are useful for the diagnosis and intervention of disorders in immune or hematopoietic systems. Similarly, the secreted protein can also be used to determine biological activity, to raise antibodies, as tissue markers, to isolate cognate ligands or receptors, to identify agents that modulate their interactions, and as nutritional supplements. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); antiinflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. The protein may also show utility in the treatment or prevention of a variety of vascular disorders, particularly embolism, thrombis, aneurysms, stroke, or athersclerosis. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:120 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 791 of SEQ ID NO: 120, b is an integer of 15 to 805, where both a and b correspond to the positions of nucleotide residues shown in SEQ ID NO: 120, and where b is greater than or equal to a + 14.

### FEATURES OF PROTEIN ENCODED BY GENE NO: 111

In specific embodiments, polypeptides of the invention comprise the following amino acid sequence: TMLFYLSSQPDWQLDFFRVSFNG PVFFIIIFNDRAGFRM QALVSQAACRRSRYKLSVVY (SEQ ID NO:453), and/or DRAGFRMQALVS QAACRRSRYKL (SEQ ID NO:454). Polynucleotides encoding these polypeptides are also encompassed by the invention. The gene encoding the disclosed cDNA is believed to reside on chromosome 1. Accordingly, polynucleotides related to this invention are useful as a marker in linkage analysis for chromosome 1.

This gene is expressed primarily in human cerebellum, and to a lesser extent, in colon carcinoma cells, activated T-cells, fetal spleen, and placenta.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, or neural disorders, particularly neurodegenerative disorders. Similarly, polypeptides and antibodies directed to these polypeptides are useful in providing immunological probes for differential identification of the tissue(s) or cell type(s). For a number of disorders of the above tissues or cells, particularly of the immune or central nervous systems, expression of this gene at significantly higher or lower levels may be routinely detected in certain tissues or cell types (e.g. neural, immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in human cerebellum indicates that polynucleotides and polypeptides corresponding to this gene are useful for the diagnosis and treatment of diseases in the central nervous system and immune disorders. Moreover, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 121 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1584 of SEQ ID NO:121, b is an integer of 15 to 1598, where both a and b correspond to the positions of nucleotide residues shown in SEQ ID NO:121, and where b is greater than or equal to a + 14.

### FEATURES OF PROTEIN ENCODED BY GENE NO: 112

The gene encoding the disclosed cDNA is thought to reside on chromosome 8. Accordingly, polynucleotides related to this invention are useful as a marker in linkage analysis for chromosome 8.

This gene is expressed primarily in testes, fetal brain, and fetal liver.

Therefore, polynucleotides and polypeptides of the invention are useful as reagents for differential identification of the tissue(s) or cell type(s) present in a biological sample and for diagnosis of diseases and conditions which include, but are not limited to, brain and liver diseases, reproductive disorders. Similarly, polypeptides and antibodies directed to these polypeptides are useful in providing immunological probes for differential identification of the tissue(s) or cell type(s). For a number of disorders of the above tissues or cells, particularly of the liver and brain expression of this gene at significantly higher or lower levels may be routinely detected in certain tissues or cell types (e.g. immune, reproductive, hepatic, and cancerous and wounded tissues) or bodily fluids (e.g. lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain and liver tissues indicates that polynucleotides and polypeptides corresponding to this gene are useful for the treatment of neural, hepatic, or metabolic diseases. Furthermore, the tissue distribution indicates that polynucleotides and polypeptides corresponding to this gene are useful for the diagnosis and/or treatment of disorders of the brain and nervous system. Such involvement may impact many processes, such as learning and cognition. It may also be useful in the treatment of such neurodegenerative disorders as schizophrenia; ALS; or Alzheimer's. The tissue distribution further indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). Additionally, the tissue distribution indicates that the protein product of this gene is useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents as such agents (antagonists) are useful as male contraceptive agents. Similarly, the protein is believed to by useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO: 122 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1006 of SEQ ID NO:122, b is an integer of 15 to 1020, where both a and b correspond to the positions of nucleotide residues shown in SEQ ID NO:122, and where b is greater than or equal to a + 14.

### FEATURES OF PROTEIN ENCODED BY GENE NO: 113

This gene is expressed primarily in apoptotic T-cells, and to a lesser extent, in the frontal cortex of the brain.

Therefore, polynucleotides and polypeptides of the invention are useful as reagents for differential identification of the tissue(s) or cell type(s) present in a biological sample and for diagnosis of diseases and conditions which include, but are not limited to, immune or neural disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. Immune, hematopoietic, neural, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:248 as residues: Arg-19 to Gly-36, Val-44 to Leu-59.

The tissue distribution in apoptotic T-cells indicates that polynucleotides and polypeptides corresponding to this gene are useful for treatment and diagnosis of immune disorders. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Expression of this gene product in T cells also strongly indicates a role for this protein in immune function and immune surveillance. Alternatively, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 123 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1364 of SEQ ID NO:123, b is an integer of 15 to 1378, where both a and b correspond to the positions of nucleotide residues shown in SEQ ID NO:123, and where b is greater than or equal to a + 14.

### FEATURES OF PROTEIN ENCODED BY GENE NO: 114

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematbpoietic disorders, particularly inflammatory conditions or immunodeficiencies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g. immune, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for the diagnosis and treatment of a malfunctioning immune system response to foreign antigens. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Expression of this gene product in neutrophils also strongly indicates a role for this protein in immune function and immune surveillance.

Many polynucleotide sequences, such as EST sequences, are publicly available and accessible through sequence databases. Some of these sequences are related to SEQ ID NO:124 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1132 of SEQ ID NO: 124, b is an integer of 15 to 1146, where both a and b correspond to the positions of nucleotide residues shown in SEQ ID NO: 124, and where b is greater than or equal to a + 14.

### FEATURES OF PROTEIN ENCODED BY GENE NO: 115

In specific embodiments, polypeptides of the invention comprise the following amino acid sequence: LAAGILNSSLPALYHSVEEISQ (SEQ ID NO:455), XYRMNT KFLESYKMSTTLSRRHQNVSLCKDMKTPAGTDTKIAFLE (SEQ ID NO:456), SYKMSTTLSRRHQNVSLCKDM (SEQ ID NO:457), ICIESLMLHYIALVFEMAF MFPLVYHEMGSDSIRFHLCQVDSCLPSMMRFFFSFPFL (SEQ ID NO:458), YI ALVFEMAFMFPLVYHEMGS (SEQ ID NO:459), and/or SDSIRFHLCQ VDSCL PSMMRF (SEQ ID NO:460). Polynucleotides encoding these polypeptides are also encompassed by the invention.

This gene is expressed primarily in melanocytes, merkel cells, synovial cells, ulcerative colitis, and to a lesser extent, in fetal spleen, bone marrow, jurkat cells, adrenal gland tumor rejected kidney from a failed transplantation.

Therefore, polynucleotides and polypeptides of the invention are useful as reagents for differential identification of the tissue(s) or cell type(s) present in a biological sample and for diagnosis of diseases and conditions which include, but are not limited to, integumentary, skeletal, or gastrointestinal disorders, particularly tumors, including melanoma, lymphoma, and adrenal gland tumors. Similarly, polypeptides and antibodies directed to these polypeptides are useful in providing immunological probes for differential identification of the tissue(s) or cell type(s). For a number of disorders of the above tissues or cells, particularly of the integumentary system, expression of this gene at significantly higher or lower levels may be routinely detected in certain tissues and cell types (e.g. Integumentary, skeletal, gastrointestinal, immune, hematopoietic. renal, endocrine, and cancerous and wounded tissues) or bodily fluids (e.g.lymph, serum, amniotic fluid, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in melanocytes indicates that polynucleotides and polypeptides corresponding to this gene are useful for detecting and treating tumors particularly those involving melanocytes, lymphocytes and the adrenal gland. The secreted protein can also be used to determine biological activity, to raise antibodies, as tissue markers, to isolate cognate ligands or receptors, to identify agents that modulate their interactions and as nutritional supplements. It may also have a very wide range of biological acitivities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anaemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating haemophilia, cardiac infarction etc.); antiinflammatory activity (e.g. for treating septic shock, Crohn's disease); as antimicrobials; for treating psoriasis or other hyperproliferative diseases; for regulation of metabolism, and behaviour. Also contemplated is the use of the corresponding nucleic acid in gene therapy procedures. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 125 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1661 of SEQ ID NO:125, b is an integer of 15 to 1675, where both a and b correspond to the positions of nucleotide residues shown in SEQ ID NO: 125, and where b is greater than or equal to a + 14.

### FEATURES OF PROTEIN ENCODED BY GENE NO: 116

When tested against fibroblast cell lines, supernatants removed from cells containing this gene activated the EGR 1 (early growth response gene 1) promoter element. Thus, it is likely that this gene activates fibroblast cells through the EGR1 signal transduction pathway. EGR1 is a separate signal transduction pathway from Jak-STAT, genes containing the EGR1 promoter are induced in various tissues and cell types upon activation, leading the cells to undergo differentiation and proliferation. In specific embodiments, polypeptides of the invention comprise the following amino acid sequence:
GGVSVQDGSLREETDVGEGGRPRGGQSEGARVTRRPSPPDSNASAFDLDLDFS PFCIWCYRLETPAEVVF SPAPLRLSGPGLAPVVFVSTLPSLQPSSFCGWD LPARPRGLSGFR (SEQ ID NO:461), FTNKSCSKMSSTHLYKGSDVLCYARS SESMSLSCGDVANAGR LTPRLHLARSASQGPPTLPRVPPRGSRPPTA GESPA PRTXSLENHKNIDHLSSNSHGKFRIYGQNDIKI (SEQ ID NO:462), QDVIYTFVQ RFRRPMLCTILRKYEPVVRGRRKRWQA HPSSAFGKKRLPRPPHPAQGAPQRE QASHSWREPGPQNTFPRKP (SEQ ID NO:463), REETDVGEGGRPRGGQSEGA RV (SEQ ID NO:464), GPGLAPWFVSTLPSLQPSSFCGWDLP (SEQ ID NO:465), MSSTHLYKGSDVLCYARSSESMSL (SEQ ID NO:466), SQGPPTLPRVPPRG SRPPTAGESPAPRT (SEQ ID NO:467), RFRRPMLCTILRKYEPVVRGRRKRW (SEQ ID NO:468), and/or RLPRPPHPAQGAPQREQASHSWRE (SEQ ID NO:469). Polynucleotides encoding these polypeptides are also encompassed by the invention.

This gene is expressed primarily in hematopoietic cells, endothelial cells, and in spleen.

Therefore, polynucleotides and polypeptides of the invention are useful as reagents for differential identification of the tissue(s) or cell type(s) present in a biological sample and for diagnosis of diseases and conditions which include, but are not limited to, hematopoietic, integumentary, and immune disorders, particularly multiple myeloma, immunodeficiencies, leukemias, and vascular conditions. Similarly, polypeptides and antibodies directed to these polypeptides are useful in providing immunological probes for differential identification of the tissue(s) or cell type(s). For a number of disorders of the above tissues or cells, particularly of the hematopoietic, immune, and vascular systems, expression of this gene at significantly higher or lower levels may be routinely detected in certain tissues and cell types (e.g. Immune, hematopoietic, integumentary, endothelial, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in spleen and hematopoietic cells, combined with the detected EGR1 biological activity indicates that polynucleotides and polypeptides corresponding to this gene are useful for the treatment and/or detection of vascular, immune and/or hematopoietic disorders including arthritis, ischemia, auto-immune diseases, host-graft rejection, AIDS, leukemia and microbial infection. Moreover, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, a utility for treating or preventing vascular or integumentary disorders may be anticipated for this gene based upon its expression within endothelial tissues in addition to its EGR1 activity. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 126 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1050 of SEQ ID NO: 126, b is an integer of 15 to 1064, where both a and b correspond to the positions of nucleotide residues shown in SEQ ID NO:126, and where b is greater than or equal to a + 14.

### FEATURES OF PROTEIN ENCODED BY GENE NO: 117

In specific embodiments, polypeptides of the invention comprise the following amino acid sequence:
RGMRGRWLVSSGAAFPIPLNGFCESREFFPDSGSVLLHWRPNXVLIEIKVFGS RSQSLISSK NLKTSLTFIYGKVEEVLNN (SEQ ID NO:470), LKLSSADSQA IMNIFSADCMPRLHIALQTEMIPNRAPQGGAAANLWHEAQYRRLPFSR APEX TDAHQASAQRGAAQLPREQ (SEQ ID NO:471, PIPLNGFCESREFFPDSGS VLLHWRPNX (SEQ ID NO:472), and/or NIFSADCMPRLHIALQTEMIP NRA PQGGA (SEQ ID NO:473). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, diseases of the immune system, including neutropenia, cancer, inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, hematopoietic, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy or may be useful in the treatment of immune dysfunction or anti-inflamatory by inhibiting infiltration of neutrophils to the site of injury or distress. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 127 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1593 of SEQ ID NO:127, b is an integer of 15 to 1607, where both a and b correspond to the positions of nucleotide residues shown in SEQ ID NO:127, and where b is greater than or equal to a + 14.

### FEATURES OF PROTEIN ENCODED BY GENE NO: 118

Contact of cells with supernatant expressing the product of this gene has been shown to increase the permeability of the plasma membrane of renal mesangial cells to calcium. Thus it is likely that the product of this gene is involved in a signal transduction pathway that is initiated when the product binds a receptor on the surface of the plasma membrane of both mesangial cells and other cell types, in addition to other cell-lines or tissue cell types. Thus, polynucleotides and polypeptides have uses which include, but are not limited to, activating mesangial cells. Binding of a ligand to a receptor is known to alter intracellular levels of small molecules, such as calcium, potassium and sodium, as well as alter pH and membrane potential. Alterations in small molecule concentration can be measured to identify supernatants which bind to receptors of a particular cell. In addition, when tested against fibroblast cell lines, supernatants removed from cells containing this gene activated the EGR1 (early growth response gene 1) promoter element. Thus, it is likely that this gene activates fibroblast cells through the EGR1 signal transduction pathway. EGR1 is a separate signal transduction pathway from Jak-STAT, genes containing the EGR1 promoter are induced in various tissues and cell types upon activation, leading the cells to undergo differentiation and proliferation. In specific embodiments, polypeptides of the invention comprise the following amino acid sequence:
TFRLVSAHLKTRKLINPEAAERRWRDWDSRQGWLSVK (SEQ ID NO:474), and/or KTRKLINPEAAERRWRDWDSR (SEQ ID NO:475). Polynucleotides encoding these polypeptides are also encompassed by the invention.

This gene is expressed primarily in bone marrow cell lines, and to a lesser extent, in human endometrial stromal cells, human adult small intestine and human pancreas tumor.

Therefore, polynucleotides and polypeptides of the invention are useful as reagents for differential identification of the tissue(s) or cell type(s) present in a biological sample and for diagnosis of diseases and conditions which include, but are not limited to, haemopoietic and gastrointestinal tract disorders and stromatosis, in addition to endothelial, mucosal, or epithelial cell diorders. Similarly, polypeptides and antibodies directed to these polypeptides are useful in providing immunological probes for differential identification of the tissue(s) or cell type(s). For a number of disorders of the above tissues or cells, particularly of the immune and digestive systems, expression of this gene at significantly higher or lower levels may be routinely detected in certain tissues and cell types (e.g.hemaopoietic, immune, reproductive, gastrointestinal, endocrine, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:253 as residues: Gly-25 to Arg-31, Ile-47 to Glu-57, Glu-120 to Arg-138.

The tissue distribution in bone marrow cells, combined with the detected calcium flux and EGR biological activity indicates that polynucleotides and polypeptides corresponding to this gene are useful for immune and gastrointestinal tract disorders, and stromatosis, particularly tumors and proliferative disorders. More specifically, polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 128 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1023 of SEQ ID NO:128, b is an integer of 15 to 1037, where both a and b correspond to the positions of nucleotide residues shown in SEQ ID NO:128, and where b is greater than or equal to a + 14.

### FEATURES OF PROTEIN ENCODED BY GENE NO: 119

In specific embodiments, polypeptides of the invention comprise the following amino acid sequence: WNYTVNNLYLFSFSIVSMKFMHVLSINIF FGRARWLT PVIPALLEAEAGGSLGQEFKTSLGKDGETPSLLKIQKLAGHGGRRL (SEQ ID NO:476, DQPGKHGETLSLLKMQKLTWCGGMPFVIP SYSRSPRPENRLNL GDRGCTELLHSSLGNRVRLSKKKEVYMMELYSK (SEQ ID NO:477), VIPALLE AEAGGSLGQEFKTSLGKDGET (SEQ ID NO:478), and/or NRLNLGDRGCT ELLHSSLGNRVRLSKKKE (SEQ ID NO:479). Polynucleotides encoding these polypeptides are also encompassed by the invention.

This gene is expressed primarily in human fetal brain.

Therefore, polynucleotides and polypeptides of the invention are useful as reagents for differential identification of the tissue(s) or cell type(s) present in a biological sample and for diagnosis of diseases and conditions which include, but are not limited to, neurological, developmental, and immunological disorders. Similarly, polypeptides and antibodies directed to these polypeptides are useful in providing immunological probes for differential identification of the tissue(s) or cell type(s). For a number of disorders of the above tissues or cells, particularly of the central nervous and immune systems, expression of this gene at significantly higher or lower levels may be routinely detected in certain tissues and cell types (e.g.neural, developmental, immune, and cancerous and wounded tissues) or bodily fluids (e.g.lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal brain indicates that polynucleotides and polypeptides corresponding to this gene are useful for diagnosis and treatment of disorders relating to CNS and immune system. In addition, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and preception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Furthermore, expression within fetal tissue indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 129 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1132 of SEQ ID NO:129, b is an integer of 15 to 1146, where both a and b correspond to the positions of nucleotide residues shown in SEQ ID NO: 129, and where b is greater than or equal to a + 14.

### FEATURES OF PROTEIN ENCODED BY GENE NO: 120

In specific embodiments, polypeptides of the invention comprise the following amino acid sequence: HASEHLAALPVNVKIGK (SEQ ID NO:480). Polynucleotides encoding these polypeptides are also encompassed by the invention. The gene encoding the disclosed cDNA is believed to reside on chromosome 5. Accordingly, polynucleotides related to this invention are useful as a marker in linkage analysis for chromosome 5.

This gene is expressed primarily in T cells/helper I.

Therefore, polynucleotides and polypeptides of the invention are useful as reagents for differential identification of the tissue(s) or cell type(s) present in a biological sample and for diagnosis of diseases and conditions which include, but are not limited to, immune, or haemopoitic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues and cell types (e.g. Immune, haemopoitic disorders, and cancerous and wounded tissues) or bodily fluids (e.g.lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:255 as residues: Ile-31 to Glu-36, Leu-59 to Glu-73, Ser-109 to Ser-121, Ser-175 to Gln-182, Lys-258 to Lys-264.

The tissue distribution in T-cells indicates that polynucleotides and polypeptides corresponding to this gene are useful for diagnosis and treatment of immune disorders. Moreover, expression of this gene product indicates a role in regulating the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 130 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1158 of SEQ ID NO: 130, b is an integer of 15 to 1172, where both a and b correspond to the positions of nucleotide residues shown in SEQ ID NO: 130, and where b is greater than or equal to a + 14.

### FEATURES OF PROTEIN ENCODED BY GENE NO: 121

In specific embodiments, polypeptides of the invention comprise the following amino acid sequence:
LVCILLVHWIPPI-GAWGLSLMLFLILEQRCGKGKWRNALLSVSFSVPQLQMQK VS LDSTPLNVNHDKMDIWKLTPKL (SEQ ID NO:481), IMIKWIFGNLLL SCD LGCISTSGLPQYQGLRLLNFEYSLGFMLRSLWSRSAIQCFFS (SEQ ID NO:482), LLLSCDLGCISTSGLPQYQGL (SEQ ID NO:483), and/or LRLLNFEYSLGFM LRSLWSRS (SEQ ID NO:484). Polynucleotides encoding these polypeptides are also encompassed by the invention.The gene encoding the disclosed cDNA is believed to reside on chromosome 11. Accordingly, polynucleotides related to this invention are useful as a marker in linkage analysis for chromosome 11.

This gene is expressed primarily in human gall bladder.

Therefore, polynucleotides and polypeptides of the invention are useful as reagents for differential identification of the tissue(s) or cell type(s) present in a biological sample and for diagnosis of diseases and conditions which include, but are not limited to, metabolic, or gastrointestinal disorders, particularly those relating to the gall bladder. Similarly, polypeptides and antibodies directed to these polypeptides are useful in providing immunological probes for differential identification of the tissue(s) or cell type(s). For a number of disorders of the above tissues or cells, particularly of the gastrointestinal tract system, expression of this gene at significantly higher or lower levels may be routinely detected in certain tissues and cell types (e.g. Metabolic, gastrointestinal, and cancerous and wounded tissues) or bodily fluids (e.g.lymph, serum, bile, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:256 as residues: Ser-18 to Gly-26.

The tissue distribution in gall bladder tissue indicates that polynucleotides and polypeptides corresponding to this gene are useful for the diagnosis and treatment of gall bladder disorders, or related metabolic conditions, such as gall stones. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:131 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 649 of SEQ ID NO:131, b is an integer of 15 to 663, where both a and b correspond to the positions of nucleotide residues shown in SEQ ID NO:131, and where b is greater than or equal to a + 14.

### FEATURES OF PROTEIN ENCODED BY GENE NO: 122

In specific embodiments, polypeptides of the invention comprise the following amino acid sequence: ASPHL FIEKWGRAFTLRKLLLVPVISKRIINIMAHQVKPPI FCAMIMCNLFCSGYEHLLFTLMRFFSFEQIFDEV VFH (SEQ ID NO:485), and/or KLLLVPVISKRIINIMAH QVK PPIF (SEQ ID NO:486). Polynucleotides encoding these polypeptides are also encompassed by the invention. The gene encoding the disclosed cDNA is believed to reside on chromosome 4. Accordingly, polynucleotides related to this invention are useful as a marker in linkage analysis for chromosome 4.

This gene is expressed primarily in glioblastoma. Therefore, polynucleotides and polypeptides of the invention are useful as reagents for differential identification of the tissue(s) or cell type(s) present in a biological sample and for diagnosis of diseases and conditions which include, but are not limited to, neural disorders, particularly glioblastoma multiform. Similarly, polypeptides and antibodies directed to these polypeptides are useful in providing immunological probes for differential identification of the tissue(s) or cell type(s). For a number of disorders of the above tissues or cells, particularly of the central nervous system (CNS), expression of this gene at significantly higher or lower levels may be routinely detected in certain tissues and cell types (e.g. neural, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:257 as residues: Ser-40 to Gly-45, Leu-73 to Arg-80.

The tissue distribution in glioblastoma indicates that polynucleotides and polypeptides corresponding to this gene are useful for the diagnosis and treatment of neural cell disorders. Moreover, polynucleotides and polypeptides corresponding to this gene are useful for the detection/treatment of neurodegenerative disease states, behavioural disorders, or inflamatory conditions such as Alzheimers Disease, Parkinsons Disease, Huntingtons Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Moreover, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 132 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 762 of SEQ ID NO: 132, b is an integer of 15 to 776, where both a and b correspond to the positions of nucleotide residues shown in SEQ ID NO: 132, and where b is greater than or equal to a + 14.

### FEATURES OF PROTEIN ENCODED BY GENE NO: 123

When tested against U937 and Jurket cell lines, supernatants removed from cells containing this gene activated the GAS (gamma activating sequence) promoter element. Thus, it is likely that this gene activates myeloid cells, including their progenitors; through the Jak-STAT signal transduction pathway. GAS is a promoter element found upstream of many genes which are involved in the Jak-STAT pathway. The Jak-STAT pathway is a large, signal transduction pathway involved in the differentiation and proliferation of cells. Therefore, activation of the Jak-STAT pathway, reflected by the binding of the GAS element, can be used to indicate proteins involved in the proliferation and differentiation of cells. In specific embodiments, polypeptides of the invention comprise the following amino acid sequence:
FAVIRFESIIHEFDPWFNYRSTHHLASHGFYEFLNWFDERAWYPLGRIVGGTVY PGLMTTAGLIHWILNT LNITVHIRDVCVFLAPTFSGLTSISTFLLTRELWN QGA GLLAACFIAIVPGYISRSVAGSFDNEGIAIFA LQFTYYLWVKSVKTGSVFW TMCCCLSYFYMVSAWGGYVFIINLIPLHVFVLLLMQRYSKRVYIAYSTFYI VGL ILSMQIPFVGFQPIRTSEHNLAAAGVFALLQAYAFLQYLRDRLTKQEFQTLFFLGV SLAAGAVFLSVI YLTYTGYIAPWSGRFYSLWDTGYAKIHIPIIASVSEHQ PTT WVSFFFDLHILVCTFPAGLWFCIKNINDE RXFGKXGF (SEQ ID NO:487), EFD PWFNYRSTHHLASHGFYEFLNWFD (SEQ ID NO:488), TRELWNQGAGLL AACFIAIVPGY (SEQ ID NO:489), TYYLWVKSVKTGSVFWTMCCCL (SEQ ID NO:490), GVFALLQAYAFLQYLRDRLTKQEFQ (SEQ ID NO:491), and/or YSLWD TGYAKIHIPIIASVSEHQPTTW (SEQ ID NO:492). Polynucleotides encoding these polypeptides are also encompassed by the invention.

This gene is expressed primarily in human colon carcinoma (HCC) cell line, and to a lesser extent, in human eosinophils.

Therefore, polynucleotides and polypeptides of the invention are useful as reagents for differential identification of the tissue(s) or cell type(s) present in a biological sample and for diagnosis of diseases and conditions which include, but are not limited to, gastrointestinal or immune disorders, particularly colon carcinoma and leukemia. Similarly, polypeptides and antibodies directed to these polypeptides are useful in providing immunological probes for differential identification of the tissue(s) or cell type(s). For a number of disorders of the above tissues or cells, particularly of the excretory and immune system, expression of this gene at significantly higher or lower levels may be routinely detected in certain tissues and cell types (e.g. gastrointestinal, immune, hematopoietic, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO:258 as residues: Glu-49 to Ser-54.

The tissue distribution in human colon carcinoma cell lines, combined with the detected GAS biological activity, indicates that polynucleotides and polypeptides corresponding to this gene are useful for the diagnosis and treatment of colon cancer and immune disorders. In addition, expression within cellular sources marked by proliferating cells indicates that this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus, this protein may also be involved in apoptosis or tissue differentiation and could again be useful in cancer therapy, particularly in modulating the immune response to cancer-specific antigens. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 133 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1529 of SEQ ID NO: 133, b is an integer of 15 to 1543, where both a and b correspond to the positions of nucleotide residues shown in SEQ ID NO:133, and where b is greater than or equal to a + 14.

### FEATURES OF PROTEIN ENCODED BY GENE NO: 124

This gene shares homology with elongation factor 1-Alpha (giardia intestinalis), which promotes the GTP-dependent binding of aminoacyl tRNA to the A-site of ribosomes during proetin biosynthesis. One embodiment of this gene comprises polypeptides of the following amino acid sequence:
MGHMLYLLGNINKRTMHKYXQESKKAGKASFAY AWVLDETGEERER GVTMDVGMTKFETTTKVIT LMDAPGHKDFIPNMITGAAQADVAVLVVDASR GEFEAGFETGGQTREHGLLVRSLGVTQLAVAVN KMDQVNWQQERF QEIT GKLGHFLKQAGFKESDV GFIPTSGLSGENLITRSQSSELTKWYKGLCLLEQ IDSFKPPQRSIDKPFRLCVSDVFKDQGSGFCITG KIEAGYIQTGDRLL AMPP NETCTVKGITLHDEPV DWAAAGDHVSLTLVGMDIIKINVGCIFCGPKVP IKACTRFRARILIFNIEIPITKGFPVLLHYQTVSE PAVIKRLISVLNKSTG EVTKKKPKFLTKGQNAL VELQTQRPIALELYKDFKELGRFMLRYGGSTIAA GVVTEIKE (SEQ ID NO:493), LYLLGNINKRTMHKYXQESKK (SEQ ID NO:494), LDETGEERERGVTMDVGMTKFET (SEQ ID NO:495), GHKDFIPNMIT GAAQADVAVLV (SEQ ID NO:496), GFETGGQTREHGLLVRSLGVTQL (SEQ ID NO:497), WQQERFQEITGKLGHFLKQAGFK (SEQ ID NO:498), TSGLSGENLI TRSQSSELTKWY (SEQ ID NO:499), PQRSIDKPFRLCVSDVFKDQGSG (SEQ ID NO:500, LISVLNKSTGEVTKKKPKFLTK (SEQ ID NO:501), and/or QRPIALELY KDFKELGRFMLRYGGS (SEQ ID NO:502). An additional embodiment is the polynucleotides encoding these polypeptides. The gene encoding the disclosed cDNA is thought to reside on chromosome 6. Accordingly, polynucleotides related to this invention are useful as a marker in linkage analysis for chromosome 6.

This gene is expressed primarily in colon tissue from a patient having ulcerative colitis, brain tissue, lung tissue, testes and endometrial tumor.

Therefore, polynucleotides and polypeptides of the invention are useful as reagents for differential identification of the tissue(s) or cell type(s) present in a biological sample and for diagnosis of diseases and conditions which include, but are not limited to, ulcerative colitis, and testes and endometrial tumors. Similarly, polypeptides and antibodies directed to these polypeptides are useful in providing immunological probes for differential identification of the tissue(s) or cell type(s). For a number of disorders of the above tissues or cells, particularly of the immune system and reproductive system, expression of this gene at significantly higher or lower levels may be routinely detected in certain tissues or cell types (e.g. reproductive, immune, cancerous and wounded tissues) or bodily fluids (e.g., serum, seminal fluid, pulmonary surfactant or sputum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in ulcerative colitis, testes and endometrial tumors indicates that polynucleotides and polypeptides corresponding to this gene are useful for diagnosing or treatment of a variety of reproductive or gastrointestinal disorders. Furthermore, the tissue distribution indicates that the protein product of this gene is useful for the treatment and diagnosis of conditions concerning proper testicular function (e.g. endocrine function, sperm maturation), as well as cancer. Therefore, this gene product is useful in the treatment of male infertility and/or impotence. This gene product is also useful in assays designed to identify binding agents as such agents (antagonists) are useful as male contraceptive agents. Similarly, the protein is believed to by useful in the treatment and/or diagnosis of testicular cancer. The testes are also a site of active gene expression of transcripts that may be expressed, particularly at low levels, in other tissues of the body. Therefore, this gene product may be expressed in other specific tissues or organs where it may play related functional roles in other processes, such as hematopoiesis, inflammation, bone formation, and kidney function, to name a few possible target indications.

Many polynucleotide sequences, such as EST sequences, are publicly available and accessible through sequence databases. Some of these sequences are related to SEQ ID NO: 134 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2143 of SEQ ID NO:134, b is an integer of 15 to 2157, where both a and b correspond to the positions of nucleotide residues shown in SEQ ID NO: 134, and where b is greater than or equal to a + 14.

### FEATURES OF PROTEIN ENCODED BY GENE NO: 125

This gene is expressed primarily in skin.

Therefore, polynucleotides and polypeptides of the invention are useful as reagents for differential identification of the tissue(s) or cell type(s) present in a biological sample and for diagnosis of diseases and conditions which include, but are not limited to, integumentary diseases. Similarly, polypeptides and antibodies directed to these polypeptides are useful in providing immunological probes for differential identification of the tissue(s) or cell type(s). For a number of disorders of the above tissues or cells, particularly of the integumentary system, expression of this gene at significantly higher or lower levels may be routinely detected in certain tissues and cell types (e.g. integumentary, melanocyte, and cancerous and wounded tissues) or bodily fluids (e.g. lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in skin indicates that polynucleotides and polypeptides corresponding to this gene are useful for the diagnosis and/or treatment of diseases relating to integumentary conditions. Specifically, polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Boweni's disease, basal cell carcinoma, squamous cell carcinoma, malignant melanoma, Pageti's disease, mycosis fungoides, and Kaposii's sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. In addition, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Moreover, the protein product of this gene may also be useful for the treatment or diagnosis of various connective tissue disorders such as arthritis, trauma, tendonitis, chrondomalacia and inflammation, autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 135 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 406 of SEQ ID NO: 135, b is an integer of 15 to 420, where both a and b correspond to the positions of nucleotide residues shown in SEQ ID NO: 135, and where b is greater than or equal to a + 14.

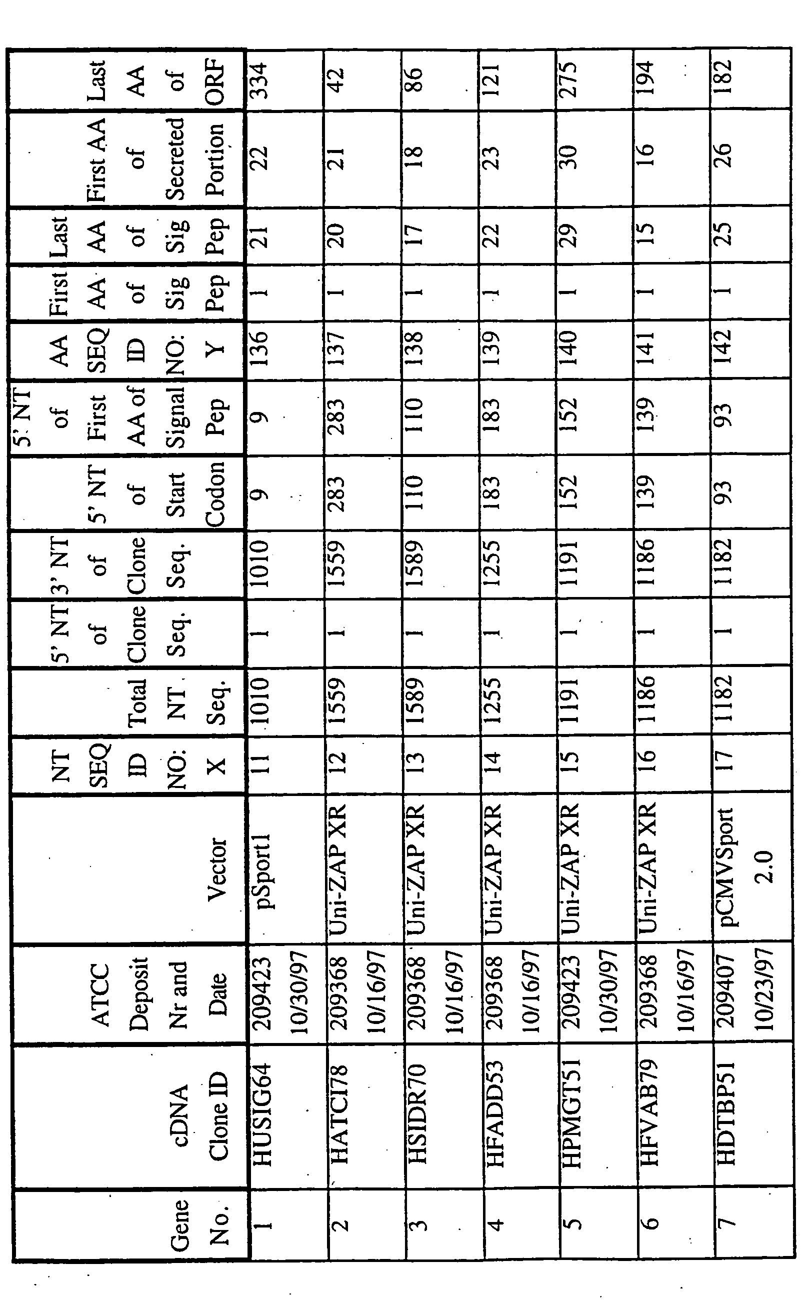

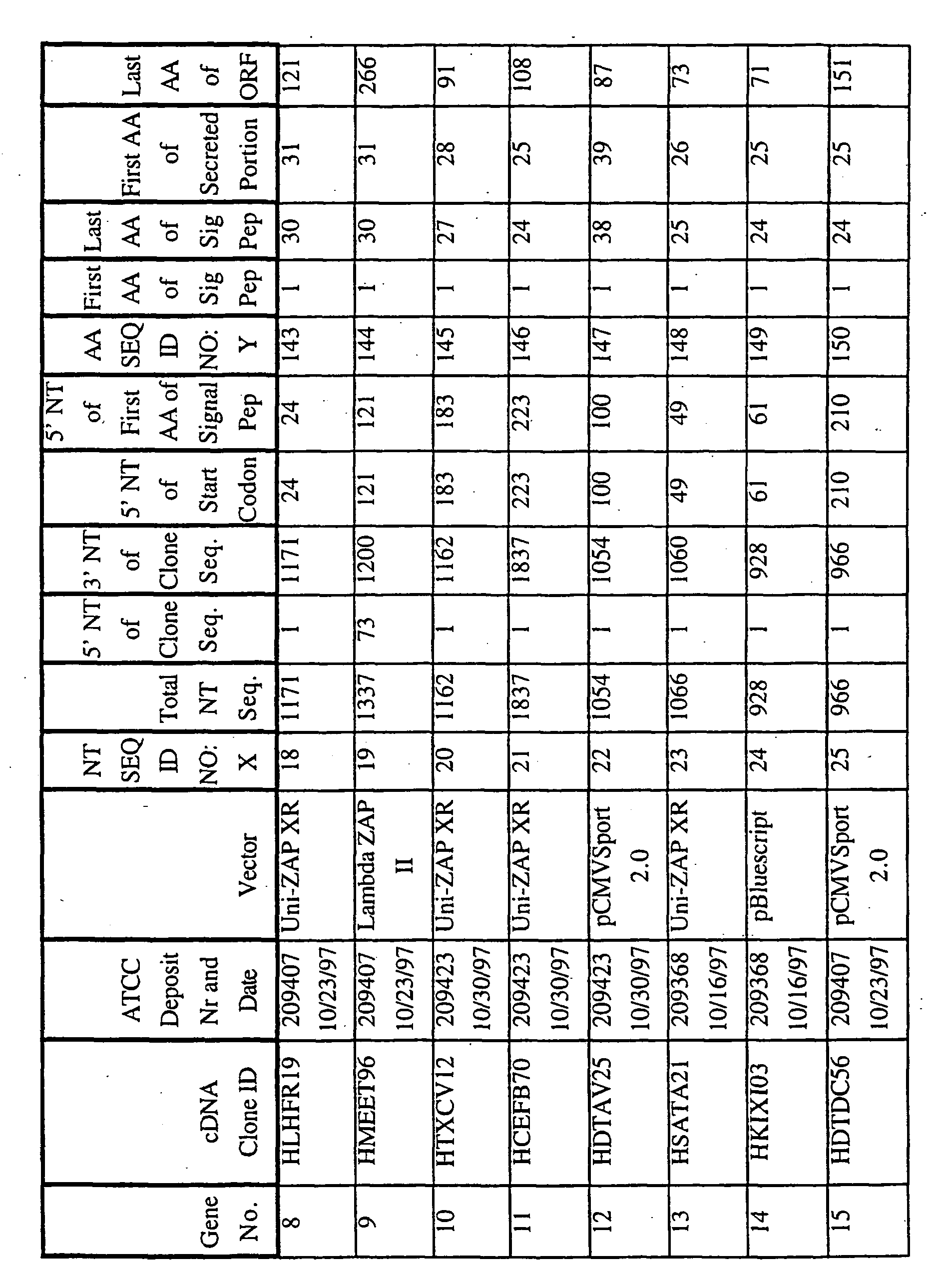

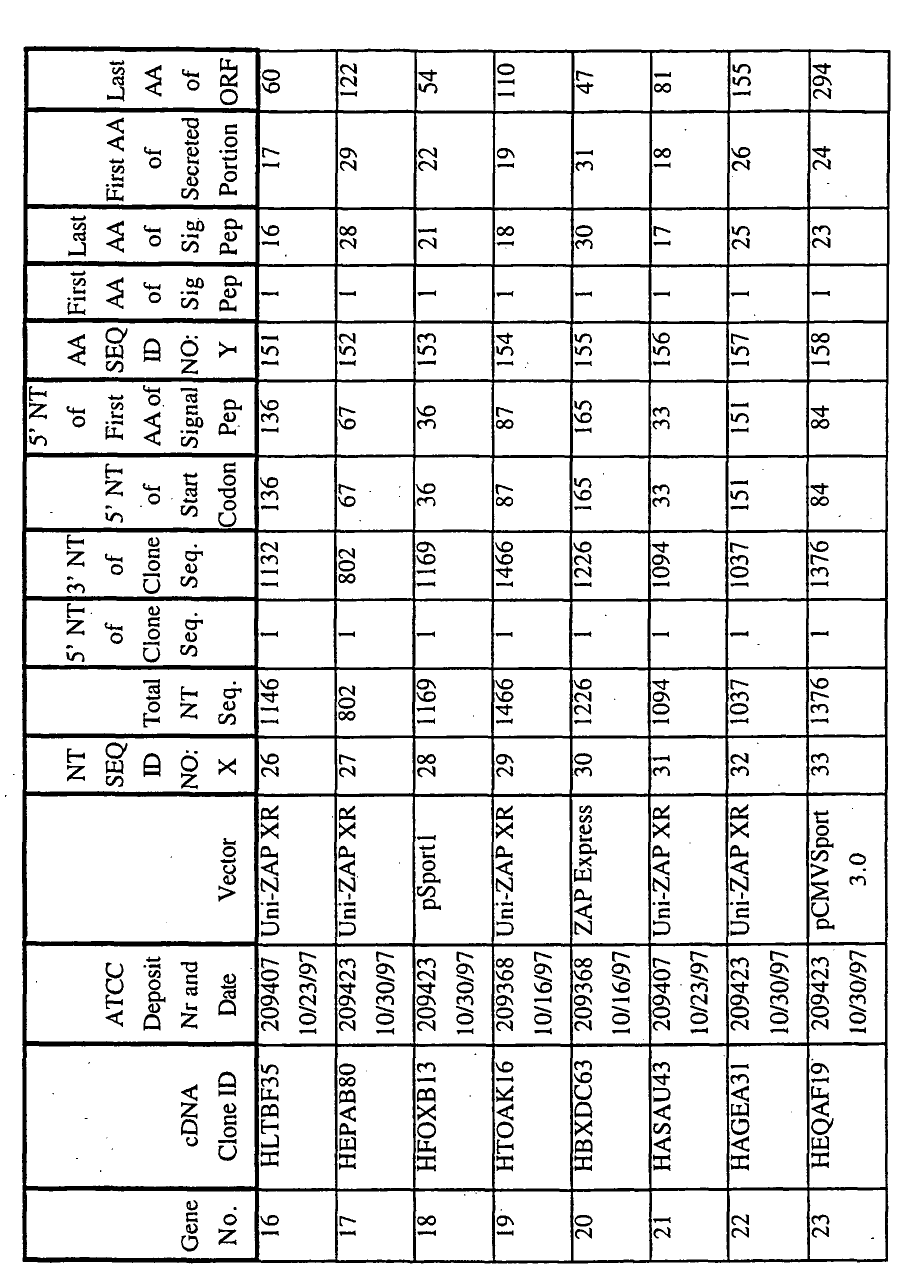

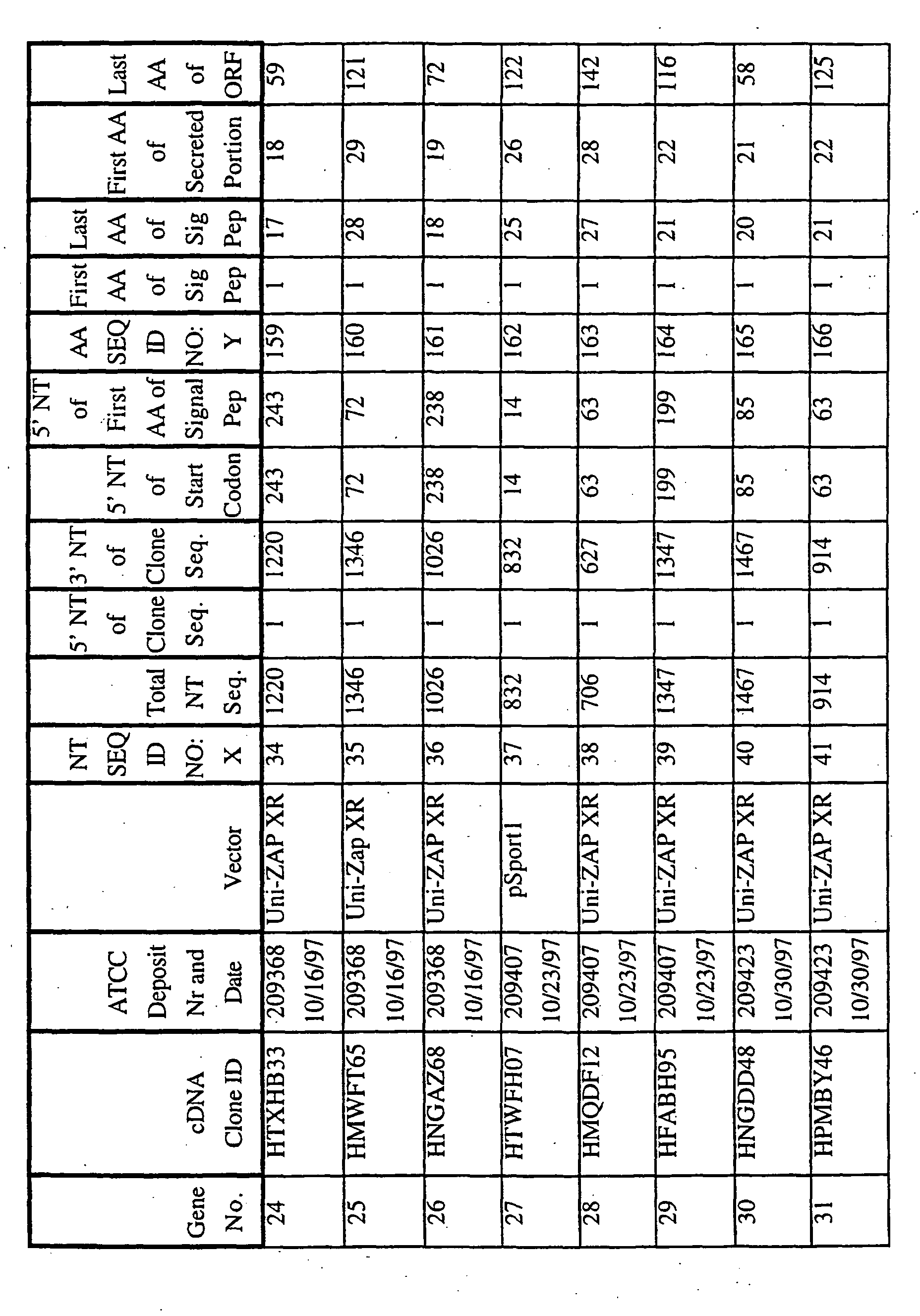

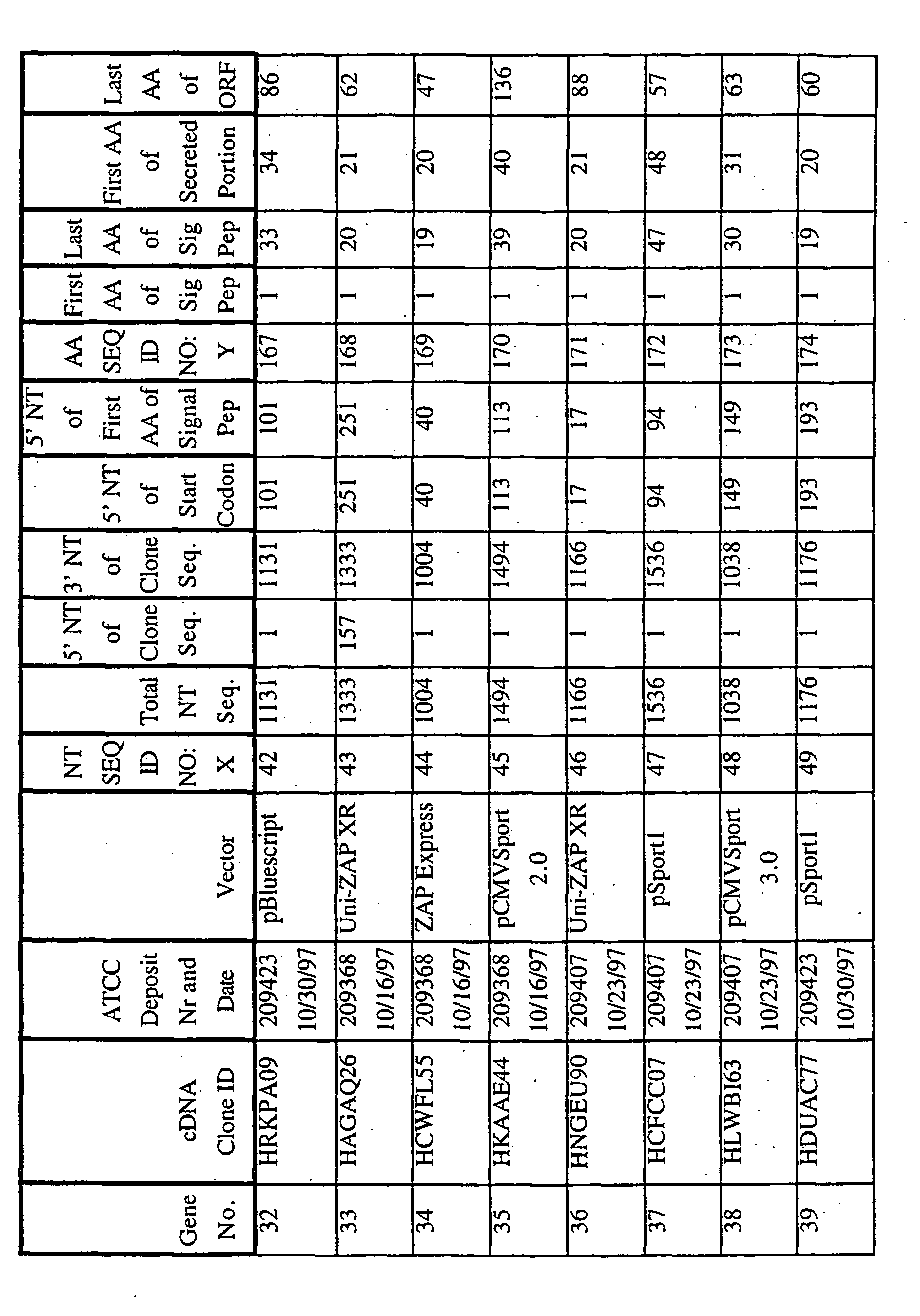

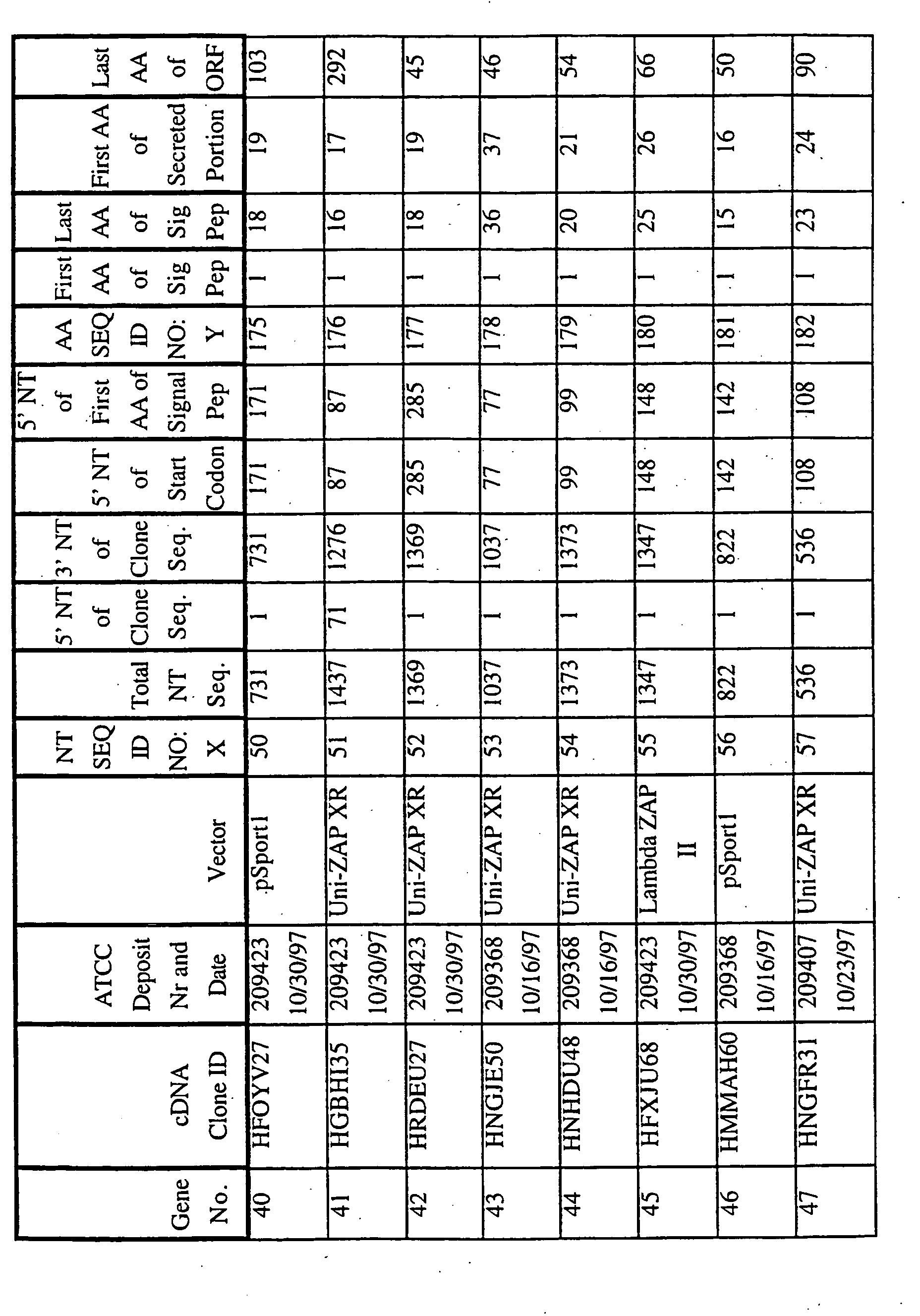

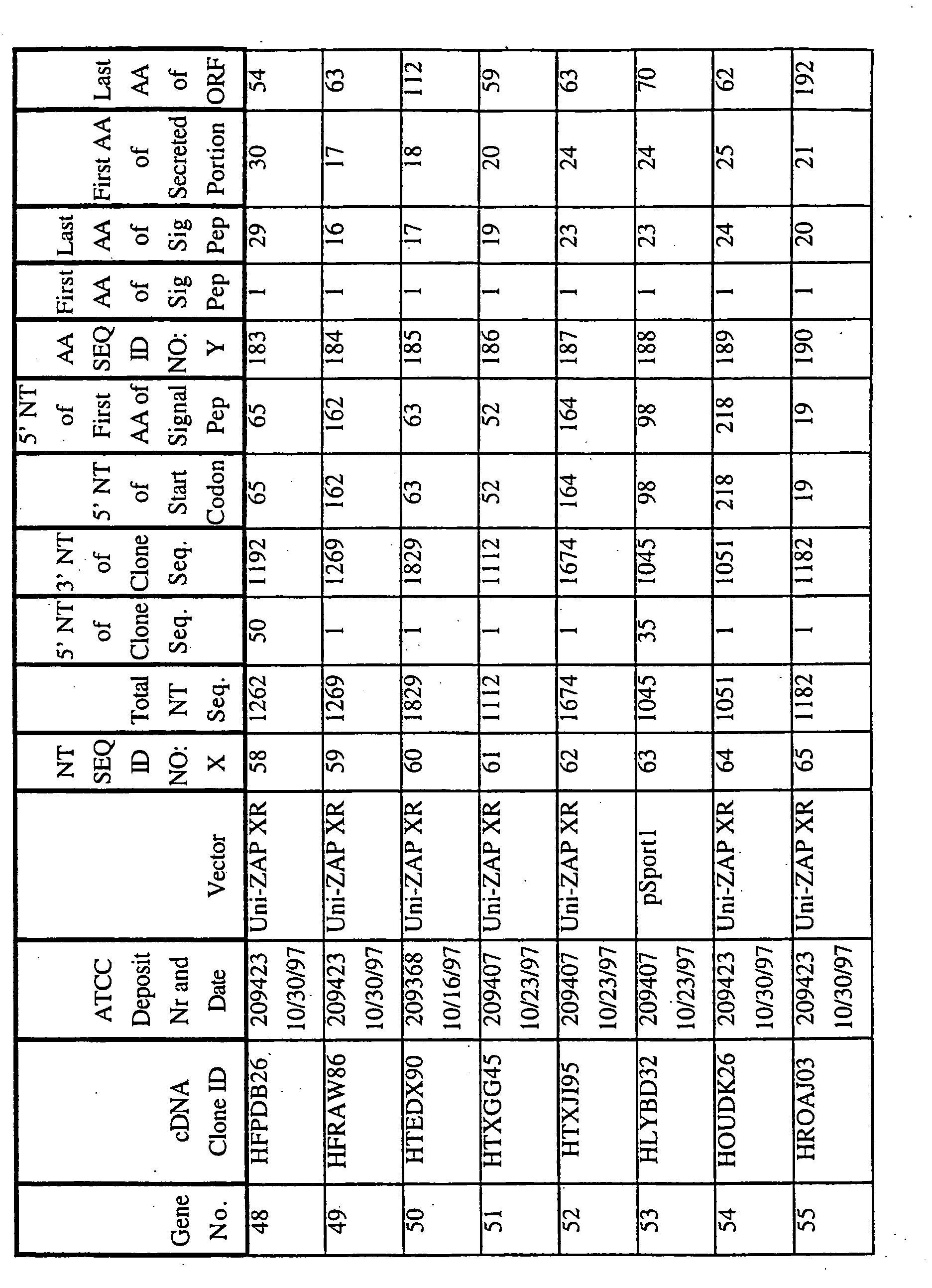

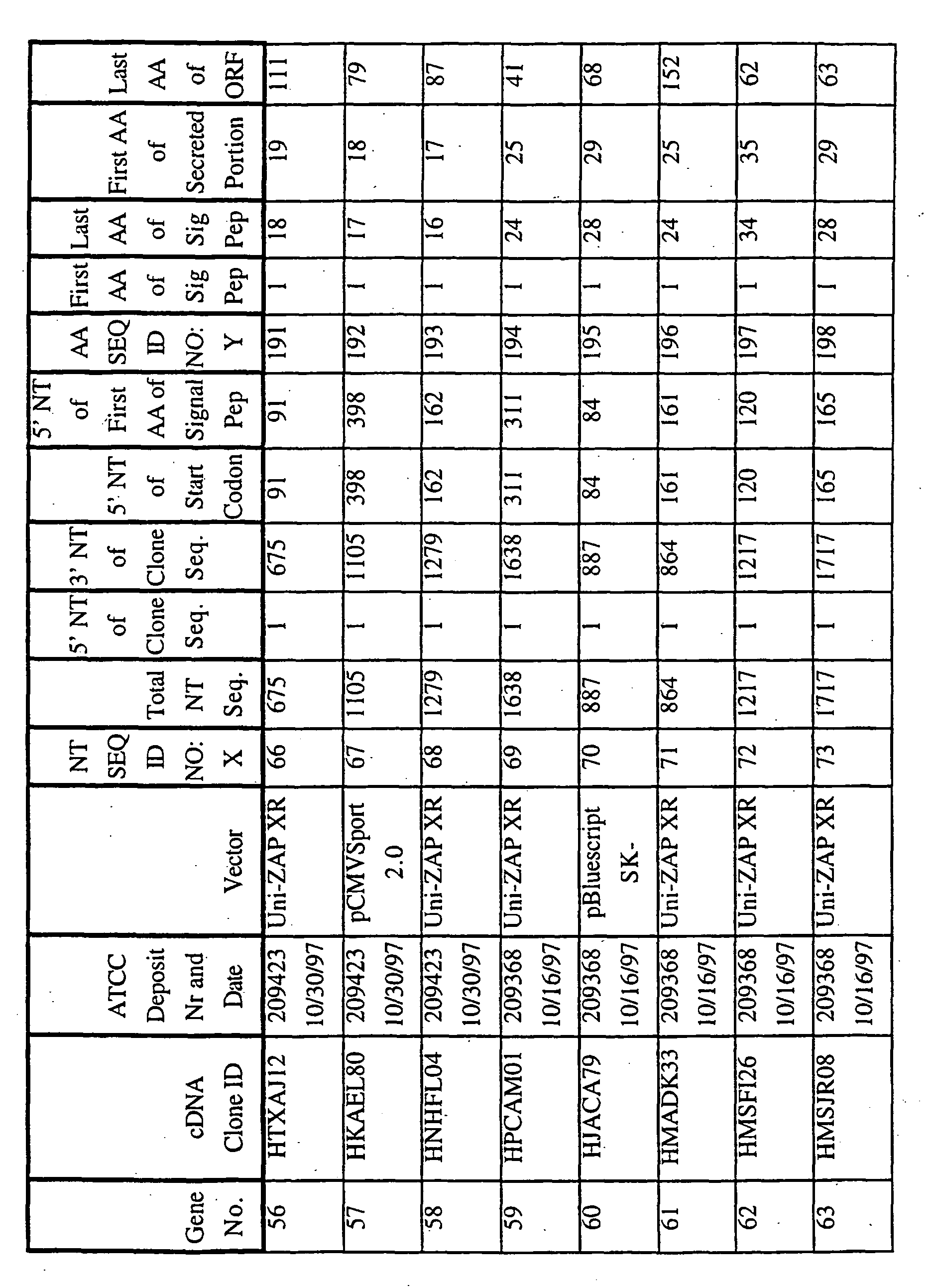

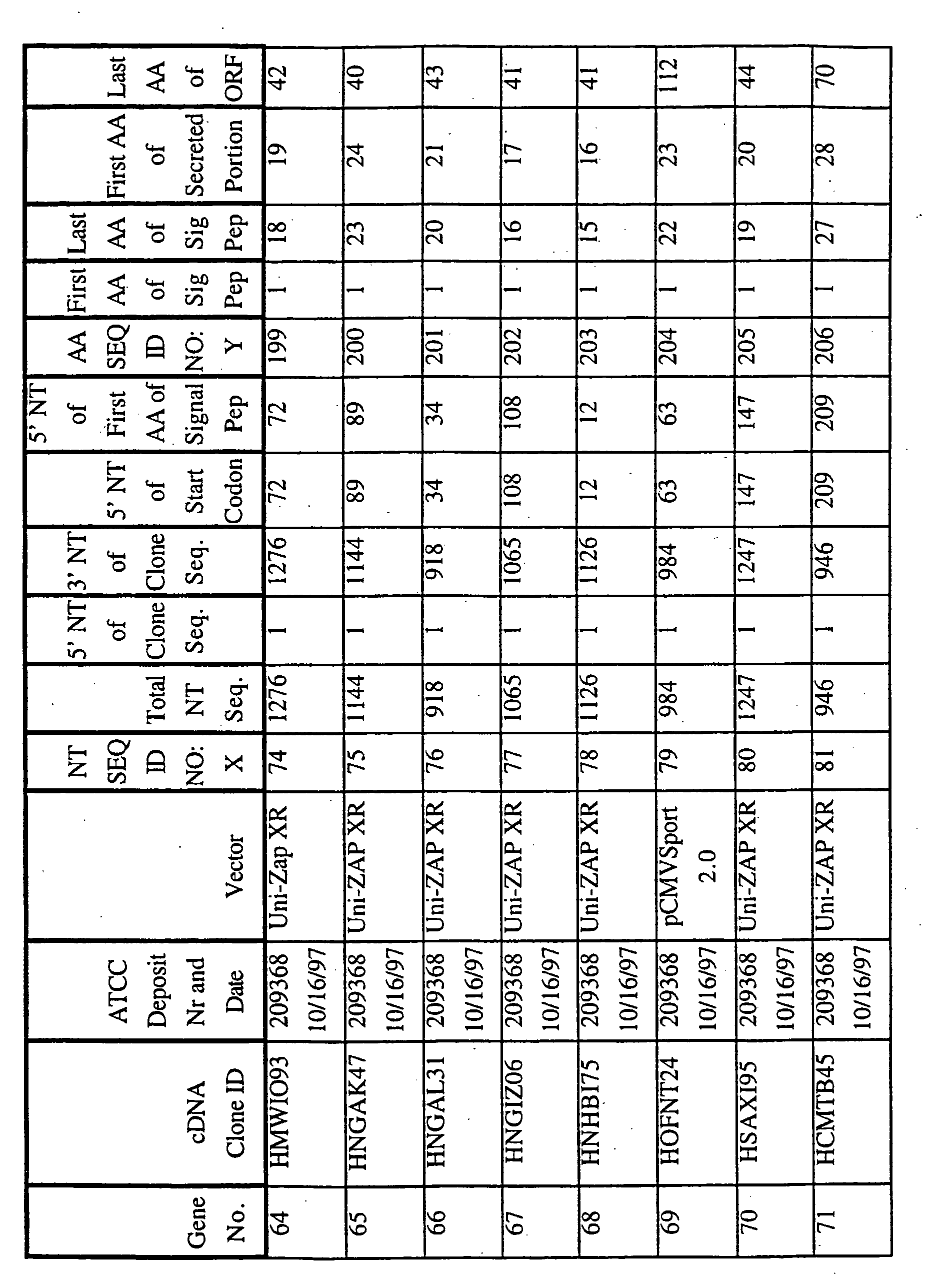

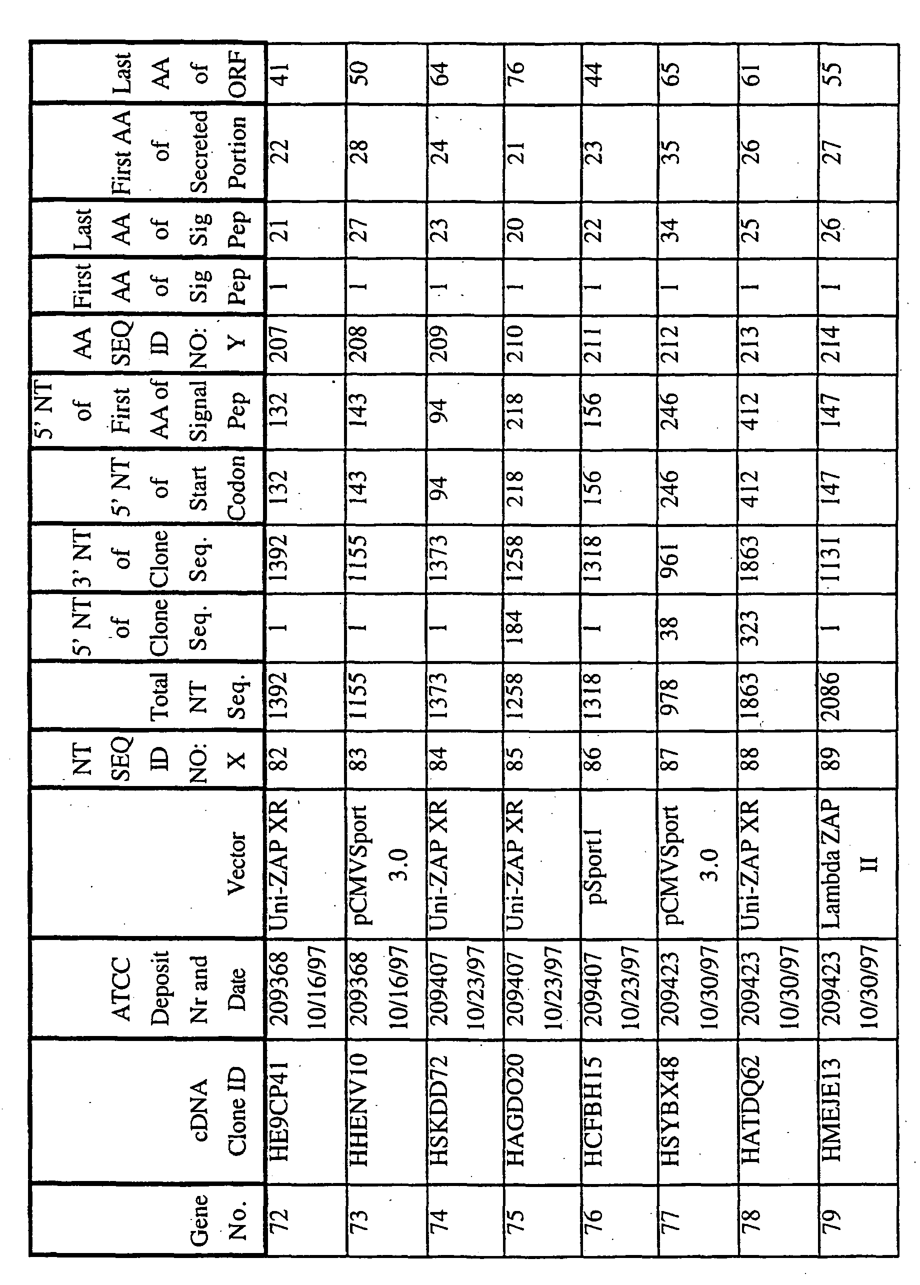

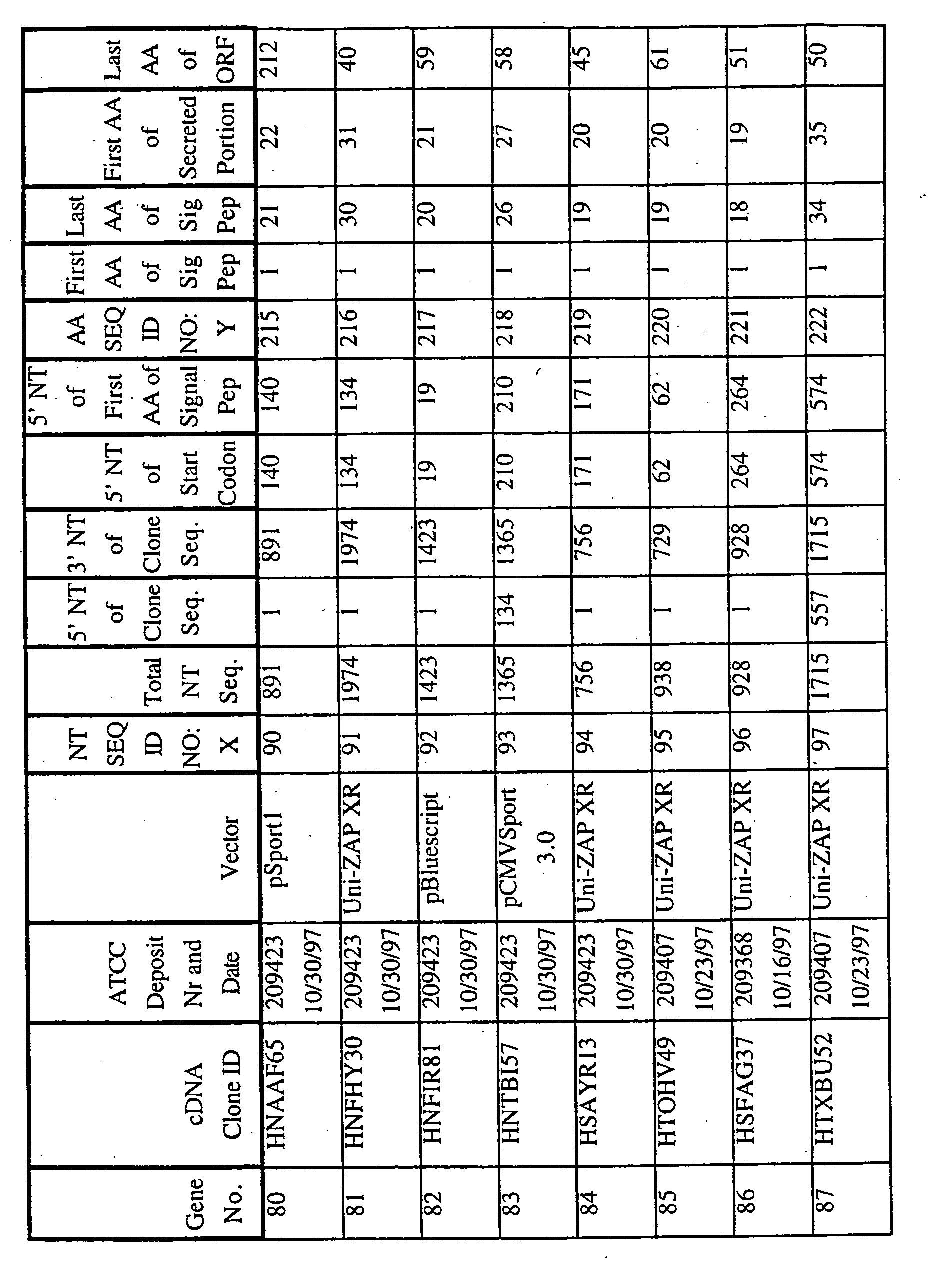

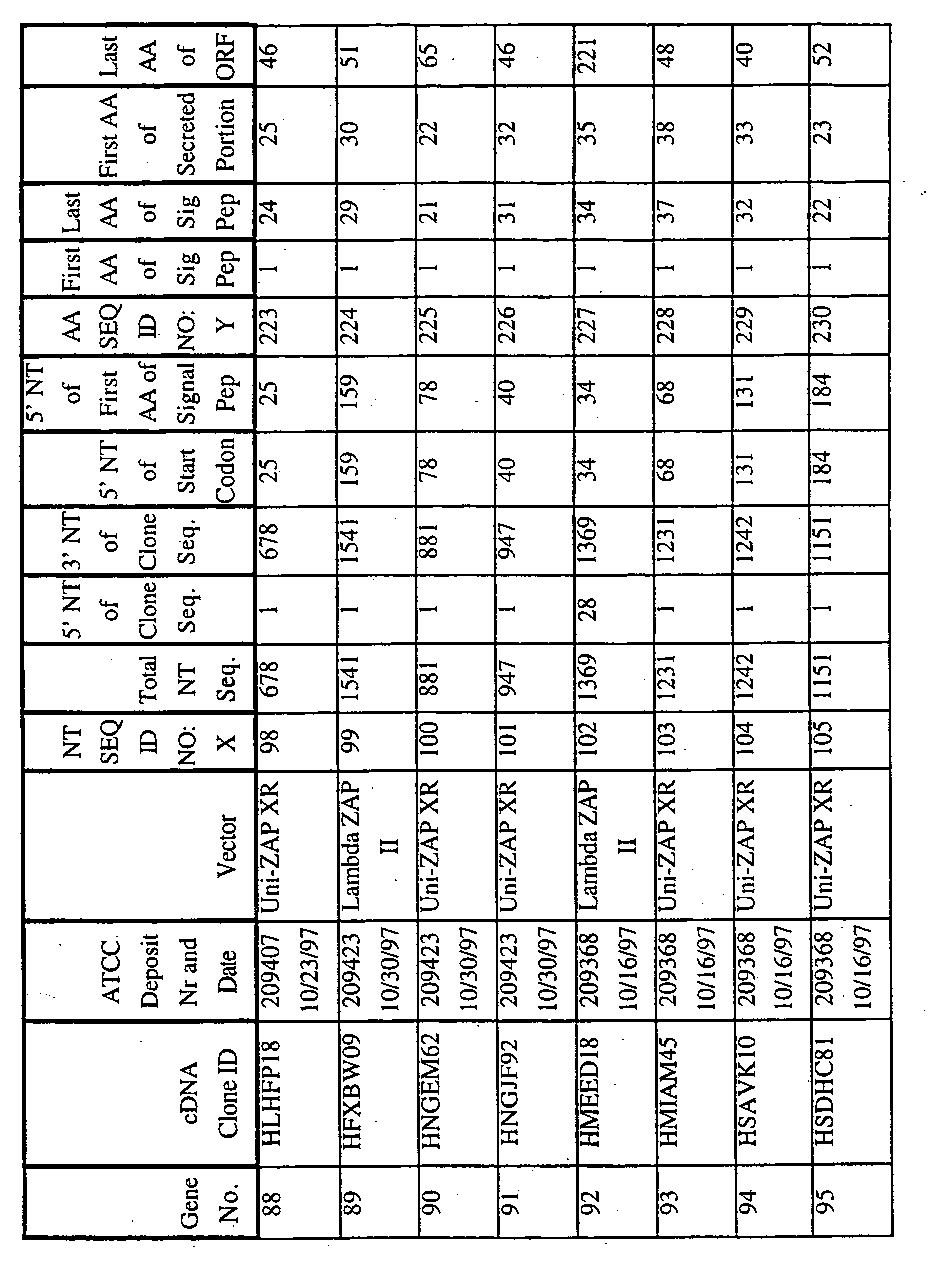

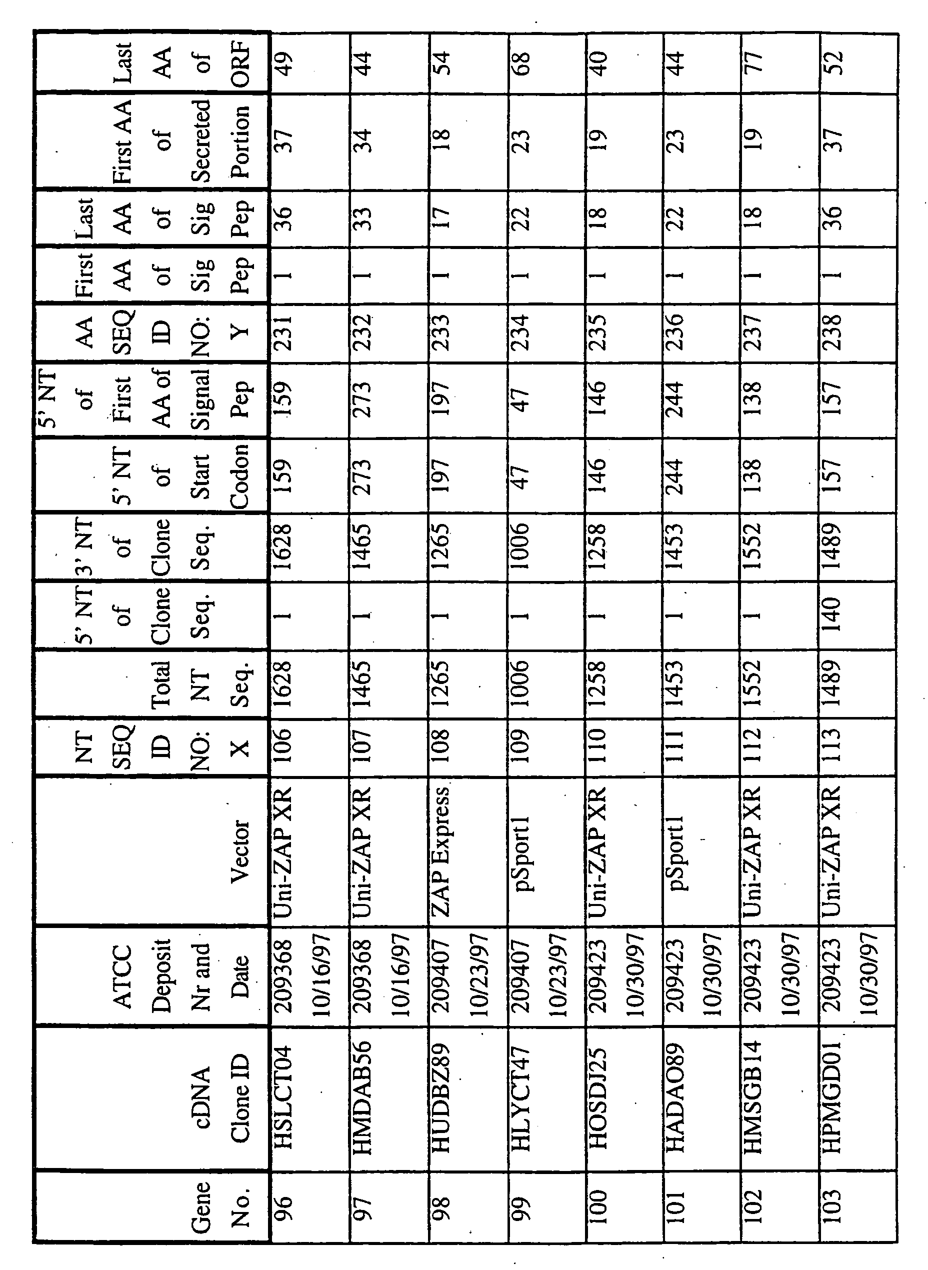

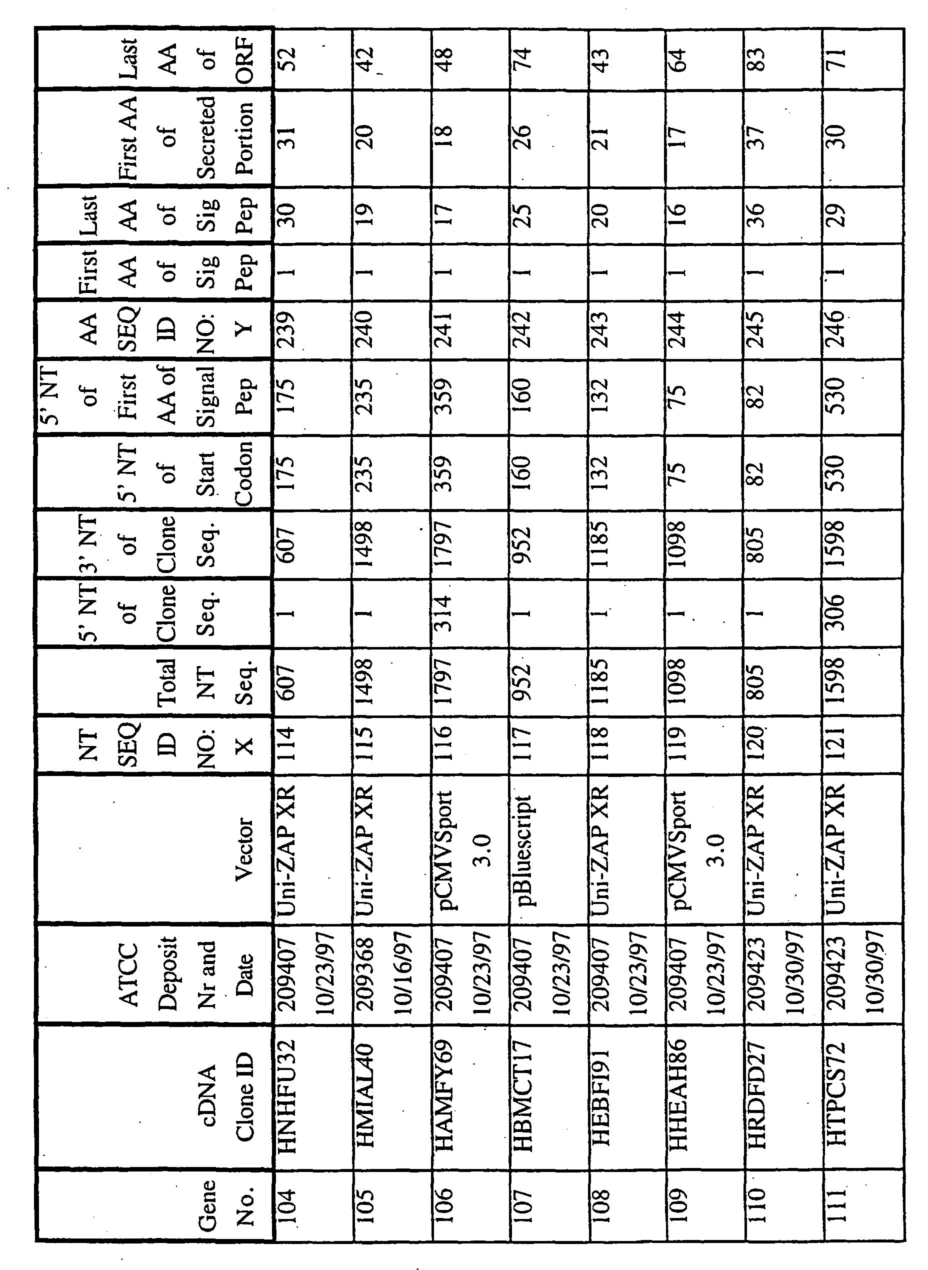

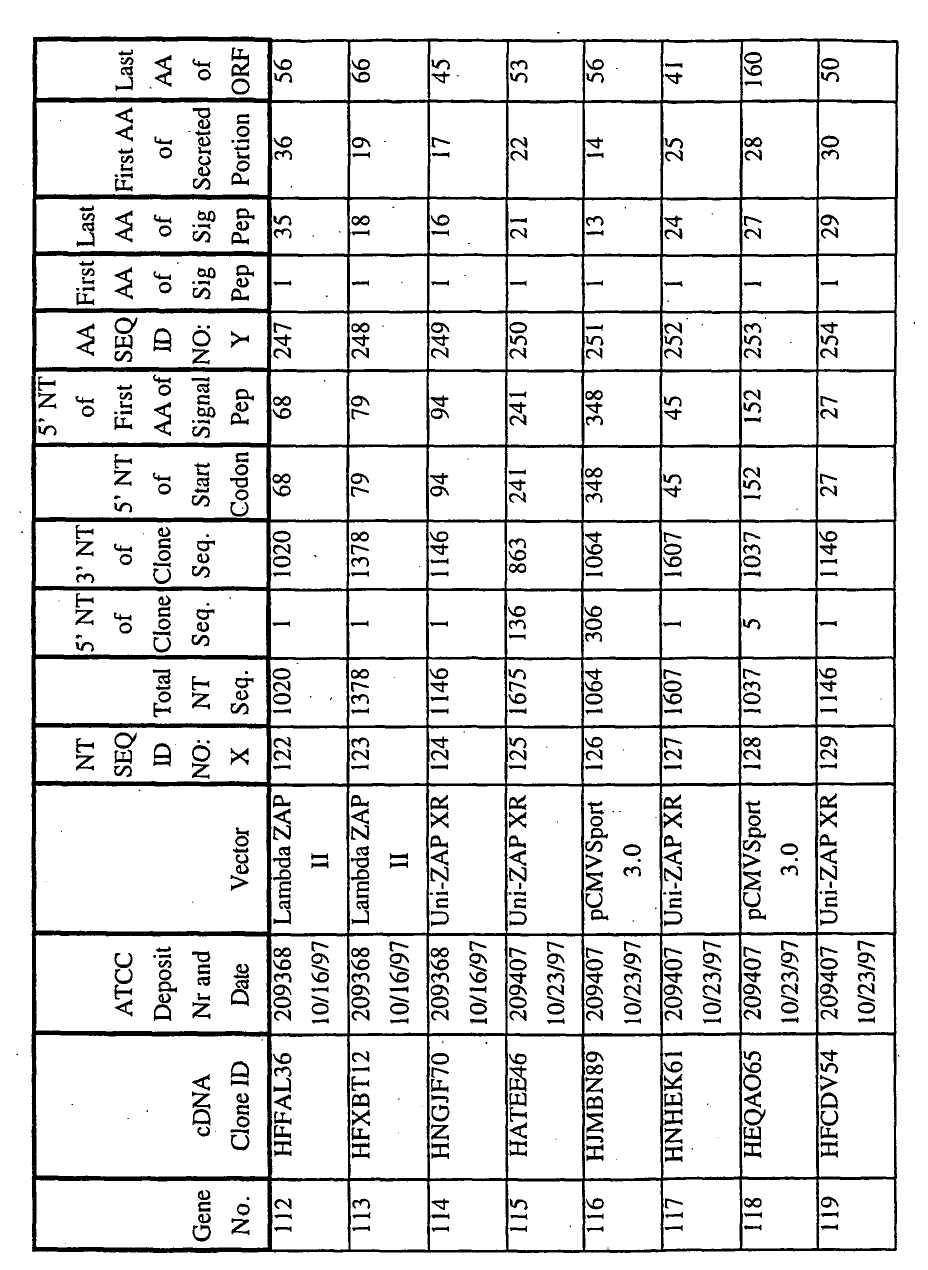

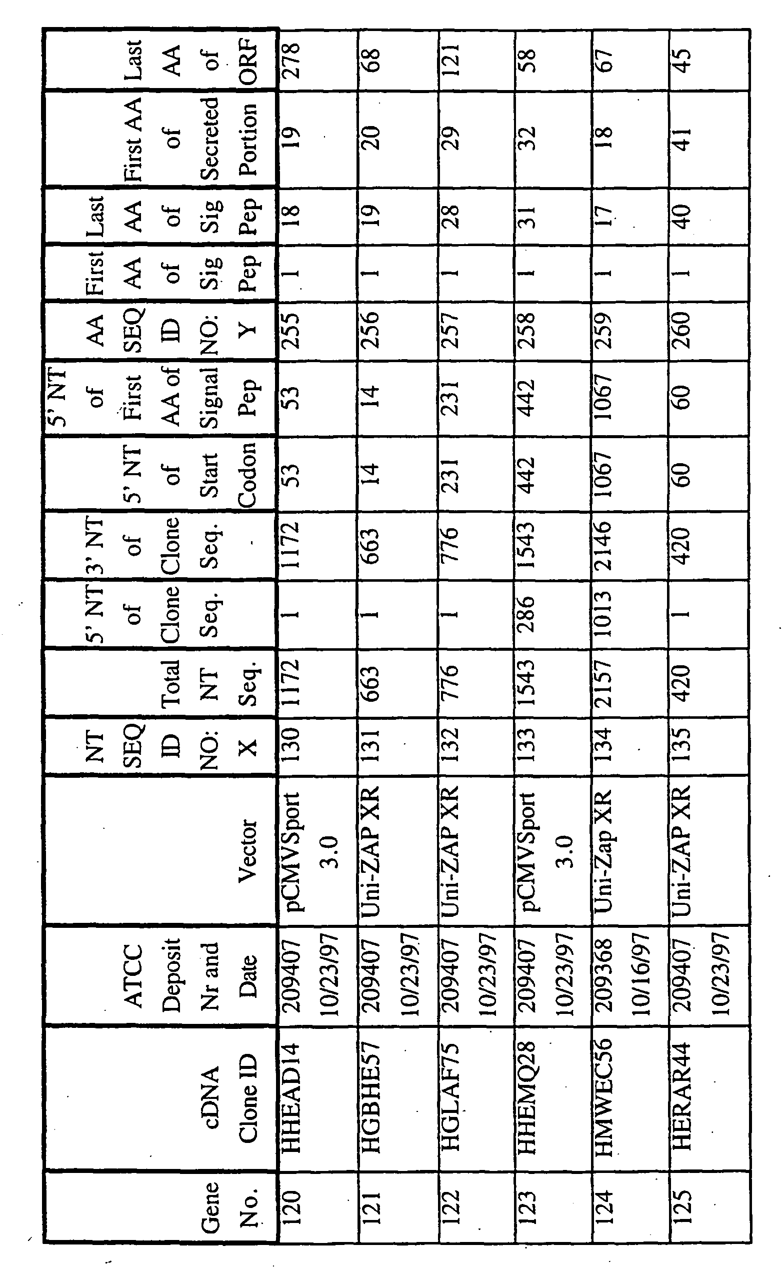

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

''Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown in Table 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus; it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991) ) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be'detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picomaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia); Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human, cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO: Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to_increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport 1 | pSport 1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Biotechnology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01 % (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime^{TM} DNA labeling system (Amersham Life Science), according to manufacturer's instructions. After labeling, the probe is purified using CHROMA SPIN-100^{TM} column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and Xbal and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra).* Proteins with a 6 x His tag bind to the Ni-NTA resin with high affinity and can be purified in a simple one-step procedure (for details see: The QIAexpressionist (1995) QIAGEN, Inc., *supra).*

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4°C or frozen at -80°C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC 19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E*. *coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4°C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra).* The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)
Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaC12 (anhyd); 0.00130 mg/L CuSO₄-5H₂O; 0.050 mg/L of Fe(NO₃)₃-9H₂O; 0.417 mg/L of FeSO₄7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂0; 6.65 mg/ml of L-Aspartic Acid; 29.56 mg/ml of L-Cystine-2HCL-H₂0; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂0; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂0; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1 %BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL- 11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteiries and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | STATS | | GAS (elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) - | | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp 140 family | | | | | | |
|---|---|---|---|---|---|---|
| IL-3 (myeloid) | - | - | + | - | 5 | GAS (IRF1>IFP»Ly6) |
| IL-5 (myeloid) | - | - | + | - | 5 | GAS |
| GM-CSF (myeloid) | - | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B- |
| CAS>IRF1=IFP»Ly6) | | | | | | |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1 ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCI (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1 x 10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC 12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC 12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF-κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAGCTT:
3' (SEQ ID NO:10)

Next, replace the SV40 minimal promoter element present in the pSEAP2-promoter plasmid (Clontech) with this NF-κB/SV40 fragment using Xhol and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and Notl.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| Reaction Buffer Formulation: | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assav Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Bibtek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assav Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1 % SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of -Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties.

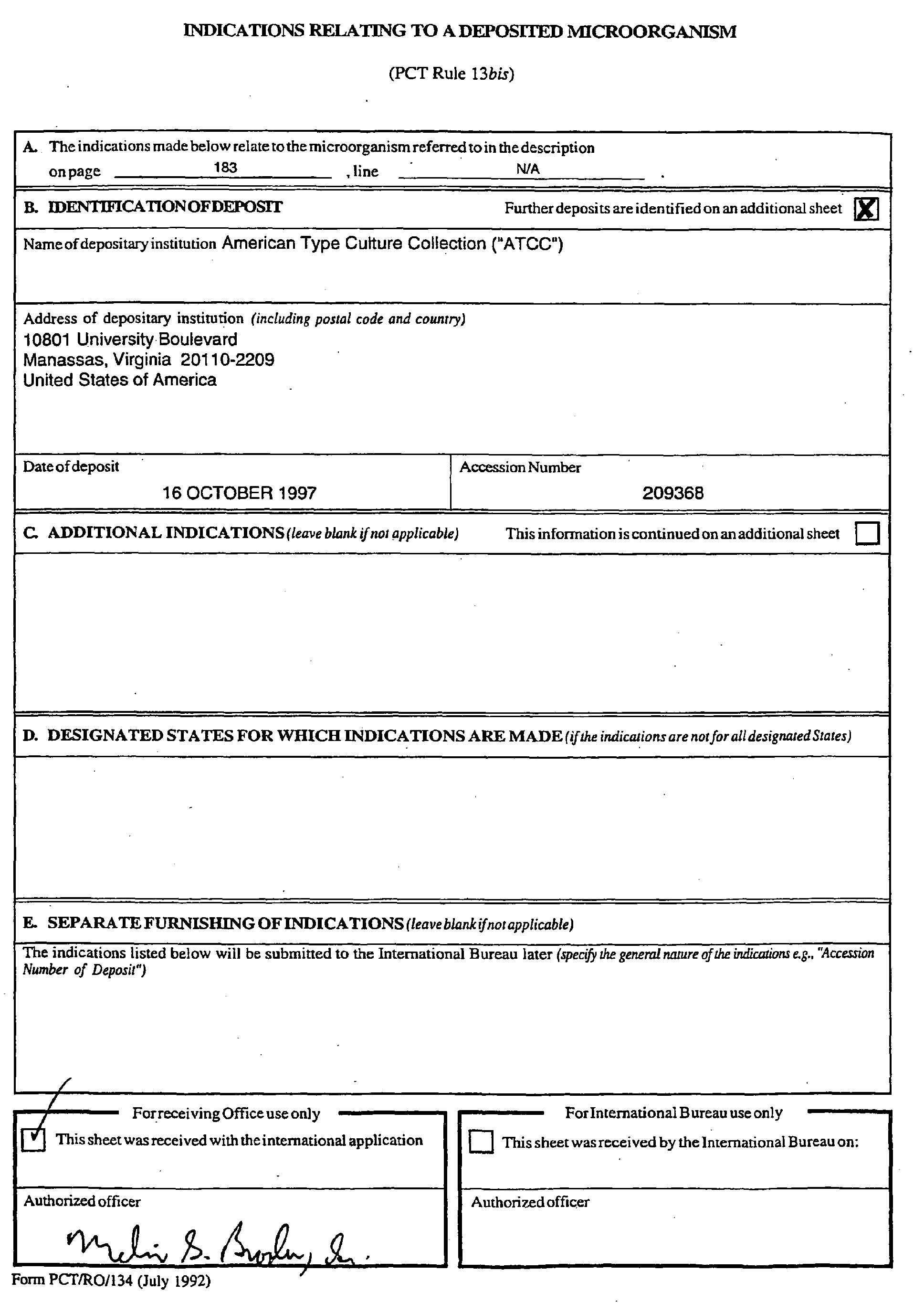
INDICATIONS RELATING TO A DEPOSITED MICROORGANISM

(PCT Rule 13bis)

A. The indications made below relate to the microorganism referred to in the description
on page 183 , line N/A .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
16 OCTOBER 1997 | 209368

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:

Authorized officer | Authorized officer
 |

Form PCT/RO/134 (July 1992)

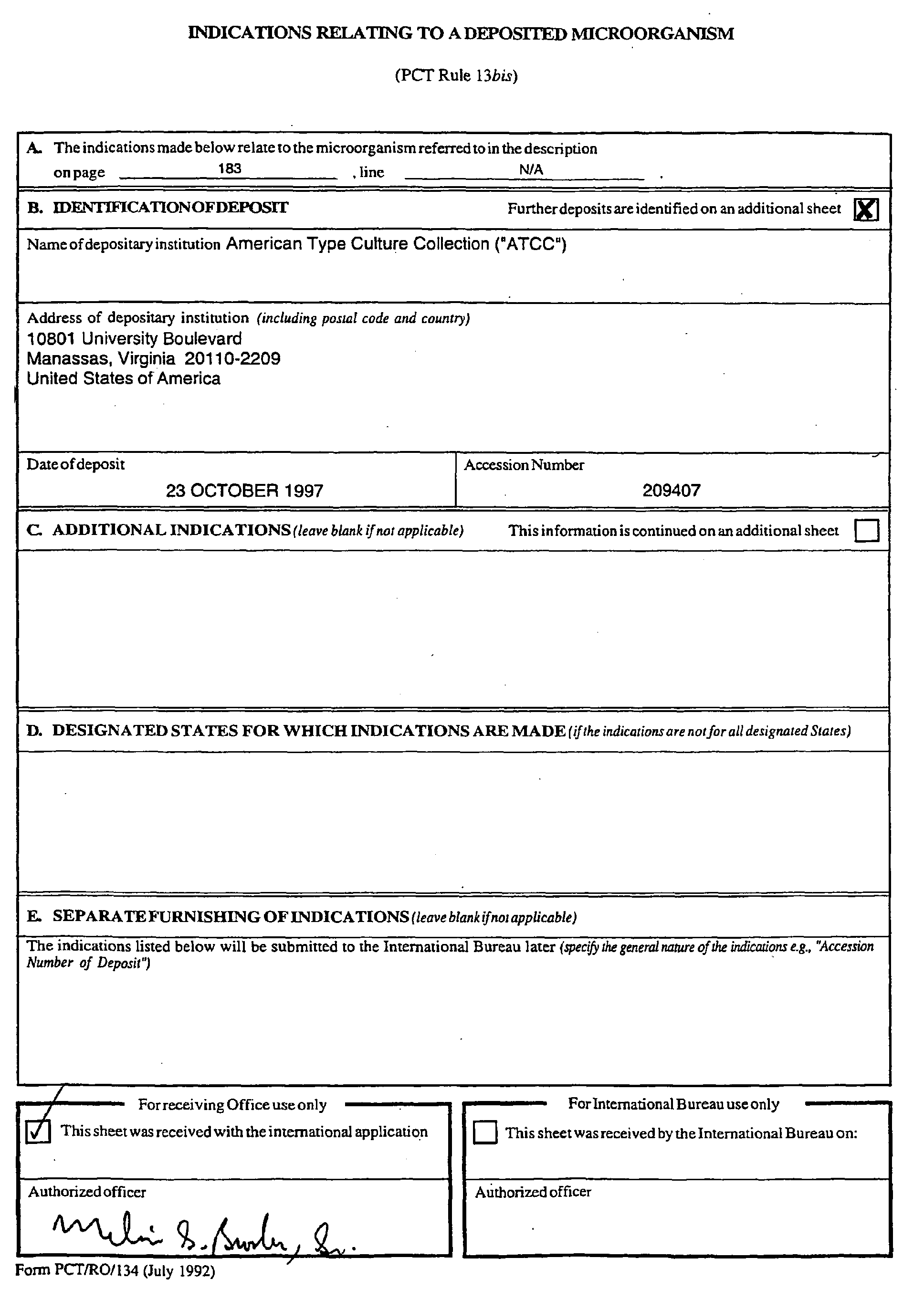
INDICATIONS RELATING TO A DEPOSITED MICROORGANISM

(PCT Rule 13bis)

A. The indications made below relate to the microorganism referred to in the description
on page 183 , line N/A .

B. IDENTIFICATION OF DEPOSIT | Further deposits are identified on an additional sheet [X]

Name of depositary institution American Type Culture Collection ("ATCC")

Address of depositary institution (including postal code and country)
10801 University Boulevard
Manassas, Virginia 20110-2209
United States of America

Date of deposit | Accession Number
23 OCTOBER 1997 | 209407

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[✓] This sheet was received with the international application | [ ] This sheet was received by the International Bureau on:

Authorized officer | Authorized officer
 |

Form PCT/RO/134 (July 1992)

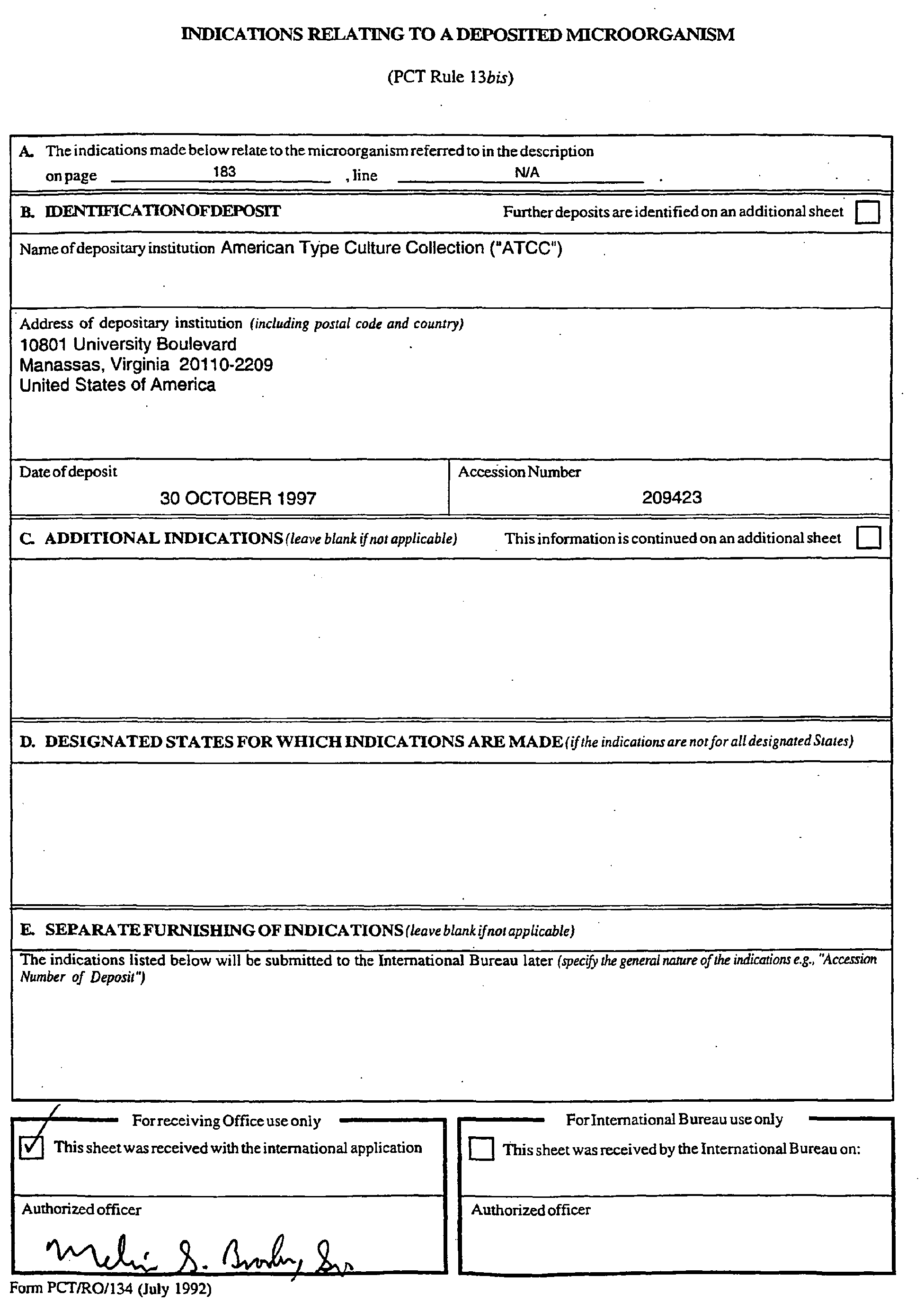
INDICATIONS RELATING TO A DEPOSITED MICROORGANISM

(PCT Rule 13bis)

A. The indications made below relate to the microorganism referred to in the description
on page 183 , line N/A .

B. IDENTIFICATION OF DEPOSIT | Further deposits are identified on an additional sheet ☐

Name of depositary institution American Type Culture Collection ("ATCC")

Address of depositary institution (including postal code and country)
10801 University Boulevard
Manassas, Virginia 20110-2209
United States of America

Date of deposit | Accession Number
30 OCTOBER 1997 | 209423

C. ADDITIONAL INDICATIONS (leave blank if not applicable) | This information is continued on an additional sheet ☐

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
☑ This sheet was received with the international application | ☐ This sheet was received by the International Bureau on:

Authorized officer | Authorized officer
 |

Form PCT/RO/134 (July 1992)

<110> Rosen et al.
      Human Genome Sciences, Inc.

<120> 125 Human Secreted Proteins

<130> PZ020.PCT

<140> Unassigned
<141> 1998-11-04

<150> 60/064,911
<151> 1997-11-07

<150> 60/064,912
<151> 1997-11-07

<150> 60/064,983
<151> 1997-11-07

<150> 60/064,900
<151> 1997-11-07

<150> 60/064,988
<151> 1997-11-07

<150> 60/064,987
<151> 1997-11-07

<150> 60/064,908
<151> 1997-11-07

<150> 60/064,984
<151> 1997-11-07

<150> 60/064,985
<151> 1997-11-07

<150> 60/066,094
<151> 1997-11-17

<150> 60/066,100
<151> 1997-11-17

<150> 60/066,089
<151> 1997-11-17

<150> 60/066,095
<151> 1997-11-17

<150> 60/066,090
<151> 1997-11-17

<160> 502

<170> PatentIn Ver. 2.0

<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733

<210> 2
<211> 5
<212> PRT
<213> Homo sapiens

<220>
<221> Site
<222> (3)
<223> Xaa equals any of the twenty naturally ocurring L-amino acids

<400> 2
Trp Ser Xaa Trp Ser
1               5

<210> 3
<211> 86
<212> DNA
<213> Homo sapiens

<400> 3
gcgcctcgag atttccccga aatctagatt tccccgaaat gatttccccg aaatgatttc 60
cccgaaatat ctgccatctc aattag 86

<210> 4
<211> 27
<212> DNA
<213> Homo sapiens

<400> 4
gcggcaagct ttttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120
gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg 32

<210> 7
<211> 31
<212> DNA
<213> Homo sapiens

<400> 7
gcgaagcttc gcgactcccc ggatccgcct c 31

<210> 8
<211> 12
<212> DNA
<213> Homo sapiens

<400> 8
ggggactttc cc 12

<210> 9
<211> 73
<212> DNA
<213> Homo sapiens

<400> 9
gcggcctcga ggggactttc ccggggactt tccggggact ttccgggact ttccatcctg 60
ccatctcaat tag 73

<210> 10
<211> 256
<212> DNA
<213> Homo sapiens

<400> 10
ctcgagggga ctttcccggg gactttccgg ggactttccg ggactttcca tctgccatct 60
caattagtca gcaaccatag tcccgcccct aactccgccc atcccgcccc taactccgcc 120
cagttccgcc cattctccgc cccatggctg actaattttt tttatttatg cagaggccga 180
ggccgcctcg gcctctgagc tattccagaa gtagtgagga ggcttttttg gaggcctagg 240
cttttgcaaa aagctt 256

<210> 11
<211> 1010
<212> DNA
<213> Homo sapiens

<400> 11
gcgtccgtat gttccagtgt gggttattgc agcagctttg tactatccta atggctactg 60
gggttcctgc tgatatcctg actgagacca taaatactgt atcagaagtt attcgaggct 120
gccaagtaaa ccaagactac tttgcatctg taaatgcacc ttcaaaccca ccaagaccgg 180
caattgtagt acttctcatg tccatggtta atgaaaggca gccatttgtt ttgcgctgtg 240
ctgttctcta ttgtttccag tgtttcttgt ataaaaacca aaaaggacaa ggagaaatcg 300
tgtcaacact tttaccttct accattgatg caacaggtaa ttcagtttca gctggccagt 360
tattatgtgg aggtttgttt tctactgatt cactttcaaa ctggtgtgct gctgtggccc 420
ttgcccatgc gttgcaagaa aatgccaccc agaaagaaca gttgctcagg gttcaacttg 480
ctacaagtat tggcaaccct ccagtttctt tacttcaaca gtgcaccaat attctttcac 540
agggaagcaa aatacaaaca agagttggat tattaatgtt gctttgtacc tggctaagca 600
attgtcccat tgcagtaacg cattttcttc acaattcagc caatgttcca ttccttacag 660
gacaaattgc agaaaatctt ggagaagaag agcagttggt ccaaggctta tgtgcccttt 720
tgttgggcat ttcgatttat ttcaatgata actcacttga gagctacatg aaagagaagc 780
taaaacaact gattgagaag aggattggca aagagaattt catagagaaa ctaggattta 840
ttagcaaaca tgagttgtat tccagagcat ctcagaaacc ccagccaaac tttcccagtc 900
cagaatacat gatatttgat catgagttta cgaagctggt aaaagaactt gaaggtgtta 960
taactaaggc tatttataag tccagtgaag aagataaaaa aaaaaaaaaa 1010

<210> 12
<211> 1559
<212> DNA
<213> Homo sapiens

<220>
<221> SITE
<222> (1547)
<223> n equals a,t,g, or c

<220>
<221> SITE
<222> (1549)
<223> n equals a,t,g, or c

<400> 12
ccggctacta ggaactagtg gatcccccgg gctgccaggt ttttattctt tatatataaa 60
atgtctccca gataccaaaa gatcaaataa aaatcccacc cctcctactg cgttcctagg 120
ttggctgtat acttatttga ggaatgtttc acagcccttg tttcagtgca gtattttctg 180
gtttatgggg ctggaatcaa agggaccgct attccctctg ccccaacaca cagacttacg 240
aatcagtgct ccctatagca ccatactgca ggattggtgt gcatgactgt ggcatccatc 300
agacatattc tggtagagat ctggttaccg atagccttag ccatgggcac ccgaggcctc 360
actcaaattg tagctgtcat tcaatccaga agccaatggg cactctcttg aagtctgctt 420
ggctcttctg ttctcttttt tagtaggtct gatttattaa tatgatcaat acaggttaac 480
ttgtgaagtc ctatatacaa cactcttcct cctctagaca actttgctat tgctacttag 540
aaatgatgta aatcattgga ggtgtgataa atttacaaat atttagtttt taacccatag 600
caagggagat attgggctag agtgtaaaac acagagcatt taatatacat aggaaattta 660
ggagtaaaat tttgatgcta attttccaaa atcatttcta ctatttctcg tcattataag 720
attgctcaca tattttgaat ggcatcccac agtagtcaca ggcaaattag aataacatat 780
gctttacaaa acaaaaaggc atatcagaaa atgagtcaaa gtaaaaaaaa tggcaggtac 840
catccattct ccagtaaata atgaatatgg agtgtattta attacaaatt tccaaatata 900
tattcgcata ataaagacaa ggaaaatgaa acaaaatcaa atgtgttttg ttgaaataag 960

cgcatgagct tgaaggatcm agtactgatg gctccaggtg ttttccatgt tagattccct 1020
ttgaaagtac tgatgccatc agcacttgtc agctggtacc caatgatgta aattttgagc 1080
ttgaatactt ttcatrggar rgcatcacat tcctaaagga gacaaatgac cactaaaaac 1140
taaccattaa tatagattac ttatggaggg ttttatcttg actactaatt aagggagtaa 1200
ttgtatctct aaactttata tcagattgcc agtggatttg ccagattatt aatgcttttt 1260
tagaatacat ggggggtggg gttataatca cataacacag tttttcatat tttttaaaag 1320
gggttggacc aagacataag tttcattgac aatcttaatg ttttcactgt agacaatatg 1380
ttagaataac tgcagtgagt cacacagcat ataacttctt aatggtaaat tcagaaccag 1440
tcttccagat aagcaagtat aacattttag ttaaaaaaaa aaaaaaaaaa aaaaaaaaca 1500
aaaaaaaaaa aaaaaactcg agggggggtc ccgtacccaa tctcctnana tgcatcgta 1559

<210> 13
<211> 1589
<212> DNA
<213> Homo sapiens

<400> 13
ggcagagctt ttgctctgag aacaatgcta ccggtagttt ccagtgtgtt tgctttgcct 60
ttttatttga attttagaat ttattatttt aaaattttat cttatttaaa tgttattcat 120
ttttcttcta ctaattttga gtatcacagc ttcgtactcc ttgacttgca ttctctcagg 180
agctggggag ccaagctcgg tctccgcttc ggtggtttca ggtcccgggt tctgtctggc 240
ggctctgctt ctaatgcgga ctggcggttt tgcagcaacg cttttgccag tagcgcccac 300
tgagcggttt ttcagttgct gcaccgttct tagcgcccaa cggaacgttt cccgtacgcg 360
gagtccataa gttgtctgtg tcgtcacttt ctcggttctg gcamcgctag tatctcctag 420
gaacttccta aamccgtttt acggtgcaga acggtaattg caccgagcct ctgtgccaac 480
tagtacttcc gtaataccgt tagccggctc ttttcctgtc acaacacatt aaaacgtgtt 540
aaaacacatt aaacggatta aacacattar acggattaaa acacattaaa acgtcacaac 600
acatttaaac agagtcggtc cattcytcca gacagtatgt ttcgggatgg aagcacccyt 660
cctaagaatc cgcagtttct ttstagcagc accgttatgg cgcytaaacg gatatttgtt 720
tggcaatacc agcgctatcc gctaggtgcc ggcgcttgct gagtctcagc gccgccagat 780
tcttgctagc gaggtccaga gcagtgcaga gacctagcgg acagttttcc ggtggcagca 840
atgctcattt cccggacaca gatggttttt ggsccctgaa gaactcttta atccctgcgt 900
tttgtagcgc tcgttctctg taaggtttgc ttartatccg tttcatttcg acttcttttc 960
tcgctgtttt tctgtcggaa ttacgatttg ttttgtttct atgtgtyctc tagaatttta 1020
ccggttttca tttgtctact aattttcgtg cattcgttac tattgagttt cataatatct 1080
gggtgccmtc tgcccackag ctccggagag cataaatacc caggctgatg gtagtgctaa 1140
aggttctgct tcatctcgtc aatcagcgca aacgcttatg tcctcctgct ctgaggctta 1200
attaagcgat ggagcaacat tttcttggct atgtgaagcc tgcttgagat tccgcagagg 1260
tggtgcctga gctccagcct cggcctattt tgaattcaga agactgtgat attactgtgt 1320
gagccaccac tttgggaggc cagcatgggc agatcacttg aggtcaggag ttggagacca 1380
gcctggccca agtggtgaaa ccccatctct actaaaaata caaaaattag ccggtgatga 1440
tgcatgcctg caatcccagc tactcgggag gctgaggcag gagaatctct tgagctcgga 1500
ggcagaggtt gcagtgagct cagatcgtgc caccgtcctc cagcctgggc agactgagac 1560
tccatcaaaa aaaaaaaaaa aaaactcga 1589

<210> 14
<211> 1255
<212> DNA
<213> Homo sapiens

<400> 14
gctgaaatgt ctcatttagc taaatggaaa cattcaccag acacttctag ctagctctcc 60
cacatcactt tgagccacac aggcacttcc atgtttggga ctgaattgcc tgggaatccg 120
tatacccaaa atctgagccc tcattcttac ccttgggtga tgcctctcca tagccctcag 180
caatgctctc cactcgctgg atggggctac atctcgtgca gattttgtgg cgctgttgga 240
ccagttcggc aaccattaca tccaggaagc tatctacggc tttgaggagt cctgttctat 300

ctggtaccca aacaagcagg tccagcggcg actctggctg gagtatgaag acatcagtaa 360
aggcaactcc ccatcagatg agtctgagga gcgggaaaga gaccccaagt gctgacattc 420
ccagaataca tcaccagctt gtcagactcc ggcaccaagc gcatggcggc tggagtccgc 480
atggagtgcc agagcaaggg acgatgcccc tcgtcctgcc ccctgtgtca tgtgacatcc 540
agccctgaca cccctgctga gccggttctg ctggaggtga ccaaagcagc ccccatctat 600
gaactagtga ccaacaacca gacccagagg ctcttgcagg aggctaccat gagctctctc 660
tggtgctcag ggactggaga tgtcatcgag gactggtgtc gatgtgactc cactgctttt 720
ggagctgatg gactccccac ctgtgcgcct ctcccacagc ctgtgtatgg ttctctttct 780
ctctttcagc attactctgg aaacagataa tgagcgtcta ctatttgcaa agaaataggc 840
tgggtactgg gaaacaaaga aaaatatgac acaatacctg cctttagaga gctttcaatc 900
taccaaggga ggcagcacag atggttttag tagagaaagg gtgtgacaag tgttaacaag 960
aggcatttaa agaaagtgtg aagagggaat ggtcaagtct gaactgacgc catggtgacg 1020
gctctgtgga ggaggtggcg cttgaacttc tccttgaagg tggagaaagt aggacagcat 1080
aactagaaga caatggggag aaaagtgcca ttgtaatgga agaagttaga gatctacact 1140
gagagcttaa ctagaccaaa aacataacca ctcattgaac acttactggg tgtccaggca 1200
ttgtatggag cagttagata ctgctcattt aatagacaaa aaaaaaaaaa aaaaa 1255

<210> 15
<211> 1191
<212> DNA
<213> Homo sapiens

<400> 15
gaattcggca cgagcggagg ccgaagagtt gagtccgttg ctaagcaacg aacttcacag 60
acagcgatcc ccaggtgttt catttggttt atcagtgttt aatttgatga atgccatcat 120
gggaagtggc atccttggct tagcttatgt tatggctaat accggtgtct ttggatttag 180
cttcttgctg ctgacagttg ctctcctggc ttcttactca gtccatcttc tgcttagtat 240
gtgtattcag acagctgtaa catcttatga agatcttgga ctctttgcat ttggattacc 300
tggaaagttg gtggtggcag gcaccataat aattcagaat attggagcta tgtcatctta 360
tcttttaatt attaaaacag agcttcctgc tgctattgca gaatttttga ctggagacta 420
tagtagatat tggtatcttg atggacaaac actactaata atcatatgtg ttggcattgt 480
gttccctctt gcacttcttc ccaaaatagg ctttcttggc tacacaagta gtttatcatt 540
ttyctttatg atgttctttg ctcttgtggt aataattaaa aaatggtcca tcccttgtcc 600
tctgacatta aattatgtag agaaaggctt ccagatttca aaygttacag atgattgtaa 660
gccaaagctc tttcatttct ccaaagagag tgcttatgcc ttaccaacca tggctttttc 720
atttctctgc catacctcaa tattgcccat atactgtgaa cttcaaagtc cttcaaagaa 780
aagaatgcag aatgttacca atacagcaat tgctttaagt tttctcattt attttatatc 840
tgcactcttt gggtacctca ctttttatgg gtctcattct gtcgcacagg ttggcgtgca 900
gtggtgtgat ctcagctcat tgcaacctct gcctcccgga ctcaagcaat cctcccacct 960
cagcctccag agtagctgag actacagaaa aggaagagat accatggaga tgtgcaccca 1020
gaggaaaggc cacgcaagga cacagcaaga aggcaactgt ttacaagcca agggaagagg 1080
cctcaggaga accaaacgtg tccacacctt gatcttgcac ttcccaacct ccagaactgt 1140
gagcaaataa atgatgttgt ttaatcaaaa aaaaaaaaat caaggggccc g 1191

<210> 16
<211> 1186
<212> DNA
<213> Homo sapiens

<400> 16
gaattcggca cgagattgaa tgttccagat aatccctttc ccagtcctgc ctgacatctg 60
ggtagggggt ttgtccctgg aattctggga cactggctgg ggtttgagga gagaagccag 120
tacctacctg gctgcaggat gaagctggcc agtggcttct tggttttgtg gctcagcctt 180
gggggtggcc tggctcagag cgacacgagc cctgacacgg aggagtccta ttcagactgg 240
ggccttcggc acctccgggg aagctttgaa tccgtcaata gctacttcga ttcttttctg 300
gagctgctgg gagggaagaa tggagtctgt cagtacaggt gccgatatgg aaaggcacca 360

atgcccagac ctggctacaa gccccaagag cccaatggct gcggctccta tttcctgggt 420
ctcaaggtac cagaaagtat ggacttgggc attccagcaa tgacaaagtg ctgcaaccag 480
ctggatgtct gttatgacac ttgcggtgcc aacaaatatc gctgtgatgc aaaattccga 540
tggtgtctcc amtcgatctg ctctgacctt aagcggagtc tgggctttgt ctccaaagtg 600
gaagcctgtg attccctggt tgacactgtg ttcaacaccg tgtggacctt gggctgccgc 660
ccctttatga atagtcagcg ggcagcttgc atctgtgcag aggaggagaa ggaagagtta 720
tgaggaagaa gtgattcctt cctggttttg agtgacacca cagctgtcag ccttcaagat 780
gtcaagtctt cgartcagcg tgactcattc gttcttccaa cagtttggac accacaaagc 840
aggagaaagg gaacattttt ctacagctgg aaagtgagtc ctatcctttg aggaaatttg 900
aaaaaagaca tggagtggtt tgaaagctac tcttcattta agactgctct ccccaaccaa 960
gacacatttg cctggaaatt cagttcttag cttaaagact aaaatgcaag caaaccctgc 1020
aattcctgga cctgatagtt atattcatga gtgaaattgt ggggagtcca gccatttggg 1080
aggcaatgac tttctgctgg cccatgtttc agttgccagt aagcttctca catttaataa 1140
agtgtacttt ttagaacatt tggaaaaaaa aaaaaaaaaa actcga 1186

<210> 17
<211> 1182
<212> DNA
<213> Homo sapiens

<400> 17
cccacgcgtc cggtaaaata taaagaaact gaaccagtgt gtcttttcac catagatata 60
agagttcgga ccgcccagca cacaaggtca gcatgctgct cctctgtcac gctctcgcta 120
tagctgttgt ccagatcgtt atcttctcag aaagctgggc atttgccaag aacatcaact 180
tctataatgt gaggcctcct ctcgacccta caccatttcc aaatagcttc aagtgcttta 240
cttgtgaaaa cgcaggggat aattataact gcaatcgatg ggcagaagac aaatggtgtc 300
cacaaaatac acagtactgt ttgacagttc atcacttcac cagccacgga agaagcacat 360
ccatcaccaa aaagtgtgcc tccagaagtg aatgtcattt tgtcggttgc caccacagcc 420
gagattctga acatacggag tgtaggtctt gctgtgaagg aatgatctgc aatgtagaat 480
tacccaccaa tcacactaat gcagtgtttg ccgtaatgca cgctcagaga acatctggca 540
gcagtgcccc cacactctac cttaccagtg cttgcctggg tctttgtgct tccattgctg 600
tgatgccacc attcctagga gaggcagaga ccagcctcta aagcacaagc caaaaactgt 660
gtgaacggtg aactttggag tgaagatcaa tcttgcactt ggtgaagagt gcacattgga 720
cctcaaggcg aaagccagtg gtttgcttgg ataaaatgtt cccgcatgag gccacaggac 780
tgaggatggg aatttggcag ggcctgagaa gatggtctga cttccaggct tcctggtcaa 840
agagagctac gtttgggcag ttctgcagag aggatcctgg caactagtcc cacctgacta 900
ggcctttagc tgaaaggatt tcttgacctc cttgactgcc tcagaggctg ccaggtcaaa 960
ccctcttgtt tatgtgatta gctcagagca tctctatgaa atctaaccct tcccctcatg 1020
agaaagcagt tttccccacc aacagcatag tcaatgagaa aggcaactgt acgaagaaaa 1080
cttccagtgg aactaatatg aaatctattt gcaaattatg gggggaaata aagcttttaa 1140
attatacaat gtaaaaaaaa aaaaaaaaaa aaaaaaaaaa aa 1182

<210> 18
<211> 1171
<212> DNA
<213> Homo sapiens

<400> 18
gcttcagacc tttgtgatac accatgctgc gtgggacgat gacggcgtgg agaggaatga 60
ggcctgaggt cacactggct tgcctcctcc tagccacagc aggctgcttt gctgacttga 120
acgaggtccc tcaggtcacc gtccagcctg cgtccaccgt ccagaagccc ggaggcactg 180
tgatcttggg ctgcgtggtg gaacctccaa ggatgaatgt aacctggcgc ctgaatggaa 240
aggagctgaa tggctcggat gatgctctgg gtgtcctcat cacccacggg accctcgtca 300
tcactgccct taacaaccac actgtgggac ggtaccagtg tgtggcccgg atgcctgcgg 360
gggctgtggc cacgtgccag ccactgtgac actagccagt gagtctgctc ctttgcctcc 420
ctgccatggt gcggtccctc ctcatctctc ccaccctgaa gcccccacca ttcatgctgc 480

ctcttgttac tcttagcata aaatgggcct taactgcaga aatgtcaaat cagaacagta 540
gctgcctagt aatgcccagt gatgggggac ccttgtgccc ttggaaaacc tcactccaag 600
tagaggctgt atctggagtg agtgtctaca gagaggggaa ttggtcagtg catggcagaa 660
cttgacatgg cagaactgtt ccgtgggccc cagagcaggg cctgccgtgc cttcctactt 720
ggtcatcctg tggctgtagg tgcactgtcc caactgctca cacactttcc agtcccctcc 780
ctccattctc accacagtta cagtcggagc agggagagac aggacaccaa gagacatggg 840
cagggccacg tgtagtctgg acagagccca cccagcccag acttggcctc ctgtctctct 900
gtgaggggca agcatagtcc gaaggcctgg attataaata ttagaacata atgaaaagga 960
aactgtgtgg caaagggata aagggataca gagaaaagaa acaaggaaga gatgaggtga 1020
cagtttggac caaggaaaat gccaagagaa gacttcacac taccttttttt ttttctggt 1080
tttgccattc ttttattcat gttgttacac tcagtatagg atagtttatt aaaatcatta 1140
tgtctgtaga aaaaaaaaaa aaaaaaaaaa a 1171

<210> 19
<211> 1337
<212> DNA
<213> Homo sapiens

<220>
<221> SITE
<222> (22)
<223> n equals a,t,g, or c

<220>
<221> SITE
<222> (1318)
<223> n equals a,t,g, or c

<400> 19
   cggggcttcg gcgccaggcc angcgctagt cggtctggta aggatttaca aaaggtgcag 60
   gtatgagcag gtctgaagac taacattttg tgaagttgta aaacagaaaa cctgttagaa 120
   atgtggtggt ttcagcaagg cctcagtttc cttccttcag cccttgtaat ttggacatct 180
   gctgctttca tattttcata cattactgca gtaacactcc accatataga cccggcttta 240
   ccttatatca gtgacactgg tacagtagct ccagaaaaat gcttatttgg ggcaatgcta 300
   aatattgcgg cagttttatg cattgctacc atttatgttc gttataagca agttcatgct 360
   ctgagtcctg aagagaacgt tatcatcaaa ttaaacaagg ctggccttgt acttggaata 420
   ctgagttgtt taggactttc tattgtggca aacttccaga aaacaaccct ttttgctgca 480
   catgtaagtg gagctgtgct tacctttggt atgggctcat tatatatgtt tgttcagacc 540
   atcctttcct accaaatgca gcccaaaatc catggcaaac aagtcttctg gatcagactg 600
   ttgttggtta tctggtgtgg agtaagtgca cttagcatgc tgacttgctc atcagttttg 660
   cacagtggca attttgggac tgatttagaa cagaaactcc attggaaccc cgaggacaaa 720
   ggttatgtgc ttcacatgat cactactgca gcagaatggt ctatgtcatt ttccttcttt 780
   ggttttttcc tgacttacat tcgtgatttt cagaaaattt ctttacgggt ggaagccaat 840
   ttacatggat taaccctcta tgacactgca ccttgcccta ttaacaatga acgaacacgg 900
   ctactttcca gagatatttg atgaaaggat aaaatatttc tgtaatgatt atgattctca 960
   gggattgggg aaaggttcac agaagttgct tattcttctc tgaaattttc aaccacttaa 1020
   tcaaggctga cagtaacact gatgaatgct gataatcagg aaacatgaaa gaagccattt 1080
   gatagattat tctaaaggat atcatcaaga agactattaa aaacacctat gcctatactt 1140
   ttttatctca gaaaataaag tcraaagact atgawawmaw agttttttat accttattta 1200
   agagaaacaa cctgacgtgc accawtcagt ctgcacatcc caacccttca cattttataa 1260
   attattgtag atcatgtttt gttaggagcc cttttatgga gaggacattt tcccatgnct 1320
   taagtaatcc agccttt 1337

<210> 20
<211> 1162
<212> DNA

<213> Homo sapiens

<400> 20
ggcacgaggc gccccggact cttctcagtt gagagtgcgg ttcctgggca ggtttccaca 60
ccagttcctt tccgcgtcct tcggccctgg ctctggctgc ctggcggagg tggggtagca 120
tttgtcattt gcacactgct ggctttatct ttggggctgc accccgaggc aacaaatgca 180
ggatgctctg tcacccacat gtccaccacc atctggtttg ccttttggct actttgactt 240
tctccttaaa tgcttcctgt gctgagcaaa cattccacag ccagcagagc aatggagagt 300
tcatggccac tcttcccagt atcagcaagc aatttggggt gatcgtttgg aagcctcaga 360
ggaaagatgt catcaggctt cctgtggctt tgtccttcag catggggctc ggcttgcttt 420
cacctgcctt aggaagattt ctggcttctg agctctgata tggggagaag ataagggctg 480
ggatctttga gtctgcccct agctgggtat gtgcgtccgg tgtgcgggcc ttggagtttt 540
tggtaatgac tcacttgtgc tctttctggg atctgtctcc ctcccacatg accccgtggg 600
gtccctgaat gactgtttta gagtacccat gtgggttccc tgagtcacag caggggatgt 660
ttaataagga ggttagcact gagcttgggg acgtgctgtc acaccagcag gacgctgcag 720
gaaggagcag gctacttcct ttcttgacgt gcaaataact cgtataggct aatcaacagg 780
cttataagtt aaaagggcta ccgctcggcc ccttggggat tccatcccct cctctgtaac 840
ttggagatgt ttgtttctgc tgcagactca gagggttgcg atgaagagtg gtgggactga 900
gttgagaagc ttatcccttc gctgggtggg aggtttctaa ttgccctgtt ctttggggga 960
tccttaagtc cagcttccag gtgggggcag cgataggacc aagttctcct agtagtctct 1020
gggaagccac ttgagggaag ctgccggtca tgcccatgca cccattggtc ttctgccagc 1080
aggccctgta ggtcgtgcca tgttccatgt ccttctgggt tcttggggga gaaggaagct 1140
gttgaaaaaa aaaaaaaaaa aa 1162

<210> 21
<211> 1837
<212> DNA
<213> Homo sapiens

<400> 21
aggaagaaat gaataatgtg agcattaggg gcggcagtgg gattactcag tgttgagaga 60
aacaacacgg aaagcccaga gcagaggagg gagaaggccc cattctctca gctttaatct 120
actgcacctc ggagggcaga gctctaacca gacaccctgg ataagagtcc gctggctcct 180
ggaagctccc tggtagaccc ccagatctct ctttgggtgc tgatggccat tctgctggcc 240
tgcttcacag ccgtcttggc cttcatctgc ctccagttct ggtgtgtccg ttgccatgag 300
ccgcgatggt cttacagggc tggccacatg gaggaggcca atgggttggt gagatggcca 360
gaggaggccc cggatcttgg tcagagggag gaagacctgc aggggctccc cctggtggaa 420
atgccacgca agaactccag agatggagct gaactggatc ccgaagccaa ccaggatgcc 480
cctgatgcgg gtgccttaca gagggggggt ggtgacccac ccgctatact gcctcattgt 540
ggggaatgag agtggggagg agagcgcccg catcatgtag cctaaagctt tccacacagt 600
aggtcgtttg tacaaatgtg tctatagact acccatttct ctcccatcaa acgtcactgc 660
tattgtaggt cacctgggtt ggatgaatgc cccatgacaa agcttctcaa gctggaaaat 720
gtatccccca gggtatgcca aggcacaaac raggcatctc agcagcagcr tccactcgga 780
ggacacagtt aagaattgaa agcattgttt taacattaaa acaaacatgg atatgttata 840
aagtagaaag caaaaatttg catgacttaa ataaaacaca gcacttgaag gaaacacttg 900
agtwacaagt aggctctcag argtacactt ttgggcaaag gtagatgtcc agtttcctct 960
gcccttgagg ggattatttg gaaataattt gggattattt ggaaataatt tgtgagaacc 1020
cttgctctgg agtgttttcc aacttttagg caatcacata ccaccgytyt ctatttttta 1080
aaaccatgga ttatcttcac tattattaat aatactttcc tttaaattga ytcatttttt 1140
aagcgtaaac ttattttcaa agacagcctt atattactcc ataagtgaaa aaccagcacc 1200
ctcytgctgc aaacagaagg gaagagacat aagaataaac atcatgaaaa caagataata 1260
ttaaataatc tgacttagct tctattgcct gccagtgatt ctgagcttaa tgcctgcttg 1320
cttgtctttg ataaaaaggg agatccagtg ttggagaggt aataaagaca tattagcacc 1380
aatatttgtc tttctccttg atatagcata agatttgaaa gagaatcgaa aagggaagtg 1440
ctgtttttac tttgcgagtc agtgttagtt aaatgccatg tctgtgaacc acctcaaatg 1500
ctgtcagtca gcttcccttg atttgagaaa attgtcttga ccagactagg caggctgttc 1560
tggagaagac gcctgtactc tccaagcatc atccaagacc ttcccagtac cctcttatat 1620

ctatagagca caaaatccca gaatcacagg acgacactgc agtgaatcaa ctaagaaaca 1680
gcaggagctg agaagccaag acgagaagcc ccacatccct attcccttgc ctacctcatg 1740
cattccctgc tcggcaccca gacttttgcc cccattcctg ctacttgtga acaaataaag 1800
attcatatac tcacaaaaaa aaaaaaaaaa aactcga 1837

<210> 22
<211> 1054
<212> DNA
<213> Homo sapiens

<400> 22
cacgcgtccg cggcacgctg ggaaagaatc ctccagtttt agctcctact tcaacagcat 60
ttccttatct atacagtaac ccaagtggga tgtctcctta tgcttctcag ggttttccat 120
ttcttcctcc atatcctcca caagaagcaa acaggagtat cacttcttta tctgttgctg 180
acactgtttc ttcttcaaca acaagtcata ccacagccaa gcctgccgct ccttcatttg 240
gtgtcctttc aaatctgcca ttacccattc ccacagtgga tgcttcaata ccgacaagcc 300
aaaatggttt tgggtacaag atgccagatg tccctgatgc atttccagaa ctctcagaac 360
taagtgtgtc acaactcaca gatatgaatg aacaagagga ggtattacta gaacagtttc 420
tgactttgcc tcaactaaaa caaattatta ccgacaaaga tgacttagta aaaagtattg 480
aggaactagc aagaaaaaat ctccttttgg agcccagctt ggaagccaaa agacaaactg 540
ttttagataa gtatgaatta cttacacaga tgaagtccac tttcgaaaag aagatgcaaa 600
ggcagcatga acttagtgag agctgtagtg caagtgccct tcaggcaaga ttgaaagtag 660
ctgcacatga agctgaggaa gaatctgata atattgcaga agacttcttg gagggaaaga 720
tggaaataga tgattttctc agtagcttca tggaaaagag aacaatttgc cactgtagaa 780
gagccaagga agagaaactt cagcaggcga tagcaatgca cagccaattt catgctccac 840
tataggtaaa ttgtatttca agtttgagtc tcaaggtgat tgcatcagtg ttctttaaat 900
agacatgttg ttaacggtgc ctgttcatca gccttaagca taattctgtc attatagtta 960
ctgtgctatg taacatagaa tgctttgtat tatataataa gcataatata aacatataac 1020
tagtagaaaa aaaaaaaaaa aaaaaaaaaa aaaa 1054

<210> 23
<211> 1066
<212> DNA
<213> Homo sapiens

<400> 23
gaattcggca cgagaaaaag caaaagtaaa ataaatatta aggaagaaat gtcaagagta 60
gtctctctct tctttttcat tctcttttcc tttttcttct ttgctttctc cttatcgtct 120
tctctttctt ttgtacacta tgagaaacta gtgcaagtga aagaatgttt ggattcattt 180
ctcaaaaaaa taaaaattaa ggaatacaag actagacaat gttatcattt aataaggtgg 240
gagaacaatg gggccaagtt gcaaagttga acacaggtgg aagcatcagc aaagcagagc 300
cgtagaagaa gtcaagcaac tttgtccaag gcagggtttt aagctcacat taggcaggtg 360
ggggctaagc agagctcctc agagggtcag atgcatttgt agatacctta ctgctctact 420
gtctcctgtt ccagaaaata cctatgcata taacattcat aaaactttaa tatacgtaca 480
aaactagtat cttgactgta gtgctagttt gattcttatg ttttgcaatt catattttgt 540
aattgaaaat atgaaagagt gtgtgtatag aagaaagaag agagaaggaa ggaaggaagg 600
aagaagaaag agaaaggaga gaaagaaaaa gagggaaaat tgagattaag gttcaagtga 660
ttgagtttgg cgtctcctcc caagggattt tacattggct ttggttcaac tctgatctta 720
aattgcaact gcctataaaa tgtattttat aaattggggc agggggtat aaaatgttat 780
tttagcttgt agaattctag tggattggag tgtagttaca gatcgaaaga actatagatg 840
ggagataatg agaagtggaa ttaagccatg caaagtaaaa atctaagagg cagctctatg 900
aaatatgact ctactgaaca ggtttaggag acagcagctg gtgaggcaaa ctcctgctag 960
gaagaatttt ctaattctaa gctactttca aatttgatag ggctgaaaaa tatctctctg 1020
aatcaaatgc agacattaaa aaaaaaaaaa aaaaaaaagg ccgctc 1066

<210> 24
<211> 928
<212> DNA
<213> Homo sapiens

<400> 24
ggcacttcat ctaaagtagt aactcagaaa gtgcacttat cttctgttga atttcctttc 60
atgtcagcct ctctaaaaaa ccacctgacc cactgctttt tgctattgct gcttaaagag 120
cttgtttccc ccaccatgat tagctttgtg cctacactaa ggcactccta cagattcttc 180
aacctcttct catgtgatgc agaaagtaca aaggagagcc ctggccgaac tgtccagttt 240
agtaaaacac ccagaggagt aactatgttt atttagggtg tttcagagct tgctgctctg 300
cctagtctat tgtaagagct ctgaaattat tagagctttt cctaaaaata tccacacttg 360
gatgtgactc agtcatagaa aaacaactga cccaagaaac agagagtctg agttttggtt 420
cttggtttgt tttgttctta ttgtgttttc attttgttgc atgaaacatg aaacctccgg 480
tcttgggata gatttgagtc ctgactcagc cagtgataag ttgtttgacc ttgggtgagt 540
tactaaactt ctctggggct aaatttcttc atctaagaag tggattagat acagcaacat 600
attatagttc gaagcttctg gctggacgtt gtggctcacg actgtaatcc cagcattttg 660
ggaggctgag gtgggaggat ctcttgaacc cagaagttca agactagcct gggcaacatg 720
gcaaaaccct gtctctacta aacatacaaa aattagtcag gcatgatgga gcatgcctgt 780
aatcccagct actcaggaag ctgaggtggg aggatcgatt gagcccagga gtttgagatt 840
gcagtgaacc atgattgaac cactatactc caacctgggc aacagagcaa gaccctgtct 900
caaaaaaaaa aaaaaaaaaa aaaaaaaa 928

<210> 25
<211> 966
<212> DNA
<213> Homo sapiens

<400> 25
   ccacgcgtcc ggtatttttg tcaatgacgc cactgaaagg gatcaagtcc gtgattttac 60
   ctcaggtttt cctctgtgcc tacatggcag cgttcaacag catcaatgga aacagaagtt 120
   acacttgtaa gccactagaa agatcattac taatggcggg agccgttgct tcttcaactt 180
   tcttaggagt aatccctcag tttgtccaga tgaagtatgg cctgactggc ccttggatta 240
   aaagactctt acctgtgatc ttcctcgtgc aagccagtgg aatgaatgtc tacatgtccc 300
   gaagtcttga atccattaag gggattgcgg tcatggacaa ggaaggcaat gtcctgggtc 360
   attccagaat tgctgggaca aaggctgtta gagaaacgct agcatccaga atagtgctgt 420
   ttgggacctc agctctgatt cctgaagtct tcacctactt ttttaaaagg acccagtatt 480
   tcaggaaaaa cccagggtca ttgtggattt tgaaactgtc ttgtactgtc ctggcaatgg 540
   gactgatggt gccattttct tttagtatat ttccacagat tggacagata cagtactgta 600
   gtcttgaaga gaaaattcag tctccaacag aagaaacaga aatcttttat cacagagggg 660
   tgtaggcgtg agttttaggt gaatttatgt ggttcctgct tgaaaacctt cccctctcca 720
   ggttcggttt agagaacttt gccacaggtc ttctggggtt tttaaggctg gctggagaag 780
   acagtgggag ggtgcccccg tctgacaccc ctggggttgc tgaggggacg gttggagtgg 840
   ggatcggcct gcgaaaggat actgtgaaat cactaattaa ctaataaacc tgtctcaagt 900
   tgaggatttg aaaaaaaaaa aaaaaaaaaa aaaaaaaaaa aaaaaaaaaa aaaaaaaaaa 960
   aaaaaa 966

<210> 26
<211> 1146
<212> DNA
<213> Homo sapiens

<400> 26
gctgacctca taatccgccc gcctcggcct cccatagtgc tgggattaca ggcgtgagca 60
ccgcgtccgg ctgtcagaat ttcttttaat gctacacata tataagcaaa taatgttttt 120
aagaagctaa ccttgatgtt aagagtggca ggtgttctcc agtttttacc tctttcatat 180

gggaccaaag tagctagttt atggaacaca tatgaaaatg tggttatgcc accaagtttt 240
actactacac ttgtcttacc acttttaagt catgaattct ataattattc ataccccttt 300
gcctgtgatc agaagtaact tttaaaatta tcacttgact ttggatggaa ctagtataaa 360
ggcaggaatt ttgtctttca gtggagattt attctgttca aggttgaagt ggacacatca 420
ttatcttggg atggtacttc tttatttaaa tagtccttta aagttctttg agtggcagag 480
aacgtttttg gtttcagtct gagaaggcta ccaaatggtt taagttcatt tcatagttag 540
gagaaaaaga ttttgagtag tctaatgtcg tcagaaagga ttaaaacgtt gtatgtacca 600
agaaggcaga atgamgaagg atcacgttca caaatgctgt atgtttaaca aaatacgttt 660
agatggtaat atccaatagt cttatcaagt gctacaatct ttttaaacag ggaatggcca 720
aatccagtgc tattgaaaca gcctgaagaa tgcaatctta atttgcctgt atgggaccca 780
agggttagtg tattattttt tcccctacca attcacactg tgcaataaca agtaaaaatc 840
atctgcataa actccaggga gacttccagc cttttactta tgaatggtca tgtccgtatt 900
acactttctt ttagataacc tcaactataa ttgtcctcaa ctataattga tgtgagaagc 960
ataattatgt accctgtaaa ccacatttaa ttgtacatag tttttaatac agattttact 1020
aacattttaa tttattctat atagaactac cttgtaagtc aaagttgtgt gggcatttgt 1080
gctttaaaaa aaataaaagg atactaaaaa aaaaaaaaaa aaaaactcga gactagttct 1140
ctctct 1146

<210> 27
<211> 802
<212> DNA
<213> Homo sapiens

<220>
<221> SITE
<222> (337)
<223> n equals a,t,g, or c

<220>
<221> SITE
<222> (359)
<223> n equals a,t,g, or c

<400> 27
ggtgggtgac cagagagtcc tgtctatcct aggaggagaa cattcagccc aaatcccagc 60
cccatcatgc acagatcaga gccatttctg aaaatgtcgc tgctgattct gcttttcctg 120
ggattggcag aagcctgtac tcctcgtgaa gtcaacttgc tgaaagggat cataggtctc 180
atgagcagac tgtcaccgga tgagatccta ggcttgctga gcctccaagt actgcatgaa 240
gaaacaagtg gctgcaagga ggaagttaaa cccttctcag gcaccacccc atccaggaaa 300
ccactcccca agagggaaga acacgtggaa yttcctngaa atgcgsctac atggtgrtng 360
acctacctct tcgtatccta caacaaaggg gactggttca ctttttcctc ccaagtgtta 420
ctgccaytac tgtaacttgg aactggacat cagggatgat ccctgctgtt ctttctagtg 480
agcctgctcc atctcagctt agccttcaca aggcctccat ctcccaggca ttctaacctc 540
tgaagaaagc tctctgtccc ctggactgcc tgtgtggagg gtaatgaact gggtccttta 600
aggaatggca cctgggtgcc cagaggcatg gccagaaggt gtctgtgggg gccatgcctt 660
agggggatgc acccagggcg gctgagagag caactgcagg agtttcccct aaaatctctc 720
ctccagatcg ttctcgaact ttccccacta cttccataat aaaatgtata cttgttgaaa 780
aaaaaaaaaa aaaaaactcg ag 802

<210> 28
<211> 1169
<212> DNA
<213> Homo sapiens

<400> 28
ggaatgtgag tgcaaatttg aatttccatg tacacatgtc cccgtgtgca catatctgtc 60

tgtatgtgct agtgtttcta tgtaatgtga ctagatgtaa atgtgttagg gcattcacaa 120
cctgggacac agagaaagtg aaatatttta tggcacactg gagtaaactg aagagggtta 180
ggggtactag agttgagtga aaaggaattt cttacatttt cctcatatta tacaattatg 240
ggaagaaaat taaaatgcag aattttaggg gagttattaa atattgaatt tgtgtacaac 300
tttcaaatga aatcttttca gttttttatg acacacttga gctcacttct agaaacatgt 360
cttagtctgt tttgtgctgc tctaacagaa tacttgagac tgggtaattt ttaacaagca 420
gagatttctt tcttacagtt ctggaggcta ggaagtccaa ggttgagggg catgcatcta 480
gcaagggcct ccttcctgcg tcatcccata gtgaagggca gaaaggcaag agaacacgct 540
tttgcatgag agagagaaag agagagaaga gaagggaagg gaagagaaag aagagaagag 600
aagagaagag aagagaagaa aagggagcca aactcatcta tttatcagga acccttctta 660
tgatagaaac ccactcccat gaaaacagga ttaatctgtt tatgaggaca gagtcctcat 720
tacctcatca sttcttaaag gtctcacttc tcagtactgc tgcattgggg attaggtttc 780
caacacatga acttcggagg acacattcaa gccgtagcat tctyccttga ctcccmaaat 840
ccatgtcctt ctcatgtcta aaatagatta atyccatccc aatwgcccca aagtcttgac 900
tcgttccagc accaactcaa aattccaaag tccagagtcc catttgaatc agacaggaga 960
gactccaggt gcaattcatc ctgaggcaaa tttctctcca gatgttagcc tatgaaaata 1020
gcaaattact ttcttccaaa atacaatggt gggacaggca taggatagac attcccattc 1080
caaaagggag aaataagcaa gaagaaaggg gtaactggtc ccaagtaagt ccaaaatcca 1140
acagaaaaaa aaaaaaaaag ggcggccgc 1169

<210> 29
<211> 1466
<212> DNA
<213> Homo sapiens

<400> 29
   ggcacgagtg cctcaaagac tattatttgg gaggatctag tgcaaatgtt agtaatgtgg 60
   atattgtgta gtgtcccagg atattaatgt ttttagcctc ttggctttta ttctgtattg 120
   ttgccccaaa agatgatgct cacttatctt tcatccagtg taaggatatc tggaaagaca 180
   acagaaagta tagctgtttt catttcaaaa gtgatcagct gcttgagcta gcaagcaagg 240
   cttgcactag cttccaggcg cagtcacgca gtttcacagc aggcgcggtt ccctcggagc 300
   acccagagct gccctgcggt agtcagcagt tgtgctgtgg ctgcactgcc aggctgggtg 360
   gcargtggat cggagccagc agatgtggct caggaagtgc cttcttggcc tctccttaat 420
   ctctttcaga stctgtgggc ccttgattgc actgtgggtt gtttcagact ccagtattag 480
   gagactgaac cccttggtgg ttttttggtg tgtgtgtgct gagmtgggtt gaggacatgt 540
   taagcaggtg gggtgcytcc cctgggtttg ctccgggtgg tacctgtggt gtggggtggt 600
   tctgagtagt tctggcccca ctgctggagt atctgcccay tcagtttgtg agatggcagg 660
   gcttcatcct ggtctggtgc ctcattttct tctttagcag tgggcttaga accaatgcag 720
   attcccaagt taagtatttt ttctgtagct taattattac aggcttctgg tacctaagcc 780
   ctttcttact ttctgttctg aggggaagag aagataatgt tgtttctccg ccccccccgg 840
   agtggcccca ggaccttgca tggcatttgc agcatttgca gcgtgcttgg gtttgcttta 900
   ctagggtgaa agtgttgcac cccccagcac ccacaaaggc acctctgctc accctccggt 960
   gaggttctga ctggccctgg gacatcacst gctccaggat cctatgtggc tcatcccagg 1020
   agagatgtgg gagggaaggg gaaaaaaggc ttacatttgc tgagtggaat tcatgtagat 1080
   ctgagttccg cattgattcc taagctgcag agcccttatg ccttggctgt tttgtgaatg 1140
   ttagtcggtc ttaacctttt tcaccgagtt agcattggct gtctcaggag gctcacagct 1200
   cctgctcctc ctccagggga gtgcgccctc ctcctctgtc ggtagctgtc aggtgcccct 1260
   ttcctctgca gcagactgtc ctgggtcctt gcctggcctt ccccttacac gtgagcctgc 1320
   agcttcattc acagcccctg tgtagaaaga taggcactaa aagcagctga ctggcagccc 1380
   tagaaacatg aagggtttca tttatagttt cagtcctttt ccttctttcg agccttaatt 1440
   taaaaaaaaa aaaaaaaaaa ctcgta 1466

<210> 30
<211> 1226
<212> DNA
<213> Homo sapiens

<400> 30
ggcagtgccc gcatggacta aaataagtat tagattctgc cttcaagctt gctaccagat 60
gcgactgtaa aattgaagta catcatctca cttgccacta taaagcctgc taccagatgc 120
agcttaatta tcacttgcca ctcactcact gatagggttt tgatatgagt ctgcaagcaa 180
ttgatttatt gtggtccctg tgcactcaaa cctctctgct aacattaatc tgtatttgca 240
gccactccca agccctatca tcatcacctc agctccacct cagatcatca agcattagat 300
tctcataggg acatgcaacc tagatcccta acatgtgcag tttacaatag ggtttgcact 360
cctatgataa tctaatgttg ccactcatct gacaggaggt ggagctcagg cagtaatgcg 420
agtgatggag aatggctgta aagacagatg aagctgtgtt tcctcacttg ctactcacct 480
cctgctctgc tgcctggttc ctaacagggt gttgagaacc ctgggattat attgtatagg 540
atgtcaccat tatgtgtcca ttatgtatca gtattatgta tccactgtat agactgatat 600
ttatttttta ccacctcaga actggtttcg gtttttagta gatatggtta catagatagg 660
gcactttgtt gagatactag gaaatgtgtt ttgaggactt attctttttc agtggtgaaa 720
tgaattcttt tggactgtta cttcccctga gaaaaattag gaattttatt tgcctaaagc 780
atgagagaac tatcaaaacc atgagaattt rtcagagatt taggagaggt acaatgtcaa 840
gggatacaak acaggcawtt tgtgtaattt taaccttgag acagttagaa atcctggaaa 900
cctcctgaga cactgagcag tccttttgtc agccattaca gaggaacaaa attagatatc 960
cagatgtgcc aaggaagagg agccttggta catacttaag gatttagtct ggcccctgag 1020
attctaaacc ctactcgtga taattagccc acctttgaat catcataatc ttttattaga 1080
ttatggtgaa atgcccataa tgtagttaga tggcaacata aaaagtaaat actttattga 1140
gtgagataac atcatatgga ggcttaaagt atttcttcta tatatcatat acaatatctg 1200
gcacaaacta aaaaaaaaaa ctcgag 1226

<210> 31
<211> 1094
<212> DNA
<213> Homo sapiens

<400> 31
ggcagagccc cagggtgaga agtttgggag acatgttcca ttttggtctg tgggatctgc 60
atttcttcct tatagttatg gctcatagag atgactgctc tttcaaaggt ggttgtggtc 120
tattagagcg tttccaatgt ccccatactt ctttctcgag tgctagtcaa aagcgacttg 180
cagatggtat ggaatgtctt tgtgagatag aaagaacaca gactaggatc agaaaaatct 240
gcctcccaac cctccatggc catcttctgg ctgtgtgact ttaccgccct aactctttaa 300
aatagcccca cacctacctg ccaggattgt cttcagaatt acataaaata acacatacca 360
aagtctctag aatagagcat gtcacataat agactcaaca aaggtcagca tctcttcttt 420
ctttttggga tgactaaagt agcctaaata aataagtatt ccgcaatgca tgacttgata 480
acataaactt ttgtaatact ctattacctc caagcataaa acaattattg agacaaaaga 540
aatacaatat tttctaagtt ccaagacaaa aaaaaaactt accaataaag aaaggagctg 600
tctgttccac aaaaccaaca gccacagttt tttccttctt aaatgtcaga ctttgaaaag 660
gttgacattt ctgttcacca gcatatacac gtgggccctc tcttactgat gaccacagag 720
tcctggggac cttcctgtgc tcccagccca gctctcctct ccggccacac tgctgcctcc 780
ttcacgcata cactgggtgg tgtgctgggc tgccctcctt accacaagtt ttattcctca 840
gcacacactt cagatcacag aaaggagaca aataaggtgg aggaaggaag atgggttgat 900
gtaactaggt cacttggtaa tttcaatttc agacgtaaat ttttctgtgt ttcagaatta 960
ttgatctgtg gaatatttct tgattcttct tggaaattac aaattaattc aaatgattgt 1020
aaagttctct gaaaggctct acagttttct taagagaata aaaaaacctg aaaataaaaa 1080
aaaaaaaaaa aaaa 1094

<210> 32
<211> 1037
<212> DNA
<213> Homo sapiens

<220>

<221> SITE
<222> (6)
<223> n equals a,t,g, or c

<400> 32
ggcggnccca tgaaagactg cgagtacagc cagatcagca cccacagctc ctcccccatg 60
gagtcgcccc acaagaagaa gaaaatcgcg gcccggagga aatgggaggt gttcccggga 120
agaaacaagt tcttctgtaa cgggaggatc atgatggccc ggcagacggg cgtcttctac 180
ctgacgctcg tcctcatcct ggtcactagc ggactcttct tcgccttcga ctgtccgtac 240
ctggcggtga aaatcacccc tgccatccct gcagtcgctg gcatcctgtt cttctttgtg 300
atggggaccc tgctccgcac cagcttcagc gaccccggag tcctcccacg agccacacct 360
gatgaagccg ccgatctgga aaggcaaata ggtaacactg aaagtctgcc catggcctct 420
ggtcacttcc cgcctgggcc cagctacagt ggggaaggca ggccgagggc tktgcaggag 480
gagctgascg ctgggaagga aggaggccag aagtcagcgt tccttagctc gctgggtggg 540
caggatgagc tgaagaagag gtgtgatata aggctggagg gacaggtatc ctggaggcag 600
gactgcaggc ccacttgagc aaagcatcag tgtgagctgt gcttctgatg tttctttgaa 660
acccaagtgt ttgattccac tctactaagc agcctccacc ccgagagatt acagttgtag 720
gttgctgccc ttactccctt ttgcaatcct tagagcatga ccagtttcca tgatataaat 780
ctaggaaagt tacatcttag gcagctttct tgtttatcca ggccaggatt gagaatttcc 840
tttatttagg tataataaac atgtatctgt gatatgtatt gagatgaata gctttatttt 900
tccttagata ttaaaaccta tactaaagtt tattacaacc cattttgaag atattaaaac 960
agatcctaat cccttacaca ataaactttt acagtttttt tttttaaaaa aaaaaaaaaa 1020
aaaaaaaaaa actcgag 1037

<210> 33
<211> 1376
<212> DNA
<213> Homo sapiens

<400> 33
ggccaccctt cccccaccca gagactgggc agctgtgtct ggtggactct gcgccccgcc 60
ccctgcagcc gtacttgcgc ctcatgcggt tggacaagcc cattggaacc tggcttctgt 120
atttaccatg tacctggagc attggtttgg cagctgaacc aggttgtttt ccagattggt 180
acatgctctc cctctttggc actggagcta ttctgatgcg tggagcaggc tgtactatta 240
atgacatgtg ggaccaggac tatgataaaa aggttacaag aacagccaat cgtccaatag 300
ccgctggaga catttcaact tttcagtcct ttgtttttct tgggggacag ctaaccctgg 360
cactgggtgt tcttctgtgt ctaaattact acagtatagc tctgggagca ggatccttac 420
ttcttgtcat cacctaccca ctaatgaaaa gaatttcata ctggcctcaa ctagccttgg 480
gcttgacatt taattgggga gcgttacttg gatggtctgc tatcaagggt tcctgtgatc 540
catctgtttg cctgcctctt tatttttctg gagttatgtg gacactaata tatgacacta 600
tttatgccca tcaggacaaa agagatgatg ttttgattgg tcttaagtca acggctctgc 660
ggttcggaga aaataccaag ccgtggctca gcggcttcag tgttgcaatg ctgggggcac 720
tgagcytagt gggtgtgaac agtggacaga ctgctccyta ctacgctgcc ctgggtgctg 780
taggagccca tctgactcac cagatttaca ctctagacat ccacagacct gaggattgtt 840
ggaataaatt tatctccaac cgaacactgg gactaatagt ttttttaggg attgtccttg 900
ggaatttgtg gaaagaaaag aagacagaca aaacaaagaa gggtatagag aataaaatag 960
aaaattaatg aatgaaattt atctaggaat ttttaaaaca ttttttacaa aatataatta 1020
gatttgaata caaaatctga tacaatatgt taaagaatta agaacctgaa gatgaagatt 1080
tagagcatat ttacctggat tttacttatt tgctagcaaa attccccctt gtcacagaaa 1140
ccagggactc ttcaggattt gagatggcct tgagtatttt agttgataca ttcttctgcc 1200
cattataatt ctcacctgaa gttatgggga ttgcacgggt tttggcactt tagaaaaagc 1260
ctgatgtggg tcttacataa atgaatgtct gtataagaaa atggactctt ttttttaggg 1320
aaaaataaaa gcaactatgg gaaaaaaaaa aaaaaaaaaa aaaaaagggc ggccgc 1376

<210> 34
<211> 1220

<212> DNA
<213> Homo sapiens

<220>
<221> SITE
<222> (803)
<223> n equals a,t,g, or c

<400> 34
   ggagaagtgc ctaacatccc atgtgtgcta aggtggcaga tcctgagctc tccagctgcc 60
   ctcactgtgg cctcacggca caaccagggc cagagtcagg aaacatcagt cactcgctta 120
   gggaagggag tccgagaacc ctcttcgtgg actcaacttc ccaggcttct gtccctgctg 180
   cagaatgccc aggccacaga gaagggaccc ccttttcagg agcttccacg tcacaggctt 240
   ttatggggcc tttcttgctt gtgtttctgt ttcccatcct gagggtgtgt ggaataatac 300
   gagagcctac ccaggactgg agcgtgttat tagaaagagc acgcttgact gctcccgggc 360
   agccacctgc cctttttccc ttggaaagtg gacctatggc cactgcacaa aacacttcct 420
   aataccaggc tgctggtgac tacattttgt ggtttctttg ttagttgata tttatagttc 480
   agatctcatc actctgccaa gtgcaataaa cattttgttt taactggaat ataactttta 540
   ttattgttat tatttttctt ttaaatgtct taaaagtgac cagcttgtgc aagtggagtc 600
   agaaacccta aatagtctaa atgccaagtc agtgcgctct gtagatcagt gtacgattag 660
   aagacaggct ctcatttatt actgcttgta tttatataat ggaagttagg taacataagc 720
   ccctctgcag gccagcttag atgttgatga tgcttaaatg gtaaaatctg tgagaatgtt 780
   caacaatgcc ctgtatttgt acngcctaaa actgaataat acatatgtga catgaataaa 840
   taggtcagtg atttttatga catattaatt tcctttacct aagttcctgg gaaatatagc 900
   tgaacagtta cagagaatga aaacaaacac tggcatctta caaacacctg ctttgattat 960
   ttagtctaat taggttaaat ttaactattt ccattttaac aaattgaaaa caattcttat 1020
   ctgttcctcg gtcatgcact ttatcagtgt tggatgcaga tgtgtttaat atgacacagt 1080
   tggtgctgtt ttctcagctc tgggtgggaa ataaaaagga attgagaaca tatttttaga 1140
   gtcttctggg gactttgtat ttctgggcct atagctatga aattttaagg aggaggaaaa 1200
   aaaaaaaaaa aaaactcgta 1220

<210> 35
<211> 1346
<212> DNA
<213> Homo sapiens

<220>
<221> SITE
<222> (537)
<223> n equals a,t,g, or c

<220>
<221> SITE
<222> (880)
<223> n equals a,t,g, or c

<220>
<221> SITE
<222> (1115)
<223> n equals a,t,g, or c

<400> 35
ggcagaggct tgtgaagggt aaagtttaaa cccctctgct tagcccctgc ctccagcctc 60
tgccaggagt aatgtgctcc catagtactc tgatccactt gtatttggtg cttccttttt 120
tttttctttt ccttccttcc tcctttcctt tcccttccty ttcctsttcc tccattcttc 180
cctccctccg tcttcctcca ttcttccctc cctccctatt cctccattct tccctccctc 240
cctccctctc acatccttta ggactcagca tcacctcctc taggcagtct ttcctggayt 300

accaccaytt atgcacaaaa cacctaagca ytaccttatg tggcctcatt tatcactgct 360
taaatatttt ttawacacgt gctgtgatgt ggcacatgca ggtgtcattc ttgakgatcc 420
actggttatt gccttgaggg gatgacaact gcccggtagg gtwacctggt gtgactgcac 480
ctaaaacagc aataccaaag ggcccattgc cagttctgtc actgaccagc tggggcntct 540
gagtatatcc cttaaccact ttggacctta atttggcatc tgtcaaatga gatggtggaa 600
cttgaggaac tctaaggccc ctactgtgca ggtcttatta atgattacaa cagcagcagc 660
agccagtgtt tactgaggac ttacaaagca ccaagcactt tgcctatcct aatccttaca 720
tcaactctac gaagttagta tggttactat ccctatttta cagatgagga gactaaggct 780
aagagaggtt atatgacttg accacaaggt cataataaag aaacagattt gaatccaggc 840
attctgactt tactgttctt agccacataa tgggcacasn ttygacacac rgttttgtgt 900
actgtttggt ggtcactcac agactccatc ccagactctg catgaaccat ccctgttcta 960
catttttaag gctcaaactg gagtctgggt gaaacctggg gacagaagac tgctatagtc 1020
acaattatta gagggaaatg ggtgaggacc agtggccagc tctgttcatg aacctttgac 1080
aattctcaca gagagtcttg ctttggacag agacnactta cgttgctgtt ttcagttacc 1140
ctctttagga ggggagagta ggcctgagtc atgcttcaga cacagattaa aatcagattt 1200
ggtaccaggt gcagtggttc acgcctgtaa tcccagcact ttgggaggct gagttaggag 1260
tatcacttga ggccagaagt ttgagagcag cctgggcgac atagtgagac atcctctctc 1320
tttaaaaaaa aaaaaaaaaa actcga 1346

<210> 36
<211> 1026
<212> DNA
<213> Homo sapiens

<400> 36
ggcacgagcc tctgtcacca aacagtatga agtatctaaa caatgtttta aaacaaatta 60
tttgtagcct tcactgttgg ctgtctccat ggcaggtgag ctgcgacctt ttgtcagcag 120
cctagctgag atggctgctg atgcctgcag gtataagtga ctgtcaattt tccttactca 180
tttatcttgc tgtctcatgt ttaaactaga agaagttgtt catctcaagt gtcctcaatg 240
tcaatctatc atgtttgcct aattttgctc ttgtacatca catctcacag tcaccagaac 300
atgagtagct gtctccaggt gcctctgtct ctgttatctt gcccactgaa gggagagcac 360
ttaagccaat ttgcaggaga ccacagtttg ccagaggtca gagacagaaa tcaccactgc 420
attttgttta aagaatcaca tcagaaaaga aaataaagga cagggaggga aaagaaggga 480
aacctaataa acacgccggt cctaaagttt gattccaaga tttatgacag aatcaggcaa 540
aactaaatta aaataatatc tgtgaaaact ggacaacctg aacataagtt gatttttcca 600
gagaccaaag aacaaatcat tgcacaaaca catacctttt caaactgaaa atgattccag 660
agttaacttc atggacctaa atatgaatat taacatctca caaatactat ttgtaatttt 720
atccttgagc agtatagtgg agaggtgact tctaagcaaa ttattagtag gaagtaaggt 780
taagtgggca tattccattt ttacatcctt tcttctccat ttatttactt cctatatgtt 840
taggggcttc caccaccctt atcctctgaa ataacactag agcttttgcc atttcctcca 900
cccaaagctt ctcagatgtt ggaccagcaa ggcgatcaag tttgttgttt gtttgtttat 960
ttgtttgttt gtttgtttga gactgggtct cactgtcacc caggttggag tcagagtggc 1020
tgcgta 1026

<210> 37
<211> 832
<212> DNA
<213> Homo sapiens

<400> 37
gctataacca accatgctag caaacttcac actgttcatt ctgactctaa tatcttttct 60
gctgttagtc tgttctccgt gtaaacatct caagatgatg cagctccatg gcaaaggatc 120
tcaagatctc agcaccaagg tccatataaa acctttgcaa actgttatct ccttccttat 180
gttatttgcc atttactttc tgtgtataat cacatcaact tggaatccta ggacacagca 240
gagcaatctt gtattcctgc tttaccaaac tcttgcaatc atgtatcctt cattccactc 300
attcatcctg attatgagaa gtaggaagct aaaacagacc tctctttcag ttttgtgtca 360

ggtgacgtgc tgggtgaaat aacagaaacc ctcaactcca tagattcaca aggggagcat 420
ggtgtgtctt ttagcagaaa agaaactgat ggtgtctaga acgttttata tttctgtcag 480
tttgttgtag tgtatgtatt tgagtaattt caaaacagat tcctaggata gtcttttata 540
tatatataat atatataaaa ttcatatata tataaaatac gtatgggtgt atatgtgtgc 600
atgtgtgtga ataataacat tgaccataaa ttatgaagcc tagtatattt catatatata 660
agtatgtgta ttttatgata gctaattgta tgatatttca tttgaagaat ttatctctct 720
ttgtaattaa gaaattacag catttatcag aaaatcattg ctgttttcca ttgtaatttg 780
taccacatac atgtacttaa ctatcaaaaa aaaaaaaaaa aaaagggcgg cc 832

<210> 38
<211> 706
<212> DNA
<213> Homo sapiens

<400> 38
ggcagaggtg acaagccccg ccaagacaga cctgcaagtc ttcgtctcaa gggacctccc 60
tcatgccagg cccctgcctc tcacagcagc accctttcct ctcattgtcc ctgttccctt 120
tttgcctgtg gatctgtttg gccagggtcc ctggggtcag gaatatttgc aagactcagc 180
cagctccttc ccagcccagc ctcttggggc tgggactttc tcaccctgcg gcaggcacaa 240
cagatgctgg gacccagtct ctgcccaggt cacagcacaa gtgcacatca gcactatggg 300
gcctatgtcc tgcccagaga cctctgctcc ttcctgctca catccacagt tcagggcacg 360
gcgcccctca agaactccag agtcacctgt ctcatcggct cccagcaagt gcctctttgt 420
ctatgatgtc cccctctctct gaggcctgga cccacccatc tttgtccctg gggcctgctc 480
ccagccactg aggcccgctc tggccagggg agaaggagct gccgtgcgtc ttccctgtgc 540
cccgtctccc tgcttggttc tcccctccct tccctggccg gctgccatgg ccaggagcta 600
agtgcctttt tgtgtgcaac cacttaccct ttctctgaaa aacctgttct caggaaggat 660
ctgataaact catttactct yaaaaaaaaa aaaaaaaaaa aaaaaa 706

<210> 39
<211> 1347
<212> DNA
<213> Homo sapiens

<220>
<221> SITE
<222> (83)
<223> n equals a,t,g, or c

<220>
<221> SITE
<222> (334)
<223> n equals a,t,g, or c

<400> 39
gggcagccct caggccctcc ggcagcctgg ccgggcccga gtggccatgg cagcactggt 60
gtggctgctg gcgggagcac atngtcaagc ctcaacaagt ggatcttcac agtgcacggc 120
tttgggcggc ccctgctgct gtcggccctg cacatgctgg tggcagccct ggcatgccac 180
cggggggcac ggcgccccat gccaggcggc actcgctgcc gagtcctact gctcagtctc 240
acctttggca cgtccatggc ctgcggcaac gtgggcctaa ggctgtgccc ctggacctgg 300
cacaactggt tactaccacc acacctctgt tcancctggc cctgtcggcg ctgctgctgg 360
gccgccgcca ccacccactt cagttggccg ccatgggtcc gctctgcctg ggggccgcct 420
gcagcctggc tggagagttc cggacacccc ctaccggctg tggcttcctg ctcgcagcca 480
cctgcctccg cggactcaag tcggttcagc aaagtgccct gctgcaggag gagaggctgg 540
acgcggtgac cctgctttac gccacctcgc tgcccagctt ctgcctgctg gcgggtgcag 600
ccctggtgct ggaggctggc gttgccccac cgcccactgc tggcgactct cgcctctggg 660
cctgcatcct gctcagctgc ctcctgtctg ttctctataa cctggccagc ttctccctgc 720

tggccctcac ctctgccctc accgtccacg tcctgggcaa cctcaccgtg gtgggcaacc 780
tcatcctgtc ccggctgttg tttggcagcc gcctcagtgc cctcagctac gtgggcatcg 840
cactcactct ttcaggaatg ttcctttacc acaactgcga rttcgtggcc tcctgggctg 900
cccgtcgggg gctgtggcgg agggaccagc ccagcaaggg tctttgagac ctgggggatc 960
tcaggagcca cctgggatgg ccctggcctg aatccagcct ccgctgtggc catagaagga 1020
atggagaaca gggctgggca tggtsgctca cgcctataat cccagcactt ccagagtccg 1080
aggtgggtgg atcacctgag gccaggagtt cgagaccagc ctggctagca tggcaaaacc 1140
tcatctctac taaaaataga aaaattagct gggcatggtg gcgcgtgcct atagtcccag 1200
ctacatggga ggctgaggtg ggaggatcac ttgagccctg gagatcgagg ctgcagtaag 1260
ccaagatcgc atgctactgc actccagcct gggagacaga gcgagacgct gtctcaatta 1320
aaaaaaaaaa aaaaaaccgg cacgtag 1347

<210> 40
<211> 1467
<212> DNA
<213> Homo sapiens

<400> 40
   gaattcggca cgagcagagc aagactccaa ctcaaaaaaa ataaaaagaa agaaagaaac 60
   atccctggca ccctgctctc catcatgtct gtgtgtttac cgctgcacct ccctttcctg 120
   atgctagcaa aggtagccac cagcttttgc agatggcagc tcacactatt tgtgtccact 180
   ttttacaaag atgccctggt tcatactgtc aatgacagaa atcaggaagc agaattggaa 240
   gcactcaaaa agtcctgttg acagtgcaca tcccctcatg ccctggaata tcacacagag 300
   agctttgcag tgagctggaa agtcctccag actcaaggcc gggcctgggs ccacaacaga 360
   ctttgacagc cctgcagtyc cgggctcacc agggaargga gccaggagtt cagcccccgc 420
   agcggcccct gctcacagca caggcaaaag ccacagccac acaggatggg gcctccctac 480
   ttcagcccaa ttgtgatttc ctgccttgca aatcatctct cttgaagctc ttcagctcct 540
   cttcatttct tacaccagca ccagcagctg gccttcttct tgagccaatt cccagaaagc 600
   aatargctga gtgtcacttt ttcagggttg ctgagagctg cccatggggg gaggcgggac 660
   aatagcagca gcrgggttac ccagcaamsc aactccctcc caagcaccag ccctgtggca 720
   ggtggacttc aaagttacct ccctgaggaa agcacaaatc atgtgctgga cctttgacat 780
   cctccccgag ggtcacacac tgacaattac actctgggta taggatccac cccgctggga 840
   ctggctaggc accctgcaga atcccaagag ggccctgccg acagctagga cccgccaagc 900
   ccattagacc tcctagcacc ttccactgtc cctgtttcct ttggtttttg ttgtgttttt 960
   tgttttgtag agataagtat cacttggtcg cccaggctgg agtgcagtgg tacaatcatg 1020
   gtacactgca acctcaaact cctgggctca agtgatcctc cacctcggcc tcccaaaatg 1080
   ctgagattac aggcaccatg cctgggtcat attttttttt ttttgagatg atgtttcact 1140
   ctcgttgccc aggctggagt acaatggcgc gatctcggct cactgcaacc tctgcctccc 1200
   gggttcaagc gattcgcctg cctcagcctc ccaagtagct gggattacag gcacccacca 1260
   ccatgcctgg ttattttttg tatttttagt agagacatag tttcaccatg ttggctgggc 1320
   tggtcttgaa ctcctgacct caggtgatct acccaccctt tagcccccaa agtgctggga 1380
   ttacaggtgt gagccactgt gcccagccaa tttaaaaaaa tttttttaag agatagggtc 1440
   tcactatgtt gcccaggctg gtctcga 1467

<210> 41
<211> 914
<212> DNA
<213> Homo sapiens

<400> 41
ggcacgagtg aaaatctact ctatcagctg ctgtggttgc caccattctc aggaccctcg 60
ccatgaaagc ccttatgctg ctcaccctgt ctgttctgct ctgctgggtc tcagctgaca 120
ttcgctgtca ctcctgctac aaggtccctg tgctgggctg tgtggaccgg cagtcctgcc 180
gcctggagcc aggacagcaa tgcctgacaa cacatgcata ccttggtaag atgtgggttt 240
tctccaatct gcgctgtggc acaccagaag agccctgtca ggaggccttc aaccaaacca 300
accgtaagct gggtctgaca tataacacca cctgctgcaa caaggacaac tgcaacagcg 360

caggaccccg gcccactcca gccctgggcc ttgtcttcct tacctccttg gctggccttg 420
gcctctggct gctgcactga gactcattcc attggctgcc cctcctccca cctgccttgg 480
cctgagcctc tctccctgtg tctctgtatc ccctggcttt acagaatcgt ctctccctag 540
ctcccatttc tttaattaaa cactgttccg agtggtctcc tcatccatcc ttcccacctc 600
acacccttca ctctcctttt tctgggtccc ttcccacttc cttccaggac ctccattggc 660
tcctagaagg gctccccact ttgcttccta tactctgctg tcccctactt gaggagggat 720
tgggatctgg gcctgaaatg gggcttctgt gttgtcccca gtgaaggctc ccacaaggac 780
ctgatgacct cactgtacag agctgactcc ccaaatccag gctcccatat gtaccccatc 840
ccccatactc acctctttcc attttgagta ataaatgtct gagtctgaaa aaaaaaaaaa 900
aaaaaaaaaa aaaa 914

<210> 42
<211> 1131
<212> DNA
<213> Homo sapiens

<400> 42
ggcacgagga ttcttctttt atatactgat tacaagactg acacctatca agtatgatgt 60
gaatctgatt ctgacagctg tcactggaag cgtcggtgga atgttcttgg tagctgtgtg 120
gtggcgattt ggaatcctct cgatctgcat gctctgtgtt ggactagtgc tggggttcct 180
catctcgtca gtgactttct ttactccact gggaaaccta aagatttttc atgatgatgg 240
tgtattctgg gtcactttct cttgcatagc tatcctcatt ccagtagttt tcatgggctg 300
cctaagaata ctgaacatac tgacttgtgg ragtcattgg gctcctattc ggtggtttta 360
agccattgac agttactggt ccacaagcct ttcctacatc actttgaacg tactcaagag 420
agcgctcaac aaggratttc cacagagctt tcacaaatgt gccttttcaa actaatgact 480
tcattatcct ggcagtatgg ggcatgctgg ctgtaagtgg aattacgtta cagattcgaa 540
gagagagagg acgaccgttc ttccctcccc acccatacaa gttatggaag caagagagag 600
agcgccgagt gacaaacatt ctggacccta gctaccacat tcctccattg agagagaggc 660
tctatggccg attaacccag attaaagggc tcttccagaa ggagcagcca gctggagaga 720
gaacgccttt gcttctgtag atgcccaggg gcttggtcag tgtgcctcag ctttggagtt 780
catgcctgga gtggttcaac agtctctggt gcaagtctaa taagagatca ggcatatata 840
tctgttcttt gcataatatt atggtgccct tattgatata tggtaagggt gtactagggg 900
attaggatga ttgtaagaga atgagaaaga tgaccaaaag gttggtggta gggaggcttt 960
ttcttatttc caaatacttg agaaattacc ttttggttta caaatctatg atcaacttat 1020
tccattaaat agatacatta aaaaaattaa aaactgaatt cttctgtcag aaaaaaaaaa 1080
agaaaaaaaa aaaaaaaaaa aaaaaaaaaa aaaaaaaaaa aaaaaaaaaa a 1131

<210> 43
<211> 1333
<212> DNA
<213> Homo sapiens

<220>
<221> SITE
<222> (411)
<223> n equals a,t,g, or c

<220>
<221> SITE
<222> (1264)
<223> n equals a,t,g, or c

<220>
<221> SITE
<222> (1319)
<223> n equals a,t,g, or c

<400> 43
cctggaacac ttcaacaacc agtatccagc cgcagaggtg gtgaactttg gcacctggtt 60
cctcttcagc ttccccatat ccctcatcat gctggtggtc agctggttct ggatgcactg 120
gctgttcctg ggctgcaatt ttaaagagac ctgctctctg agcaagaaga agaagaccaa 180
aagggaacag ttgtcagaga agagsmtcca agaagaatat gaaaaactgg gagacattag 240
ctacccagaa atggtgactg gwtttttctt catcctgatg accgtactgt ggtttamccg 300
ggagcctggc tttgtccctg gctgggattc tttctttgaa aagaaaggct accgtactga 360
tgccacagtc tctgtcttcc ttggcttcct cctcttcctc attccagcga nagaagccct 420
gctttgggaa aaagaatgat ggagagaacc aggagcactc actkgggacc gagcccatca 480
tcacgtggaa ggacttccag aagaccatgc cctgggagat tgtcattctg gttgggggag 540
gctatgctct ggcttctggt agcaagagct ctggcctctc tacatggatt gggaaccaga 600
tgttgtccct gagcagcctc ccaccgtggg ctgtcaccct gctggcatgc atcctcgtgt 660
ccattgtcac tgagtttgtg agcaacccag caaccatcac catcttcctg cccatcctgt 720
gcagcctgtc tgaaacgctg cacattaacc ccctctacac cctgatccca gtcaccatgt 780
gcatctcctt tgcagtgatg ctgcctgtgg gcaatccccc taatgccatc gtcttcagct 840
atgggcactg ccagatcaaa gatatggtga aagctggcct gggagtcaac gttattggac 900
tggtgatagt aatggtggcc atcaacacct ggggagttag cctcttccac ctggacactt 960
acccagcatg ggcgagggtc agcaacatca ctgatcaagc ctaacgccaa gtgtacaaac 1020
tggcccaacc acaggagctg ccagtatcca gcagtatctg gaccacaggc aaagaaaacc 1080
actaggacca ccaggagcac acaaccccag acccacgccg gagggcatcc ctccaccaga 1140
agattccgcc acctcaagtg aactgcagga atcctccaac aaccacaaac acatgcttcg 1200
ctgttagtgt cttcttcctg ccctcagcac cacagctcaa gaaaacctaa agtttcaata 1260
caanccatag gctcacaaaa aaaaaaaaaa aaaaaaaaaa aaaaaaaaaa aaaaaaaana 1320
aaaaaaaaaa aaa 1333

<210> 44
<211> 1004
<212> DNA
<213> Homo sapiens

<400> 44
gcttcactgg agttctaagt tttctcccct ctgttttgaa tgagtcagct ctgcttctca 60
ctactgcttt cttccacatg ccacggaggg gttgccagcc tcttgacctc agaccttagc 120
tctcagtccc atcgtttctc catctgcact aatgtgaatc actctaagta ttctagtctc 180
tgatgtgttt tgaaggcaga agcagtcaga gggcactgct caccaggctg ggctgggcag 240
gcagatcaca cggaagccct gccctgtcac agttgttaat actgcaggga gatggtgggg 300
agacactatg gggaacttga ggagtcatgg ttcacaatgt acttctaaac cactgtgagt 360
ttttttgctc ttgtctttgg aatataatac tttattgctg ggggataatg agtatttact 420
ttaaaaaaca gatgcatttc taagtccctc tgttttgtct tgacttccag ctccccaaca 480
tactcacatt ccactactta ttctctattt taactttact gcttctttta ctttttttta 540
gttttacttt tattttttat ttttttgaga cagartcttg ctctgtcaca caggctggag 600
tgcaatgacg cgattttggc tcactgcaag ctccgcctcc caggttcatg tcattctcct 660
gcctcagcct cccaagtagc tgggactaca ggtgcccgcc accacgccct gctaattttt 720
tgtattttta gccgargcag gtggatcacc tgargtcagc agtttgaaac cagcctggcc 780
aacatggcga aaccccatct ctactaaaaa tacaaaatta gcagggcgtg gtggtgcact 840
cctgtaatcc tagctacttg ggaggctgag acaggagaat cacttgaacc caggagccag 900
aagtcgcagt gagccgtgat catgccattg caccccagcc tgggcaaaaa gagcgaaact 960
ccatctcaaa aaataaaaac aaaaaaaaaa aaaaaaaact cgag 1004

<210> 45
<211> 1494
<212> DNA
<213> Homo sapiens

<400> 45

gtcgacccac gcgtccggcg gcggcaggcg cgggcgaggg ccacggggag aggagacgca 60
gccccgcggg tggcacgctc ggccgggccc cggcccgcgc tcaacgggcg cgatgctctt 120
ctcgctccgg gagctggtgc agtggctagg cttcgccacc ttcgagatct tcgtgcacct 180
gctggccctg ttggtgttct ctgtgctgct ggcactgcgt gtggatggcc tggtcccggg 240
cctctcctgg tggaacgtgt tcgtgccttt cttcgccgct gacgggctca gcacctactt 300
caccaccatc gtgtccgtgc gcctcttcca ggatggagag aagcggctgg cggtgctccg 360
cyttttctgg gtacttacgg tcctgagtct caagttcgtc ttcgagatgc tgttgtgcca 420
gaagctggcg gagcagactc gggagctctg gttcggcctc attacgtccc cgctcttcat 480
tctcctgcag ctgctcatga tccgcgcctg tcgggtcaac tagcctcacc gaggtgccgg 540
agagggagcg ctggacaact agaatgttga cctcgagccg aggccctact tgcagcgcac 600
cggaggagag gctctctagt ctgaaggcac cgccggcttg cgccgagctg agtgccgggt 660
ttccctattc caatcctgtt tgaaatggtt tcttcagcag ggcttaaaag agcagccttc 720
atcctgaaaa tgtatttcct tttgtttaat gctttgagta gataatcctg aattgaggtc 780
atgaggaggc cccccaggcc agacagtcct gaacccctct gacacttgga aactgaatat 840
aagtaaaatg tccaggtgga ctctgagtat ttcctgtgga tcctgggaaa gtactgttgc 900
acaaaggctg caaagctgga ctcaggaatg tcctccaacc agcagcgctg acctaagagc 960
tccctgtgcc gtctatccag accagacttc ggtagatgcc tttgttagat ctatcacatg 1020
taaacgagct tgtatctcct tccctgtgcc acgagagaga ttggcttttt attccagtct 1080
aggcagagac agaagaatgt tgaataagag cacgattaga gtcctgtctg gttatctgtt 1140
gcccaagaaa agaactctgc tgtccaggca ctgcttggct tactatccca gcaaagactg 1200
cagttttgtg gacttttgac caccttgggc tggcactctt agcacacctg agacagattt 1260
aagcctccct aagagactga agagaggaac aggtgtcaga tactcatagg cactgagatc 1320
tacaaatggg aagcttgtga gtggcccatc tttgttggcc tacgaacttt ggtttgatgc 1380
cagtcaggtg ccacatgaga acctttgctg agatgcaaat aaagtaagag aatgttttcc 1440
tgaaaaaaaa aaaaaaaaaa aaaaaaaaaa aaaaaaaaaa aaaaaagggc ggcc 1494

<210> 46
<211> 1166
<212> DNA
<213> Homo sapiens

<400> 46
gagacctgcc catcgaatgg aattgagctt cgtcaggcgc ctacttctct ttacattctt 60
cttctccaca ttcagcccac ccccacccac cccatgcttg gaaggtctta tgtcctgcct 120
gccttctcca ytaayyaaga acacggcggg ctcccaaacc aaatccctaa gggagatagg 180
aacgggaata tcagacactc acgtatcacc ttcccctgct caagctccac tttgcagccg 240
gagtcccacc tgggattcat cagatccaaa ctccatggac tagttaggcc tggaaaagac 300
cttagaggta ggaggtcttt acagcttagc aaacattctt tgagtacctg ctatatgttg 360
aggtgggaga cttataaaca agtaagttac actgcagtgt gaaaatgaca gtgtagatga 420
taaagctgaa gcttaagcga tgctgacaca tagacttctg ttagctctag ggctttttcc 480
tgccttaact gacagacttt gacaaatctt ctaggactgt gtgaggaatg aatatagatg 540
atcgaaaggg gaattctttt aagacttgtg aaagaagcta cattggagaa ctgaccttcc 600
catgtactga ggacggcctg tgtgtgtgta tgtgtgcaca tgggcatgtg cacacatgtg 660
tgtaggaacc tgtgtacatt tcttttatca ttctcctgat gcctttgtta gtgtttctgg 720
ctccctgcct gttcttaata aaatccacag tttatcttgt agcttracct ttcttgaggc 780
catgctacat ttcaattttg gtataataga aagatgttgt caaagctgat gggggctagg 840
tcctaagctc attgaagact ggaggctgtg ccttgttttc ctctacatcc cctaagcctt 900
ttgtacagtg tgtggcacct agagggtgct tactgtcagc actgaattgc gcccagtgcc 960
caccagggac ctgcacctct catcaggaat gactttggga ttgccagctg ttctatctgc 1020
tcctctcagc atgaacacta agcagagtgc cttgttttta ctgcacttgg gagccatatc 1080
ctgttgcctt tttggatgat tcgggggaaa ttccttgtct gtggttccgt atgtgttgca 1140
ggctgagggt tggcttgctc cctcga 1166

<210> 47
<211> 1536
<212> DNA

<213> Homo sapiens

<400> 47
ccacgcgtcc ggggcagaaa ggaaccgggt tgtcttgggc cgggcagggc gggtggtgac 60
tctcaaaagg aaataggatc atggcagcag atgatgacaa tggtgatgga acaagtttat 120
ttgatgtctt ttctgcttct cctcttaaga acaatgatga aggctcactg gacatatacg 180
ctgggttgga cagtgctgtt tctgacagcg cttccaaatc ctgtgtacca tcaagaaatt 240
gtttggactt atatgaagag atcctgactg aagaaggaac tgcaaaggag gcaacatata 300
atgatttgca agtagaatat ggaaaatgtc aactacaaat gaaagagctg atgaaaaagt 360
ttaaagaaat acagacacag aatttcagct taataaacga aaaccagtct cttaagaaga 420
atatttcagc acttatcaaa actgccagag tggaaataaa ccgcaaggat gaagaaataa 480
gtaatcttca ccaaaagatt gtcctgagtt tccacatttt cgaaataatc ataaaactgc 540
aaggacattt gatacagtta aaacaaaaga tcttaaatct agatctccac atttggatga 600
ttgttcaaag actgatcacc agagctaaaa gtgatgtttc taaagatgta catcatagca 660
cttcactgcc aaatctggaa aaggaaggaa aaccacattc tgataaaagg agtacttcac 720
atttacctac atctgttgag aaacactgca ctaatggtgt ttggtcacgt tctcattatc 780
aggttggcga gggtagctca aatgaggata gtagaagagg aagaaaagat attagacata 840
gccagtttaa cagaggaact gaaagagtac gaaaagactt aagtactggc tgtggtgatg 900
gtgaaccaag gatattggag gctagtcaaa ggctacaagg gacatcctga gaaatatggt 960
aaaggtgaac caaagactga aagcaaaagt tcgaagttta aaagtaactc agattctgac 1020
tataaaggtg aacgcattaa ctcttcttgg gagaaagaga cccctggaga aaggtcacac 1080
agtcgagtag actctcaaag tgacaaaaaa ctagaaagac aaagtgaaag atcacaaaat 1140
ataaatagga aagaagttaa atcacaagac aaagaagaaa gaaaagttga tcaaaaacct 1200
aaatcagtag taaaggacca agatcactgg agaagatctg aacgagcatc acttcctcat 1260
tccaagaatg aaataacatt ttctcataat tcaagtaaat accatctaga agagagaaga 1320
ggatgggaag attgtaaaag agacaagagt gtaaacagtc atagttttca agatggaaga 1380
tgtccatctt ctctttcaaa cagtagaact cacaaaaaca ttgactctaa ggaagttgat 1440
gccatgcatc agtgggaaaa tacaccttta aaagcagaaa gacatagaac tgaagataag 1500
aggaaaagag aacaagaaag caaaaaaaaa aaaaaa 1536

<210> 48
<211> 1038
<212> DNA
<213> Homo sapiens

<400> 48
ccacgcgtcc gagacattta aactagattc ccagtcctct ccttcaaaag cttggtcttt 60
gtttttccta tagggaaaaa agtcaaaata agttccaaaa actatcctca aagtagtatt 120
gtgcttgtag taaatgaagg ttggatggat ggatactgac aatggtggca ggcatttcaa 180
gccttttaaa ttagtacttt ttgtcgtctt gcttattaaa attttgttaa ttttagcaaa 240
gaccaattgt tgtgataaac tggtgttttt tggatgcttc aagcacacgt taaccaattt 300
tttaattccc cttttggttc ctcccattgt tctaaaatag gactttcata ttattaaaac 360
ctcaaaagat gatccaccca ggatgaacaa agatcaccaa ggggaaagaa aacatttttt 420
atctttacag aaaacatgtt aagattatat atagatgtat tctttacatt ggatattgta 480
ttagagtcct ccttacaaga aatgaaatag tttttagcac tcttagcatt agagttccta 540
gattggtgtt gatagctaca gttttaaaat gtataacctg aaaatgaagg ttaattttgc 600
attgtaagag cacatttgat ctatgtaaaa agtgtccatt tggtgtattt ttttaaaaaa 660
gagaaagcac tttcatatta agtagcatgt gtatgaattt agattttcat atttgttgtg 720
tctgtattca gtgaagtaaa ttgagcattt aaatgtttgt tgatggcaac attaactatt 780
aaattaaagc accttatact ctgctgctta acttgcttgt aattgcacct ttgttacctg 840
cacattttca tatagaatat tgttgtaaca ttgcttcatg tgggtctgga tggaagatta 900
gtgggcctac aggatcattt atttatattg tttatattac aataatatat tgtagatcag 960
ttgtaagttc atttctttac aaataaaagc ctcttccatt tgaaaaaaaa aaaaaaaaaa 1020
aaaaaaaaaa aaaaaaaa 1038

<210> 49

<211> 1176
<212> DNA
<213> Homo sapiens

<400> 49
ccacgcgtcc gaggacaaaa aacaggcctt ttaaaattct gttcagaacc agaagatgat 60
caagaaaggg aaatgcccac tagcagcttc tactgtaagg ttagagctga cagagaaaga 120
actccttaac tactatttgc ttcattctct acaaaggaaa ctagagaagt gggttgatgt 180
aatagaaaga acatgtgttt gtggggccag gcaaacctgg gtttaattct gtttcaacac 240
tgcttaacaa aatttatggg aggctattgt tttggattgg gctcctgcac caggcccctc 300
cgggaccaaa ccaaaatgga gtcactcata ctaaaactcc aggtcactga accaaaacta 360
agttgtttta tctgaccttc caagaaatca ggagggagaa aacaaccaaa tctccaaaca 420
ggccagtttt aatcagcgtg ataaggaagt cctctctttt ttaaccctat aaagaaagta 480
actttttgaa atgatcaata cactttgtat tccttagttc tgctttcttt agcccctttc 540
tgcctataaa gcccacttcc tctgctcaac ttactgaagc agtattccat tttatagaat 600
gagatgctgc ccaattctgg aatcactaat aaaagccaat tagatcttta catttgttga 660
aattttgtct ttgacaacat tactagttat attattctgg gtatcctttt ccccatctgt 720
aaaatggaca tagcgatatc cttcccatca gatttttctc attaatagaa gtaatacatt 780
caaaacactg agccaggctc acaccagtga gcaacttgtt aatattactc agaagcatat 840
tatagatatt gacagaaagc aatactgttc ctaattaggg gaagaaattc taagagagta 900
cctaagagtt tgaatttaga ttataacatt tgctctgagt attttacatt acagcctttg 960
gggggaaaag tacaaatgag atctgagaac agtggtactc atctttgagg aattatggaa 1020
aacgtaatag aacactaaac atgggaaaac atcggccttc aggttgaaaa gtggaaatct 1080
caatccctga attttttttt ttttttacta agtaactttt ttgcccattg gtgtcattta 1140
accaaaagaa gaagaaattc caaaaaaaaa aaaaaa 1176

<210> 50
<211> 731
<212> DNA
<213> Homo sapiens

<400> 50
ccacgcgtcc gagacgccac ctgggcggac agccaggagc tctccatggc caggctgcct 60
gtgtgcatgt tccctgtctg gtgccccttt gcccgcctcc tgcaaacctc acagggtccc 120
cacacaacag tgccctccag aagcagcccc tcggaggcag aggaaggaaa atggggatgg 180
ctggggctct ctccatcctc cttttctcct tgccttcgca tggctggcct tcccctccaa 240
aacctccatt cccctgctgc cagccccttt gccatagcct gattttgggg aggaggaagg 300
ggcgatttga gggagaaggg gagaaagctt atggctgggt ctggtttctt cccttcccag 360
agggtcttac tgttccaggg tggccccagg gcaggcaggg gccacactat gcctgcgccc 420
tggtaaaggt gacccctgcc atttaccagc agccctggca tgttcctgcc ccacaggaat 480
agaatggagg gagctccaga aactttccat cccaaaggca gtctccgtgg ttgaagcaga 540
ctggattttt gctctgcccc tgaccccttg tccctctttg agggagggga gctatgctag 600
gactccaacc tcagggactc gggtggcctg cgctagcttc ttttgatact gaaacttttt 660
aaggtgggag ggtggcaagg gatgtgctta ataaatcaat tccaagcctc aaaaaaaaaa 720
aaaaaaaaaa a 731

<210> 51
<211> 1437
<212> DNA
<213> Homo sapiens

<400> 51
cgcccgacgc cggaactgcg agctctcagc gggagccgag acggtgcagg gccggagaag 60
caccttcact cccagcctgc gccccgatgc tgcgcgttct gtgcctcctg cgcccctgga 120
ggccccttcg ggcccgcggc tgcgcttccg acggggcggc cgggggctca gagatccaag 180
tgcgcgccct ggcgggtccg gaccaaggga tcactgagat tctgatgaac agaccttctg 240

cccgcaatgc cttggggaat gtcttcgtca gtgagctgct ggaaactctg gcccagctgc 300
gggaggaccg gcaagtgcgt gtcctgctct tcagaagtgg agtgaagggc gtgttctgtg 360
caggtgcaga cctgaaggag cgggaacaga tgagtgaagc agaggtgggg gtgtttgtcc 420
agcgactccg gggcctgatg aatgacatcg cagccttccc tgcacccacc attgcggcta 480
tggatgggtt tgccttgggc ggaggcctag agcttgccct ggcctgtgac ctccgagtgg 540
cagcttcctc ggcagtcatg ggactgattg agaccacgcg agggctcctc ccgggggcag 600
gagggactca raggctgccc cgttgtctgg gggtggccct ggcgaaggag ctcatcttca 660
cgggccgacg actgagtgga actgaggccc acgtactggg gctggtgaat cacgctgtgg 720
cccagaacga ggagggggac gccgcctacc agcgggcacg agcactggcc caggagatcc 780
tgccccaggc ccccattgcc gtgcggctgg gcaaagtagc cattgaccga ggaacggagg 840
tggacattgc atctgggatg gccattgaag ggatgtgcta tgcccagaat attccaaccc 900
gggaccggct agagggcatg gcagccttca gggagaagcg gactcccaaa tttgttggca 960
aatgaccccc attttaacct tcagcatggg agatgcatgc cctgaagagc aggatccaga 1020
aggaagattt gtggccagat tgccttcatc atttcacctc tccagacttc catttcttca 1080
caaggatgat gatggaaata aaatgactgg cgtgatgcct ggaaccaagg tgctgatcct 1140
accacctact gctaccttcc ttagcttcac cctggctaga aataatcacg agggttgggt 1200
ttgctttgga aaatgcctgt ctctctactt gaatgataaa gaattaaatt agatctctct 1260
gagtcttggt atcattggct ctcagcccct gacctctctc agttatcagg cactcattag 1320
agatgtcaga agattttaag atacccctag tttcttcctg tggaacaaca gaggtaataa 1380
ataaactctg gacatcggtt gaaccagtgt caggggtcag actgcagatc ccagtct 1437

<210> 52
<211> 1369
<212> DNA
<213> Homo sapiens

<220>
<221> SITE
<222> (3)
<223> n equals a,t,g, or c

<400> 52
agncagacgt agaacgtagt ggatccccag gggtgcagga attcggcacg agatttgata 60
cccagtgcca tattgtccct aagaaaggtt gcacaaattt acactcccac caagagtgtg 120
ggagagccat ttgccacata gactcaccag tatttttttt ttaattttg atccatttgg 180
aagttatttg ggaacttggg tgtttttccc caaaagcaaa ggcaattgcc tcaacaccag 240
ttatcaaagg atccctacag atctattttc cctgtagatc tgaaatgctg tcttcattgt 300
atcttttgct gatgcccccg tacaaattta caggtgagct ccatcctcct gtagcagcca 360
cctgcctact gacagtccta ctcgggtgtc tgataggtgt ctcaagtgat ggatggatat 420
gacagtagag tccttgattt actgtcccat ggcccctgct catcttctct ttcttgattg 480
atggtgccat catctaccca gttactttga ccaaagcaat gggagtcatc ctggattcct 540
tkctttytct ccacttctaa gccatcagta tactggcact gtgttaaagc catatttcaa 600
accagaccac ttgtcaccat tcctgtcact tctacctctt ttatcccaac atcacctctt 660
gggtagacca ttgcaaactg gaagtgcatt tccagaatat tcttgttggt atagaaccct 720
ctgtctacat agtagctaga gcagtttttt ttttttttag atgttaaaca gatacatcgc 780
tctgctaact aaaacccttt aaagtgtttt ccatctcaat tagaatagaa ttcatagtcc 840
tcaccagcca ctgcaaggtt tatataatct agcccctgcc tatcttcctt gcctcttctc 900
tgttacccac aacctgcttt ttgtttcatg atgtgtgaac tcatttcaac cttagggtcc 960
tgctcttgcc tcttcctttg cctagaatgc ttttcccttg tcctaaatca tctgtgttat 1020
gctagttttt argtctcaac tcatgtcacc ccgttgscct ttatctcatt gtctggcttt 1080
attttctcta aaacacttgg cactatgtar atgttctatt tatttactta ttttaagggt 1140
agaatcttta tctgttttgc ttggtgccaa ttattcaaca tgttgaatag tgcctggcac 1200
ctagcaggca ctggagccta tttctggaat ttcatgttgc accattgccc tctctgtttg 1260
ttctccatta ctaaattcct ttcaagccaa ccccatggcc tccatgactt tttcaaaaaa 1320
aaaaaaaaaa aaaaaaaaaa aaaaaaaaaa aaaaaaaaaa aaaactcga 1369

<210> 53
<211> 1037
<212> DNA
<213> Homo sapiens

<400> 53
ggcacgagct cgtgccraat tcggcacgag ggtcatagtc cacagaggta aaagttaaca 60
attctgatgc tcttgtatgt gcataccaga ggctctaggg aagaattccc tctttctttc 120
ttccaccttc ttgtggctgc tggcattctt tggcttgtgg tcacatcact cctatcttga 180
aggccagcat cttcaaatct gtttcttctt cacatagcct tctgtgtgtg cagtgccctc 240
tacctctctc ttataaagac atttgtgatt aaatggaggg tttaggataa tctcgtcaag 300
atccttaact taatcacaac tgcaaaaacc tctttcccaa ataaggtaac attcacaggt 360
tccagggatt aggacctatt atctttggta agtattattc agcctaccac aatagctaaa 420
acaattctga aaaagaagaa taaagtgaga gaaatcagtt tatctgattt cgatacttat 480
tgtatagcta tggtaaataa ggctgcatgg tattaaagaa aggacatata tgaatgaaac 540
agaatagagg acccagaaat agacccacac aaaggagccc aaattatttt taaccaaggt 600
agaagacaat ttattggagg aaagacagcc ttttcaacaa atggtactat aacaattaga 660
tatccatagg caaaaaaaaa aaaaagaatc ttgatctaag gctcacacct tatataaaat 720
aatattaaac tcatggccag gcacagtgac tcatgcctat aatcccaata cactgggagg 780
ctgaggcaag agtatcactt gaggccaggg gttcaagact agcctgggca acacagtgaa 840
actctatctc tacaaaaaaa ttataaacta gctgggcatg gtggcacatg cctgtagtca 900
caactactca cgaggctgag aagatcactt aagctgagtt gttcaaggtt ctaatgagct 960
acaatcgtgc cactgcactc cagcctaggt gacagacaaa gaccccatct caaaaaaaaa 1020
aaaaaaaaaa actcgta 1037

<210> 54
<211> 1373
<212> DNA
<213> Homo sapiens

<400> 54
ggcacgagag caaagagaag tgtccaccag gcccgctgca ccaacgctgc gtctttaaca 60
gctctggggc tgggcgcgtc atggctacga gaaagcgcat gaccacttcc ctgtttggtt 120
tggtttgtgt tgtgtgtcag ggcgcagggg tttctgcttt cactcaagtt aatttatttt 180
ccttttcctt ggtaattgtg aaaaaacaaa ataaaacctc ctgtgagcct tttggaactt 240
ctggaaaagt ccctttgctg tgarcsgctg actctgagaa gagctttgag cagggctgga 300
aaccattttt ctgcaacctt ttctttcctg ggtatgtctt gggtgcacac aggctcccca 360
caaggcaaag gctgtccctg gatggttggc aaaatgcgcc acaccagagt gggtttgtgt 420
tggcaggagg catgaraaaa ccttgctgat ggcaggggag gacggcgaca ccacgatggg 480
aacaaaatcc tcctccttac ytctaattac aaagaggaaa aagtcactga aaaaaaaagt 540
ttaaaatgtc ttaatataag agtcatatat aatccaaagc taccaaaggc caagtgttta 600
gggggaagtt tctggtggtt aaccccactt caggggggatt taaagtggtt gtggtgagga 660
tttggttcca ggtatgcgtc ctgccaacct gggtgggtgt tccctttggt ggagcctctt 720
gaaaaatgar ggartggctg ggtgcagtgg ctcatacctg taatcccasc actttgggag 780
gccgaggcgg ccagatcacc tgaggccagg agttcaaaac cagcctggcc aacatggtga 840
aacttcatct ytacaaatat acaaaaatta gctaggcatg atggcaggct cctgtaatcc 900
cagctacttg ggacgctgaa scaggagaat ctcttgaacc caggagtagg aggttscagt 960
gagctgagat tgtgccactg cattcccgtc tgggcgacag agcaagactc catctcaaaa 1020
aaaaaaaaaa aaaaaaagag ggagtggctg ggtgcagtgg ctcatgcctg tatcccagca 1080
tattgggagg ccaaggaggg aggattgctt gagcccagga gttccagatc agcctgggca 1140
atctsscaaa acccgggcag tacaaaatcc ataaaaatta gcggggcatg gtggtgcgcg 1200
cctgtagttc cagctacttg ggaggctaag gttggagagt tacttgagcc taggaggttg 1260
aggctgcagt tagccatgat tgtacctctg tacgccagcc tgggtgacaa agcaagagcc 1320
tgtctcaaaa ccaaaaccaa aacaaaaaac aaaacaaaaa aaaaaaactc gta 1373

<210> 55

<211> 1347
<212> DNA
<213> Homo sapiens

<400> 55
ggcacgagct ggtctatagt gaagttactg gccattccaa aggctatgga tttgtggaat 60
acatgaaaaa ggactttgct gcaaaggcta gactggagct attgggtaga cagttgggag 120
catcagcact ctttgcacaa tggatggatg ttaatctatt ggcttcagag ctcattcatt 180
ctaagtgcct ttgtattgat aaactcccca gtgactacag ggattcagaa gagctgttgc 240
aaattttttc cagtgtccat aaacctgtgt tttgccagct tgcacaggat gaaggtagtt 300
acgttggtgg ctttgcagtg gttgaatata gcactgcgga gcagctgaag aggtccagca 360
gcagcagacg gtatgaccat caaggcagca aagtccaggt ttccttctgt gctcctggag 420
cgccagggcg aagtacatta gcagcattga tagcggctca acgtgtgatg cacagtaatc 480
aaaagggctt acttccagag ccaaatccag tacaaattat gaaaagttta aacaaccctg 540
ccatgttgca agttcttcta cagccccagt tatgtggacg agctgttaaa ccaggtatgg 600
accatgtatg tatactgatt aattaaagaa tgctagcatg aagttaattt tttcacatgt 660
gaaatatgga aaaatacatt gatttgtgaa aaatatattt aaattagtat aaaatattta 720
atttttctag gacctgttaa tgaattttaa caataatctg aggtgatata ttttgactat 780
ttttgacata tacaacctgc ttatgtggta ttttttacta ttaataaagc taaatatcaa 840
accttctgtt tagctcttag ttgacatgaa tttatagacc aaggtaaagg ttaacacact 900
acattgttat aacctatatt aacaaagaat taatactctc tatgtaatat ttttagcaga 960
attattttgt tgaaaagtgc caagtgtttg tttcctcttt gttcttcccc tttttgttgt 1020
aaaattgttt cactttgtag caaatgatga aaacattatt attttctaag tgttatgcaa 1080
atctttataa tatcagtata cattaaatat ctacctattt agtattcttt ctctagtaag 1140
agcttacctt ctgtgcattc tgaaatgtac aactttttat gtacaaaaat gtctgtttta 1200
gcattatgag gaaatgaatg cctatacagt ggtctcccct tatctatgtt cttgtgttcc 1260
acagtttagt tacctgcagt ctgaaaatat taagtggaaa attccaaaaa taaactactt 1320
ataggtttta aaaaaaaaaa aaaaaaa 1347

<210> 56
<211> 822
<212> DNA
<213> Homo sapiens

<400> 56
ccgggttgac ccacgcgtcc gcggacgcgt gggcaaatat tggtaatgct gggaaaaggg 60
agttcagaat gccaaaacgt ttctggtttt atttgtcttg ggtgaggacc cagaggggtg 120
ggagatggag gtgtgagcag catggtctgt tgtggttttt tcttgttgtg gagtagagtt 180
agatcataca tgaagctctc tgggcatagg tggagtagca gctgtccaca ccattgctat 240
tcaaagtgtg gtttgcacac cagtaatgga aaatcatctg tgcacactgt ttagtttaac 300
tgatactttt tttttcatag caagatttct taatgaagga agtaatgtat tgatttacat 360
tctgactcat tgtctttatc ttgtctttga tcagtttgta gactggcact ggtccacact 420
ttgaataaca ctattcttca ttctactttc catgtacccg gatgccaggc aaacagggag 480
ttttacgctg ggtggagaac ggaacattct gctgactcct tgaaagggct tatctcacca 540
ggcatggtag ctcacgactg taatcccagc tctttgggag gctgaggtgg gaggattgct 600
tgagctcagg agtttgagac cagcctgggt aacataggga taccttgtcc ctacttaaaa 660
aaaaaaaaaa aaaaattagc tgggtgtggt tgtgcacacc tgtagttcca gctattccar 720
aggctgaggc aggaggatag gttgagcatg ggargttgag gctgcartgt gccttgatgg 780
cgccactgca ctccatcctg gttgacaaaa aaaaaaaaaa aa 822

<210> 57
<211> 536
<212> DNA
<213> Homo sapiens

<220>

<221> SITE
<222> (536)
<223> n equals a,t,g, or c

<400> 57
ggacgagtgg ggagctggaa ggaggatgga gtgggaagat aatcttccct tggagttcag 60
ctgtcccgtg accaaactcc tctctgtccc cagctggact cctctagatg ctcagatgct 120
ccttctcttc tttccttctc tgtcacacca ttcttctgtt ccttggctct tctgctcatc 180
tccttgtgga gscawaggtt tggggtttat atgagtacag gataggtgac atggtggatc 240
aaaaggcaac attttgtgtg caaaaacagg aatgcctgtt cccattaggg tcatgggttk 300
ccagggttga gggtggggcc tttgctaggg aaccaccctc ttctacccag tattttcctg 360
tctcctgtct gtatcaatag gtacacaata twtattaaat taatkaatga ctatacatta 420
tgaaatggga aatgcaaggt ataaaggaga attgctgtcc ttgaaaagaa atttagtttg 480
tttttttgtt gagatggagt cttgctctaa gctagagtgc agaatgtaat caaggn 536

<210> 58
<211> 1262
<212> DNA
<213> Homo sapiens

<220>
<221> SITE
<222> (12)
<223> n equals a,t,g, or c

<400> 58
atcctgcagt tnttctacgc aaataaaatt ctgtaagcgc agagcagcag ggaaatggtt 60
ctgtatgagt gcattgctga gccatcatgt tcccctgttt tatctcactg gatgcctctt 120
ctcactgctt gcctcttggg attgtaatgg gaaagagggt gctgggagag caatcaaagg 180
caaaaataat acatggaatt gtatgatttt atctaaagta aaattctaga ctgctttcac 240
ttatttcatt tcctccccat acaaatttga tgagcaggta cacactttct ttaaaacatt 300
ccaagtgtat atagattcag tgcttatttc tcagcttttt ctttcttaag ttcatctctg 360
tcacctagct tttattttta atactcaatt tctgaggctt aggaaatact tgttacctta 420
agcgtttttt tgtttttttt ttgttgttgt tttgtttttt tttagtgtat ttgctattga 480
tactttgcta ttgatacctt tacttgccaa gatttattta aagttggcca agataccaag 540
aaggtggctt gcaggggcat ctttggcttc tcataatttt aaacagtcat tgctttcaag 600
atcatcctaa acaagaatta atagagagaa aaaaatctat aaagcatgtg ttgaaaatcc 660
tcaggctcat gataacttac gggataacaa tagtaggtaa caccgagtgc ttacaattca 720
gtgggcacgg tcaaagtgct ttttgtaagt tctatttaat acttgcgaca accctgagat 780
agtaagtatt attattgttc tcattttata gatgagaaag ctaacacata gggaaattag 840
gtaactttcc ccagataact cagtaaacag tggagctgga attcaaacat gtacggtctg 900
acttcttgag cccatactca ataatcatgc catcctgcct ttccatggaa taactgatgg 960
atggttagaa atgttaaaaa caaagcaaat gcaaacatct ctgcttctgc tcctctacta 1020
agcccagtta acttttgatt ttatatgtac taagtataca tttttattaa tttatgtgag 1080
ctgagtgttt ttattaatta ctttttggct attaatgtca attattttgg tgatcaatcc 1140
tagttatcca aggagtcaca tcactcgtaa aaattacaag aaaaatctct aagtccctct 1200
caccctccct ctaaccagat tgcatcccaa gcttctatag aaactctggt accactcgtg 1260
cc 1262

<210> 59
<211> 1269
<212> DNA
<213> Homo sapiens

<400> 59
gaattcggca cgagarataa cgagatgcta ctagtctggg taccagctgt acgaggaagc 60

atacagctaa gtaggtcagt gattaccaat cagtgtggtg agtacatgaa acagcaggca 120
aggtttggga cagccctgtt tgaaatttgg aagtgaaaat catggtgcac aaagccatcc 180
ttgctctgct tccctgggga ttctctgctg atgaattact ggcttcccta atgatgtktc 240
ttacagagaa gtatcagaac tgcagttcta ccacagacat astgaatcaa caactcagga 300
gcttgggcca gaactttatg tttcaacaaa atctccagtt gattctgatg tagcctaaag 360
tttaagaacc acattgctat agagcataaa ttatttgagg gtagtgctca cggattattt 420
aaactgatat ttctagtgtc cagtgcttga cctaaagtaa gaattccaga catgtttatg 480
aaagagtgaa gagggtcaaa gattttgctt ttgaaccttt tgagtttctg tatgactcct 540
ttgggtcatc taggaagatg ggattatgaa gctgtcaggg tccatatwac aacsaaaagt 600
aaatgtccag gaaagacctg tgactgaaaa agtcatgcta ttcacccttc aagtcccact 660
ccaagtcata cctgctttgc agatctttct aacttaaacc ccgkgtccta tgatcctcct 720
gtctgcgtcc attctttatt atttaagtga ccatatccta actgaatatt gaccatcatt 780
actcattttt ataccttcaa cagctggcac actttttaga atagtgttaa tgcagaacaa 840
atctttcctc attaataagg atttgtggca tacctatgtt aatgttgaat taaatcagtg 900
ttgaaattat tagcaaagct attcctatca gacttagtcc tgaagacaag ccaggtcttg 960
gtaattttac accttacatt attttcaaac agatcagggt acagtccarg agccaggttt 1020
tattgggcac cttccttctc ttttaacatt attgtttgtt ctctccctgc tgtctcttga 1080
ccagggaaat gtcttcaata tcatttattt tgagatcttg ctcgaaactg tcattggaga 1140
tgttagctgt taatgaatgc aaaaagttga taagttttag ctttcttttt ttgatatgtt 1200
ggctttagaa agtcaaaatt ctaagactaa aagaccttca agagagaaat taataaaaaa 1260
aaactcgta 1269

<210> 60
<211> 1829
<212> DNA
<213> Homo sapiens

<400> 60
ggcacgagtg gccctgatag ataacggaag tgaggaagga tgacctgaaa gtggtcagga 60
ttatgatgac gtcgagcctt ggcctttcct tcctccttaa cttaatcctg ggtatgaaat 120
tcacctatct gattcctcaa aataaatata tacaactctt cactaccatc ctcagtttct 180
tctcaggagt cctctctctc ctagagtgca agttgtctac cagtagctgt acctgcctga 240
acatccataa atctgacaac gaatgtaagg aatctgagaa ttctatcgaa gatatttcat 300
taccagaacg cactgcaatg cctcgtagca ttgtccgtgc acacactgtg aattccctaa 360
acaaaaaagt ccaaacacgt cacgtaacct gggctctgtg atttggaatc tatttcttgc 420
agtatatgct catctttatg gaaaaagctt tgtgggtgtg tgctgtgtct ccaaccatgt 480
tgtctctatt tggaattatg gttggggttt gtaaaaagat ctggaagatg gttttttaaa 540
aaatcctggc ctgctgaatg aatagtttct cctgcaacat tgtggttaat ataataaata 600
ttatcatata aataatactt cctgtattgt taagtctata catcacaaac acttatgata 660
tataacagtt gtatagtgaa atatttcaaa catacataag aaatacaaaa tgatgatcaa 720
acacctatgt acacatcaac ccaactttat aaaattttaa cattatttcc tggagaagtt 780
tttccgttaa atttatagat ataactgatg ttcctctaaa aacttctcca atttcatttc 840
catctcttgt ttcctatcat aaaacacttt taaggatgcg ttgctgatga ttctacattc 900
atgttgacat tttcactaca aatttataca tctataaaaa ttatatagac cagcttttca 960
aatgtttgaa agttggtcaa tattacactg tagagatcat gttgtattta ttttcataaa 1020
tgctgaaata aatcttctaa ttattttaga attcctgaat ctatctatgt aagtaaaact 1080
tggtccttca tgcctttttg tggttcatac tttgtggggc taagtaatat tccatcgtaa 1140
gactgtacca cagttggttt atctatttca cctactgaag gatatcttgg tgttccaagt 1200
tggggcagtt atgaataaaa ctgttataaa caccggtgtt gcaaggtttt ggtgtgaaca 1260
ttaagctttc actttttgtg gggtaaatac ccaggaacat ggattgccgg ggttatatgg 1320
agaagaatgg gtttagtttg taaaaaactt ccaaactgtc ttccaaagtg gctataccat 1380
tttgcattct accagcaata tatgagagtt cctgccgtac cacatccttg tcagcatttg 1440
atgttgtcag tattgtgaat tttgaccact ttaatagatg tgaatgtgtc ttattgttta 1500
aatttgcaat tctctagtga tttataatgt gaaaagcatc ttttcatatg gttacttggc 1560
atctatgagg tatctgttcc attcttttgc cctgtttgta atcagccttt tcattttttt 1620
ggtgagtgtt taaatgttat ttgtatattt tggataacag tcctttatca gatatgtctt 1680
gcaaatactt tctcccagcc cgtggtttgt attttcattc ttgacaacgt cttttgtagc 1740

acactggttt ttaattttaa taaagtccag cttattaatt attaaaaaaa aaaaaaaaaa 1800
aaaaaaaaaa aaaaaaaaaa aaaaaaaaa 1829

<210> 61
<211> 1112
<212> DNA
<213> Homo sapiens

<400> 61
   ggatagcagg ggctcagtca tattttttgg atgaatgaac aaaccctgaa gatgctacat 60
   ctgactctat atcttcattt tatccttttc gtatttccta ttacctctaa tttctcttcc 120
   ctgcacccat ttctatttat ttcatcccag tttacctcct gctgccagat taattttcct 180
   aatgcacagg ctctatcata tcatgagttt ctcattgcta catatgacta atttgccaat 240
   atttttgcac atcagaatgt gtatcacttt gaggctggtt ctgtgtttgt tttagtttag 300
   gaaaagctgt tcagattgtc tgtaaatccg tatggggatc tttgcatagg attttaaagc 360
   agccacacat cttgtacaaa atgtataaga ttaattttct atgttaggac catttgtttt 420
   caccaattcc atagagctcc aatgtgtaaa agaagacact gatctaactc ttgtgttaaa 480
   tatttagtaa ctcatttatc tggaagaaag caaaacaaaa caaaaataca aggaataaaa 540
   atcactggga gtgcttttca ttcactgaat aatgagtttt gcaaggagca cgtggatggt 600
   gacattatat cttttacatc tttattttct gtttcttttt tgactcctta tcagtgaatt 660
   tatcttattt tatactttta ctttctattt ctttcttgac tctttgttgg tgaattggta 720
   gcaagagact tactgtctga tcagaacttt gaatcttcct gcctctcttt ctttgaggtt 780
   gacagggata aagataatta agatagcgct tgggtgtgat gacactggaa gacaggctgg 840
   gtcagggcct gtagtarara cttcccccct ctattgaatg ttaatctgaa agtgaatctg 900
   aaagcagatg gtcatgaact acccagggtc tccattaagc ccatgaagtt tattttaaaa 960
   ctcttaaaat agattgagat tcaaattgag attcatgtct attttttaaa cattgtgtct 1020
   taacaaagta gatgttcagt catacagtta ggcaaatgtt ctaaggaaag atgtttmcca 1080
   tgctaagtta aaaaaaaaaa aaaaaaactc ga 1112

<210> 62
<211> 1674
<212> DNA
<213> Homo sapiens

<220>
<221> SITE
<222> (734)
<223> n equals a,t,g, or c

<400> 62
gagaagtcct agtggctctc tgctggtgct gaggcaggaa gggccagaag ctggtgagct 60
tccctgaacc ctggtgtgcc tgggagtcag ttccgaagaa ggcagctgtc ccagagatgt 120
gacaggacct ggctgttgtt tctctctgac cctaacagga tttatgccct gcatccgcgg 180
tgtttttcac tgttttattc ttattattct tattctcttg gcgtcacatg cgtttagtgg 240
atctggaaat caaaggctga aggaagcgct gacattgatc gtatctgtta atgtggatat 300
tgccagacac aggcccttct tggagcgtat acatgttaag aagggcaaca cttagccccc 360
aaaacagtac cccagtgttg cgtttacatg cagcagaaat agctggatgg agagagattt 420
tgagccaggg tctgccaatg ccacttcagc ctcarggctg agctgggcct ggagaggaaa 480
gctgctgagc caccgttgga gcaaatgact gaggacctct gctgccccta cttctgcccg 540
agagctgagc tgacctgatt gtgctttggc atcaccaccc actctggccc caacatctag 600
ccttgccaag ttcaggtgct agtcatgacc atgtagagct caccgtgtac ccaaagactg 660
tggcagcttc ctggctgctg gagctttcca ggccccctat cttctgttta ttcactcasc 720
cagcattaac tgancatgga ctggataggg ttgctgccct tgtggaatgc accatctgga 780
rarararagg aatgtggtat cgcagacgct acagttgctg ccagtttagg gatagaagta 840
tcagggatgt cttcccagaa gcagtgatgc tccaacaaca tctcaggcac ttggctgtgg 900
ccacatatcg gtgcagaagg agaagcccat gcaaggcacc cacagttgag gaagcagagg 960

gtggcaagcc acgagcagtg ccctcaggca caggattcca gaagcatggg caagagcctg 1020
gagggtccac atccccacat tggttctggg gacatttgca actccttgtc ttgagtgtca 1080
acaacaggca gctctttgtt cagggcaggg cagggtattt ggagatgact ggtttgccct 1140
gtccaaagct gctgctcaca ctgttgagag gcttgacgcc tggggtggga catgggctgt 1200
gtgcatatcg gcgaggctgt ctggcctggc ggctggacam agcctcctag tggagatgat 1260
atttggcctg tgaactgaat caggtgaagg ataggagaag aaamcascat atttggagac 1320
ttagtggcca gagcatgtct tggcagagaa actggaagga ggctggaatg gctgcaccag 1380
gcagaaggaa gactcctaaa gtggggatga cgggtaacct gggtcacatg tgcgagatct 1440
tcttgtagcc tgggggagga gcttacactg tcctgagagc agtggggagc ctcaggagcc 1500
tgcctccaga gtatctaggc attagttaca tgccatgtta cagatgagat tgcccaggca 1560
cagtggctca tgcctaaaat cccagcactt tgggagacca cagtgggaag atcacttgaa 1620
gccaggagtt ccagaccagc ctgtctctac aaaaaaaaaa aaaaaaaact cgta 1674

<210> 63
<211> 1045
<212> DNA
<213> Homo sapiens

<400> 63
   tcgacccacg cgtccgagat gcacgaactg attaattcat ttgttctagg gctctgagga 60
   gtcgtctact taaccttttg ggttgctggt cttacctatg ttctcacgcc tccatttctt 120
   cacccactca ctcagccttc tccatttacc ctcccaagtc tttggcgagg tacactcatc 180
   ctgcgtatca tcactgccat gtcctgatac cccagctctg ccatattgcc cttctttttt 240
   gcggtatgat gaccacatag aggcccaacc tcttaaacac atcaatacca atgatcacat 300
   ttcaatctag acttctaagc aacggctgaa atctctccag gccaaaggag agtttgtatc 360
   accttaccag aagcttctcc ggaacaattg gccagaagcc tagagttcag aaacccagac 420
   acatgcagta agcaatttcc agtttctcta taatttagaa gaggacacca tgatatgtaa 480
   tgcggggtct ggaggttgga atgcctccat aaaacacctg ccatattttt tggtccaagc 540
   cttagtgtta taaatcaaga aggctgtaaa taagacttca gcyttttgtg ctggtgaagt 600
   ttgtttcctt taacttatcc tccaagagta ccgaggcacc gagatctacc atttgccacc 660
   tcatccattt ctatggcaga acaccgcctg gggagaggaa ttcgattccc cgaatcagga 720
   tgactgtgtg gggcttctgc aaaggttgca tcacgagtcc tatttctgag ctatctgaga 780
   tccccattaa gaatttaaaa gcaataaaat aacggagatt tttgactatc aacatgaatg 840
   ctgtgtgggc ttttacagtt aatgattgcc cttgagtgct gaataatctg tggcctgaaa 900
   aaagaaatgt tcttatcttc taaatttggt aatcaagaac aagatagagt aatgaatgta 960
   aaggaacact gttgcaagtt gagtgtttcc aaaaaaaaaa aaaaaaaaag ggcggccgct 1020
   ctagtaggat accaagtctt tacgt 1045

<210> 64
<211> 1051
<212> DNA
<213> Homo sapiens

<400> 64
ggtttttccc gggatacatc tgtgttgagt cactttgcat tcaacagtgc ctcgccacca 60
aaatcataca taagaggaaa actaggactg gaagaatatg ctgtctttta cccaccaaat 120
ggtgttatcc cttttcatgg attttcaatg tatgttgcac cactttgttt tctataccat 180
gaaccttcca aattgtatca gatattccgt gagatgtatg tgcgtttttt cttcagactc 240
cattccatct cttctcatcc ttctggtatt gtgtcactct gtctgctgtt tgaaactctt 300
cttcaaactt atcttcccca actcttttat catctacgag aaattggggc tcaaccactt 360
cgcatatcat ttaagtggat ggttcgagct ttctctggat acttagctac agatcagctc 420
ttgcttttat gggatagaat cctaggatac aactctctgg aaattcttgc tgtgctggca 480
gctgccgtgt ttgctttccg agcagtgaac ctgatggagg tgacatcact ggctgcagct 540
gaaaatctag ctgcccacag tgaacagttc tgcactgctc ctctattccc tgagctttac 600
agagtccaga tccctgtact gctgaactca ggcagaaaga agagtgcagt ttattggact 660
ccaatctcat tcaacagaac aaagaagttg aggttgcaag gaagaaccta taatgatggg 720

tcatggaata taacctagaa aagaagagaa ataaaagaga ctgtgtttca ccatgttgcc 780
caggctggtc tcgaacttct gagctcaagc aatccaccct cctcagcctc cagaagtgct 840
gggattacag gcatgagaca ccaagtccag ccataaggtt cttattctat atatacatga 900
aatgatatca cttgaaggta gactgtgata agttaaatac gtatattttt taaatcttca 960
aacaaccact aaaataaaag aacaaagagt tacaactaaa aaaaaaaaaa aaaaaactac 1020
gtaggggggg acggcgtacc caattacgcc c 1051

<210> 65
<211> 1182
<212> DNA
<213> Homo sapiens

<400> 65
ggcacgagcc cccagcacat ggaagccctg ttacagtccc tcgtgatagt cttgcttggg 60
ttcaaatcct tcttaagtga agagctgggc tctgaggttt tgaacctact gacaaataaa 120
cagtatgagt tgctttcaaa gaaccttcgc aagaccagag agttgtttgt tcatggctta 180
cctggatcag ggaagactat cttggctctt aggatcatgg agaagatcag gaatgtgttt 240
cactgtgaac cggctaacat tctctacatc tgtgaaaacc agcccctgaa gaagttggtg 300
agtttcagca agaaaaacat ctgccagcca gtgacccgga aaaccttcat gaaaaacaac 360
tttgaacaca tccagcacat tatcattgat gacgctcaga atttccgtac tgaagatggg 420
gactggtatg ggaaagcaaa gttcatcact cagacagcaa gggatggccc aggagttctc 480
tggatctttc tggactactt tcagacctat cacttgagtt gcagtgcctc ccccctccct 540
cagaccagta tccaagagaa gagatcaaca gagtggtccg caatgcaggt ccaatagcta 600
attacctcaa caagtaatgc agaagcccga caaaatcctc cacctaacct ccccccctggg 660
tccctggtga tgctctatga acctaaatgg gctcaaggtg tcccaggcaa cttagagatt 720
attgaagact tgaacttgga ggagatactg atctatgtag cgaataaatg ccgttttctc 780
ttgcggaatg gttattctcc gaaggatatt gctgtgcttt tcaccaaagc aagtgaagtg 840
gaaaaatata aagacaggct tctaacagca atgaggaaga gaaaactgtc tcagctccat 900
gaggagtctg atctgttact acagatcggt gatgcgtcgg atgttctaac cgatcacatt 960
gtgttggaca gtgtctgtcg attttcaggc ctggaaagaa atatcgtgtt tggaatcaat 1020
ccaggagtag ccccaccggc tggggcctac aatcttctgc tctgtttggc ttctagggca 1080
aaaagacatc tgtatattct gaaggcttct gtgtgacagg aaacccaagc ctaagaaaca 1140
attaagtggt tctcatctct aaaaaaaaaa aaaaaaaaaa aa 1182

<210> 66
<211> 675
<212> DNA
<213> Homo sapiens

<400> 66
ggcacgagct gctcttcttc ttcaacatgc tcttctgggt gatttccatg gtgatggtgg 60
ctgtgggtgt ctacgctcgg ctaatgaagc atgcagttct ccctctgcct caccgctgtg 120
ttcctgctgc agctggccgc tgggatcctg ggcttcgtct tctcagacaa ggctcgaggg 180
aaagtgagtg agatcatcaa caatgccatt gtgcactacc gagatgactt ggatctgcag 240
aacctcattg attttggcca gaaaaaggta tgggtcagcc agtggtctgg gggactgtgg 300
gtaaaagtga atgtcatccc aagagatgcc tcaccctcta tgcctgtggg gctcttcatt 360
acctgccagg taatggcttc tgggaagggg tttggcaaaa aaagcacacg tagcagagtg 420
ctttaaatgt acttttaaag acacagaaca gtatatatag taatctactg tgttataaat 480
ggttacttac agggggtgag gaactgggca gattcttgaa tattacctct tcaaaagtga 540
cattttaggc tggtccaaag ggagtgagtt atctcatttg attgttcaca gtcagctaca 600
gatccaactc cttgttctac tctttccccc cttctcagtg ctgcacttga ctagactaaa 660
aaaaaaaaaa aaaaa 675

<210> 67
<211> 1105

<212> DNA
<213> Homo sapiens

<220>
<221> SITE
<222> (797)
<223> n equals a,t,g, or c

<400> 67
   gggggaaaaa aggacacgtt gaattctgtt gctttaaatg tatatttttt tattgtgcta 60
   aaatgcacag aacataaaat ttgccattag taacactgag tacattcaca gtgtcgtgca 120
   accatcagca ctgtctagcg ccagaacttt ttcatcaccc caaagggaaa ccccgtatcc 180
   atgaaggact cactccccat tcgccctctc cagcccttgg cagccaccag aatgctttct 240
   gtctccataa attcattttt aataagtgca attctgtgtg actttaaaat aaataaacat 300
   gagcacgatg agttgcttat tggaaggata tccatgcggg gaggccggcg tgtggagtgc 360
   gtargcctcc ggacgggcag gagttgaagg ggcgtggatg tgccgccctc tcctcccctt 420
   gctctttcct tggggtcact gcctgagtat ccctctttgc aaatggcccc aaataatgtc 480
   tcagccccca cgtctgcatc gcctcctagc ttcaggaccc tccaccaaaa aacattccaa 540
   gcttcagact cactcctggg aaaattccaa tggcctcact ctcccttttg agccagccag 600
   atcccatggc ctgtggcggg ctgcctttga gtcctgagca cctgtgagyt agggaagcag 660
   gacaggcaca cccagggaag gggaagagtc gtcgtcagtc acagtaattg atatctttgg 720
   aatcgtctaa gagatactta gcgtgtgcct aaaacattca tttctttttt tgtttgtttt 780
   ttgwgacgaa gtctcgntct gtcgcccagg ctggagtgca gtggcgtgat ctcagctcac 840
   cgcaacctcc tcctcccggg ttcaagcgat tctcctgcct cagcctcctg agtggctggg 900
   actgcaggca cacactacca cgcctggcta gttttttgta twtttagkgg agacggggtt 960
   tcactatgtt ggccaggctg gtctcaaact cctgacctcg tgatctgcct acctcggcct 1020
   cccaaagtgg tgggattaca ggcatgagcc actgcaccca gccaactagt cttaaaaaaa 1080
   aaaaaaaaaa aaaaagggcg gccgc 1105

<210> 68
<211> 1279
<212> DNA
<213> Homo sapiens

<400> 68
ggcacaggtt aaacgttgta taaaatgttt cagtggggct ggagacagtg gcttcttggc 60
atcagctgca cagggttgga ccacagagca ggccccgcga agtgttattc cgagcagccc 120
cgcctcagct cgcagcctgg actccaagtg ttactgtgtc catgctgagt ataattgatt 180
tgcttttcct actttctcca actttcggtt taatcacaga attgcttttc agtccagaag 240
ttcccaaagc tctttcctgc cctctgaagg ctctgggagg tggttctcat tcacatgagc 300
ccctgggrat gtttgctcca gttcctccag gctgtgaatc aagtactcca ttccccaagg 360
gcctgggggc cagtaagatc ctcaccctgg gggctcaggc tgaattcagg aggaggagtc 420
actgaactgc attcaggaaa ataaacacat cttgctcagc tgagtgagcc tttgaatttt 480
tctccctggg aagtcacatg tggggtggcc araggtcacc gtgtggtcag ccagggatct 540
tggctgggca acgatgcctc ccacagaccc ttatcaagac cacggcagtc ttcactttgg 600
ccatctcctc tggtgtgtga gaagaagcca ttctctgggt tgaactgagg ggcttcagat 660
gaacaattct gttccttcac ccttggctaa tggcccactg gcatgcagac ctgctagtga 720
attcaggagt ttcccagctc taaggtgagt cctggcagct ctactgggcc tggccaactc 780
catctccagg tcaggtttcc cgtcacaagg caactactag ggagaccccc gamccccgtc 840
cagctcccac caggctgaat taragtcccc stkgctgccc ctcacccayt cccccatatt 900
caccattcca gttacttttg ctgtgaagta ggccacccaa actagggaga ataaaacaac 960
aaccatttta tgcagctcat ggctacagtg tgggaagaat ttaaacaggg catggaggag 1020
atgtctcctg tctgtctcaa aatgtctgga gtctcagcta ggaagactcm aagtctggga 1080
gtartttggc atctgggggt tgggatcatc tggagactcc tttactcctg ggtctttctg 1140
gtccacaggc tggaagggck tcagaattag gaccgccagc cacagtcctg agcttggcgt 1200
ctccatgtgg cttggcatcc tgcagcacac acagcagcct ctgggctcga gggggggccc 1260
cggtacccaa tcgcctgag 1279

<210> 69
<211> 1638
<212> DNA
<213> Homo sapiens

<400> 69
ggcacgagag aaggtgctca tggtggagct gttcatgcgg gaggagcaag acaagcagca 60
gctgctggaa acctggatgg agaaggagcg gcagaaggac gagccgccgt gcaaccacca 120
caacaccaaa gcctgcccag acagcctcgg cagcccagcc cccagccatg cctaccacgg 180
gggcgtcctg tagctatgcc agcggggctg ggcaggccag ccgggcatcc tgaccgatgg 240
gcaccctctc ccagggcagg cggcttcccg ctcccaccag ggcccggtgg gtcctgggtt 300
ttctgcaaac atggaggacc actttctgat aggacatttt cctttcttct ttctgttttc 360
tttcccttgt ttttgcacaa agccattatg cagggaatat tttttaatct gtagtattca 420
agatgaatca aaatgatggc tggtaatacg gcaataaggt agcaaaggca ggtgctttgc 480
agaaagaatg cttggaaact tgagtctccc tagaagtgaa aagtgagcag aggcccctag 540
aaaccctcct ctgaatcctc ctaattcctt aaaatagatg caaaatggta agccgaggca 600
tcgcgcaaaa gctggtgcga tgcttcaggg aaaatggaaa acccacgcaa gaataatgat 660
tgattccggt tccaaaaggt gtcacctacc tgtttcagaa aagttagact ttccatcgcc 720
ttttccttcc atcagttgag tggctgagag agaagtgcct catccctgag ccacacaggg 780
ggcgtgggag catcccagtt atccctggaa agctagaagg ggacagaggt gtccctgatt 840
aagcaggaaa cagcaccctt ggcgtcccca gcaggctccc cactgtcagc cacacacctg 900
cccccatcac accaagccga cctcagagtt gttcatcttc cttatgggac aaaaccggtt 960
gaccagaaaa tgggcagaga gagatgacct cggaagcatt tccacagatg gtgtcagggg 1020
tttcaagaag tcttagggct tccaggggtc ccctggaagc tttagaatat ttatgggttt 1080
ttttttcaaa tatcaattat atggtagatt gaggattttt tttctgtagc tcaaaggtgg 1140
agggagttta ttagttaacc aaatatcgtt gagaggaatt taaaatactg ttactaccaa 1200
agatttttat taataaaggc ttatattttg gtaacacttc tctatatttt tactcacagg 1260
aatgtcactg ttggacaatt attttaaaag tgtataaaac caagtctcat aaatgatatg 1320
agtgatctaa atttgcagca atgatactaa acaactctct gaaatttctc aagcaccaag 1380
agaaacatca ttttagcaaa ggccaggagg aaaaatagaa ataaatttgt cttgaagatc 1440
tcattgatgt gatgttacat tccctttaat ctgccaactg tggtcaaagt tcataggtgt 1500
cgtacatttc cattatttgc taaaatcatg caatctgatg cttctctttt ctcttgtaca 1560
gtaagtagtt tgaagtgggt tttgtatata aatactgtat taaaaattag gcaattacca 1620
aaaaaaaaaa aaaaaaaa 1638

<210> 70
<211> 887
<212> DNA
<213> Homo sapiens

<400> 70
tttttttttt ttttttttat tagaataaat gttgttgcca aatgaaaaca tgacactgta 60
tataactgct gaggcctatt ctcatgttta accttcctaa gccagttttc ttaagttggt 120
ggagatggaa gactatagta attttcctag catgtctggc atctgctgct ataaaagaga 180
cagcagtaag catgaagact gtgtttccca tatttgtcca aatcactttg attttgcttt 240
tagagtcaag agtcttaaaa attggtgatt tttccaattt tttctgctaa tagtatattt 300
aaaaattagc atatgcttta ggtacatgaa actttaaaaa agtaattata atgtacagtt 360
aaaaatttat agtaagtgga tctcacaatt cattttctaa ttagtaattg atagttttac 420
ccattaaaat gacaatgaaa atatttttac tatgggtttt cctcccattt gtttctaatc 480
atacctttga taatatttta taaaggctta taatcatagc agggaattaa tttactactt 540
aaattttatg tatatgtaca gtacatttaa aaattaagta gtcagggcat ggtggctcac 600
gcttgtaatc tcagcacttt gagaggccaa ggtgggtgga tcatgaggtc aggagttcaa 660
gatcagccta gtcaacatgg tgaaaccctg tcactactaa aaatacaaaa attacccggg 720
cgtggtggtg catggctgta atcccagctc ctcaggaggc tgaggcagga gaatcacttg 780
aacccaggag gcggaggttg cagtgagctg agatcacacc attgcactcc agcctgggtg 840

acatagtgag actctgtccc cctctcaaaa aaaaaaaaa aaaaaaa 887

<210> 71
<211> 864
<212> DNA
<213> Homo sapiens

<400> 71
   ggcacgagcg gaccgggccc gcggggctgc tgcggggcga tcgggccggg ccgctgccgc 60
   gccatggact cccgtgtcca gcctgagttc cagcctcact gagtggccac ccccaaagtg 120
   ctgccagccg aggaagcccc cagcactgac catgtctatt atggaccaca gccccaccac 180
   gggcgtggtc acagtcatcg tcatcctcat tgccatcgcg gccctggggg cctttgatcc 240
   tgggctgctg gtgctacctg cggctgcagc gcatcagcca gtcagaggac gaggagagca 300
   tcgtggggga tggggagacc aaggaaccct tcctgctggt gcagtattcg gccaarggac 360
   cgtgcgtgga gagaaaggcc aagctgatka mtcccaaacg gsccggaart ycacggstga 420
   vccaggatgc aaaggccycc tggtccctgt ttgcaagccg gccaagargg ggctgggagg 480
   ggcaaaamcc atacggatgc gctgctgtct gagaggaagg gctgacactt gctggcatgg 540
   cctctgcggg tttcgtccat cgcatgcact gatgcccggg gacttggctg tcctgggctt 600
   cccctcggcc tccaggtgag gctgcccatt gcaggcactg ggtaggcctg accttgctgg 660
   ggctcatggc cctgtagcgc ttttgttact tgaatgtcta gctgagcctg tttttgatgg 720
   agctactact gtaatgcgtg aactaacaaa cctgtgaact gtaaataggc ccctggaagc 780
   acgtgcttaa gcccttttgc tgatttttaa aaatatcatc tagcgcaaaa aaaaaaaaaa 840
   aaaaaaaaaa aaaaaaaaaa aaaa 864

<210> 72
<211> 1217
<212> DNA
<213> Homo sapiens

<400> 72
   tctggaacct ctctcttaat tcatatttcc cgtaagtctg tccatctgtt gtgtggaatc 60
   tcagtttgtg atattggata gatgcaaata agcaaagctg ttacttttca tagtttcaaa 120
   tgaaaaactc aacatcacta ctgtataaat tattttctag tctatctgtg tttattttta 180
   aattcctttt actattctat acattgcaca ttgctctggg ggtaaaaatc cartataaac 240
   cattagctca ttttattgac cattcttgta ttcagcaagt atcccaagta cagtggtcca 300
   taccttgaat tttttttcac tttttaagtg agatataatt tacataccat aacaacttag 360
   tgggtttcag ttatttcaaa tacaaggttg twcatatatc atcactgtct aattccagaa 420
   cattttattt ttattttttt tcagcagtgg ggtcctgcca tgttgcccag gctggtcttg 480
   aactcctggg ctcaagtgat cctcctgcct cagtctccca aagtcctggg attacaggtg 540
   tgcgccacca cacccaccct caaaacattt ttatttccta aaaaagaaac cccacatcca 600
   taggcagttc cacattccgt tcttcctatg atccagctct tggcagctac tatagttkgt 660
   ttyctgtttc tgtggatttg tctattctgg acatagcatg taattggagt catacaatat 720
   atggcttttt gtgcctggct tctttcactt agcataatgt ttttaagatt cattcatgtt 780
   gtagcattat cagcactttg tttcttttat ggctaaataa cactgcattg tgtgsacata 840
   ccacattttg tttatccgtt aatcagttga tggatatttg ggttgtttcc acttcggggc 900
   tattatggat gatgcttctc tgaatatttg tgtacaagtt tttgtgtgga catttgtttt 960
   tagttcactt gagtatgtac ctaggatgga attactgggt catgtggtaa ctgttttaat 1020
   ttcgtaggaa ctgccaaatt gtttcctaaa gtggctacgg tattttacat tcccatcagt 1080
   actgtatgac agttccgttt atccacatcc actccaacac ttgttgttat ctgttttgat 1140
   tatataatag ctattttagt gggtatgaag tcgtatttca aaaaaaaaag gaattcgata 1200
   tcaagcttat cgatacc 1217

<210> 73
<211> 1717
<212> DNA

<213> Homo sapiens

<220>
<221> SITE
<222> (712)
<223> n equals a,t,g, or c

<220>
<221> SITE
<222> (721)
<223> n equals a,t,g, or c

<220>
<221> SITE
<222> (903)
<223> n equals a,t,g, or c

<400> 73
gctcctaggg gcaggacttg gcagttgctc aggagatgtt tgagaggtgg gcctttccgg 60
agaagtggcc ctttaaccct ggactgtggg ccatttagga gcatcacacc agttttagaa 120
tgtcagtagg gacactgcga cacagacagt gacctgggag caggatgcag gagccacatg 180
gcaagtttct gtcctggggc aggtggctct ggtggtggtc tcttgccgca ccagctctgg 240
ttcaggctgt taacatgcct cccgcataca tccagataga gaactggtac atgatgctcc 300
tgatgggctg ggagacaaaa tgttgccatg tcaggagtct gtgggtggga acataatgaa 360
agggaccccg acgtggggac agtgtggcca ggcagcatgg agaagtgggt gccactggct 420
cagcctgggc ctgagctcac tggggaccgg agctactttg catgttgctg agggatgggg 480
agggcagggc tcttccttca cagaatgctc tttctcttgc ttggtaggat gttctttggc 540
aaaaacaagg tgatgatggt ggccttgggt cggagcccat ctgatgaata caaagacaac 600
ctgcaccagg taagtctctg gccctcacgg ggtggagcta aggcaaagcc gccctctctc 660
ccactactcc catgtgacag cctcccccac cattggcagg ccagaagctc anggararca 720
nggcctggct taaactctgg gttytccytt macytccgra cccaaggaat arcasttcct 780
gcytcccayt cyttcttgct atgacaacca aaaastcttt aaatgttgcc aaatgtaccc 840
ggtgagcaaa aacgtgctta gtagagaacc aytgttctaa tgtgaccaag ctgtcctcac 900
tcntgatttg taggtcagca aaaggttgag gggtgaggtg gtctcctgtt caccaaccgc 960
acaaaggagg aggtgaatga gtaagtactg ctgaggaacg gagggaaaga ggcgggtgag 1020
aggggatttc agggagggaa tgatgcagga acttcgttcc atatgtggca tcaagttgga 1080
actgctcctg gaagcctgtc actgagctag tggcctggga ccacagccct ctctgtcctc 1140
tccagcatgg tgctctgggc tggctgctgg ccagcagcca aggtctgtga tgggggaagg 1200
cccagggcca cgggacactt gggggagcct gactcatggg ctggtggcgt ggccaggagc 1260
atctcctcct accacttctc ttttccctta ggtggttcac gaaatacaca gaaatggact 1320
acgcccgagc tggtaacaaa gcagctttca ctgtgagcct ggatccaggg cccctggagc 1380
agttccccca ctccatggag ccacagctca ggcagctggg cctgcccacc gccctcaaga 1440
gaggtatggg cagccctgga gccaaaaggt cacagcctag agtccaagag cacgggcctg 1500
caagctcatc cctttctagc tgagcaagtt atttctacaa gcttccattt tctcatgaga 1560
aaatggggct aagagtgcat gcctcacagt gggggtgaaa gcagacagta acttacagcc 1620
aaaatgcagc ccttgaggcc catcaggggc cttgcgcttg tttaaaaaaa aaaaaaggga 1680
attcgatatc aagcttatcg ataccgtcga cctcgta 1717

<210> 74
<211> 1276
<212> DNA
<213> Homo sapiens

<400> 74
ggcacagtga aaacttggta tttaaggaaa atgttgggag aagcccacag ataacacact 60
tgctctgttt aatgctaata aattgcattt tttctttatt gttattattg tcacatgcag 120
atgggatgca tttatttata agttctgggg ataggatact tttttgcctg tactttttac 180

attccagagt ttgtgcttga cttcatcttg actaagacag atgagaatat ttcctagaat 240
ttgtttttag tcttatttgg atcctgtcac actgcagcat tttattgtcc tcagctgtct 300
gtgatcctgg aaacatacgt gtgactgaag ctcccaaaca cccaatctct gaagaactgg 360
aaactcccat aaaagacagc cacctgatcc ctacgcctca agcccccagt attgcctttc 420
cactcgccaa cccccctgtg gctccgcacc ctagagaaaa gattataacg atagaggaga 480
ctcatgaaga attaaaaaaa cagtacatat ttcagttatc atctctgaat cctcaagaac 540
gtattgacta ttgtcatctg attgagaaac taggtaccag tattttactt aaatccaaaa 600
tgtcccatat aataaccata tttggaagtc aaatgtagtc aatattaata tcatttgtaa 660
agtctagaag tagaggaaaa gaacatgggt aacttgtcta aggctataca ttgagtcagc 720
attggaactt tgattttaaa aagctctaaa gtcagtagat catgggattt aaaattcaaa 780
tggaatctag cagttgaggt ttaagaatct tctgtgctgt ttgactattc attcactaat 840
gaagctttaa agacttacta tataaataaa accacatttt aatgaacttg aaaggttaat 900
aatatctaga gaggagctcc ctcttgtttt tttgtttgtt tgtttccagt tagaaacagt 960
gtggaggcca ggcacggtgg ctcgtgcctg taatctcagc actttgggag gctgaggtgg 1020
gcggatcacc tggggtcagg agtttaagac taggctggcc aacatggtga agccctgtct 1080
ctactaaaaa tacaaaaaaa tttgcgagac gtggaggcac atgcctgtga tcctagttac 1140
tcgggaggct gaggycagga gaatcgcttg agcctgggag gcggaggttg caatgagcca 1200
agatagcgcc actgtactcc aggctgggtg acaaagcaag actctgtctc aaaaaaaaaa 1260
aaaaaaaaaa ctcgta 1276

<210> 75
<211> 1144
<212> DNA
<213> Homo sapiens

<400> 75
gcacacatac gtatgcatat aaggattatc atatataaat ttatataaca atttttatgc 60
atgagtgtga ataaatatat gcatatatat gtctgtatat gtaaacataa tgcatatagt 120
aatttacata tatctgtgtg tatatatgtg tgtggcacag tcacacacac acacacaaat 180
atgtatacag atgcttcctg gcttacaata ggatttcatc ctgataaatt catcgtaaat 240
caaaagtatt gcaagttgaa aatgcatttc ataccccagt aagttcatca tttgktcaaa 300
agtattgtaa gtcagaatac atttgacatc tggataagtc cattataaag tcaaaacatt 360
ttaagtctaa tcattgtaat ttgggtaccg tctatgtaga tacgtaaatc atacattaag 420
ggtgactagg tgccaggttg aatgttatga aaatgaattt caagtctcac aggcacattc 480
acccattaca aatatgtacc acattcacct attacaaata tgtacacatg tatgtgttca 540
tgttcatact acaatggcag agttgcataa ttgtgacaga aatcaaatgg cttacaataa 600
ctaaggcatt tctacatagc cttttaaagt aaaaagttta ttcattgttg gtctacataa 660
cgtggaggaa tttgtagcgg acaggctatt acagtcagtg aattgaaagg aagggagaag 720
ttgggggaga ctagtagctt tttgaaggta ttattttaga gatttatgaa kttttggaga 780
acaagggatg aggaaaaagt attgaagaat ttgggagagc aggatatcaa ttagtttctg 840
actttattgg gaatgcagat cagagaaagg ctgggataga aaactgaaat aataattata 900
gccttcggtg aatatcagca ggactgatgg gactataggg agggtagact aggtgataga 960
gcccattgtg gcagtttcgg taggacatca ttggtgtata cgtatatgtt atttgtgatt 1020
ttgtttatct ttttttaata agcaaaagga aaagtgtcct gatatgtttt ggctttgtga 1080
ccccatccga atctcacctt gaattgtaac aaagttttac catgttaaac aggctagtct 1140
cgta 1144

<210> 76
<211> 918
<212> DNA
<213> Homo sapiens

<400> 76
ctgcaggaat cggcagrgca gttttcatca tctatgatct taagtttcct catgctcttt 60
ctcattgtta agacaatacc acttatttta gcttattgtt acaatagtat ttcatttttt 120
tctaacaact tggtattagt caagatggga tataataaca aatgactctt gaaatcttca 180

tgggtgccat gggaagtttc aagaacacaa cttg[?][?][?][?][?]g aattgcatga ctttgttctg 240
tgtttcattg accactactc attcagtgag cctgaaagta actgtgtata tcactgttag 300
tatactatgc atgccagata cccaagactc aaacttttcc tttcctttag ataccaccta 360
cttagtcatc aattttggtt caacctactc cactaaatag ctttgacttc cattcactta 420
ccactttaga ttagtgctat agactcctat tttacctcct tcatatcaat ccccttaaaa 480
ctcccaatag cttccattgt ttcaaccaaa agctcaaatt cctttaatat aaagtgttat 540
atgaactggc accctatata ctctatatcc taatctctca tctttcattt atttctttaa 600
ctcctgactc atgtaacaaa aatgtattta tctcagcacg tatgtactcc cttgttatgc 660
tctcccaaat gtccctgtat ttttcattga atagcaattg ccacatttta ttttatatgc 720
ttgtgttacc atttatatat atatttacta cagactctgc ttttagaagg catacactgg 780
ccaggcgcag tggctcacac ctgtaatccc agcactttgg gaggctgagg ctggcggatc 840
atgaggtcag gagatcgaga ccatcctggc taacacggtg taactccatc tctactaaaa 900
ataaaaaaaa aactcgag 918

<210> 77
<211> 1065
<212> DNA
<213> Homo sapiens

<400> 77
   ggcacgagag agaggtgggg ttaatgtgat ggaaagttgg cattgcttag tttctggggc 60
   tgccttctag atagcttcta aaccccaaag cttctctcca cagtgccatg cgtctactga 120
   gtactctgct tagcttttac cccttcagca actgcttttt gctcagtttc tgtgattctc 180
   acccacccgt gtggcttagg aattcacaag tgtttccaga ggaagttgtg tgaaaggtgg 240
   ctagaggacc agaatctttt tccctcgtgt cctggctgtc ctgtggccct ggactccaca 300
   tttttatcac tagcccactg ggcccccacc ctgttgggtg tcagtgctcc ccaaactgac 360
   aagtgccctg aggaggaaac gctcggatgc aggcgcagct cagttcattt cctttctatg 420
   tgggatcctc accttctatg tctttcttgg tctccaaagc ctcacacagc tgttccttgc 480
   tgttggcatc acttagtctc gcagtccctg gtgtttccat caggaaagtc aggcaggcgt 540
   tagcttcact gccaggaggg aatctcctgt ggcctccatg gcgctgtgtt ttcttccagt 600
   ctttttctta tgcacagaca catcatcccc tttgtttcct tttgtgatgc tgtttctaaa 660
   aggatcttta tttctagaag aaactttagg aggcaaaaac agcgcagccc ccttaacaga 720
   gtggctctgg tggcaacttt cccttgttaa tttgtcctgt agcccctact ttcccaagcg 780
   cttgctgttt gtggggctac aggacacagc agctgaaagg ggctgtgggc atcgccagca 840
   tgtaccctct tatcccattt gctgacaagg atcctgaagg cccaagcata gaaagaagtt 900
   gctatggctg ccatgtgtca gcagcatagc catggccaac tcagggccct gactcctacc 960
   tgaccccctt ctgaatgaca ctcaaggtaa gggtcccctt cccactcaca ggtgaggtga 1020
   aacatttcac cttgaaaagc ctcttgcccc cagcctcccc tcgta 1065

<210> 78
<211> 1126
<212> DNA
<213> Homo sapiens

<220>
<221> SITE
<222> (1124)
<223> n equals a,t,g, or c

<400> 78
ggcaggactg catgactggc aagctttggc tgttgctgcc taggttgggc catgctgcag 60
ccgcccccac cactgcactc tctggatcag aactggaagg tacctcgata tctttgctta 120
tagcattgga cagatgaagg gctggttacc tgtcaggaag tgaagagcgt ggttaagagt 180
cctctatgct aggttgtcac agtcaccagc tactagactc ttggctacaa catttctcac 240
caagagcagt gtccttgggg aaaaactaaa cagggatgag aaaggggtta ggaataaaac 300
tytctcctag agaccaggtc agaatacata atgggtttaa cttcgcaata aagtgacaag 360

gtgcacttga ataagccacc ctgatacacg gaaagcactg ggcacagaag taactttccc 420
attgaatcag gagttgatcc cataaacctt actattagcc aagtttacat ttatgaacat 480
tttacacaca ctactcagtt atatattaaa gacaaaaatt gataaaatac ttatactttg 540
gtaagccata gagccaattc tcttttcaac ctagttgttc atttcaccag tgggcaaaaa 600
tcattatttt taaaggtttc caatttaaga gcacagacca cccagctatt atagagctct 660
atagtttagc cctcgaaggt gagtcccaga tgcagttcag ggatggtctg aacctttgaa 720
cagggcaaat ccaagcactc taactcctgg ttccctgctc tatccctcat ccatgccacc 780
tttttaagc atccatatgg attagtgaat ctcatttcca aatctatgct tggttagcat 840
aatctctcat ccagaactcc cctttgaatt tgagatccta tatctaaggg cccacctcca 900
ttcctccctt caggtctcac agacacctta aagtcaatgt cttatctgtt tccctttcac 960
gctcccaaac gagtcattgt tcatcttcca ctccttattt cagtaactgg aactccatcc 1020
ctccagaagc acaaaacaga acctgggagt catccttgat tcttgctatt tcctcacctc 1080
ccatatccaa cctatcccca agtcctgatg actttacctg ctgnaa 1126

<210> 79
<211> 984
<212> DNA
<213> Homo sapiens

<220>
<221> SITE
<222> (232)
<223> n equals a,t,g, or c

<220>
<221> SITE
<222> (332)
<223> n equals a,t,g, or c

<220>
<221> SITE
<222> (333)
<223> n equals a,t,g, or c

<220>
<221> SITE
<222> (929)
<223> n equals a,t,g, or c

<220>
<221> SITE
<222> (943)
<223> n equals a,t,g, or c

<400> 79
tcgacccacg cgtccgcgcg tccgtcggtc ggtgcgtccg ggccgccggc ttcgmcctcg 60
ccatggcacc ctggctgccg ctgctgtckc tgctggggct gctcctgggc rccgctcccg 120
ccccgccccg ccgagcagcc gacgctcagg cccgggaggc ggcgtacccg gagctgctgg 180
ggcccgcccg cttcgcgctg gagatgtaca accgcggccg ggcagccggg angcgggcga 240
cgctgggggc cgtgcgcggt cgcgtccgcc gggcgggcga ggggtcgctg tactccctga 300
gggcgaccct ggaggagcct ccctgcaacg annccacggt gtgccagctc cctgtgtcya 360
agagaccytg ctctgcagct ttgaagtctt ggacgagcta ggaaaacaca tgctactgag 420
gcgggactgc ggcccagtgg ataccaaggt cacagatgac aaaaacgaga cattgagttc 480
agtccttcca ctgttgaaca aggaaccccct gccccaggac ttttctgtga aaatggcctc 540
aatcttcaag gagttcgtta ccacctataa tcggacgtat gagtcgaagg aggaaaccca 600
gtggcgcatg tctgtctttt ccaacaacat gatgcgagca cagaagatcc aggcactgga 660
ccgtggcaca gctcagtatg gggtcaccaa gttcagtgac cttacagagg aggarttcca 720

<210> 82
<211> 1392
<212> DNA
<213> Homo sapiens

<400> 82
attcggcaga gcagaaaacc agactgcact tgctttataa aacagagctt tatttttcct 60
tcataataag cagagttgca gtgttgctgg tattgattca ctggcgtggt ggtatcagga 120
cagatgtctc tatgattaat ttttggcctg tcactcatgt ttgcatatgg ctgttgtggc 180
tccaagcatt ggaagcaaga ggacagggaa gcaacattga ctgtaccagg aactccaaaa 240
cagtcttcac atcttaatgg ttggacaatg ccaaatggtc actcttttct ggaagttgac 300
tggggacaag atagtggtaa ggattagatt tggccagaaa gtttctgcca cagtgagctt 360
tcctgtctaa atccttattt taactgttgt cacttaatat tcacactttg gaaggacatc 420
tactgttggt tacaattatg aaaccaactt gaatactttt tagttgaaca tttcagtagt 480
cttaattatg tttaaatagg tttcacaatt tactgttttt agtttagttt ccggctcccc 540
ccaaccccca acttttgyta gagagttact ctcttaactt ttgctagaaa gtagcaaagt 600
tctctactct acatgttcag ggctggctgt agaatttcgt tttttaagga aacaggaaga 660
cagaactaat tatgcaagtc ttcatttagc tttttaaaaa aacagcttta ttgagttaga 720
attgacatgc agtaaatggt acatatttaa agcgtacaat ttgttaagtt ttgacataag 780
tatacattgt gaaaacatca gtcaccacaa tcaggatact tattttaaaa aacaacttta 840
tttaggatta gtatactgat aatgtgtcca ttgtaagtgt acattttcag ttttgacaaa 900
tgtatagatt tttgtaacta ccaccaccag tcaagatgaa aacgtttcta gcactccaga 960
aagttccctt gtgtcccttc ttggtcagtt attcccacca tgctctcagg caaccacagt 1020
tctgcttcta tcactatata agtgacagaa tttttctaca gaatttcaca tagatggaat 1080
catacaatat gtactgttct gtctggcttc ttgaggtaag ccaaatgtct tttaagagtc 1140
atgcatgttt ttgcatttat tagtagttta ttcttttttt gttggtgagt agcattcatt 1200
gtatggatat attccagtct gttttattca ttcacttttt ggacatttgg gttgttatca 1260
attttgggct cttttgaatt aatccctccc tccttccctc cttcccyccc tccctccttc 1320
cctccctccc tccctctctc cctccctcct tccttccctc cctccctccc tccctttttt 1380
ttttcggcac ga 1392

<210> 83
<211> 1155
<212> DNA
<213> Homo sapiens

<400> 83
ccgggtcgac ccacgcgtcc ggtgagtgca ctctaggatg ttcacatgat gacaaaatca 60
cctaacaatg tagacgcttc agaacatata ccctttgtta atcgatgcat cactgtatat 120
atgtgtgtat atacacacat atatgtacat atatttaata catttgtgta tgtgtgtgta 180
tatatatata tatatacttc tcattattta tactctagac ccagagcctc ctagctggtc 240
tccaaaattg gactctcatc tctctttgag acagccttca aatgatcgtt tttaaagtgc 300
taattaactc ctcttctcaa aatgcttcaa tggcccacta atctctaccg aatcaaggaa 360
ttcagccata ctgtcccaag atatctttcc ttggccagtt ggagcctcat ttcagctgct 420
ctgggtttat cccctgtctc ttctttccca cttccaagcc tgtgctcagc ccacctcctc 480
ttctggggat gccccacacc ccactctgcc atatctgcca aacctttcat ctccccgtga 540
agctcttgac accaaataca gtttacttta gaaaatgtat tttttccact ttctcaacta 600
aacttttcct tgtgtgatct gcttttccgc tgccaaggca catcgttttt aattctctac 660
agcactgctc atatcttgcc cagtattata gcttctacat attggtcttg cttcttattt 720
ttgagcacaa aaactaagcc actccacttt ctcttaccag tgaatccagc ttaaaaaaac 780
tgtgagcaac ctatcagtat tttgttgaca tgaactctat agaaacctta gtccctggat 840
cttcgactct gcctcccctg acatttatct gctcccacaa agcacgcagg tgtgggaaga 900
gaagtggctg tttttttgagg tcacatttca gccctgattc atcctaatgt cttcaccctt 960
tttatccttt ggscactgtg tycctagaga tgtgaattca attccgcacc attctctcct 1020
ttacaatgat gccaatattc tcaggctttt aagactaaat tttaaattac gagaaaattt 1080

gatcttcaaa cttaagttgg acctagaaag aacaatctca tgaactcaaa aaaaaaaaaa 1140
aaaaaaaaaa aaaaa 1155

<210> 84
<211> 1373
<212> DNA
<213> Homo sapiens

<220>
<221> SITE
<222> (877)
<223> n equals a,t,g, or c

<220>
<221> SITE
<222> (897)
<223> n equals a,t,g, or c

<400> 84
gatgtgctgc tgcctctgct gtacctcctg gtccggaagc acatcaacag agcgggtatc 60
gggaacacgt ttcagggagg tgccaactgc atcatgttcg tcctctgcac ccgcgccgtc 120
cgaactcggc tcttctctct ctgttgctgc tgctgctctt ctcagcctcc caccaagagc 180
ccggctggca ctcccaaggc tcccgcgcct tccaagccag gagaatctca ggaatcccaa 240
gggaccccag gggaacttcc aagcacctgg agcttttgtc ctttctagtt ctgtggcata 300
ggtgctgcct tcctgggggt aggcamttct gtgagtccct gstgcagggc agaagtgcat 360
gtctgctgca ccagaagccc ccgctattga tgctctcggc tccattggga gagcagctgc 420
caactcagct tcttctacct cctagatcct caggcagcaa gttccamcgg taccagcgtc 480
ctggcccacg ggtgggcgtt catctgcata agggtagcag tgagattcgg gaagccggtg 540
gcccacagct gtggccacag tgcccccacc cagtggacct tgatgtcctg aggaccacac 600
agcactgttt acaatctgag ggacccacat ctgtgcacct atcctctgtg tgattaaata 660
agctgtcagg ggccctcaga gatgtgtccc catccacaca tcatggagca gattcccggc 720
tggtcagggt gttaagattc tctggagaac ccggctttcc ttggagactt tgctcaacag 780
aggaaccakt tgccattcac caggttgaag gtyccctgar gttgcatggt gaccaccagg 840
ggcaagcaca gcccggcgca ctgctcaggg acagcantct ccccgggsct cytggcntct 900
ccytccatgg gctgctaacc cttgccgtag cttgggtttg agtgtcagca caggccagaa 960
tccacactcc tgtccccact gtcatcactt cagacacagg ttccctcctt ccaattccaa 1020
agctggaaca agggcaggaa gaggcgtctc agggaacccc cgaagacccc actgtctctc 1080
cagctccttt cttagaatcc tcctcttctt ggggagacca aaggggatga gtagggaggg 1140
gttctggctt atttacttct tagtttagtt ttttgtaagt aaagtggccg gagagatgag 1200
taatccaaat acgattcctc aggccgggca cagtggctca cacctgtaac cctagcactt 1260
tgggaggcca aggcaggcag atcacctgag gtcaggagtt tgagaccagc ctggccaaca 1320
tggtgaaacc ctgtttctac taaatataca aaaaaaaaaa aaaaaaactc gta 1373

<210> 85
<211> 1258
<212> DNA
<213> Homo sapiens

<400> 85
tgcactgtgg gtgtccaccc acaccttgtg ttcctcatgg cctaccccag cctttcttct 60
ccactgggtc ccactgttcc ctggagacag agggctagca tgctgtcatt tatctgaagg 120
ttgtggctga cccattctcc tgggatttcc caggccacct ctcctttccc tttccctcac 180
ttaacccaga cttgctcagc tgaggctatt gtccctgatg ttggctttac ttgtaggagg 240
tttagtggct gctctggcct gccatggaat tttggctgca attttggcag tgtgtggaga 300
actggtatca ggaaagggaa ccaggagtag tgatgaagat gatggtgggg atggggacag 360
aggacatagg ggactgtccc tcttgaactc tgcctttggg cacatgggag atggggacag 420

gaaagatgat aacagtggaa ccctgtgaag aataggagaa tgggaattct aaaataccag 480
ttcccaaaca aaccatctct ccattggaaa aggagctgtt gcaaaccgtg ttcatttaag 540
cttctgatga aaagcaatgt atgtttataa gcttgcaagt aacagacaag atgttccaag 600
gccgatgagg aaaatatcgc ttccctgtct ctgcctctac cttgctgtgc ctctctggct 660
ttagcagcat tggatgttga attggtttgg ttcatttctc tgaagtcgga ctgtcaggag 720
gagccagagg ttggctttct gggatgtgaa ggggacagtg tcagcctaag cctctggaga 780
tgcttaggat gctgaatttt aaccccttct ttcactgcat ggcccttcaa acagggattg 840
gcaggctccg cgagttacag agtgggtgag tcttggagag aaccaacgac taaggtagct 900
tagctctctg ctttgttctt gggagccaag agaatgcaag atcctggtgg gtgagcagtg 960
gtatgaagag gggacactgg tgggtgaaca gtggcatgga agtcactgat gcagcctctg 1020
gccctcagtc tccctatcgg caaagtgggg ggctcttcct atctctaaag agtttcatcc 1080
ctgacaggtg gatgggtgaa agatccagag agtgggggtg aggggtgggc cctgaagact 1140
ttttgtctgt agctcttgca tattgagtgt ataaacccgg cttctggacc aacccaagat 1200
gaataaactg gggcagaaaa ttaaaaaaaa aaaaaaaaaa aaaaaaaaaa ctcgtagg 1258

<210> 86
<211> 1318
<212> DNA
<213> Homo sapiens

<400> 86
cccacgcgtc cgaacagctc ttaattggtg ctttcagttt ttaatggact tcctgatgtt 60
aagagagtga gagagcagtg gtttacttaa tcaccggggc ccttttcata gtttcgtctt 120
gtgtgctgtg ctttttaccc agctctagga gggagatgtt tgtgggtacc agggttttgt 180
tagtccctct cccctttttt agtataagtg gcatgctggc aatagacaaa taccttcata 240
aaaagctgct tctaaatgaa ataattacta cctccacttg ggcattatga taattatcaa 300
tagaatattg aatcagcttg gtttttcagg aaaaaaaaat ccaaacaaac cttccttaaa 360
caaggaaggt tttatttcag gtctccaatt agagtaaaca agcatttggg gcttttcctc 420
ttgttttctg tgttaggatt gtgaatacta gtctgctata gaaggttgct agttactttc 480
tgtaatgtag gcagtttctg gggattggag acacagatgg gagtaaaggg tctggattct 540
tctctttccc acctgcctgt ttggctttcc ctcagcctcc caccctgtgc ccatgtgccc 600
cttctctgat ctctgtgctc agatggcagg gaccatagga gttcatgtgt ccagaggtaa 660
cactacccga gtctgtgata ctcagaagct tccagttcct tattaaaacg ggaaattgaa 720
cagtaaattg ttactgccta ccagatttca cagcaggaga ctgtcattac acctttcatt 780
tgactcagag agagtgtagc catcagatgc ccagattttg ccattcttta gtcacttgga 840
aaatgcaccc attgtcagca ttttgagtta ttaggattcc tcaaatgaaa aaaaggcaaa 900
caatatattt tgatggattg aaatccacac tgttgtgctg aaaatagggg aaggaaaaaa 960
ggaagccaag attttggcag aaaactgaac actcttaaga ataactcctc agccgggcgc 1020
ggtggctcgc gcctgtaatc ccagcacttt gggaggccga agtgggcgga tcacgaggtc 1080
aggagatcga gaccatcctg gctaatgtgg tgaaaccccg tctctactaa aaatacaaaa 1140
aattagccgg gcgtggtggc gggcgcctgt agtcccagct actcgggagg ctgaggcagg 1200
agaatggcgt gaacccggga ggcggagctt gcagtgagct gagatagcgc cactgcactc 1260
cagcctgggt gacagagcga gactccgtct caaaaaaaaa aaaaaaaaaa aaaaaaaa 1318

<210> 87
<211> 978
<212> DNA
<213> Homo sapiens

<220>
<221> SITE
<222> (977)
<223> n equals a,t,g, or c

<400> 87
   ggcacgaggc gcgccaaggc gtcagtcgag gagtcaaggc agcaatgaat cgtgtcttgt 60
   gtgccccggc ggccggggcc gtccgggcgc tgaggctcat aggctgggct tcccgaagcc 120
   ttcatccgtt gcccggttcc cgggatcggg cccaccctgc cgccgaggaa gaggacgacc 180
   ctgaccgccc cattgagttt tcctccagca aagccaaccc tcaccgctgg tcggtgggcc 240
   ataccatggg aaagggacat cagcggccct ggtggaaggt gctgcccctc agctgcttcc 300
   tcgtggcgct gatcatctgg tgctamctga gggaggagag cgaggcggac cagtggttga 360
   gacaggtgtg gggagaggtg ccagagccca gtgatcgttc tgaggagcct gagactccag 420
   ctgcctacag agcgagaact tgacggggtg cccgctgggg ctggcaggaa gggagccgac 480
   agccgccctt cggatttgat gtcacgtttg cccgtractg tcctggctat gcgtgcgtcc 540
   tcagcactga aggacttggc tggtggatgg ggcacttggc tatgctgatt cgcgtgaagg 600
   cggagcagaa tctcagcaga tcggaaactg ctcctcgcct ggctcttgat gtccaaggat 660
   tccatcggca agacttctca gatccttggg gaaggtttca gttgcactgt atgctgttgg 720
   atttgccaag tctttgtata acataatcat gtttccaaag cacttctggt gacacttgtc 780
   atccagtgtt agtttgcagg taatttgctt tctgagatag aatatctggc agaagtgtga 840
   aactgtattg catgctgcgg cctgtgcaag gaacacttcc acatgtgagt tttacacaac 900
   aacaaatgaa aataaatttt aattttataa tatgggaaaa aaaaaaaaaa agggcggccg 960
   gtaacccatt gcgcccna 978

<210> 88
<211> 1863
<212> DNA
<213> Homo sapiens

<220>
<221> SITE
<222> (82)
<223> n equals a,t,g, or c

<220>
<221> SITE
<222> (112)
<223> n equals a,t,g, or c

<400> 88
tgggggttgg gatgcagagg catgaccccc tttcttcccc tgccccttcc tgacatgaga 60
tgctctatga tagaggtagc tngggcgtgg agtgaaacag aatgtgggtt tncggggagg 120
aagtgttggg gtcacatgct gcttctcctg ctttcttaca taggtgcttc agcgaagagt 180
cctgcgtctc catccctgaa gtggagggct acgtggtcgt ccttcagcct gacgcccccc 240
agatcctgct gagtggcact gctcattttg cccgcccagc tgtggacttt gagggaacca 300
acggcgtccc tttgttccct gatcttcaaa tcacctgctc catttctcac caggtggagg 360
ccaaaaagga tgagagttgg cagggcacag tgacagacac acgcatgtcg gatgagattg 420
tgcacaacct ggatggctgt gaaatttctc tggtggggga tgacctggat cccgagcggg 480
aaagcctgct cctggacaca acctctctgc agcagcgggg gctggagctc accaacacat 540
ctgcctacct cactattgct ggggtggaga gcatcactgt gtatgaagag atcctgaggc 600
aggctcgtta tcggctgcga cacggagctg ccctctacac caggaagttc cggctttcct 660
gctcggaaat gaatggccgt tactccagca atgaattcat cgtggaggtc aatgtcctgc 720
acagcatgaa ccgggttgcc caccccagcc acgtgctcag ctyccagcag ttcctgcacc 780
gtggtcacca gcccccgcct gagatggctg gacacagcct agccagctcc cacagaaact 840
ccagtacgta agcctggtgg ggctgggcag ggaggggcag gtggcaggtg agtgtgttgg 900
gacaggtatc ctccccctcc acctctggga gaggacaagg taggtggagc aatgggttct 960
gttccctgtg gtacctgcct tagttgacag ctatggacca atccctctct ccctttggat 1020
cattcacttt cccttgtgaa gaacatgagc cttgtgtcat tgtagggatg cttatgcctg 1080
cartcataaa gtcagtgtgg gtttaggcgt gtttgttata cctgatgatg cctccttccc 1140
cagccccaag caatatcaaa ttagccctgg acctaggatc ytctgtggtg aaactatcaa 1200
gtatttacta agcacytact atgtactagc actgtcctaa gatgatctaa atgatttata 1260
ggatagatca gtccctatcc ccaaggagag aattggtact attacatgta aaattatgtg 1320
ctacgtatgc agtcacaaga atatttctaa aaaatatatg caaatatgca aagcgttaca 1380
ttcagatgga gtgctgccct tcaaagctgc ccttcaaagc aacctggccg tggtcccaaa 1440

ggttcccggt catggtcctt tacgaatggt gcaattttat tttcaacttt tgagttgcgt 1500
aattgtagaa aaaccagaaa atatcaaata tttcttttaa agaagaaaaa tcaggccagg 1560
cacagtggct catgcctgta atcccaacac actaggctga gtcaggaggc tcacttgagt 1620
ccaggagttt gagaccagcc tgggcaacac agtgagactc tgtctttaca aaaaataaaa 1680
aaaattagct gggcatggtg gcaggtgcct gtagtcccag ctacttggga ggctgaggta 1740
ggaggatctc ttgggcctgt gaggccaagg ctgcagtgag ccatgatcac accactgcac 1800
tccagtctgg gagacagagc aaggctctgt ctcaaaggaa aaaaaaaaaa aaaaaaactc 1860
gag 1863

<210> 89
<211> 2086
<212> DNA
<213> Homo sapiens

<400> 89
cgaggaatgg agccggtagc tgctgcggcg agtsccgcgg ctcctccgta gacccgcgga 60
gcaccttcgt gttgagtaac ctggcggagg tggtggagcg tgtgctcacc ttcctgcccg 120
ccaaggcgtt gctgcgggtg gcctgaatgt tcgcatctta ccacatacag ttctttacat 180
ggctgattca gaaactttca ttagtctgga agagtgtcgt ggccataaga gagcaaggaa 240
aagaactagt atggaaacag cacttgccct tgagaagcta ttccccaaac aatgccaagt 300
ccttgggatt gtgaccccag gaattgtagt gaytccaatg ggatcaggta gcaatcgacc 360
tcaggaaata gaaattggag aatctggttt tgctttatta ttccctcaaa ttgaaggaat 420
aaaaatacaa ccctttcatt ttattaagga tccaaagaat ttaacattag aaagacatca 480
actcactgaa gtaggtcttt tagataaccc tgaacttcgt gtggtccttg tctttggtta 540
taattgctgt aaggtgggag ccagtaatta tctgcagcaa gtagtcagca ctttcagtga 600
tatgaatatc atcttggctg gaggccaggt ggacaacctg tcatcactga cttctgaaaa 660
gaaccctctg gatattgatg cctcgggtgt ggttggactg tcatttagtg gacaccgaat 720
ccagagtgcc actgtgctcc tcaacgagga cgtcagtgat gagaagactg ctgaggctgc 780
gatgcagcgc ctcaaagcgg ccaacattcc agagcataac accattggct tcatgtttgc 840
atgcgttggc aggggctttc agtattacag agccaagggg aatgttgagg ctgatgcatt 900
tagaaagttt tttcctagtg ttcccttatt cggcttcttt ggaaatggag aaattggatg 960
tgatcggata gtcactggga actttatatt gaggaaatgt aatgaggtaa aagatgatga 1020
tctgtttcat agctatacaa caataatggc actcatacat ctggggtcat ctaaataata 1080
attaaagtgg ctttcataat atgtaacttt tgggttctgc ctttttcaga aaatggaaac 1140
ttgggccatg tgtatttcaa acaaaaataa ctttagatat atcttttttg tagcyttgat 1200
tgatgctcta agatcacatg agggtagtat ttaatatatt agatgaagga caactttgga 1260
cataacactg actaggagtt gagagctttt gcatcaggca gaagcaaact gattatagtt 1320
gtgttgcacc agatcatgta gctgctgtgt aacatgacct taaatagtct tcctgcatag 1380
gaagagcaaa agggtattca tcaataggat atagatttaa gacattccct gactacccct 1440
tgcgttgtta ggtgatgtct tttagcagaa tcatgaagac cttttttctc ccttaataaa 1500
ggagaaaaat atactgatgg ctggagaaat ttttctctgc ctttcagttt tatgaatttt 1560
ttcagaagta acaatattat tattgacttt ttacttattt gataaaaatt aaagaactat 1620
ttttgttttg gtcagataaa ttgacaagac taatcagtat tttattataa gtaaaagatt 1680
tttcttcttt ccttaaaaat attttttttt cacctaggtc taaatagcta actaactggt 1740
agaccagagt attacatcat cttattttgg ttttatacca ataaaacata gcgtggaact 1800
cattcaggta atgttttgca tttcattgct tttggatgaa caaaggaagt aaactaatcc 1860
tttataaatg aaaacccaga atagttggta tgtcagctag tcattcctgt catattccca 1920
gtagaatgat tttcaagttt gaatttctgt wcaaatatct aaataagaga tgtgcagaga 1980
gcaccaattt tccttcaata tccattcttt acttttcaca taatgataga acctttgatt 2040
tttcaagtgg gtatgcctcc tagaataaag actacatttc ccgagt 2086

<210> 90
<211> 891
<212> DNA
<213> Homo sapiens

<400> 90
gaaattatgc atgagctgtg attgcaagta atttttaaac gtctgcatgt ggacaaagta 60
aatacataaa aatgaaaatg gctgtgtaag agagtaggat gaacaataat aattgttttc 120
cccactgttg tgaattatga tgttatcttt taacttcaca tggatggttt gggtttctct 180
agttcttaaa agtcaaagag ccaagttagc cctccattcc ctccatcttc atcaagaggt 240
caggctcaga atgtcacgga gagagtcgcc tggcaggcct cttcgttgtg gggtgagagg 300
gaacatgggt gccagaaccc cagtgccaac cgctgactat ccttctccct acaggacatt 360
gccgagaatg gctgcgcccc caccccagaa gagcagctgc camagactgc accgtcccca 420
ctggtggagg ccaaggaccc caagctccga gaagaccggc ggccaatcac agtccacttt 480
ggacaggtgc gcccacctcg tccacatgtt gttaagagac caaagagcaa catcgcagtg 540
gaaggccgga ggacgtctgt gccgagccct gagcaaaaca ccattgcaac accagctaca 600
ctccacatcc tacagaaaag cattacccat tttgcggcca agttcccgac gagaggctgg 660
acctcttcat cacattgact tacgccgttg cttttccaga ctgggcagag gggctgactt 720
cgcagtgtgt gccaaagarc cggtgtctga taatcccatt ttcctgctta tcacctgaac 780
tgtgtcagta tcacttttag ttttgttggt tggttggttt gttgtttgtt taatatgccc 840
tgttttctac ttctgttgga aaatatttgg ggttgaaata aaccagtggg a 891

<210> 91
<211> 1974
<212> DNA
<213> Homo sapiens

<220>
<221> SITE
<222> (654)
<223> n equals a,t,g, or c

<400> 91
aaaattgcta attaatatta gtatttggtt gctttatctt aaaacaatac ctacctctgt 60
taataggaaa attagaaatt atatctattg ttcctaaaga tacaatttgt ttctctaact 120
ccaagggaag tgaatgaata tgtttcagac tatacttgtc tgtgtattat ttgtttttgt 180
aaggtggttt tttcttttgc ttcaaattga gagcatacaa acaaaattcc actgtataag 240
cagtcaattt tggtaataat aacacatgcc tagagcactc agattaaatt attacacata 300
ctaataaaat tattcacagt agtactcctt ggtaatgttg gtattaggga acagggatag 360
aacccatctt caaagtcaac cctattactt caagcctttg caggtcctgc tgtttccaag 420
gatacaatcc aatgcttaat ttacaggaat ccgaaatcat ttgctgtgtt aaaaacttga 480
gattagccct tgctgccccc ttccaggttg gcctttggaa gcgaaaggtc tttaagctta 540
agtaaactgg agaatatttc tgagtaagca ggctccaggt ggagggtaca ctccaagata 600
ccttcacttg ccaaaacaac actccctgca aggttatcag cgaaaaggca gtanaaagag 660
ccctcctgct gccagaaggt cttgttttgc aaggcttctc agcctgcctg cagccattgg 720
gtgaggagag gaaaggacac ctcacctggc agagcagcac aaaggtcagg catgtttccc 780
tcctgacctg gaagctgggt cctgctcctt cacatgaact gtccagggct tggtcatata 840
cccaggtccc ctcagtcctg cccagartga ctccactggt ctccccagaa aacaccaaca 900
gcagararaa atgaartgtg tctgttttct ggtttcatat tgcarctcct cttgctcttc 960
ytaggacaga aataacatct gcmatgccar aramctgtga agtaataaac aacctttccc 1020
ccagctcttg acaagcatta ccttgcttkg aggtgcaatc agttytgaca agtttatggy 1080
tttgtgtcyt tcaacccaat ggacattacc ytctggggga gtttaatgac ccytytcagc 1140
ttcacactca cttgaaggaa agagctagaa gtcagtaatg atgtgttatg acttgcatgg 1200
cttcaggtag ttcagaacct gcaacctgta gttcagatta caggagaaga actcagacga 1260
taaattggct tccaaagaag gccaaataga aaaaaaaaag gttcttcaga ccaatagctg 1320
agtgacctga aatttaaatg caaaaaatgc attaaaaccc acaaaaccac ttcctttaaa 1380
attcttgata ccaaacatgt tacagcatta agtgaacctt aatctaagca aaattcagag 1440
aagagcattc acacctttga tagaagagcc agccagccag ggtgctgggg ctcagctctt 1500
gagaaaaatg ttgatgtaaa agccttggtg gcctggttca gcccctgagc ctccctggta 1560
agcacatggc aggctcagaa catccataac tgaatgctct gagccctgta aaaatgcagt 1620
tacaatggga aatcaactct taagtatgct atgggatgct gtgataaaat ttgagtttct 1680
ctaccttctg tttgcacttt ggttcattta gaaaagagtc cataattgtg tgcttcaaga 1740

gtgcaattcc atggctgtaa cagaatagag tcctctttct tctggccttt gcctctttgg 1800
ggatattcca actctgtgga aggtgatcat ttgcgattat gatcagttat ttatatttga 1860
ctgtaaatga aaacttcaga gtcagtttca aaaaacaaga gatggacata aggacatgtg 1920
cttatgagta ggggacaaat aactagagac aaaaaaaaaa aaaaaaaact cgta 1974

<210> 92
<211> 1423
<212> DNA
<213> Homo sapiens

<400> 92
ggcacgagat actttcttat ggaacttgta tggtttcgtt ttttacattt aaaccttctt 60
ccccgtggtg tgtgttgtgg aatctgtgtt tgtgtgagga ggggcatggt gctctcagaa 120
cccacctcct gtggccagag agccctgtcc tgtgagggtg gttgtcacag tggcagggtt 180
caattcagaa gaccttgagg gcaggctgat gtttcctgaa tgggcccctg gttgttgctt 240
gtccctgact ctccatttcc ccatctgagt ggatttggac ctaatagggc actggagctg 300
gttcgaatcc tgactggact acttggcaac tttatgtctg ggagcaagtt acttaacctc 360
cccaagcctg tgtctgtgaa atgcgggtaa atgaatgtag atgtttggca gcagctactc 420
cttgttgagc tctcacagtg aactctcctg cctctgccct ccttccccgc ctcccctggt 480
gcctagcgtc aggtctagcc acttcctcct gggcccctct ccctttctcg tggctggctg 540
cctgcccgcc tggcgctgga cctttcatgt aacgggaatc agcatgtata ttctggtctg 600
gtctgtttct acacttaatt ttgtttccag tagtatttcc ctgtaccggc agagttcaca 660
aacacatttg aagaggcttt ttctcaggat tcttaacctt cccaaaggaa gtcccatgga 720
tgggtttcta gaagtctata aaatgctctg aaattgtatt tttcctgtgg aaagcataac 780
tttcatctgc ttgttcgtgc tcaaaaaaga tcatgaatga atgattgcat gattttatgc 840
cattgtgctt atactaaagg atatgtagcc catctcttga gctgktaaac tgttttgact 900
actttaaatc gtgcagctgt gagcatctct gtaaatttag tgtacacatg tatcccctgg 960
agtggcattg cctcggcagt gagcacttat ggttttataa ctctcttcac agactcaaat 1020
gactccagaa agctacactt cctgttgtga gtatatgata tccatttccc tacatagcca 1080
ctaacatcag gtttttacaa ttttatttat ttcttgctac tttaagaaat ttttgtggtg 1140
aaatacatat aatagaagtt gactatctga atcattttta agtatacatt cagtagtgtt 1200
aagtatgtcg ccattgttgt acaaccaatc tccagaactt tttcatcttg caaaacaaac 1260
tctgtaccca ttaaataaca ttaaacattc cattccctcc agcctcagca accccattct 1320
actttctgtt tctgtgagtt tgactattcc aagcacttca tatcagttaa atcatgaagt 1380
atttgtctgt ctgtgactgg cttatttctc tgagcacagt gtc 1423

<210> 93
<211> 1365
<212> DNA
<213> Homo sapiens

<400> 93
ggcagagcta acccgagtga agccacttcc gggcttcccg ggcgccttcc gcagtcctct 60
tccgggtgat ggcggccggg tgccccggat gtagccctgg cgcaagatct cttctttttt 120
ccacctcgcc ttccgcggat tcccagcttg agaaacacct ctttgccccg tcatgccaaa 180
gaggaaagtg accttccaag gcgtgggaga tgaggaggat gaggatgaaa tcattgtccc 240
caagaagaag ctggtggacc ctgtggctgg gtcagggggt cctgggagcc gctttaaagg 300
caaacactct ttggatagcg atgaggagga ggatgatgat gatggggggt ccagcaaata 360
tgacatcttg gcctcagagg atgtagaagg tcaggaggca gccacactcc ccagcgaggg 420
gggtgttcgg atcacaccct ttaacctgca ggaggagatg gaggaaggcc actttgatgc 480
cgatggcaac tacttcctga accgggatgc tcagatccga gacagctggc tggacaacat 540
tgactgggtg aagatccggg agcggccacc tggccagcgc caggcctcag actcggagga 600
ggaggacagc ttgggccaga cctcaatgag tgcccaagcc ctcttggagg gacttttgga 660
gctcctattg cctagagaga cagtggctgg ggcactgagg cgtctggggg cccgaggagg 720
aggcaaaggg agaaaggggc ctgggcaacc cagttcccct cagcgcctgg accggctctc 780
cgggttggcc gaccagatgg tggcccgggg caaccttggt gtgtaccagg aaacaaggga 840

acggttggct atgcgtctga agggtttggg gtgtcagacc ctaggacccc acaatcccac 900
acccccaccc tccctggaca tgttcgctga ggagttggcg gaggaggaac tggagacccc 960
aacccctacc cagagaggag aagcagagtc gcggggagat ggtctggtgg atgtgatgtg 1020
ggaatataag tgggagaaca cgggggatgc cgagctgtat gggcccttca ccagcgccca 1080
gatgcagacc tgggtgagtg aaggctactt cccggacggt gtttattgcc ggaagctgga 1140
ccccccctggt ggtcagttct acaactccaa acgcattgac tttgacctct acacctgagc 1200
ctgctggggg cccagtttgg tgggcccttc tttcctggac tttgtggagg aggcaccaag 1260
tgtctcaggc agcgaggaaa ttggaggcca tttttcagtc aatttccctt tcccaataaa 1320
agcctttagt tgtgtaaaaa aaaaaaaaaa aaaaagggcg gccgc 1365

<210> 94
<211> 756
<212> DNA
<213> Homo sapiens

<400> 94
agcacgaggg tgggaatgtg aagagggcag cccaggccct gtgtttcggg ggtgtgcctc 60
tccccgcact cccgtttctg ggaatgctgt tccttctacc ttcagggcct gcccgccctg 120
cggtgtagcc gcactcctcc cgggtgtcat ttcttcagag tctttccttc atgccctttt 180
tcctcctcac gttccccctc gtgctttacc cacatctgtc ccgtggttcg gatccagttc 240
tcccgtgcgt tatgggtatc cacgtgtttg gtcttagcca tcactccagg aaagtggctc 300
ctccctgagg acagggctct gtctctgatg ctgctggcct ccctccagtg ctgccctcct 360
ccttttgggg cttggtggat gcaagtgctg acacacaaag gcaggcaggc aggcctcggg 420
ccaggggtgt cctccaggcc cctctgatgg cagggaccag cgagacctgg ggaaactgca 480
cagtctgtgc ctgtctgtgc tctcaggaga ctgagggaat tagtgggtgg gagagatttt 540
atgttggatt aaaaactgca cctccaggcc gggcatggtg tctcatgcct gtaatcccag 600
cactttggga ggccaaggtg ggaggatcac ttgagccctg gagtttgagg cttccaggaa 660
gctatgatta caccactgct ctccagcctg ggcaaacaga gtggcaccct gtctctaaaa 720
acaaacaaac aaacaaacaa aaaaaaaaaa aaaaaa 756

<210> 95
<211> 938
<212> DNA
<213> Homo sapiens

<220>
<221> SITE
<222> (479)
<223> n equals a,t,g, or c

<400> 95
ggcacgagtg gaggttcagg gtcagggtcc aggggcgaag gtggacgctg gagaagggca 60
gatggacagg gtcaggttca gatcttggct cttgtatcct tgctgtgtgg ctctgggcca 120
agaacttggc ctctctgcgc ctcagtggct cattacagaa aatggatgcc cagcacttgc 180
cttagtgggt tgttttgagc caactgcagg ctcagggagt agctggcatg atgtgttcct 240
accctgaagg gcagaaaaag gggaaggagg ccaccagatc ccacaggtgg gtccccaggt 300
ccctccccgg gatgggcagc akcctggctg ctccccacag caacccctgg ctggccccat 360
tggcgctgct ggaaatcccc camccagttc tttgtgaatg gaaaaggaaa ctgattgcgc 420
tagaagaggt ctccgaatgc cggccggggg tggggggcgg ggggggsttc ctctcccant 480
gcaggagggg ccacctcagc ttcctttctg gggccccata cccccttttt cctatctccc 540
ctctgasttg aggaggaggc tgtggccccc gcccactgtt tggcctctgg aaccactggg 600
tgacagtgta catcggggcg attaagggcc acctggcctc tggacctccc tcgctgtggc 660
tgctgcctgg gaccccccac cccatgcctg actcgaaccc caacctcagc tctacacaca 720
ggggcaccac tgtaggagga gagagaaacc tctctctagg gaaccacctg gaaggggcc 780
ctgccctgtc ttcatctggg taccccatgt aatctaggaa actgtcttta acttgccgag 840
ggcctccatg tctgagttcc taatgttttt tattttgctt tttcaattaa taaagctcat 900

ggcagaaact atttaaaaa aaaaaaaaa aactcgta 938

<210> 96
<211> 928
<212> DNA
<213> Homo sapiens

<400> 96
ctgcaggaat tcggcacgag gcagggtggg tgcatcaagg gggttgtgcg gtggctgaca 60
agaggttgta atgtggggaa gggcaggagc gggctggtct ttacccactg ccatccccgt 120
cctcttatta aatagagatt cctagtattg ctgttataaa aaatgtcctg ctaaacatag 180
ttctctcaaa aatatttttc tgagattctc tgacaattaa atctggatcc cagccccaat 240
attcacctgc aattacatta tggatgaaat taaatgtgca ctttctatgg tgtacattta 300
tttttcaaac ctcaggaagc catattgagc tcttaatctc aggtcaagtg tcttcttata 360
tcccctccct tgatttttgt actcataagg ttgtatccag agagaagttt gaggaataat 420
tcattacctg ggaattaaag taaaccttga gcttgggagt cctaacatat aaccatctct 480
aaaattctgc aactgtagat ttttaatcat ctaattttag ggacttcaaa atatttttct 540
gactttacct acattcgaat taagttaaaa tagcactgat aatggatagt aggatccaaa 600
cagaaacatt ttaaatgaat ctagttaagt attgagccgg gcacagtggc tcacacctgt 660
aatcccagca ctttgggagg ccgaggcggg cagatcacct gaggtcggga gttcaagact 720
agcctgaaga aaccccatct ctactaaaaa taaaaaatta gctgggcgtg gtggcacatg 780
cctgtaatca cagctactcg ggaggctgag gcaggagaat cacttgaacc cgggaggcag 840
aggtttcagt gagccgagat cacaccattg cagtccaccc tgggtaacaa gagtgaaatc 900
cgtctcaaaa aaaaaaaaaa aactcgta 928

<210> 97
<211> 1715
<212> DNA
<213> Homo sapiens

<220>
<221> SITE
<222> (17)
<223> n equals a,t,g, or c

<220>
<221> SITE
<222> (34)
<223> n equals a,t,g, or c

<220>
<221> SITE
<222> (40)
<223> n equals a,t,g, or c

<400> 97
cacggcctat ggtttangtt aaaaccctta attntttttn cggtaatcac ttttaacact 60
gctaatggaa attatgtttt catgtgtttc acatgaaaaa gtatatatat acaaggttaa 120
tatagtgggt aaaatacttt acatagtcac acatttacaa atttttcaag aggttagcca 180
ctaagacttt aataatttta caagggaaaa agcctttttt tttytttgat atacagtttt 240
ttcttcttag ttctgcatta gaaatggcat ctgttttagg tctcaaaata taactcggct 300
gtttcactgt atatgtacat tgttttctgt aggaataggr taatgatata taggatcatg 360
atattccttc tatccatgtg ccaaatgggt gtaatgttta tttactgatg ctttatgtta 420
ccaaaacata cagtaaaaaa gtagaaattt atgaaatacy tttgataaaa agtttatttt 480
gtgcttacca aaaggaatgc tttcacaata gtgtatcagt tcttttgttt tgttaaagtt 540
ggaatttatt ctgttgccag catttaagta gtcatggcaa gtcctgtttt taagaccttt 600

tggagactgg agctttctgt tccattaagt cttttgttta tactacaaat tgtcacctca 660
cttagttcag atgaaatctg ttactctaca aggaaggtgt tcatcattag gaggcagctt 720
tactaagctg gtgctttgca tggtagcaag tgctgccctt tatcagcacc ctgggtcata 780
gtgtaggcta grgttaaggc actggcagac ttagggatgc tggacagacc tgtagttcgt 840
tttaagtcat gttcacagga atttctacaa taataaaccc atcatctcca taggtcagat 900
cgaagtgcat tccaatgcta aatagaagtt atgagtgggt ttaacaattt tagatgattc 960
agcttttgtt ccattactgt tgaactatat gaactattcc attactgcag agatttaagt 1020
atctgtttta ataagctctt tttgttattt aaaggctgcc catgggtttc tgcctagtgg 1080
taaagctgat tgttaccctc ctttgaaatc ccttctagtt ctgagatgct ttgagggtaa 1140
ctggattcga ttttgggata tcttttctca cattcagact tacacttaat ggtgttagaa 1200
atcaacaaaa ctccttttta aaaagaaaag atattaagcc tgcctacttc tacaatgcat 1260
tctgttacct atttgaacag tatgtttgta actatggcaa tgaagtcagt agataggaaa 1320
ccagttattc cttctacctt taaaaatttt gagaacttgc caaccaggga ttaaagctat 1380
tatcttgaac agagtcccta aagctagtct agtttttgcc acatctgcaa tgattattgt 1440
ttaatttcaa aagaatcctc aggctctaca atctaggggt ggtaaatgtg tttccactat 1500
acttgggaaa aggtcagtag gatgtgcatc ctagggaaga taaaatcgta tatggtaaag 1560
gcatttgagt taattttgca ttatatctag gaaccatatt atttaaaatt tgaatcctat 1620
taatgctgag agatcctaag agctagtatg ttgtaaaacc tgccacctga ataaaatgaa 1680
aaaaaaaaaa aaaaaaaaaa aaaaaaaaac tcgta 1715

<210> 98
<211> 678
<212> DNA
<213> Homo sapiens

<400> 98
acatttctca tgtagtaaac atgtatgtgt atgtgtgtgg gtgtgtgtct aattactttg 60
ttggacagat tcctgtggtt tggaactgct ggggccaagt ttatacaaaa atctacattt 120
ttgagtaaac tccccatgac tctagtaagt ttccacagta tttgagtatt ctttatttcc 180
taaagctgta caaacatcag ataatcaata tttgtaagga tcgataatat aatttataat 240
gaaattgctt tatcagctat taatgttaac tacatcatct tcatatagcc ttataactca 300
tttgtgctat tccattttcc tctgttcctt ttattttcac ttcccttgta atgttagtct 360
ctttgtactg atttctgaag agttcattta tgattaaaca tgttaacatt ttgtctagaa 420
ttgcaaatat gtttttctc attcatttta ctatggtgtt ttattttttg gttatacaga 480
agttgtatat tgaaatataa tcttgttctg ttttatgact ttggagtttt gtggtttttt 540
aaaacatgtt tacattggta tcatttattt gtattgcctt ctatttcagc agttagtttg 600
tcatttcttt ttccattaat catcattctg gtgttaatga atgcattaaa tatttaaagt 660
aaaaaaaaaa aaaaaaaa 678

<210> 99
<211> 1541
<212> DNA
<213> Homo sapiens

<400> 99
ggcacgagtc gcatcaaagt cagaaccagc gccctccgtc cttgctctca gcccagtgcc 60
aggcttcctt gttaggagta ttcttctcat gaaattctac tgatttctta cattgtagcc 120
cagatatgat ttgctgaggg atatctgaga gaaagcctat gtgtcctttc cataaagcgt 180
accttgactg cttttttcaa atctctctct tgctactgat ctttctaact taccttgata 240
ttgggaagtg tggtctttgg agccatgaat ggagaattag ggaattaggg aaacatgaga 300
ggtggtggaa ttaattcatt gtatcttgat tatggtggcg actatgcagg aatatgtgat 360
gtcaaaatca ttgacttgtt cacctaatgt gtgtccatct taatgcttgt aaattatacc 420
ttaacaacgt taactaaaga agtaattaaa ctcccacatt ggacaaggag ggatctagag 480
cactgaatct gcacagattc tctgagctca gtctaggaag agaagtgggg ctgaactata 540
cagaagatgt tgaaattaga aacgtacttt ccaggaataa attaaaaatt gagtttttgt 600
ccacctacaa taatcaggca ctgtgccagk tcctaggact gtaaagaaaa ctatctctga 660

tataagctta taatctaaca gagaagacaa aaagtagaaa gcttaaattc agtgagtaag 720
tgctatgata gaggcaggag tgggttgcaa aggagaagca tcaaatacag atgaatggca 780
caaaaggctt tctgtaggac attcatatag agcctgagaa atcaggagaa ctcctccagg 840
atcactagct gtattagttc attctcatgc tgctaataaa gacaaaccca agactggggt 900
wacttataaa ggaaagargt ttagttgact cacagttcar cagggtgggg aggcctcagg 960
cagcttacaa tcatggagga aggggaagca aacgcgtcct ccttcacatg aaggcaggaa 1020
agagaagtgc ccaagcaaaa gggggaaaaa gtcccttata aaaccatcag atctcatgag 1080
aacagcatga aggtaaccac ctccatgatt aaattacctc ccaccaggtc cctcccacta 1140
cacctggaga ttataggaac tacaattcaa gatagatttg ggtggggaca cagtcaaacc 1200
atatcactag ccaaagcagc aggtgtgtgt gtgtgtgtgt gtgtgtgtgt gtgtgtgtgt 1260
gtgtgtgtgt gtgtattcag tatcttgaac tagaccactg ctcttgaaga tctcttgacc 1320
atgtagaagc caggataaca catttagctg gaagctaatt gtgaagctgg gaaattatgc 1380
atgtatagtg atgttgggca caaactcatg ttatgttcag gtaacaagca gagcattttg 1440
ggttcctttc tttaggagtt gtctaatttt acttcatttg ttatttctga ttatattggt 1500
taccagctcg tgccgaattc gatatcaagc ttatcgatac c 1541

<210> 100
<211> 881
<212> DNA
<213> Homo sapiens

<400> 100
ggcagagccc agctgtttct tacacagtga catcccaggt gccctggggt ttgggactcc 60
tggcaggaga gaagagaatg aaccaaccca ttctgagaag tcaggcattg ttatggccct 120
ggagatgggt ggtgaaggca aaaccatgtg tgtgtgtgtc tatggatgcc tggattcctg 180
accgcagtca gcattgccca tcaattccag gtcaaaagaa ggaaagggct gggtcccatg 240
gtcaccaagc ccttgccgyt ctgctcttct tgtgaaagtg accttcagag tttcatcagc 300
tgatagcaga cagccagatt gccctctagt aaagcctagt ttaagatcag tttcaggaat 360
tttctgggtg tttttatctt tgcagtgaag ccataaaagc cctcatagca gctgggcarg 420
ctgcaaagtt gatgcttccg agtggctcat tagagtgtaa tggcattcct gagagtagat 480
gttgctgcaa gcagcaggca ggaagctcat gcggcagcag cctgatggat actccgcgtc 540
aagggggttc tggtggatgc gtggcaggca ggcagcggct acactccatg ggcggtgctg 600
ggttgcgaaa ggggctgaca gtgctgctct ccggcagagg ggtgggggca ggtgcatgca 660
cattgcagat gaaaaggtga ggggtctcag tgggtgtgat gggagttaga caacagcact 720
gtttaggagg tggtttaaag ctttttctct aatttaaatt gggtagtaaa ttctgaccct 780
aacatgagta ttgtatctga tgggattcag tttggggaaa aattcagtct tgaagtctag 840
caaattccag tttcggtctc aacctttggg tcatctcgta g 881

<210> 101
<211> 947
<212> DNA
<213> Homo sapiens

<400> 101
ggcacgagca gattgtttct ggcttagtag catcatctaa tggcgagcag gggcactgca 60
gcccctggac gcaccttcct ggcgatgatg gtcacgtcct ttttcttttg tatgagatgg 120
ggatcatggg ctgagcagat gccccaaagg tgtctgccat gctgtatgca ggaatgctga 180
ggtttcatgc aaaaacgtat gttgggatca cttgacgggg tgcatggaaa gtatcctagc 240
agagttttgc tagtagagaa tgtgccctcc gctagtactc tagagtcaca gcccagatcc 300
tagccttcgg gtggaggtgc cgccctatga acggccatca gactgtccta ttgttcagag 360
actccttttc gtcccttact acttcatctc aggcctgtga ggatctctac caaagcagag 420
caagcccata aataaatagc tttgtgctca ccaatgcatc agtggcgctg aaataactgt 480
gcagtggaca ccactctccc tccctctggg cctttcagct gctctgtgtg tccacgctgt 540
tgtgtgccct gaaagaactt tgagatttgc tgaaaggtct ccagctgcaa tttcagacca 600
tgtgggtaac tcaaatgtct aacatttaaa tatggctgaa aggggtcgac tgctgggctg 660
gatggtggca gaagtagcag gctttccctt ggatctcttt acagggcaga gatgactcct 720

tacaaatggc accagtaatc cctctgcctc ctgataccca tctaacagaa aagtgcaccc 780
tgctcataag aaaaccccag gccacatttg tcttatgtga ccctggtctc tggctacagc 840
tgacttgaca caaactaaga caatcagatt tttttcttct ggaacttgct gtgagacaca 900
gagacacagt ggactggatg acaatgctgg gatgtgaagg atgtgtc 947

<210> 102
<211> 1369
<212> DNA
<213> Homo sapiens

<400> 102
cccacgcgtc cgcccacgcg tccggctggc aagatggcgg gaggggtgcg cccgctgmgg 60
ggcctccgcg ccttgtgtcg cgtgctgctc ttcctctcgc agttctgcat tctgtcgggc 120
ggtgaaagta ctgaaatccc accttatgtg atgaagtgtc cgagcaatgg tttgtgtagc 180
aggcttcctg cagactgtat agactgcaca acaaatttct cctgtaccta tgggaagcct 240
gtcacttttg actgtgcagt gaaaccatct gttacctgtg ttgatcaaga cttcaaatcc 300
caaaagaact tcatcattaa catgacttgc agattttgct ggcagcttcc tgaaacagat 360
tacgagtgta ccaactccac cagctgcatg acggtgtcct gtcctcggca gcgctaccct 420
gccaactgca cggtgcggga ccacgtccac tgcttgggta accgtacttt tcccaaaatg 480
ctatattgca attggactgg aggctataag tggtctacgg ctctggctct aagcatcacc 540
ctcggtgggt ttggagcaga ccgtttctac ctgggccagt ggsgggaagg cctcggcaag 600
ctcttcagct tcggtggcct gggaatatgg acgctgatag acgtcctgct cattggagtt 660
ggctatgttg gaccagcaga tggctctttg tacatttagc tgtggtgtgt gcttcagaaa 720
ggagcagggc ttagaaaaag cccttttgtc cgtagagttg atgtggtgtg agtgatatat 780
ttctatgttt ttaatgtaca gcatctgtac tttgtttgcc ttgataaagg taagataaat 840
gaaacgctga actatgctaa tctggaattt gtttttattt gcctgaaata tatttttttc 900
tgtgaaaaaa ttaaaacgta cttaagccag gagaatgaat tatacagtga ttgaaaatcc 960
atttaattcc tatgactttt gttttgtatt gcccaagtca aactacatca cttgtatctc 1020
cagcccaaat gtagtctgcc ttgaaaagtc tttcagctgt gactgcagga agtgggagtg 1080
tttttattgt tagctaattg ctgtgactgc aggaagtggg agtgtttctg ttgttggcta 1140
attgaagtta ttaggctcag cttcagtcat gtgtaagttt tgcagtgtaa tacatatgta 1200
gtctggtctg tatatatgaa aatttgaatt aaactgcaga atgtttatgt ctagttatgg 1260
tttaaatttt cttagtagta tataaaaggt aagagtactg aaaaattaat aaaattgcaa 1320
gttaaraaaa aaaaaaaaaa aaaaaaaaaa aaaaaaaagg agggggggc 1369

<210> 103
<211> 1231
<212> DNA
<213> Homo sapiens

<400> 103
ggaggaagga agcaattcta aaaacaaatt ttcaaagcta tttttacatt aacattattt 60
tttctaaatg tgcatccatt atagtagagt tatcttttcc ttccttaagc tcagaattaa 120
gtcaatttcc tggtatgcaa tgtggcttta ttttttttgt tatttaaatt gtttagccaa 180
agtgagatca gcaactactt atttatatgt ttgagggcac tgtgagctga aataacaaat 240
tttgtataac ttatctaact aaaaatattt ataatacttc tcaataatga ttctaatgaa 300
gatgtattga gtaatttatt accatctttt atgttttaag agttctgata gtacaagtta 360
aaaaaaaata gcctacttct actcttttac tgaaatcaaa tagatggcat gtgatgatta 420
acataaaaaa gaacacattc aacataatat attgcgatag tggcagtcta atagaaaaat 480
agcaactatt tatttacttt tatttattcg ttttatttat aatagatatg tacaaaatgt 540
aaaattattt ctaagaagat ttttctttaa acaccaagca ttacagtaga tgttgtctga 600
atgtgtgtaa ttgagaataa taatattgta taataatatt aatatgattg tggctagaag 660
gaatttattt gatcacataa gtacagcaaa ttaagaataa gatctcagca tatcctggct 720
actacataaa tgtagcaaag ttattttgct accttgaaaa aatacaagtg gacaaaaaat 780
tgcctaattt catggaaaaa ctataatttt gccagttaat acatgagatt ctcaaattca 840
cgaatttaaa atgtttaaat aattatactt ttgctgcctg aaaatatatt ggatcaaaat 900

ttcaagtaaa tatcatcagg ggatttggat ggggaagagc aagaggggaa gaaggaggag 960
aggagattta gtctagcact atttccagct gatccccacc cagcattgcc aagctacagg 1020
acaccaattc aataggatga agtcagtcaa atcctctttt aagtaatggt tgaataaaat 1080
aaaatgaggg gaagaaaatc aagtactcta aatgattaaa gcgtatgaat aagtgaaaaa 1140
tgtttgcaaa attgagaagg aaggagaatc gggaagtggt tatttatggt gtctcaatat 1200
gtaagggtaa aaaaaaaaaa aaaaaactcg a 1231

<210> 104
<211> 1242
<212> DNA
<213> Homo sapiens

<220>
<221> SITE
<222> (288)
<223> n equals a,t,g, or c

<400> 104
ttcgtatcca ctaggatggc tctaatcaat aacaaagtat tgtcaaggat gtagaaaaat 60
tggagccctc ctgccttggt gggagtgtaa tatggtgcca gatacaacct ccatcctgaa 120
gctcatctgt atgcttcctg tttgtgtttt taaactttta ctatatcttt atgtcctcat 180
aagaatatgt actatcattt ggtgttttaa agtgtacata aatgctgtca tcctgaacaa 240
atcctctcgc taactgcatc tttaactcta tactatattt tcaagatntg tccatgttga 300
tccacgtagc tccctagttc cctttaactg ctataagata ttctgttgcg tcaatatatg 360
acaatttatg catgctttgt tgacaggtaa ttggattttt agtgttttgc ctttacaaaa 420
atcactgcat cttttgcaca tgtctacttg tgcatatgaa ctgaggtaaa attgctgggc 480
cttactgtaa atatgttgtt ttaattcact ttgcgctgct gtaacagaat accatagact 540
gggtgcttat aaagaaaaga aatttatttc tcatagttct ggagaatggg aattccaaga 600
tccattcaca ggttcggttg tctggggaar actttcttca cacatcctca cttggcagaa 660
cggaagggcc tgggttgatg ctgtgtgaam cctcttttat aagggcctta gtctcattcc 720
caaggaggag ctctcataac ctaatcacct cttaaaggcc ccccactcaa tactatgaca 780
ttgaatttca acatctgaat tttagagggg acactgcaaa cctgtcatat gtctttatct 840
ttactatcac taaattgtcc aaagtgattg caacagtgat ttatatactc aacccacaga 900
gtataagaat ttctcctttc tagctgggca cggtggctca cgccagtagt cccagcactc 960
tgggaggccg agatgggcgg atcacttgag gccaggagtt caagaccagc ctggccaaca 1020
cagtgaaacc ccatctctgc taaaaataga aaaagttagc tagctatggt ggcgcacacc 1080
tgtaatccta gctatttggg gggctgaggc aagagaattg cttggacctg ggaggctgag 1140
gtagcagtga actgagatcg taccattgca ctccagcctg ggtgacagag cgagactctg 1200
tctcagaaaa aaaaaaaaaa aaaaaaaaaa aaaaaactcg ta 1242

<210> 105
<211> 1151
<212> DNA
<213> Homo sapiens

<400> 105
gcagggacag ccccacgcat gggaggtggt ggccagccat caccccaggg tgaatttctc 60
taagagcagt cctaggccag ggggtgagtg tgggaaagtc agggccgttt ccatcagtta 120
acgtttcaga gcgggttccc gctggaagtg ctggaaatac ccagatgcca tctgtagcat 180
tttatgaact gtggcggatc cactctctgt gtcttgagct tctgctctgt ggtctgctca 240
gtggaagcat cctgtcagtc cactgtacag tggggtggag ccgcagccag ggtgggtgtg 300
ccgtttgatt ggtcacggaa tgaacagggc aaaggtcact aagtagatat gatactgcaa 360
gtaggattgt ggtcataggt atttttatga atttgtgtta tgtgaagttg aggatttgaa 420
tgttgtgatt attatatcca gataaagttc tagcctggca cagtgcaggc acgtgcctgt 480
ggtcccagct gcttgaagtg ggaggagagc ttcagctcag gatttccagg ctatagggag 540
ctgtggtccc accattgcac cccagcctgg gtgacagagt gagaccccat ctcaaaaaga 600

aaagaaaaga ggctaggcgc agtggctcag gcctgtaatt caagcacttt gggaggctga 660
ggcaggcgga tcacttgaag tctggagttc gggaccggcc tggccaacat ggtgaagccc 720
ccccatctct attaaaaata caaaaatcat acttacccgg caggggagat accatgatcg 780
cgaaggtggt tttcccaggg caaggcttag ccattgcact ccggatgtgc tgagcctgcg 840
ttttccccaa atgtgggaaa cttgatgcgt aatttgtggt agtggaggga ctgtgttcac 900
actgtccccc ccgcccaaaa aaaataaaaa tacaaaaatc agtcgggcgt ggtggctcac 960
gcctgtcatc ccagcacttt gggaagccga ggtgggtgga tcacctgagg tcaggagttt 1020
gagaccagcc tggccaacat ggtgaaaatt gggaggccga ggcgggcgga tcccaaggtc 1080
aggagatcga tacatcctgg ctaacacagt gaaaccccgt ctctactaaa aaaaaaaaaa 1140
aaaaactcgt a 1151

<210> 106
<211> 1628
<212> DNA
<213> Homo sapiens

<400> 106
gaggaaatat cctctccatg aggcatacac caagtaaatg actttgtaac tttacttcat 60
cctcttcatt tacacagggc atacatgaag taaccaatgg aatcctctag gggtatttta 120
aactcccaaa aattctgtaa cggggccctt gagcccctat gcttgggtcc attcccaaac 180
tgtggagtgt actttcattt tcaataaatt tctgcttttg ttgcttcatt ctttccttgc 240
tttgtttgag cgttttgtcc aattatttgt tcaagacgcc aagaacctgg sacaccctcc 300
accggtaaca ctcttacaat tatgcagttg tgcagtgcat agcccgtgcc actatatgtg 360
gcagtgttgc ccacttagca atgaggagcg catattttcc tgcattatca ccaaaacaat 420
gttatcatct tttatttatt attatttttt tgagtcaggg ccttgccctg tcacccaggc 480
tggagtgcag tgacgcagtc tcagctcact gcaacctctg cctgtcaggc tgaggtggga 540
tgatcacttg agtccaggag tttgagacca gcctgggcaa catggcaaaa ccccatctct 600
acagaaaata attagctgga tgtggtgatg catgcctgta gtcccagcta ytcaggagac 660
tgagatggga agatcacttg agcccaggar ttagargctg cagtgagcta tgatcatgcc 720
accgcaatcc agcttgggca acagaatgaa aytctgtcaa aaaaaaaaaa gaagagagaa 780
aagaaaaagg aaagtaaaaa ttgctaaaca cytccaaagt ctatgtagga aatatgtaaa 840
tggtggtcca tttccaagat gaagtgcctc aagtaggcct gggtctgcca gctacagaag 900
gacagaatat cytaggccct tgcttcaata gctggagcct gcttgttggg gtgcccttag 960
ttgctctcat ccgaacctaa gagtttagtc tagaatgaaa atttactagc ctgcaaaata 1020
gctcacttta tctattcttt tatcagcttg cctgactacc taggtcatag gtcaaatact 1080
taaaaagccc ttgagcagac tataattgca atgcattatg ggctgcaaca aaatgcaccg 1140
agacaaccct aaagaaaaca cccaaaaccc ctacctggcc aggcgcggtg gctcatgcst 1200
gtaatcccag cactttcgga ggccgaagcg ggtggatcac ttgtcaggag tttgagacca 1260
gcctggccaa cgctggtctc cataatactc agcctatgag gaaccaggag agggacctgc 1320
acactagagg ataaattgct tgttgtaact gtgcggggta tgcctgccca ccagacacct 1380
gatcttgcaa gactgtatta aaagtctcac ttccgctatt ctccgtgtct ctgagtccat 1440
tctttgggtt tggacgggtg agtttgtttc tcacagtcta gactctagat gtgaagaact 1500
ttgatcatat caccaaagga gatggtggta tgcaatttta taagtaaaaa tacactagtg 1560
tcagtttttt ttacaaggga aacctgattt gcatcttttt aattaaaaaa aaaaaaaaaa 1620
actcgtag 1628

<210> 107
<211> 1465
<212> DNA
<213> Homo sapiens

<400> 107
   ggcacgagcg agccaagttt gcaccactgc actccagcct gggcgacaga gcaagactca 60
   gtctcgaaaa aaaaaaagtt ggaagcagaa gtaaaaaaca tggtaaagaa tgagaactaa 120
   ataaatataa taattgagag gtctgcatta gatgtggcag ggagaacaag caaaaagaga 180
   tttcagagaa gatcactgga attggcagag gccttgaagg gcagagtcta gcatacagaa 240
   gatgtaaagc cacattctgt gaaggtaagt agatgtgttt acctcttttg cactgtactg 300
   gtgcattatg gggtaaatrt gtattacttt tcctgtattg cttagcacag agttttgcct 360
   atagcaggca ccagactgtg ggcttggtag tacatgacta ttggtgatta cagatcaaaa 420
   aggacttgaa atgatcagtt taaggtcttg atgggtattg aagactcaaa ggatgatggc 480
   accctgggag tgatccacag aaggacagat tatttgaaga tgttaataac taaagacaac 540
   atggatgtta aatgatgaaa aaaagttgga tggaaaataa accattggat ctgcytctgg 600
   agtccaagaa gaatattatt cttcctacct cccccttact ctggctcttc ctattgtagc 660
   cacatgggtc agtaatgcca ttgaaaaaca aaattttaga ctaagtgggg tcgcagaaat 720
   tttggtctat cttaaattga tgacatctta ttaaagaaty tattgtataa agtgtgctta 780
   ttctggcatt tttttaatga agaaaaagtg taattcagtg cacatttatg aatttcaaag 840
   atcaataaaa atgggcaaag tatatgaacg cataatccat agaagaagat atctgacaaa 900
   tgcagttcaa taaatatttt tttaaataaa aattagcctg tggtaagaat tgaaatggag 960
   aaagaaatag aaggtagcca ggttacctac agctttgtat atgacactat agagctagga 1020
   ctttattcca tagatgaggg aagtctgcta aagtgtctac attgaaccca ttattttgct 1080
   agcattgtag ttgatgtacc taaaacagac ttgagccggt agagtaagta ggcagacttg 1140
   tccaagtgag aaaaagatga aactgtggtg aggataaaga gagaaaagga gcagatttaa 1200
   gaaatattaa aacttgaaag tactaagact tgatgatgaa ctagatgtgt tagataagag 1260
   atagcatgga gtctagtaaa agttctgttt ttctcactcg tgtgactgcc tcaataacac 1320
   aaagcttgat aggaaataaa catgagatag cacatggatc tattacaagt ttttgaaatt 1380
   gagcttgaaa agctacttca aaaaataaat tctaggccag gtgtgagycc atgcgcttga 1440
   ttaaaaaaaa aaaaaaaaac tcgta 1465

<210> 108
<211> 1265
<212> DNA
<213> Homo sapiens

<220>
<221> SITE
<222> (766)
<223> n equals a,t,g, or c

<400> 108
ggggcagatg gaaatgtctc ggattttgat aatgaagaag aggaacagtc agtccctccc 60
aaagtggatg agaatgacac ccgtccagat gtggagccac cactgccatt gcagatccaa 120
atagccatgg acgtgatgga acgctgcatc cacttgttgt cagataaaaa tctgcaaatc 180
cgcctgaagg tcttggatgt gctggatctg tgtgtggttg ttcttcagtc ccacaaaaac 240
cagctgcttc ccttggctca tcaggcctgg ccctcgctcg ttcaccgact cacacgggac 300
gcccccctgg cagtgcttag agccttcaag ttttacgtac cctgggaagc aagtgtggtg 360
actttcttcg cagccggttc tgcaaagatg tcctgccaaa gctggctggc tccctagtca 420
cccaggcccc catcagtgcc agggctggac cagtttactc gcacacgctg gccttcaagt 480
tgcagctggc tgtcttacag ggcctgggcc ccctctgtga gagactggac ctaggtgagg 540
gtgacctgaa taaagtggct gatgcctgct tgatttacct cagtgtcaaa cagcccgtga 600
aattacaaga ggctgccagg agcgtcttcc tccacttgat gaaggtggac ccagactcca 660
cctggttcct cctgaacgag ctttactgcc ccgtgcagtt cacacctccc caccccagcc 720
tccaccctgt gcagctgcas ggggccagcg ggcagcagaa cccctnacac gaccaacgtg 780
ctccagctgc tcaaggagct gcagtgaccc tgctccccca ccacagaggc caccgatccc 840
tcccctactg ccagccagaa gctgggctga ccccaccccg gccataggcg gtggcagcgg 900
cagcagagaa ggtgaattag ttagccaatc gatttataaa ttgatcgatc acacaactgc 960
ttagaaatgg attgaaggaa agtagctgac tattatttat atttcatacc ttgtgttttc 1020
aagtgacatt gtctggtggc tctaagggtt taacccctta gcctaccatc tctatagccc 1080
cagctccctc acaggccaca cacacacaca cacaagaggt cagttcccct ccatctgcat 1140
acacctccct gtcttcaaat aatgagatgg aactaatttg ttttacctaa cctgatcttt 1200
gggaaacaaa cggaaataaa gacacttctt ggatgaaaag taaaaaaaaa aaaaaaaaac 1260
tcgag 1265

<210> 109
<211> 1006
<212> DNA
<213> Homo sapiens

<400> 109
   ccacgcgtcc ggcaataatg acccattgtg gtttttaact tatctcatga aaagacttag 60
   gtttgttctc agggtatttc agatgactgc ctttataact ggggcacata cgattactaa 120
   ctatagtgat aggcgtttat acatttcccc tttgagccat ttctttatga acagtggttc 180
   ttctgctcaa agtgttctgt ctcattctta tgtttctcaa atcttcttta aaaatgtaag 240
   caaatatttt taaagaattt ttatgttttc caaaattagg attttagact ttagggattt 300
   tgatctttgg ggatttcaac attcgggatt atggtgttca gtgtgtattt tggggggatt 360
   atgatcagca tcccatacag tggaatatca tttggcaata aaaaggaatt aaatattgat 420
   tcatgctaca acatggtgaa cctaaaaaac attatgttca gtgaaagaag ccaaacctaa 480
   aaggcctacg tactgtgtgg ttaaatggta aaaatggtga atttatcaca atcaaaacca 540
   ataaacctct acaggaaaaa ataaggacaa agaaaggctg ttccctatta atggagacta 600
   aagagacatg acaactaaat gcagtttatg attgtggatt acatccttga tcagggtgaa 660
   aatagctata aaggaaatta ttgggacaat ttgctaaaat ttgaaaatgg actggatttg 720
   gctggtcgca gtggcttaca tctgtaatcc cagaactttg ggaggccaag gtgggtggat 780
   tacctgaggc caggagttca agaccagcct ggccaacatg gcaaaacccg tctctactaa 840
   aaatacaaaa attagatggg cgtggtggtg tgcatctgta atctcagcta ctcgggaggc 900
   tgaggcttga acccgggagg cagaggttgc agtgagctga gatcacgcta ctgcattcca 960
   gcctgggtga cagagcgaga ctccatctca aaaaaaaaaa aaaaaa 1006

<210> 110
<211> 1258
<212> DNA
<213> Homo sapiens

<400> 110
aaccctcact aaagggaaca aaagctggag ctccaccgcg gtggcggccg ctggctgacc 60
ggcctaaaac taaaatgaca tttattccct agctacaaac atcagcgtta ttatgttaat 120
tataccttgc cctctatcat tataaatggt tgccatggtg tttctaaaaa taagtgtttt 180
accattaatg tgtagagggc aaacaaagca taaagtacta agggatcatg cttatcctag 240
ggtctcacag aagagaggac atatttaatt aatcttgtga attacagaac aggttgtggt 300
ccagacacca agaatcatag ggtttttttt ttaaaaaacc taatagaagt agggtgacct 360
ctctctttgg tctaagagtt ctaaaggaag gtaggcatct gtttaattag ttggttcacc 420
ctggctttac ctctggttaa tgcttgtgtt aataggaagg aaaaatcact ttatcttttc 480
ttccaagccc ctccctgcct gacttaccca gactgggatt accagatacc aggtgattta 540
tgtggagatg atttttcacc tttaaactct aagccaagtg taagaaactc ttgatagcta 600
tgtctatttt atatcagtca ctgagacttt tttttaagtt tttatttatt attaagacaa 660
ctttgccaaa aaagtcccct aagcacaact atttacattt ctttatagcc tcttctgatc 720
tctaacacat atgcagtttt aactgttatt ttcatagtaa ctgatctttt gtctaaggat 780
ttttacctga aagcacaatg tattgagtct cttgaaaatc atctttcaga tctttttaca 840
gaatgaactt atgcactgct actgtagtat tctcaaggaa tatatgtaaa cacaaatgta 900
tgcctgaggt tggtttttgc agaaaacagt ctctgcttct aaaaacttct atgtctagtc 960
ttccatagga aatcctcact gtttaaccat gtgaggagcc taagtcatta aacggatcat 1020
gtctgtacat tgtgtaatga atgaaaagca cataaatgta atctactttg aactttgtaa 1080
aaatgatgtg tggaggctat tcttgtttct ccatctcaag tcctgtgtgt gcacgtgtgt 1140
gcaagtgcac atgtgtgtgt gtaataacac attgtaaaga acagaaatta ctttaaaaaa 1200
taaacagaaa tggagacctg aaaaaaaaaa aaaaaaaaaa aaaaaaaaaa aaaaaaaa 1258

<210> 111
<211> 1453
<212> DNA
<213> Homo sapiens

<220>
<221> SITE
<222> (946)
<223> n equals a,t,g, or c

<400> 111
ggtcatcttt ctcttgctcg tacagagagg agacacccgt agaaatggag atttctacta 60
cagatgaaaa tttcttttat aaaagggtaa cttctctgta ttatcctgtg tttgctattt 120
ctgaaaataa taagctgaaa atattcctct ttggcataag gattatttgg tgtggcatgt 180
tctgaacctc cactgttggc atcctttctt gattagcaga aacctaggaa cattgttgta 240
ataatgacta aattattgtc actgtcacat ttgttagtaa ctttttttaa tataattgcc 300
attaaatgta aaaagcagca tctaagacat tcaaaatgta atttkgatac tacttttaaa 360
aataagatgc taaattaata gataaggtgg gtttcctcag tatattttca ttctaaacca 420
tccactaaag tagggctaaa gaggaattta gagtaggaag acttaggttt tgtattctgc 480
ctttgttcag tatcagtgtg actttggcca agttacctga cttctgaact gcattttgct 540
tttctctaaa taagtggggg taatacctat attagaggat tatgataaaa agatgtgaac 600
atattataaa attattttat aaactagaag acatttcaaa gaagttaagc tgccactgtt 660
agtttcacag acttgggtgt attagatgaa cagcttttca gttattgctt ctatagttgt 720
cctcttgccc tttcctggat tatcagtttc tgcctgtcta cctagtcatt cccatcagtg 780
taaaacattt ataytgttat ttcttccaag ttcagaaaaa accctctyty gaytcccccc 840
atcccattcc agcactttgg gaggccaagg cgggcagatc atgaggtcag gagatcgagm 900
ccatyctggc taacatggtg acccccatct ctactaaaaa tacaanacaa attagccggg 960
cttggtggtg ggcgcctgta atcccagcta ccggggaggc tgaggcagga gaaaggcatg 1020
aacccaggag gcagagcttg cagtgagcca agattgcgcc attgcactcc agcctgggcg 1080
acagagtgag actccatctc aaaaaaamga awaaaaaaaa caacttattt taaattattt 1140
tcctagaaat tatgatgtca gcagaggtag ctaggtggta ttatggttga cttttgttat 1200
ttttaagaca gcttccgtat ttcttaggag ttttgctgaa gaacatggta tggggagaac 1260
atataatatt ctcatacact tcttaggatg ggatagatcc ctgtaacaga atattggtta 1320
acaagagaaa aacaagtttt aagacatgta tacctcatat atacatggga gatactcggg 1380
ggaagtgagt aaatctctca gaggtggctt aaataccatc atgtcctgaa aaaaaaaaaa 1440
aaagggcggc cgc 1453

<210> 112
<211> 1552
<212> DNA
<213> Homo sapiens

<220>
<221> SITE
<222> (1035)
<223> n equals a,t,g, or c

<400> 112
ttgcctaagg cccactgtgc caaattagat aatacaagaa gttcatttac actgtagacc 60
agtgacgtca atgactgttt gctctgtgat accgtttcaa aaatccaaaa tgcagacttt 120
tctctgtgcc atgcaggatg cagctgtgtg tgatatggtt tacagtaata tttctttctc 180
aaagtagcag gcttgttaag gaaaagataa gcaacacatc tggggaaaag ggcaggtggc 240
cagcaatcga tgtggtagct ctttgcccct ctcggacagc aggaattagc ttccccaggc 300
attttctgta tgtgagttgt attgtgggat gtacaaatat catctgttcc tttgggtttc 360
caggccagta gctctctatt ttgggttcaa acatgggttc tcaggccggg cgcggtggct 420
cacgcgtgta atcccggcac tttgggaggc caaggcgggc ggatcacgag gtcgggagat 480
ggagaccatc ctggctaaca tggtgaaacc ccaactctac taaaaataca aaaaattagg 540
caggcatggt ggcgggtgcc tgtgktcccg gctactcagg aggctgaggc aggagaatgg 600
tgtggacccg ggaggttgga ggttgcagta agccgagatt gcaccactgc mctccagcct 660
gggcaacaga gcgagactcc atctcaaaaa acaaacaaac aaacaaacaa aaacatgggt 720
tctcaaaagg catgcccact gtctcccatg gagcttgaca gcccatgcca ttagctctca 780

ctgttaggtt tctggggaag gttcttctac ttgattggaa aatttccaaa taaatctttc 840
cagaagatac tatgcacaca gctaagtggc ctgtctgtgg agtaaccctt ttgtaaacaa 900
acagaaacct aaagcttgat gttttggggg gctgcctgtc atctataggt tcatttaggt 960
gtatttagga agaggatcca tgaaaccact ggtttcctgt tacataataa tcattaataa 1020
tgatttaaaa tgtgnacatt gatttttttr aattccraaa tacaagcgta tatggtawat 1080
taagtcaaat ggtatgttca gtgagcgaga tggggcttgg ggcaaaacaa tactttgctt 1140
ccaaagagga tacaactctc aaggagattc tttcatcttg cctttaaggt catttaaact 1200
aattcacata atcttcagaa aactaattca catcatctat tcatgtgtaa aatcaaaagg 1260
aagactgttt tcttagtctc tcgttgccta actggccatt tatactacta ggttgattaa 1320
gggatttgcc tttttctgct gatatgggaa caaaaagtct taagcatttt taaaggcaat 1380
ggaaaattca gccacatggg ggaaaattga tattgtcacc attgagttgc tctgtttctt 1440
ggtgaagagt gaatctaatc tgatttcctt cttcatcaga tatgcctctt taacaacaaa 1500
aaaaaaaaaa aaggaattcg atatcaagct tatcgatacc gtcgacctcg ta 1552

<210> 113
<211> 1489
<212> DNA
<213> Homo sapiens

<400> 113
gaattcggca cgagtgccca gctcctgctg taattagctc cacgtgtacc cccttcattc 60
cctccctccc accgagccat ccctgaccca ggaactttcc gcagactcgc cgccatctgg 120
gagtgaagca acatggatgc agtcagccaa gtccccatgg aagtcgtgct tcccaagcac 180
atcctggata tctgggttat tgtcctcatc atcctggcca ccattgtcat catgacctcg 240
ttgttgctgt gcccagccac tgcagtaatc atctatcgca tgcggactca tccgatcctt 300
agtggggctg tttgagagcc tcccaagagg gccgggtgag ggatgaggac aggcatccta 360
tccccagcct cttcctgtct tcagaaaagc agcaggaggg actttggggc atggacctga 420
gttctggttt tgattctgcc acgagccagc tgtgtgaatt tggtcaaggg acctaactct 480
ctgagttcca ggttccttat ctttcaaatg gggatggtga tccctgccct ttctacctca 540
tagggatgtg agaaccacct gacttagtgg atgtgaaagc tgtttgtgat cagtaaagct 600
accacagata taagggtgtt atgctgaatc ctgagaagct ttcaagaacc agagaacctg 660
attgctgatg atggccttaa aggtggtgag ggagatactg ggggcagagc agactttgcc 720
agtgcccctc aggtcaaacc aagccaagag caccctgtcc ccattccaag gggccagcag 780
cactttggcc caaagtattt tctttaaggt gccattcctt catgttttct cagtttggag 840
ggtgatgggt agagctttcc agaaccttct ccattccaga atctctgccc ctgtgtaatc 900
tgaaggaagg ctgtgccatc tttgggcact gccaagggag ttggggtgat gggcttcttt 960
ctgcactgga gtctcacatc tgttagcttt gacactcaag caatgttgga aaatgcaggg 1020
tgactgagtt ccctgcccag ctttcgggat ctctggcccc catccccttg tgtgtgtccc 1080
tctgcccagc tcctgctgta attagctcca cgtgtacccc cttcactccc tcccaccagc 1140
tctgcagcca gcctatggca attatatttt aagaggtgtt cccaggactt ttgggaccta 1200
ctaaaacaat gatggttatt ttagatgtga tgatttatat ttatgtagag atatttctgg 1260
accactcaag ctcttcgata ccaaaatcag gagcatcttg ggatttatta aattatgtaa 1320
gaagatagca cagatatcgg gatattattg tgtgaaaatg ctgcttttac tttgatgtga 1380
tctcattgat gtacacaacc aagttccaat aaagtgctag aatgtgaaaa aaaaaaaaaa 1440
aaaactgcga gggggggacc cgtaacccta atcgacctta atgagtgta 1489

<210> 114
<211> 607
<212> DNA
<213> Homo sapiens

<400> 114
ggcacgagtt tcaacttgag atttggaggg gacagacatc caaaccgtat cattaaattt 60
aatagtttta tgcagttttt ttggctctag atctgtttag actcctgcag tcaggtgtct 120
gtaactagcc tctggtcctt tttgagagtt cacagtttgg tgcaaaccct ttggatgtat 180
tatttgggaa aatgggatat ctggcagcct gtgtccctgc tttacattat cctttttgct 240

gcctgcccca gcctcctcat tagcatccct gccaaggcca gtggagaagg atggagatgc 300
ggtgacattc agctgacagt tgtcacagat tgataatagc taacagcaca tctctccccc 360
ggctccttcc ctagtgcacc aattagccca gcctcatctg cacctgggac tcaagttgcc 420
taaacatatt tcatttccca tagcagaaga tgccatccat ctagagtgag actgaaaata 480
caaacaattc agaagttgtg actttccatg ctctgcacac agaggctacc aaatgctaag 540
ggcgcttcct ccccagcacc aggcttatgg ttctaagctc cagaaaaata tcaaataaac 600
cctgccc 607

<210> 115
<211> 1498
<212> DNA
<213> Homo sapiens

<220>
<221> SITE
<222> (791)
<223> n equals a,t,g, or c

<220>
<221> SITE
<222> (895)
<223> n equals a,t,g, or c

<220>
<221> SITE
<222> (915)
<223> n equals a,t,g, or c

<220>
<221> SITE
<222> (936)
<223> n equals a,t,g, or c

<220>
<221> SITE
<222> (1017)
<223> n equals a,t,g, or c

<400> 115
gcatccccat ttctgttcct ggaccctctt ctgtcacccc atcgacaagt aaagaaatca 60
agaaatccaa actgattcga agccagtctt ttaataatca agcttttcat gcaaaatatg 120
gcaacttaga gaaatgtgct agtaaaagtt ctacaacagg atatacaact tctgtctcag 180
ggttaggaaa gacttctgtg ctttcactag ctgatgattc attccggact cgtaatgcca 240
gtagcgttcc atcttccttt tctcctaata ctcccttacc gagtacttcc cgtgggacag 300
gtaactcagt tgaccccaag agcagtggaa gtaaagatac acaaccacgg aaggctacct 360
taaaatccag aaaatccaat ccttaaatca actgcttgat gaaggaggca aaacaaaggc 420
agcaggagat aatgtgattg gtacacaaaa gctaaagcag tggctggggc tttgttttta 480
aattttgggt tttttttttg ttgttgttaa tgagcagaaa gagagacata atgacagctg 540
atgttaaact tttcatattt caaattagat tccctaggag gtataatata tatttcttga 600
gtaataatgt ggttacggaa ttccaatgtt atagtgaagt gtaatgaaaa acatctctag 660
gaatgtgctt taaccactgc tgcaaaagar acaagtctgc atttatttgt gcaggaaacc 720
accatttaat tgttctagar ttttagcatt taaaaatcgt atgaaagtct acatcagctg 780
aattgtccta ncttgataag cacttggagg gggacttgga aggtgagaaa gatactgcat 840
tttctcatga gtctccaagc ccacttgaaa agtcacactg aaaggatgca aatanccgtc 900
tgcgattttg gcggnccctc cggattgtgg tgacantatg ttgtacccgg acattcccaa 960
atttaaattt ggcagtgraa attccacaaa gatttctggt aacttkgagc tataatncct 1020
taaaaataat mgattgatga ttttctttat ttccagagaa tttattttat aaatactttg 1080

tttacatttt agattgtact tgtctttatt taaatgatga atctagtctg aatcagctgt 1140
tattccagct atcatgctta agctatgtca acagcattta tttgtactaa tgctaatatt 1200
tccatcaaaa tttgcctgtg gaatatatac agttcttaat tttaaactgt cagttcttga 1260
gatataccgt gtgaagctat tgtcagcttc tctatttcaa aatgcaatca gcatataact 1320
atattgatat tagaagatat ttgtttttta aaatatattg atgtatgata aagatgatct 1380
aatttgtgaa tgcatatgta tgtgtggtta ctttttataa tgtgaaataa tgaataatga 1440
atttactcaa aataaaacat aggttaatga gaaaaaaaaa aaaaaaaaaa aactcgaa 1498

<210> 116
<211> 1797
<212> DNA
<213> Homo sapiens

<400> 116
ggaacacaaa aaaaacactg ggaaaaagaa atgaaatctt agcagaagct ggaacttttt 60
atacttataa aaatagtatg ttcttatgtt tctccttatg catttatgtg ttcacttaaa 120
aatgttttta aatctaaggt tttctttgtt tatgttcagg taaggaactg ttgtcatgat 180
ctggaaatgt ttaaaacaat gtttgttagc attctgtgag cagcaaaact tatagtgata 240
aaaatcgatt gttgttaata tgatgtttac catgtgcaca tagtaatgaa aaggaacata 300
aaagcccagc aggctcgtac caaagtcaca gcagtaatgc tatgtactgc agagtctgat 360
gagctggcct ttgtgcacac ttttattttc atgggattgc atcttagctg ttaaaacttc 420
tagattgaaa tttgacagcc agggttacat attggggact tttaaagtgt ctttccaaag 480
agatttcatt aaccgtttag attagaatat ctttcccaat tgttacagtg acatatatgc 540
tgcaatattt aacaactgga gtattagcca catgggttat tttttcaatc tgtgttttga 600
atttttttat tgtgtgttat ttaaaatatt acatatgcag ctgggagaac tacacctttg 660
tgcacataga tttatatatt aatttgtaga aaatattttc tttatatatt tccttaccat 720
acaaggtgcc ttgttcatca ggaaaacttt tgttttgtat tttgacaaga aaggcacctt 780
cagagtttct ttttaagtat agttgacaag tgtataaatg ttacacttac tttcagagtt 840
ctttttagat ctaaagaagt cagttcaaaa atggaaatca acaatgttag gagaaatctg 900
aattctgtta agttagtaag tattatgtat agcatctgtt tttaaccatt tccattctta 960
tccctagtgt atcagttgat cacactaaga aagcttaaag attgagcatt tgaaataaat 1020
gctctttata aatgattaga tttttgaagg gatattgaaa tcattgcgct gtgatttcat 1080
ctgtgatgtg aaaaatcaat ttattatcct tggtgctttc ccccccacca atgcacaaat 1140
aattgtgaac agcttgaaat gacttaaact gtaatccaaa tgggacaatc tgataagaat 1200
ttcatgcatt ggtagttaaa taacttaaat tgctaaagct ttagcttgaa atttatgttt 1260
agaaaaacta ttgatttccc atggtatgaa tataactatt gtaattcttc aaatgagact 1320
cttctcacct taaatagtca tatattaatt aacttatagg aaataagcat actatatgtt 1380
agctgtttta aaaggtacca gatgtaagag tcataaatat atgcaattaa agaagttcat 1440
agatttcaca tgaatgtaaa tgtgttatat ggagacatgt cttgtaaaca gttgaatgta 1500
tgtaagtttt ctgtttgtga aaatgtagtt aatgtactca ctgtggaggt cataaggaag 1560
ctactttttt tttaaagtgg aacctaatta aaatatttcc agaatcaaag agacttaaat 1620
ggtaaatttt taaaattttc ttatctctta ctttttagtt ttcaaagtag aaaaaatcag 1680
gaattttttt attaactagt acttacatat taaataaaat ttattattgg ctaaaaaaaa 1740
aaaaaaaaaa aaaaaaaaaa aaaaaaaaaa aactagttct agatcgcggg cggccgc 1797

<210> 117
<211> 952
<212> DNA
<213> Homo sapiens

<400> 117
ggcacgagcc tggcacatag aaggtgccca ttaattggat gaacgaatgg gggagttaga 60
gtcagtacta gaaaaagcat gggattatac ttttaagagg ccaaatgaaa atttggggaa 120
tagccatttt ccaggaattt aaaaggagcc attttttaaa tgtcaataat aatttattgg 180
ttattatttt ttaagcactt attatgggtg cttattatag gcatggtaaa agcacttcat 240
ccacattatt taaatctcag aatctatgag tttggtgaga tcactgcagt tttacagagg 300

aaaaaacagg gcagagagaa cggtaatttc ctcaagttct cactcttgtc acttaataga 360
tctagaattc caacccagat ctgatggtga agtcagtata agcttcctgg agaaaggagt 420
agagttaagg tgggggatgg ggcttgaaga cttgatagga ttagggttgg gagtgtcaac 480
tcggagatcc acagtcaggc ggaaggaacc cacaaaggca ggaatgcaca cagcatgctc 540
agaggagatg gaacctgaaa atagagagaa ttagcaagta gtgcccattg gatggagtaa 600
agagccatac ctagaaggtt atcactgggt aaccatgata agagttttgg cctggtgctg 660
tggctcacgc ctgtaatccc agcactttgg gaggccaagg caggaggatc acctgaggtc 720
aggagttcga gaccagcctg gccaacatga tgaaacccca tctgtactaa aactacgaaa 780
attatctggg tgtggcggca ggcacctgta atcccaccta ctcggaggtg acgcaggggg 840
aattgcttga accggggagg cagaggtggc agtgagccac gatggtgcca ctgcactcta 900
gcctgggcga cagagcgaga tccgtctcaa aaaaaaaaaa aaaaaactcg ag 952

<210> 118
<211> 1185
<212> DNA
<213> Homo sapiens

<400> 118
ggcacgagct tatgtctatg ttttgagata gaatccttct attttatagc agggatgggc 60
aaaccacaag caaggggcca aatccagcct gctgcctgtt tttgctaaaa aagttttatt 120
ggaacacagc catggccatt cacttccata tcatccaatg gctgcttttg tgctacaatt 180
gccaccatgc ccagtggggc ctgtggcaca caactgcaga agtgagtggt tgtggcagaa 240
atcacttagc cttcaaagcc taaagcactt actatctgac cctttccaga aaaagtttgc 300
tgacctctgt tttagagata caaactaaca tacttacaga taagtgatct gaatatgaag 360
caaattttca acataatctg ggttgatggg gaaccgtgtt acatataaac aaatccagag 420
ctaatcattg ccaggtgagg gctgtgcaga cgatcacgac actattctct tttgcatatg 480
ttttccataa taaaaagtct tttgaaaaaa tcaatgattt aggggtttaa atatactgta 540
gtcactttgg aaaacagttt ggcagttcct caaaagttag acatagaatt accctatgac 600
ccagcagttc cctttctaga tataccccca agagaattaa aaacacacat tcacacaaaa 660
acctgcacac aaatgttcac aggagcattg ccttaatagc aaaacaagcg aaacaaccca 720
aatgcccatc aggtgacggg tggataaaca cagtgcggtc tgtccataca tggaatgtga 780
ctcagccaca ggcakgaatg aagcgctgac acacgcagca acacggatga accgtgaggt 840
cacggagtgg agtgaaagat accagtcatg aagtcacaca ccgtatgatg ccattgacat 900
gaagtgttca gagcaagtaa atccttacag atggaaggca gagcggtgac tgccagggac 960
taggagtggg gggccagggg gtgactgcta atgggtatgg gatttcattt cggggctggt 1020
ggaaacgttc cggagccaga gagtagtgat agctgcacaa ctctatgtat atgctatgaa 1080
tcaccaccga atggtatatt tttaaaggac gaatttatgg tatgtaaatt gtgtctcaat 1140
aaagctgcta tcttaaaatt caaaaaaaaa aaaaaaaact cgtag 1185

<210> 119
<211> 1098
<212> DNA
<213> Homo sapiens

<400> 119
tcgacccacg cgtccgcaga actctagatc tgggaataac cctgtatcta tttctttaca 60
tttttctttc attaatgtat ttatcactat tttttttttg tttttctttg caggcctcag 120
ctgttgaaga acgctctgca gagagcagta gagaggggcc agttagaaca gataactggc 180
aaaggtgctt cggggacatt ccaggaactc cgacccacct ggtgcagagg agtcttgtct 240
tgacctgctt tgggagagtg ttgtcttgat gcttttctct tcctccttgg aaaacagctg 300
aagaaatcag gggagaaacc cctgcttggt ggaagcctga tggaatatgc aatcttgtct 360
gccattgctg ccatgaatga gccgaagacc tgctctacca ctgctctgaa gaagtatgtc 420
ctagagaatc acccaggaac caattctaac tatcaaatgc atttgctgaa aaaaaccctg 480
cagaaatgcg aaaagaatgg gtggatggaa cagatctctg ggaaagggtt cagtggcacc 540
ttccagctct gttttcccta ttatcccagc ccaggagttc tgtttccgaa gaaagagcca 600
gatgattcta gagatgagga tgaagatgaa gatgagtcat cagaagaaga ctctgaggat 660

gaagagccgc cacctaagag aaggttgcag aagaaaaccc cagccaagtc cccagggaag 720
gccgcatctg tgaagcagag agggtccaaa cctgcaccta aagtctcagc tgcccagcgg 780
gggaaagcta ggcccttgcc taagaaagca cctcctaagg ccaaaacgcc tgccaagaag 840
accagaccct catccacagt catcaagaaa cctagtggtg gctcctcaaa gaagcctgca 900
accagtgcaa gaaaggaagt aaaattgccg ggcaagggca aatccaccat gaagaagtct 960
ttcagagtga aaaagtaaat tttataggaa aaaagggtat catgatgaaa ttcaaaatct 1020
tattttctaa gcacttttga tatcaagcaa gtggcttcct tttgagata ttaaaaaaaa 1080
aaaaaaaagg gcggccgc 1098

<210> 120
<211> 805
<212> DNA
<213> Homo sapiens

<400> 120
ggcacgaggt ccctaattgt cttgtaccta gccctagggt gaccagggca ggggaatcat 60
ggcgagaagc gtaagggcct gatgaagaag gtgtgctggg tgtgggctct agcccacttg 120
gttttgtgtg agaggtggct gacagcaggt tgtttgctgt atgtaggagt tatccagccc 180
tgcaagggca gtccctccag tgtctgcaaa gcccgaagat gtctgcatcc aaaatacaga 240
ataaaaagat atggttacta caagtactca gtaagactga taatctgtca tcatcatcct 300
catgccctta aagcagagct aactgatgat taatatatgc ttctatgtta acagtcttgg 360
actttattaa tggtgggtgg aagttaactt aatgtatgta tgcaaactaa aaagtggcat 420
ccttttcatt aatgacccaa ccattattca agagctatgt ctagttaggg acttcagact 480
tttgaaagaa atgaagaaat aatgccagat acatgggctc gcacttggaa tcccagctac 540
ttgggggacc gaggtgggag gaccgcttga gcccaggagt tcgagaccag cctgggcaac 600
atagcgaaac cctgcctcag ttttaaaaaa gaaaaaaaga agtagtgaag aaattggaaa 660
ggattctgag aagaaatatg caaggtggaa aagagcctag aaagaaaggt gacagatgct 720
gggatttggt cgtcagaaga gatatctagg aaatagcatg gcagccctca agtactagct 780
ccacttaaaa aaaaaaaaaa aaaaa 805

<210> 121
<211> 1598
<212> DNA
<213> Homo sapiens

<220>
<221> SITE
<222> (1067)
<223> n equals a,t,g, or c

<220>
<221> SITE
<222> (1069)
<223> n equals a,t,g, or c

<220>
<221> SITE
<222> (1577)
<223> n equals a,t,g, or c

<400> 121
aggaaagaac aaaggttatt tcctggagaa aagacaattt attcaacacc aacragggac 60
tcatcatatg ggcacaactc tggtgtcctt ctatggagaa aacctcaagt aaagttttat 120
tctgcctttr aaaatgcttc caaaagtaga ccctgtcccc acacaggtca agactacaga 180
gaaggctttg tagaaatgtg tcacctatgt acacctgcta cttacacatt tcctcttttg 240
gaaaaatgag atacttagaa taacargaaa attaagacat actggcctgg tgccagcaga 300

tggcttttct atagacaaac taggttagtg tggaag[?][?][?][?][?] [?]ggttaaaat aaactatgct 360
gttttattta tcttcccaac ctgattggca gctagacttt tttagggtct catttaatgg 420
ccctgttttt ttcattatta tatttaatga tagggcagga tttcgtatgc aagctcttgt 480
ttctcaggct gcctgcagaa gaagtcgcta taaattatct gttgtctaca tggtacaagg 540
cccattgact catctgatgc ttgttttgtt aatttcttta atatttttat cacggggcag 600
tgggagggct tgggctttta gccacagctg ttttaagact tctgatctcc tgccctgcag 660
gaataggtgg gaagtcattg aatttttaca ctatagtaat ttgcattccc acataagttt 720
gagtgttacg aaaacattcc tttaaaggga tctgtgctac acaaaatatg ccaggacctc 780
acagacaaag ccattgctag aaatgtcatt ccaatgatca gatctggaaa caggctgcca 840
taaccacttt tccttcttgt agactcagct cacctgtata tttaaactgt tcttggcatc 900
ttgaaacacc tatttctact caggtactca ttgtcctgtt actgattcac ctttctgatc 960
cttttcaacc agttttcccc caagggggga aattttactt aacctctagt atttgaacaa 1020
ctcaatattt gaattgttgc cccatttgct tttacctgta ctgtatncnt ggtcatctca 1080
aatggcgtct aaacccagct actttgcatt ccagaagttt ccattccctc caattccacc 1140
taatttttca tctgtcctag ttactggctc tttcttcatg tcttatttct cttgctttgg 1200
gagcttaaaa gattttacaa gacctaattt tgggttcctt ccttggagcc atagttaccc 1260
tgccaagaag agtagaaaat gggttcaact cctgtttcgc tccaccaaca cctctgtgag 1320
tctcatcatc agctgagcga tgatgcctta caggttgcat agcactggaa ctttcctaga 1380
gtaacggctc tgctgccagg gtttctctgg gctcattctt ccactgactt aattatgatc 1440
tatgcctaac agagccccag tacaactatt ttgcagaatg gctgttaccc tagaattact 1500
atagcacata ttgagatata gttgtactcc ctagtagata ggaactgacc ccaacaataa 1560
actttgataa taaaganaaa aaaaaaaaaa actcgtag 1598

<210> 122
<211> 1020
<212> DNA
<213> Homo sapiens

<220>
<221> SITE
<222> (3)
<223> n equals a,t,g, or c

<400> 122
cgncacgagg tgaagttcaa cccaatgcaa ctttccttca gtctttccgt gaaagcgcct 60
gtgaaaaatg aggtcatatt tccctttctc agtctgcccc ttcccgtttt gctctccggt 120
tttcttcttt gtcttcacag atgtttacct atgttttttc tttgtttttg ctgttggaag 180
acatctaagt gatccttttc ccattctctt tttcactcat aaatgtcctg atgtttagca 240
aaaggcagtt ctctttgcta cttgagcttg taaactgttg ttaaatgagt aaccaaaagg 300
aaagtccttg cgaagttggt taccatttca gatacaagaa ccgtttatct tcccacgctg 360
acgaattttg cgagtgagat gattattttt ccttgtgttt gtaatttatt taagtaaatt 420
ccttgtttgt ttttcttttc agtacaccag gggtatatat tttcaatatg acatgtacct 480
ttggttcagg gctaagttag agtctgaaaa atgaagcctg taggattcat ggcagtgatc 540
taattgtgat tcatcttact gattgtaggg caagaagagt ggactaactc aagacacaag 600
gcaccttcag cgaggacagc aaagggcgtc tacagagacc agccatatgg cagatactga 660
ttgtactgtc tgatgttgtg aaatagccaa tctccaccag tcctgtatac tgttcaaagt 720
aatttttttc tatgaacaat ccctttttaa ataaatcaaa atgcttaaaa tctgaatgga 780
tggaacttaa aactactttg ttgaaacatc aacctgggca gaaaaaaaaa aaaaaaagac 840
atgtaaaatt ttgttatttc cagtctgtat atgaaaaaat aggtcatcaa aaggaaaaaa 900
aataactttg attaactagt gttaaacaaa aaataggttt actaaatctc gtgccgaatt 960
cgatatcaag cttatcgata ccgtcgacct cgtagggggg gcccgtaccc aatcgcctgt 1020

<210> 123
<211> 1378
<212> DNA
<213> Homo sapiens

<400> 123
atcttagcaa tctccttggc ccaaaacttc accccatctt ggaagggagg ggagagagaa 60
tgttctgatc tatatctgat gagggcgtgt ggttgggacc tgagcatcct cctggttggg 120
ctagtgatgg ggagagaggg ctgttactca cgactccctc caacagaata ccagaaacag 180
gcaggcagct caggtgtatg taaggatgtg aggccaagaa accagccctc accaagttac 240
ccctgtaaat ccttgtctcc ccatgcacct ctactttgag tcagaaatgg attcattgca 300
ggctcagttg tttgtattat gtgaatgaac tgaacgtaac caagcaccaa gagagcccta 360
aagacacagt agacctcctg tagaagggct ctgatggacc ttcaaacatt gctyctccaa 420
ctttatggtg cacacaaatc acctgtgcat gttaaaatgc agacggtgac ttacagatct 480
tgggggaggc caagtgtctg catttccaac aagctcccag atgatggcca cactgctgat 540
ctgaggacca cattttgaac agcaatcctt aaaacacaca aggctgtggg accgcactcc 600
tgagaagaga cctcaytctt ggaagggatt ytaggaggct gcattcaaac catcccagag 660
acagctcaca attttgttag ggacctgata ctaaaaatta ccaaatcaca atcgtgtgct 720
gagggcttac tttgtgcttt tcagatgaca tttcatttga tgctctcaat aaccctgaag 780
ggatttcaga gatggggatt atgatggagc ttcagaatta aactgattaa aactggccca 840
gagtcacaca actatgaaga ggctgagttg ggatttcttt taatttttat tttttgaaac 900
agggtctcac tatgttgccc agactggtct cgaactcttg agcttgagca atcctcctgc 960
ctctgcccct gagtagctgg gactacagat gtgagacagc atgccgagcc tgctgagttg 1020
ggatttgaac cccaggtttg tctggctgta agtcctccag tgtggaagta cgatagctac 1080
ccatctcagc cacggaaaac agcagagttt tggttcaggc aagtagcact aagtagaaag 1140
cccaggtatg aaccagatct gcaggagccc aaagtcattg ctcagaacca ccccctaccc 1200
gctgagaagt tcttcctgtc cctggaagtt ctctctgaga tccacccaaa tgtgtcctca 1260
ctaaggcgaa atctggcttc tcctgtttgg gcctggggag aaacataaac ctagcccttt 1320
gtacacaccg cctcgtgccg aattcgatat caagcttatc gataccgtcg acctcgta 1378

<210> 124
<211> 1146
<212> DNA
<213> Homo sapiens

<400> 124
tcttttctgt aacttcattt agcactcaaa acaatatcac tatttaatgt tacactttga 60
ctacaaactt atctttctcc ttacaagctt tttatgtatt taatattatc ttggctattt 120
ctgtgcaaac tagttaaatg ttactttgaa attcttcttt tctctacatc acctcagtta 180
ctccagtgga cagtaattgt tacttactgt ggtcctctgt tacggttttg aatttcttca 240
aatgcctttt gaatttaatg aaaatactac atgaaataat actggtggct acataatttt 300
cttccacttt ttcttaagtc tctgcaatga aacagctgac agtaaggtgt gcgtgagtgg 360
tgagatatgt taactcctat acttcacttt agcttatgtt gtcagggagt gatctccaaa 420
tgcaaaaata taaagtactc tattcttggg gaacaatgtg agcaatggaa ggcttggcct 480
ttcttaggca gatccctcag attcaaaagg aacacatctt gatttaatat caatctctat 540
acaggggcct gagaatggga tgaggagagc tgctggtctt tgtactgcaa tttaattcac 600
tctgattact ttctatcctc ccacccccac tcttcctcca gtctctttct gattagttat 660
catagtcttc cccaagactc tatatatgct ctctgggttc ctgtcaagag gaaatcctgt 720
acacaccccc tactcctcta tcactgcttc ctggtctggg cactctcact gtgcagcttc 780
ttctgctctg gtttagccaa caatataatc catagatttt ctgaattcca tcatagcatc 840
tggtctttgt gtgagtcctc tttcattccg tgtctattac gggtattttt aaatatcttt 900
actgttgata cagtgagatt tgtgggaaag aggatagaaa cataagccaa tctcccctct 960
tgaaatggac atctattatt aattaacact catcaaaata aatgttctta ttgttatatc 1020
ttagttggat aaagatgaaa agataagacc aaaatcctgc tctctaatca cgatttattg 1080
taacaataat attattgtat tattgggggg ggcccgtacc caatcgcctc acatgcatcg 1140
tataca 1146

<210> 125
<211> 1675
<212> DNA

<213> Homo sapiens

<400> 125
   cttaaagacg ccaggtagag acacacagaa cgtatgtatt aagaatatcc tctctgggct 60
   ctgaaatttt aggagtgatt cttatccact ccaagttgta agtatttgta gaaatttgtg 120
   caaacaaaca aaaactatca aatgaaaaga aaatgtactc aacctaactt atagttagca 180
   gctggaattc tcaactcttc cctgccagca ctataccaca gtgtggaaga aattagtcaa 240
   atgcttgttt tcctgcttct cttttcaact gttactgtgc tttgtttgaa agtagttttc 300
   tctctcaaag ccgttgctta tatcgttaag aatgaaggtt tgtgtttaaa atttattgca 360
   ttgcaaaggg tagtttcact gaagtcatgc accattaaat aagatgaaat atttgtattt 420
   attgtcctac ttcctaagcc gtaacttctt ttcctctgtg aatttgcatt gagtcactca 480
   tgctacacta catcgcttta gtatttgaga tggcatttat gtttcctctc gtttatcatg 540
   aaatggggtc agattccatc agattccacc tctgtcaggt ggactcttgt ctgccttcca 600
   tgatgagatt ttttttctcc ttcccctttc tttaagagag gctgacagat ctaggtgtca 660
   atcaattgga aaccagtctc tgattttttt tcattagtta ttttctatca ttagtttcac 720
   tgtgtaaatt agatatcaac tgcacttctt taaaaaaaaa tacatctccc tattacctcc 780
   ttgaaagatt tacttctgta ggcctttttc aataggctca tgactgcaga caaggaaaaa 840
   aaaagtaaaa acaaaaacag tatgtgcctg aaaatgacaa aaaaaaaatt tgtaacattt 900
   aaaaaagaaa cctgaatagc ctttaattct ttaataatac acttaaattt tatgtaaatc 960
   ggttttcgcc acgtgtgttt gttcacattc taaatgactt aatgggattc tcacggtctg 1020
   tgtctttgtg tcacgtgtat aaaatgggct tgtgatgtaa gcgtttcatc tggtcagtgg 1080
   ttcctttgat attgtactgc tgctgggagt gggctgtgga acctgccttc gggtaactgg 1140
   gttcctcttg ggtagattgg agagatgggg gtgggcgtgg gcaaattctc acacatgttt 1200
   tcttaaccta tttgcagaaa ctttcaaaag gcatttgatt aaacctcttg gcagtacagt 1260
   attcttgtat ttgttaacgt ctgtgtttag gtactggtac ctttttgttt taaaatgttc 1320
   taagtgttgg ctttaaagtg aatttatctt tagtatgata gttatatgaa aattatagga 1380
   tttgtgtgca gagaattttt ttataaagtg ctttgtaaaa aaaaaaaaat gtattctagc 1440
   ttttgcggta catatgtgtg ataactttaa tacccatgac agttaagtgc aattatttca 1500
   tcactctaaa aatgctattt ttgtgtcagt tcctgcaggt gttttcatgt ctttgcaaag 1560
   tgacacattt tgatgccttc ttgataaagt ggtagacatt ttgtagcttt ctagaaactt 1620
   tgtattcata cggtatcaat gaaaaataaa gaaaatgaaa gtgtgggtca aaact 1675

<210> 126
<211> 1064
<212> DNA
<213> Homo sapiens

<400> 126
gcggaggggt ttctgtgcag gacgggagtc tcagagagga gacggatgtg ggggagggag 60
gccggccacg cggtggacag agcgagggtg ccagggtgac ccgaagaccg tcaccacccg 120
acagcaacgc aagtgccttt gaccttgatt tggacttttc tcccttttgc atttggtgct 180
acagacttga gacaccagca gaagttgtgt tcagcccggc cccgctgcgc ctgtccgggc 240
cggggctggc gccggttgtg tttgtgtcca ccttgccttc tttgcagcca agcagttttt 300
gtggatggga cttacctgca cgccccaggg gtctttcagg attcaggatg acttttcttt 360
tacaatggtt tcctctcggc agagcccggg ttgtggggga tctgtgtggg ttctcaacgc 420
agatccatcc tggggtctcc cgggcaggga tggctgacct cgagtcccct cccttcccga 480
gaacctgctc tgtcccgagg gcagctaaca agggctgagc cccaggtaca ggttgcctcc 540
tccacggcag gaatttttac caaaaccaca agcaaaaaac aaaacagacc accacgacca 600
acaacaaaga tggggggtar ggttttgtaa aggttctgtt aggttcatat ttttatatca 660
ttttgcccat aaatgcggaa tttgccgtgg gaatttgaag acaaatgatc tatgttttta 720
tggttttcta gggaargtgt tctgggggcc gggctctctc cagctgtggg aggcctgctc 780
cctctggggg gcaccctggg cagggtgggg gggccttggg aggcgcttct tgccaaatgc 840
agacgagggg tgagcctgcc agcgtttgcg acgtccccgc acgacaggct catactttct 900
gaggatcgtg catagcatag gacgtctgaa cctttgtaca aatgtgtaga tgacatcttg 960
ctacagcttt tatttgtgaa ttaaagatgc attgatggtt aaaaaaaaaa aaaaaaaaaa 1020
aaaaaaaaaa aaaaaaaaaa aaaaaaaaaa aaaagggcgg ccgc 1064

<210> 127
<211> 1607
<212> DNA
<213> Homo sapiens

<400> 127
ggcacgagga aaaacttatt tatcgataga cctcatttat gcaaatgttt gtaaagtatc 60
atgttattat ggtaattatt tttattttca tacttatcac atcagacaag catggtgaaa 120
taatatatat taaatacata gatagagtta tcataacaga gagaatctag taaaaaaaaa 180
agacatgaaa aacatcccat taaagaaaaa gcctttatat gggacattat tcacatttct 240
gcaaatggaa ttttctgatt aactgccata tattatatta tttgaataac tttaggraag 300
rttaatttcc aagctctgtc cctggtagta ttttttcctg gctgaaattg ggctttaaaa 360
caaaacgtca gcaatgcaag ctgacgattc cagttacagg ttcagaaact tcgagtgcag 420
gcagttaaaa gtgatttaag aaaggcaaaa tcattctttc acaaatgtgt atgttaaact 480
tgtaaattga tatatcaaaa ccacaaccgg aatatagcaa tttgtctctc tttgcctgct 540
gtggtgtctc ccgcagctgc ctgccagata atatactgat tgtaactttc ctaatttaca 600
gagcctttaa gtctaatctc ctgggccagc ctaaagggct tcttcatcca ttgaaatgcc 660
agaataattg aaattgtctt cagcagacag tcaggcaata atgaatattt tctctgctga 720
ttgcatgcca aggttgcaca tagcactgca aacagaaatg atcccaaacc gagcycccca 780
gggaggggca gccgctaacc tttggcatga ggcacaatac cgacggcttc ccttctcacg 840
ggctcctgaa rtcacggacg cccaccaagc ttctgcacaa cgaggtgcag cacagttgcc 900
gcgtgaacaa tgatgccatt ggcttccctg actctccaga ggcagcttaa aggctgggaa 960
aggttgtggt tatggatttt ctgatggggg cagcgggaga gatgagcaga ggaggctgct 1020
gttggcatta agaggaaata taccaatagt taatcaaaag aaacccggcg ttgaaggggc 1080
atgagaggac gctggcttgt cagctctgga gcagcatttc ccataccttt gaatggtttt 1140
tgtgaaagcc gggagttctt tcctgactca ggatcagtgc tgcttcattg gcgtcctaat 1200
grtgtgctga ttgaaattaa agtgtttggt agccgaagcc aatcactaat atcttcaaaa 1260
aatctaaaga ctagtttaac cttcatctac ggaaaggttg aagaagtttt gaataattaa 1320
tataagcaac taatcttaga ttatggctac ttccatgata tgcgttagag gtggaaattg 1380
ggggatgaga aaggaactac cctaagacaa tatggattta agccacatag gaagtgtgca 1440
gattaaacag ccgggcgtgg tgatgcacct ctgtaatccc agcccagcta ctcatgaggc 1500
tgaggcagga gaatcgcttg aaccctggag acggaggttt cagtaagccg agattgcatc 1560
actgcactcc agcctcaaaa aaaaaaaaaa aaaaaaaaaa aactcga 1607

<210> 128
<211> 1037
<212> DNA
<213> Homo sapiens

<400> 128
agctactccg tctggccccg ccttttctct gctctcctga acctttaggc ttgtctcggc 60
ccatttgaag accaggaagt tgatcaatcc cgaggctgct gagagacggt ggcgcgattg 120
ggacagtcgc cagggatggc tgagcgtgaa gatgcagcgg gtgtccgggc tgctctcctg 180
gacgctgagc agagtcctgt ggctctccgg cctctctgag ccgggagctg cccggcagcc 240
ccggatcatg gaagagaaag cgctagaggt ttatgatttg attagaacta tccgggaccc 300
agaaaagccc aatactttag aagaactgga agtggtctcg gaaagttgtg tggaagttca 360
ggagataaat gaagaagaat atctggttat tatcaggttc acgccaacag tacctcattg 420
ctctttggcg actcttattg ggctgtgctt aagagtaaaa cttcagcgat gtttaccatt 480
taaacataag ttggaaatct acatttctga aggaacccac tcaacagaag aagacatcaa 540
taagcagata aatgacaaag agcgagtggc agctgcaatg gaaaacccca acttacggga 600
aattgtggaa cagtgtgtcc ttgaacctga ctgatagctg ttttaagagc cactggcctg 660
taattgtttg atatatttgt ttaaactctt tgtataatgt cagagactca tgtttaatac 720
ataggtgatt tgtacctcag agcatttttt aaaggattct ttccaagcga gatttaatta 780
taaggtagta cctaatttgt tcaatgtata acattctcag gatttgtaac acttaaatga 840
tcagacagaa taatattttc tagttattat gtgtaagatg agttgctatt tttctgatgc 900
tcattctgat acaactattt ttcgtgtcaa atatctactg tgcccaaatg tactcaattt 960

aaatcattac tctgtaaaat aaataagcag atgattctta aaaaaaaaaa ataaaaaaaa 1020
aaaaaaaggg cggccgc 1037

<210> 129
<211> 1146
<212> DNA
<213> Homo sapiens

<400> 129
ggcacgagat ttttggtcag gtttttatgt tgtttttcag tttgaaggag tcgttatata 60
tttttcatac tgctattttg ttagtagtat gctttgcatg tgcagtagta tgtcagtatg 120
taatagtacg tgtatgtgca gtagtatttt gcttttcaaa atctcagtct ttgatctgaa 180
tttaaagaat agtatttttt tgtgtaagag gttattttta tcttttgcta tttaaaaaat 240
caagtgccat ttgtatatga tagtacttaa gtcttcagtt tgaattctaa aaatgtgtat 300
acctatataa tccacacacc tgtcatccca tcatcctatc aacacccctg aaaattccct 360
tgtgcctctt cctagtcaac cccaccactg cctcaagtaa gtactcttct gatttctgtt 420
ctcatagatt agtttcactt gctctagaac atcatataat ggggctgggc gcgatggctc 480
acgccagtaa tcccagcact tgggaggctg aggtgggcag atcacttgag gtcaggagtt 540
tgagaccagc ctggcaaaca tggtgaaacc ctgtctctac taaaaatgca aaaattaacc 600
tggtgtggtg gcatgccttt tgtaatcccc agctactcca ggagtccaag gcccgagaat 660
cgcttgaacc tgggagacag aggttgcact gagctgctgc actccagcct gggcaacaga 720
gtgagactgt ctaagaaaaa agaagtttat atgatggaat tatacagtaa ataatctgta 780
tctattttca tttagcattg tatctatgaa attcatgcat gttttatcta ttaatatttt 840
ttttggccgg gctcggtggc tcacgcctgt aatcccagca cttttggagg ctgaggcagg 900
tggatcacta ggtcaggagt tcaagaccag cctaggcaaa gatggtgaaa ccccatctct 960
actaaaaata caaaaattag ctgggcatgg tggcaggcgc ctgtaatccc agctactcag 1020
gaggctgagg cagagaattg cttgaacctg ggaggcagag gttgcagcaa gctgagatcg 1080
tgccactgca ctccagcctg ggcgacagag cgagactcca tctcaaaaaa aaaaaaaaaa 1140
aaaaaa 1146

<210> 130
<211> 1172
<212> DNA
<213> Homo sapiens

<400> 130
ccacgcgtcc gaacaccttg cagctttacc tgtgaatgtc aagattggca agatgcttat 60
ttttggtgcc atatttggct gccttgaccc agtggcaaca ctagctgcag ttatgacaga 120
gaagtctcct tttaccacac caattggtcg aaaagatgaa gcagatcttg caaaatcagc 180
tttggccatg gcggattcag accacctgac gatctacaat gcatatctag gatggaagaa 240
agcacgacaa gaaggaggtt atcgttctga aatcacatac tgccggagga actttcttaa 300
tagaacatca ctgttaaccc tagaggatgt aaagcaggag ttaataaagt tggttaaggc 360
agcaggattt tcatcttcca caacttctac cagctgggaa ggaaacagag cctcacagac 420
cctctcattc caagaaattg cccttcttaa agctgtactg gtggctggac tgtatgacaa 480
tgtggggaag ataatctata caaagtcagt ggatgttaca gaaaaattgg cttgcattgt 540
ggagacggcc caaggcaaag cacaagtaca cccatcctca gtaaatcgag atttgcaaac 600
tcatggatgg ctcttatacc aggagaagat aaggtatgcc agagtgtatt tgagagaaac 660
taccctaata accccttttc cagttttact ttttggtggt gatatagaag ttcagcaccg 720
agaacgtctt ctttctattg atggctggat ctattttcag gcccctgtaa agatagctgt 780
cattttcaag cagctgagag ttctcattga ttcagtttta agaaaaaagc ttgaaaatcc 840
aaagatgtcc cttgaaatga caagattctg cagatcatta cggaattgat aaaaacagag 900
aataactgaa actgaaattc atggtcaact gctttaaaaa ttaagatgaa gatacagtca 960
tgaaattatc tgaaaatggg tcatcacatt aagtatttca ttacttaaaa tgttggtact 1020
agccattaac ttaaaggtgg tgggaaaaaa gcacatactt taaacatgta taattttcta 1080
gttccttttt aatgatgatt attctgaatg tatttgccac tacatttaca ataaattctt 1140
tggtattatg aaaaaaaaaa aaaaaaaaaa aa 1172

<210> 131
<211> 663
<212> DNA
<213> Homo sapiens

<400> 131
ggcacgagaa accatgaaag tcctttcttg gatccacttt atcttgatta gtctgcattt 60
tactagttca ctggatccct cctctagggg cctggggact ttcactgatg ctcttcctga 120
ttctagagca aaggtgtggg aaggggaaat ggaggaatgc cctcctgtct gtgtcgttct 180
ctgtgccaca gctacagatg cagaaggttt ctctggatag cacacctctg aatgtaaatc 240
atgataaaat ggatatttgg aaacttactc ctaagctgtg atttagggtg tatttctact 300
tctggactgc ctcaatatca agggctgaga cttttgaatt ttgaatattc gttgggtttc 360
atgttaagaa gcctgtggtc taggagtgct attcagtgtt tcttttcctg ataaacactt 420
tgaatatttt ttttgtgttt ttgtttcctt ttctgaagct gttcctcctt ttaaatattt 480
ttaatcacat tgataaaatc tatccttcac cacctctggt tctactatag ttgattttta 540
ttttaaatgt ttaattgtat ttgattaaac acttaactgg attttggaat aataaaactc 600
tcgtccaatt tggcttttaa aaaaaaaaaa aaaaaaaaaa aaaaaaaaaa aaaaaaaaaa 660
aaa 663

<210> 132
<211> 776
<212> DNA
<213> Homo sapiens

<400> 132
   ggcacgagct gatttctatt tttaggagct acttggattt gtatgtattt tttctacgtg 60
   aaaatatatg tactcttcac ttttgttcca gtactataat tgctcatgca ctctttctcc 120
   cctttgagaa cattcagtga aatacaactt catcaaagat ttgctcaaag gagaagaatc 180
   gcatgagtgt gaaaagtaga tgctcgtagc cagaacagaa aaggttacac atgatcatgg 240
   cacagaagat aggaggtttg acttggtggg ccataatgtt tattatcctt tttgaaataa 300
   cagggaccag cagcagtttt ctcaggataa atgctctacc ccacttctct atgaacaggt 360
   gtggggaggc ttactttcca ttttcatatt tatacacctc tctacaaaag caatttttaa 420
   tgaaggttag tggaattgtt aaaaatctga gagggaatga tgactggagg tgttttgggg 480
   tttttttctg tattcatttt ttaatgagaa aagttttaaa tgtagtacag gttagaccca 540
   actactacct tactattata ggacgattct atgtttctgt taaagtattc aagtagcttt 600
   ctctggggga aaaagtacca cttggacact taaaggaatt gggatttttg tctactttgg 660
   ataaggcagt tgacttctta agtaaaagca atagtgtaaa atgtcatttt gtttggaatg 720
   ttaagtgagc aaataaaaaa catgttgaaa ttgtaaaaaa aaaaaaaaaa aaaaaa 776

<210> 133
<211> 1543
<212> DNA
<213> Homo sapiens

<220>
<221> SITE
<222> (1055)
<223> n equals a,t,g, or c

<220>
<221> SITE
<222> (1143)
<223> n equals a,t,g, or c

<400> 133
cttcgccgtc atccgcttcg aaagcatcat ccacgagttc gacccgtggt ttaactatag 60
atcaacacat catcttgcat ctcatgggtt ctatgaattt ttaaattggt ttgatgaaag 120
agcatggtat ccactaggaa gaatagtagg tggtactgtt tacccagggt tgatgataac 180
cgctggcctt attcattgga ttttaaatac attgaacata actgttcaca taagagacgt 240
atgtgtgttc cttgcaccaa cttttagcgg ccttacatct atatctactt tcctgcttac 300
aagagaactt tggaaccaag gagcaggact tttagctgct tgttttattg ctattgtacc 360
aggctacata tctcggtcag tagctggatc ctttgataat gaaggcattg ctatttttgc 420
acttcagttc acatactatt tatgggtaaa atctgtaaaa actgggtcag ttttttggac 480
aatgtgctgc tgcttatcct atttctatat ggtctctgct tggggtggtt atgtatttat 540
catcaatctt attccactgc atgtatttgt gttgttactg atgcagagat acagcaaaag 600
agtctacata gcatatagca ctttctacat tgtgggttta atattatcaa tgcagatacc 660
ttttgtggga ttccagccaa tcagaacaag tgaacacatg gcagctgcag gtgtctttgc 720
attgctgcaa gcttatgctt tcttgcagta tctgagagac cgattaacaa aacaagagtt 780
ccagaccctt ttcttttttgg gtgtatcact agctgcaggt gctgtgttcc ttagtgtcat 840
ctatttgact tatacaggtt acattgcacc atggagtggc aggttttatt cattgtggga 900
tactgggtat gcaaaaatac acattccaat tattgcatca gtgtctgagc atcaacctac 960
gacttgggtg tctttcttct ttgatctaca tattcttgta tgtaccttcc cagcaggcct 1020
ttggttctgc atcaaaaata tcaacgatga aagantattt ggtaagarag gtttttaatg 1080
actactttga tatggaatag ttatttttct ttttgagatt atttacttta aatttttgtt 1140
ttnctatgtt tgactctata tattcaagat aaattttctc ctttattttg cataggtgct 1200
taaccaagaa aaattcactg agaggctggg catggtggca cacgcctkta atcccagcac 1260
tttgggaggc cgaggcgggc ggatcacctg aggtcaggag ttcgagacca gcctggccaa 1320
catggtgaaa ccttgtctct actaaaaata caaaaattag ccgaacatgg tggtgcatgc 1380
ctgtaatccc agctactcag gaggctgagg caggataatt gcttgaacct gggaggcgga 1440
ggttgcagtg agtcaagatc aagccactgc actccaccct gggaatcaga gcgggactct 1500
gtctcaaaaa aaaaaaaaaa aaaaaaaaaa aaagggcggc cgc 1543

<210> 134
<211> 2157
<212> DNA
<213> Homo sapiens

<220>
<221> SITE
<222> (309)
<223> n equals a,t,g, or c

<400> 134
caaaaaggac cgcccattga agatgccatt gcttcttccg atgttcttga gactgcttct 60
aaatctgcta atccacccca cacgattcaa gcatcagaag agcagagttc aaccccagca 120
ccggtgaaaa agtctggcaa gctgaggcag caaatagatg tgaaggcgga actggagaag 180
cggcaaggag ggaagcagct actcaactta gtggtcattg gtcatgttga tgctgggaaa 240
agtactctga tgggccatat gctttatctt ctgggtaata taaacaaaag aactatgcat 300
aagtatganc aggagtctaa aaaggctggc aaagcttcgt ttgcatatgc atgggtcttg 360
gatgaaactg gcgaagaaag ggaaagggga gtaaccatgg atgttggtat gacaaagttt 420
gaaaccacaa ccaaagttat tacattaatg gatgctccag gccataagga cttcattcca 480
aatatgatta caggagcagc ccaggcggat gtagctgttt tagttgtaga tgccagcagg 540
ggagagtttg aagctggatt tgagactgga ggacaaacac gagagcatgg actcttggtc 600
cgttctctgg gagtgacgca gcttgcagtt gcagttaata aaatggatca ggttaattgg 660
caacaagaaa ggtttcaaga gattactgga aaacttgggc actttcttaa gcaagcaggt 720
tttaaggaga gtgatgtagg ttttattcct acaagtggtc tcagtggtga aaatctaatc 780
acaagatctc agtcaagtga actcacaaaa tggtataaag gactatgttt attagaacaa 840
attgattcct ttaagcctcc ccagcgatct attgacaaac cttttagatt atgtgtgtcc 900
gatgttttca aagatcaagg atctggattt tgcataactg gtaaaataga agctggttat 960
atccaaactg gtgaccgact actggcaatg cctcctaatg aaacttgtac cgtgaaagga 1020
atcactctgc atgatgaacc tgtcgactgg gcggcagcag gcgatcatgt tagtcttact 1080

ttggttggga tggatatcat caaaatcaat gttggctgca tattttgtgg ccccaaagta 1140
cccattaaag cttgcactcg tttcagagcc cgaatcctca tctttaatat tgaaattcct 1200
atcactaaag gatttcctgt gctgttacac taccaaactg tcagtgaacc cgccgttatt 1260
aaacgattga ttagtgtctt aaacaaaagc acgggtgaag tcacaaagaa aaagcctaag 1320
tttttgacta aaggccagaa tgcattggta gagctacaga cacaaagacc aatagctctt 1380
gagctatata aagactttaa agagctgggg aggttcatgc tacgttacgg tggttctaca 1440
atagctgctg gtgttgtcac tgagataaaa gaatgatggg tcmgaatttc taccacgttt 1500
ctggatacag tgaaatagct aacctctgty tcaagaatgc agttattaag tcaaaggaac 1560
aatgtgcaat tgatatgttt ttagatgaga gagaaaaatt aaagctaaaa ttagctgcaa 1620
agaagtatta ataatcacct ctgcaaaaat tctaagttgc caactggcaa agraagtcta 1680
atgttaaaaa caactttgcc tttgaamcgt taataaatgg atttactttg ctaagattta 1740
tggcaagtgt caaaaatagt atctgaagat actgaatcat catgaaatga actctacttc 1800
tggccaaagc acaatgtatt tgcagttttc tcttttgatt caattatact gcacatgttt 1860
taaggaaaag taacttaatt gggtttttca ggcagttgat atttgaccta agcttttttt 1920
tttttttttt ttccagttaa tgctaagaaa agatttgggg aaggttataa taaaagtatt 1980
ttgtggtgac cataagaatg tccctcccca aacaagtaaa cttgtgaaag tttaatttgg 2040
aattagtgga agctgttcct ttgaaagcca agatattatt taagttgtaa agccagctaa 2100
taaaatgcct tagtttgagc ataaaaaaaa aaaaaaaaaa aaaaaaaaaa actcgag 2157

<210> 135
<211> 420
<212> DNA
<213> Homo sapiens

<400> 135
ggcacgagag agagcagagc tatacatagc tatccaggtc taacttcacg aagaatagaa 60
tggtttcttt tcattttcaa tgtacatcat actttgtcag actttttttt cagttgcagc 120
tcttcgttgg actggtgata gtattggctt tattaatctc tcattctctc acttattcat 180
tccacaaaca tttgtagaag gccaccaagc tctagggaga ggaaaatggt tttataaatt 240
agtgctttct gggataaagg aaatttataa tctgtactac ttaatagtag ccactagcca 300
catgtggttt tcgaacaaga tttccatcac ctctccaacc actttctcct cattggtcag 360
atctagaccc cgagaaactg ttcctttcat tgttttctcc gccttctaca aactgagata 420

<210> 136
<211> 334
<212> PRT
<213> Homo sapiens

<400> 136
Met Phe Gln Cys Gly Leu Leu Gln Gln Leu Cys Thr Ile Leu Met Ala
1               5                   10                  15

Thr Gly Val Pro Ala Asp Ile Leu Thr Glu Thr Ile Asn Thr Val Ser
            20                  25                  30

Glu Val Ile Arg Gly Cys Gln Val Asn Gln Asp Tyr Phe Ala Ser Val
        35                  40                  45

Asn Ala Pro Ser Asn Pro Pro Arg Pro Ala Ile Val Val Leu Leu Met
    50                  55                  60

Ser Met Val Asn Glu Arg Gln Pro Phe Val Leu Arg Cys Ala Val Leu
65                  70                  75                  80

Tyr Cys Phe Gln Cys Phe Leu Tyr Lys Asn Gln Lys Gly Gln Gly Glu
                85                  90                  95

Ile Val Ser Thr Leu Leu Pro Ser Thr Ile Asp Ala Thr Gly Asn Ser
            100                 105                 110

Val Ser Ala Gly Gln Leu Leu Cys Gly Gly Leu Phe Ser Thr Asp Ser
        115                 120                 125

Leu Ser Asn Trp Cys Ala Ala Val Ala Leu Ala His Ala Leu Gln Glu
    130                 135                 140

Asn Ala Thr Gln Lys Glu Gln Leu Leu Arg Val Gln Leu Ala Thr Ser
145                 150                 155                 160

Ile Gly Asn Pro Pro Val Ser Leu Leu Gln Gln Cys Thr Asn Ile Leu
                165                 170                 175

Ser Gln Gly Ser Lys Ile Gln Thr Arg Val Gly Leu Leu Met Leu Leu
            180                 185                 190

Cys Thr Trp Leu Ser Asn Cys Pro Ile Ala Val Thr His Phe Leu His
        195                 200                 205

Asn Ser Ala Asn Val Pro Phe Leu Thr Gly Gln Ile Ala Glu Asn Leu
    210                 215                 220

Gly Glu Glu Glu Gln Leu Val Gln Gly Leu Cys Ala Leu Leu Leu Gly
225                 230                 235                 240

Ile Ser Ile Tyr Phe Asn Asp Asn Ser Leu Glu Ser Tyr Met Lys Glu
                245                 250                 255

Lys Leu Lys Gln Leu Ile Glu Lys Arg Ile Gly Lys Glu Asn Phe Ile
            260                 265                 270

Glu Lys Leu Gly Phe Ile Ser Lys His Glu Leu Tyr Ser Arg Ala Ser
        275                 280                 285

Gln Lys Pro Gln Pro Asn Phe Pro Ser Pro Glu Tyr Met Ile Phe Asp
    290                 295                 300

His Glu Phe Thr Lys Leu Val Lys Glu Leu Glu Gly Val Ile Thr Lys
305                 310                 315                 320

Ala Ile Tyr Lys Ser Ser Glu Glu Asp Lys Lys Lys Lys Lys
                325                 330

<210> 137
<211> 43
<212> PRT
<213> Homo sapiens

<220>
<221> SITE
<222> (43)
<223> Xaa equals stop translation

<400> 137

Met Thr Val Ala Ser Ile Arg His Ile Leu Val Glu Ile Trp Leu Pro
1               5                   10                  15

Ile Ala Leu Ala Met Gly Thr Arg Gly Leu Thr Gln Ile Val Ala Val
            20                  25                  30

Ile Gln Ser Arg Ser Gln Trp Ala Leu Ser Xaa
        35                  40

<210> 138
<211> 87
<212> PRT
<213> Homo sapiens

<220>
<221> SITE
<222> (87)
<223> Xaa equals stop translation

<400> 138
Met Leu Phe Ile Phe Leu Leu Leu Ile Leu Ser Ile Thr Ala Ser Tyr
1               5                   10                  15

Ser Leu Thr Cys Ile Leu Ser Gly Ala Gly Glu Pro Ser Ser Val Ser
            20                  25                  30

Ala Ser Val Val Ser Gly Pro Gly Phe Cys Leu Ala Ala Leu Leu Leu
        35                  40                  45

Met Arg Thr Gly Gly Phe Ala Ala Thr Leu Leu Pro Val Ala Pro Thr
    50                  55                  60

Glu Arg Phe Phe Ser Cys Cys Thr Val Leu Ser Ala Gln Arg Asn Val
65                  70                  75                  80

Ser Arg Thr Arg Ser Pro Xaa
                85

<210> 139
<211> 122
<212> PRT
<213> Homo sapiens

<220>
<221> SITE
<222> (122)
<223> Xaa equals stop translation

<400> 139
Met Leu Ser Thr Arg Trp Met Gly Leu His Leu Val Gln Ile Leu Trp
1               5                   10                  15

Arg Cys Trp Thr Ser Ser Ala Thr Ile Thr Ser Arg Lys Leu Ser Thr
            20                  25                  30

Ala Leu Arg Ser Pro Val Leu Ser Gly Thr Gln Thr Ser Arg Ser Ser

        35                  40                  45

Gly Asp Ser Gly Trp Ser Met Lys Thr Ser Val Lys Ala Thr Pro His
    50                  55                  60

Gln Met Ser Leu Arg Ser Gly Lys Glu Thr Pro Ser Ala Asp Ile Pro
65                  70                  75                  80

Arg Ile His His Gln Leu Val Arg Leu Arg His Gln Ala His Gly Gly
                85                  90                  95

Trp Ser Pro His Gly Val Pro Glu Gln Gly Thr Met Pro Leu Val Leu
            100                 105                 110

Pro Pro Val Ser Cys Asp Ile Gln Pro Xaa
        115                 120

<210> 140
<211> 276
<212> PRT
<213> Homo sapiens

<220>
<221> SITE
<222> (131)
<223> Xaa equals any of the naturally occurring L-amino acids

<220>
<221> SITE
<222> (276)
<223> Xaa equals stop translation

<400> 140
Met Ala Asn Thr Gly Val Phe Gly Phe Ser Phe Leu Leu Leu Thr Val
1               5                   10                  15

Ala Leu Leu Ala Ser Tyr Ser Val His Leu Leu Leu Ser Met Cys Ile
            20                  25                  30

Gln Thr Ala Val Thr Ser Tyr Glu Asp Leu Gly Leu Phe Ala Phe Gly
        35                  40                  45

Leu Pro Gly Lys Leu Val Val Ala Gly Thr Ile Ile Ile Gln Asn Ile
    50                  55                  60

Gly Ala Met Ser Ser Tyr Leu Leu Ile Ile Lys Thr Glu Leu Pro Ala
65                  70                  75                  80

Ala Ile Ala Glu Phe Leu Thr Gly Asp Tyr Ser Arg Tyr Trp Tyr Leu
                85                  90                  95

Asp Gly Gln Thr Leu Leu Ile Ile Ile Cys Val Gly Ile Val Phe Pro
            100                 105                 110

Leu Ala Leu Leu Pro Lys Ile Gly Phe Leu Gly Tyr Thr Ser Ser Leu
        115                 120                 125

Ser Phe Xaa Phe Met Met Phe Phe Ala Leu Val Val Ile Ile Lys Lys
    130                 135                 140

Trp Ser Ile Pro Cys Pro Leu Thr Leu Asn Tyr Val Glu Lys Gly Phe
145                 150                 155                 160

Gln Ile Ser Asn Val Thr Asp Asp Cys Lys Pro Lys Leu Phe His Phe
                165                 170                 175

Ser Lys Glu Ser Ala Tyr Ala Leu Pro Thr Met Ala Phe Ser Phe Leu
            180                 185                 190

Cys His Thr Ser Ile Leu Pro Ile Tyr Cys Glu Leu Gln Ser Pro Ser
        195                 200                 205

Lys Lys Arg Met Gln Asn Val Thr Asn Thr Ala Ile Ala Leu Ser Phe
    210                 215                 220

Leu Ile Tyr Phe Ile Ser Ala Leu Phe Gly Tyr Leu Thr Phe Tyr Gly
225                 230                 235                 240

Ser His Ser Val Ala Gln Val Gly Val Gln Trp Cys Asp Leu Ser Ser
                245                 250                 255

Leu Gln Pro Leu Pro Pro Gly Leu Lys Gln Ser Ser His Leu Ser Leu
            260                 265                 270

Gln Ser Ser Xaa
        275


<210> 141
<211> 195
<212> PRT
<213> Homo sapiens

<220>
<221> SITE
<222> (138)
<223> Xaa equals any of the naturally occurring L-amino acids

<220>
<221> SITE
<222> (195)
<223> Xaa equals stop translation

<400> 141
Met Lys Leu Ala Ser Gly Phe Leu Val Leu Trp Leu Ser Leu Gly Gly
  1               5                  10                  15

Gly Leu Ala Gln Ser Asp Thr Ser Pro Asp Thr Glu Glu Ser Tyr Ser
             20                  25                  30

Asp Trp Gly Leu Arg His Leu Arg Gly Ser Phe Glu Ser Val Asn Ser
         35                  40                  45

Tyr Phe Asp Ser Phe Leu Glu Leu Leu Gly Gly Lys Asn Gly Val Cys
     50                  55                  60

Gln Tyr Arg Cys Arg Tyr Gly Lys Ala Pro Met Pro Arg Pro Gly Tyr
65                  70                  75                  80

Lys Pro Gln Glu Pro Asn Gly Cys Gly Ser Tyr Phe Leu Gly Leu Lys
                85                  90                  95

Val Pro Glu Ser Met Asp Leu Gly Ile Pro Ala Met Thr Lys Cys Cys
            100                 105                 110

Asn Gln Leu Asp Val Cys Tyr Asp Thr Cys Gly Ala Asn Lys Tyr Arg
        115                 120                 125

Cys Asp Ala Lys Phe Arg Trp Cys Leu Xaa Ser Ile Cys Ser Asp Leu
    130                 135                 140

Lys Arg Ser Leu Gly Phe Val Ser Lys Val Glu Ala Cys Asp Ser Leu
145                 150                 155                 160

Val Asp Thr Val Phe Asn Thr Val Trp Thr Leu Gly Cys Arg Pro Phe
                165                 170                 175

Met Asn Ser Gln Arg Ala Ala Cys Ile Cys Ala Glu Glu Glu Lys Glu
            180                 185                 190

Glu Leu Xaa
        195


<210> 142
<211> 183
<212> PRT
<213> Homo sapiens

<220>
<221> SITE
<222> (183)
<223> Xaa equals stop translation

<400> 142
Met Leu Leu Leu Cys His Ala Leu Ala Ile Ala Val Val Gln Ile Val
 1               5                  10                  15

Ile Phe Ser Glu Ser Trp Ala Phe Ala Lys Asn Ile Asn Phe Tyr Asn
            20                  25                  30

Val Arg Pro Pro Leu Asp Pro Thr Pro Phe Pro Asn Ser Phe Lys Cys
        35                  40                  45

Phe Thr Cys Glu Asn Ala Gly Asp Asn Tyr Asn Cys Asn Arg Trp Ala
    50                  55                  60

Glu Asp Lys Trp Cys Pro Gln Asn Thr Gln Tyr Cys Leu Thr Val His
65                  70                  75                  80

His Phe Thr Ser His Gly Arg Ser Thr Ser Ile Thr Lys Lys Cys Ala
                85                  90                  95

Ser Arg Ser Glu Cys His Phe Val Gly Cys His His Ser Arg Asp Ser
            100                 105                 110

Glu His Thr Glu Cys Arg Ser Cys Cys Glu Gly Met Ile Cys Asn Val
        115                 120                 125

Glu Leu Pro Thr Asn His Thr Asn Ala Val Phe Ala Val Met His Ala
    130                 135                 140

Gln Arg Thr Ser Gly Ser Ser Ala Pro Thr Leu Tyr Leu Thr Ser Ala
145                 150                 155                 160

Cys Leu Gly Leu Cys Ala Ser Ile Ala Val Met Pro Pro Phe Leu Gly
                165                 170                 175

Glu Ala Glu Thr Ser Leu Xaa
            180

<210> 143
<211> 122
<212> PRT
<213> Homo sapiens

<220>
<221> SITE
<222> (122)
<223> Xaa equals stop translation

<400> 143
Met Leu Arg Gly Thr Met Thr Ala Trp Arg Gly Met Arg Pro Glu Val
1               5                   10                  15

Thr Leu Ala Cys Leu Leu Leu Ala Thr Ala Gly Cys Phe Ala Asp Leu
            20                  25                  30

Asn Glu Val Pro Gln Val Thr Val Gln Pro Ala Ser Thr Val Gln Lys
        35                  40                  45

Pro Gly Gly Thr Val Ile Leu Gly Cys Val Val Glu Pro Pro Arg Met
    50                  55                  60

Asn Val Thr Trp Arg Leu Asn Gly Lys Glu Leu Asn Gly Ser Asp Asp
65                  70                  75                  80

Ala Leu Gly Val Leu Ile Thr His Gly Thr Leu Val Ile Thr Ala Leu
                85                  90                  95

Asn Asn His Thr Val Gly Arg Tyr Gln Cys Val Ala Arg Met Pro Ala
            100                 105                 110

Gly Ala Val Ala Thr Cys Gln Pro Leu Xaa
        115                 120

<210> 144
<211> 267
<212> PRT

<213> Homo sapiens

<220>
<221> SITE
<222> (267)
<223> Xaa equals stop translation

<400> 144
Met Trp Trp Phe Gln Gln Gly Leu Ser Phe Leu Pro Ser Ala Leu Val
1               5                   10                  15

Ile Trp Thr Ser Ala Ala Phe Ile Phe Ser Tyr Ile Thr Ala Val Thr
            20                  25                  30

Leu His His Ile Asp Pro Ala Leu Pro Tyr Ile Ser Asp Thr Gly Thr
        35                  40                  45

Val Ala Pro Glu Lys Cys Leu Phe Gly Ala Met Leu Asn Ile Ala Ala
    50                  55                  60

Val Leu Cys Ile Ala Thr Ile Tyr Val Arg Tyr Lys Gln Val His Ala
65                  70                  75                  80

Leu Ser Pro Glu Glu Asn Val Ile Ile Lys Leu Asn Lys Ala Gly Leu
                85                  90                  95

Val Leu Gly Ile Leu Ser Cys Leu Gly Leu Ser Ile Val Ala Asn Phe
            100                 105                 110

Gln Lys Thr Thr Leu Phe Ala Ala His Val Ser Gly Ala Val Leu Thr
        115                 120                 125

Phe Gly Met Gly Ser Leu Tyr Met Phe Val Gln Thr Ile Leu Ser Tyr
    130                 135                 140

Gln Met Gln Pro Lys Ile His Gly Lys Gln Val Phe Trp Ile Arg Leu
145                 150                 155                 160

Leu Leu Val Ile Trp Cys Gly Val Ser Ala Leu Ser Met Leu Thr Cys
                165                 170                 175

Ser Ser Val Leu His Ser Gly Asn Phe Gly Thr Asp Leu Glu Gln Lys
            180                 185                 190

Leu His Trp Asn Pro Glu Asp Lys Gly Tyr Val Leu His Met Ile Thr
        195                 200                 205

Thr Ala Ala Glu Trp Ser Met Ser Phe Ser Phe Phe Gly Phe Phe Leu
    210                 215                 220

Thr Tyr Ile Arg Asp Phe Gln Lys Ile Ser Leu Arg Val Glu Ala Asn
225                 230                 235                 240

Leu His Gly Leu Thr Leu Tyr Asp Thr Ala Pro Cys Pro Ile Asn Asn
                245                 250                 255

Glu Arg Thr Arg Leu Leu Ser Arg Asp Ile Xaa
            260                 265

<210> 145
<211> 92
<212> PRT
<213> Homo sapiens

<220>
<221> SITE
<222> (92)
<223> Xaa equals stop translation

<400> 145
Met Leu Cys His Pro His Val His His His Leu Val Cys Leu Leu Ala
1               5                   10                  15

Thr Leu Thr Phe Ser Leu Asn Ala Ser Cys Ala Glu Gln Thr Phe His
            20                  25                  30

Ser Gln Gln Ser Asn Gly Glu Phe Met Ala Thr Leu Pro Ser Ile Ser
        35                  40                  45

Lys Gln Phe Gly Val Ile Val Trp Lys Pro Gln Arg Lys Asp Val Ile
    50                  55                  60

Arg Leu Pro Val Ala Leu Ser Phe Ser Met Gly Leu Gly Leu Leu Ser
65                  70                  75                  80

Pro Ala Leu Gly Arg Phe Leu Ala Ser Glu Leu Xaa
                85                  90


<210> 146
<211> 109
<212> PRT
<213> Homo sapiens

<220>
<221> SITE
<222> (109)
<223> Xaa equals stop translation

<400> 146
Met Ala Ile Leu Leu Ala Cys Phe Thr Ala Val Leu Ala Phe Ile Cys
1               5                   10                  15

Leu Gln Phe Trp Cys Val Arg Cys His Glu Pro Arg Trp Ser Tyr Arg
            20                  25                  30

Ala Gly His Met Glu Glu Ala Asn Gly Leu Val Arg Trp Pro Glu Glu
        35                  40                  45

Ala Pro Asp Leu Gly Gln Arg Glu Glu Asp Leu Gln Gly Leu Pro Leu
    50                  55                  60

Val Glu Met Pro Arg Lys Asn Ser Arg Asp Gly Ala Glu Leu Asp Pro
65                  70                  75                  80

Glu Ala Asn Gln Asp Ala Pro Asp Ala Gly Ala Leu Gln Arg Gly Gly
                85                  90                  95

Gly Asp Pro Pro Ala Ile Leu Pro His Cys Gly Glu Xaa
            100                 105


<210> 147
<211> 88
<212> PRT
<213> Homo sapiens

<220>
<221> SITE
<222> (88)
<223> Xaa equals stop translation

<400> 147
Met Leu Leu Arg Val Phe His Phe Phe Leu His Ile Leu His Lys Lys
  1               5                  10                  15

Gln Thr Gly Val Ser Leu Leu Tyr Leu Leu Leu Thr Leu Phe Leu Leu
            20                  25                  30

Gln Gln Gln Val Ile Pro Gln Pro Ser Leu Pro Leu Leu His Leu Val
        35                  40                  45

Ser Phe Gln Ile Cys His Tyr Pro Phe Pro Gln Trp Met Leu Gln Tyr
    50                  55                  60

Arg Gln Ala Lys Met Val Leu Gly Thr Arg Cys Gln Met Ser Leu Met
 65                  70                  75                  80

His Phe Gln Asn Ser Gln Asn Xaa
                85


<210> 148
<211> 74
<212> PRT
<213> Homo sapiens

<220>
<221> SITE
<222> (74)
<223> Xaa equals stop translation

<400> 148
Met Ser Arg Val Val Ser Leu Phe Phe Phe Ile Leu Phe Ser Phe Phe
  1               5                  10                  15

Phe Phe Ala Phe Ser Leu Ser Ser Ser Leu Ser Phe Val His Tyr Glu
            20                  25                  30

Lys Leu Val Gln Val Lys Glu Cys Leu Asp Ser Phe Leu Lys Lys Ile
        35                  40                  45

Lys Ile Lys Glu Tyr Lys Thr Arg Gln Cys Tyr His Leu Ile Arg Trp

50                  55                  60

Glu Asn Asn Gly Ala Lys Leu Gln Ser Xaa
65                  70

<210> 149
<211> 72
<212> PRT
<213> Homo sapiens

<220>
<221> SITE
<222> (72)
<223> Xaa equals stop translation

<400> 149
Met Ser Ala Ser Leu Lys Asn His Leu Thr His Cys Phe Leu Leu Leu
1               5                   10                  15

Leu Leu Lys Glu Leu Val Ser Pro Thr Met Ile Ser Phe Val Pro Thr
            20                  25                  30

Leu Arg His Ser Tyr Arg Phe Phe Asn Leu Phe Ser Cys Asp Ala Glu
        35                  40                  45

Ser Thr Lys Glu Ser Pro Gly Arg Thr Val Gln Phe Ser Lys Thr Pro
    50                  55                  60

Arg Gly Val Thr Met Phe Ile Xaa
65                  70

<210> 150
<211> 152
<212> PRT
<213> Homo sapiens

<220>
<221> SITE
<222> (152)
<223> Xaa equals stop translation

<400> 150
Met Lys Tyr Gly Leu Thr Gly Pro Trp Ile Lys Arg Leu Leu Pro Val
1               5                   10                  15

Ile Phe Leu Val Gln Ala Ser Gly Met Asn Val Tyr Met Ser Arg Ser
            20                  25                  30

Leu Glu Ser Ile Lys Gly Ile Ala Val Met Asp Lys Glu Gly Asn Val
        35                  40                  45

Leu Gly His Ser Arg Ile Ala Gly Thr Lys Ala Val Arg Glu Thr Leu
    50                  55                  60

Ala Ser Arg Ile Val Leu Phe Gly Thr Ser Ala Leu Ile Pro Glu Val
65                  70                  75                  80

Phe Thr Tyr Phe Phe Lys Arg Thr Gln Tyr Phe Arg Lys Asn Pro Gly
                85                  90                  95

Ser Leu Trp Ile Leu Lys Leu Ser Cys Thr Val Leu Ala Met Gly Leu
            100                 105                 110

Met Val Pro Phe Ser Phe Ser Ile Phe Pro Gln Ile Gly Gln Ile Gln
        115                 120                 125

Tyr Cys Ser Leu Glu Glu Lys Ile Gln Ser Pro Thr Glu Glu Thr Glu
    130                 135                 140

Ile Phe Tyr His Arg Gly Val Xaa
145                 150

<210> 151
<211> 61
<212> PRT
<213> Homo sapiens

<220>
<221> SITE
<222> (61)
<223> Xaa equals stop translation

<400> 151
Met Leu Arg Val Ala Gly Val Leu Gln Phe Leu Pro Leu Ser Tyr Gly
  1               5                  10                  15

Thr Lys Val Ala Ser Leu Trp Asn Thr Tyr Glu Asn Val Val Met Pro
             20                  25                  30

Pro Ser Phe Thr Thr Thr Leu Val Leu Pro Leu Leu Ser His Glu Phe
         35                  40                  45

Tyr Asn Tyr Ser Tyr Pro Phe Ala Cys Asp Gln Lys Xaa
     50                  55                  60

<210> 152
<211> 123
<212> PRT
<213> Homo sapiens

<220>
<221> SITE
<222> (89)
<223> Xaa equals any of the naturally occurring L-amino acids

<220>
<221> SITE
<222> (91)
<223> Xaa equals any of the naturally occurring L-amino acids

<220>
<221> SITE

<222> (94)
<223> Xaa equals any of the naturally occurring L-amino acids

<220>
<221> SITE
<222> (97)
<223> Xaa equals any of the naturally occurring L-amino acids

<220>
<221> SITE
<222> (98)
<223> Xaa equals any of the naturally occurring L-amino acids

<220>
<221> SITE
<222> (123)
<223> Xaa equals stop translation

<400> 152
Met His Arg Ser Glu Pro Phe Leu Lys Met Ser Leu Leu Ile Leu Leu
1               5                   10                  15

Phe Leu Gly Leu Ala Glu Ala Cys Thr Pro Arg Glu Val Asn Leu Leu
            20                  25                  30

Lys Gly Ile Ile Gly Leu Met Ser Arg Leu Ser Pro Asp Glu Ile Leu
        35                  40                  45

Gly Leu Leu Ser Leu Gln Val Leu His Glu Glu Thr Ser Gly Cys Lys
    50                  55                  60

Glu Glu Val Lys Pro Phe Ser Gly Thr Thr Pro Ser Arg Lys Pro Leu
65                  70                  75                  80

Pro Lys Arg Glu Glu His Val Glu Xaa Pro Xaa Asn Ala Xaa Thr Trp
                85                  90                  95

Xaa Xaa Thr Tyr Leu Phe Val Ser Tyr Asn Lys Gly Asp Trp Phe Thr
            100                 105                 110

Phe Ser Ser Gln Val Leu Leu Pro Leu Leu Xaa
        115                 120


<210> 153
<211> 55
<212> PRT
<213> Homo sapiens

<220>
<221> SITE
<222> (55)
<223> Xaa equals stop translation

<400> 153
Met Ser Pro Cys Ala His Ile Cys Leu Tyr Val Leu Val Phe Leu Cys
1               5                   10                  15

Asn Val Thr Arg Cys Lys Cys Val Arg Ala Phe Thr Thr Trp Asp Thr
            20                  25                  30

Glu Lys Val Lys Tyr Phe Met Ala His Trp Ser Lys Leu Lys Arg Val
        35                  40                  45

Arg Gly Thr Arg Val Glu Xaa
    50                  55

<210> 154
<211> 111
<212> PRT
<213> Homo sapiens

<220>
<221> SITE
<222> (93)
<223> Xaa equals any of the naturally occurring L-amino acids

<220>
<221> SITE
<222> (111)
<223> Xaa equals stop translation

<400> 154
Met Phe Leu Ala Ser Trp Leu Leu Phe Cys Ile Val Ala Pro Lys Asp
1               5                   10                  15

Asp Ala His Leu Ser Phe Ile Gln Cys Lys Asp Ile Trp Lys Asp Asn
            20                  25                  30

Arg Lys Tyr Ser Cys Phe His Phe Lys Ser Asp Gln Leu Leu Glu Leu
        35                  40                  45

Ala Ser Lys Ala Cys Thr Ser Phe Gln Ala Gln Ser Arg Ser Phe Thr
    50                  55                  60

Ala Gly Ala Val Pro Ser Glu His Pro Glu Leu Pro Cys Gly Ser Gln
65                  70                  75                  80

Gln Leu Cys Cys Gly Cys Thr Ala Arg Leu Gly Gly Xaa Trp Ile Gly
                85                  90                  95

Ala Ser Arg Cys Gly Ser Gly Ser Ala Phe Leu Ala Ser Pro Xaa
            100                 105                 110

<210> 155
<211> 48
<212> PRT
<213> Homo sapiens

<220>
<221> SITE
<222> (48)
<223> Xaa equals stop translation

<400> 155
Met Ser Leu Gln Ala Ile Asp Leu Leu Trp Ser Leu Cys Thr Gln Thr
1               5                   10                  15

Ser Leu Leu Thr Leu Ile Cys Ile Cys Ser His Ser Gln Ala Leu Ser
            20                  25                  30

Ser Ser Pro Gln Leu His Leu Arg Ser Ser Ser Ile Arg Phe Ser Xaa
        35                  40                  45

<210> 156
<211> 82
<212> PRT
<213> Homo sapiens

<220>
<221> SITE
<222> (82)
<223> Xaa equals stop translation

<400> 156
Met Phe His Phe Gly Leu Trp Asp Leu His Phe Phe Leu Ile Val Met
1               5                   10                  15

Ala His Arg Asp Asp Cys Ser Phe Lys Gly Gly Cys Gly Leu Leu Glu
            20                  25                  30

Arg Phe Gln Cys Pro His Thr Ser Phe Ser Ser Ala Ser Gln Lys Arg
        35                  40                  45

Leu Ala Asp Gly Met Glu Cys Leu Cys Glu Ile Glu Arg Thr Gln Thr
    50                  55                  60

Arg Ile Arg Lys Ile Cys Leu Pro Thr Leu His Gly His Leu Leu Ala
65                  70                  75                  80

Val Xaa

<210> 157
<211> 156
<212> PRT
<213> Homo sapiens

<220>
<221> SITE
<222> (108)
<223> Xaa equals any of the naturally occurring L-amino acids

<220>
<221> SITE
<222> (113)
<223> Xaa equals any of the naturally occurring L-amino acids

<220>
<221> SITE
<222> (156)
<223> Xaa equals stop translation

<400> 157
Met Met Ala Arg Gln Thr Gly Val Phe Tyr Leu Thr Leu Val Leu Ile
1               5                   10                  15

Leu Val Thr Ser Gly Leu Phe Phe Ala Phe Asp Cys Pro Tyr Leu Ala
            20                  25                  30

Val Lys Ile Thr Pro Ala Ile Pro Ala Val Ala Gly Ile Leu Phe Phe
        35                  40                  45

Phe Val Met Gly Thr Leu Leu Arg Thr Ser Phe Ser Asp Pro Gly Val
    50                  55                  60

Leu Pro Arg Ala Thr Pro Asp Glu Ala Ala Asp Leu Glu Arg Gln Ile
65                  70                  75                  80

Gly Asn Thr Glu Ser Leu Pro Met Ala Ser Gly His Phe Pro Pro Gly
                85                  90                  95

Pro Ser Tyr Ser Gly Glu Gly Arg Pro Arg Ala Xaa Gln Glu Glu Leu
            100                 105                 110

Xaa Ala Gly Lys Glu Gly Gly Gln Lys Ser Ala Phe Leu Ser Ser Leu
        115                 120                 125

Gly Gly Gln Asp Glu Leu Lys Lys Arg Cys Asp Ile Arg Leu Glu Gly
    130                 135                 140

Gln Val Ser Trp Arg Gln Asp Cys Arg Pro Thr Xaa
145                 150                 155


<210> 158
<211> 295
<212> PRT
<213> Homo sapiens

<220>
<221> SITE
<222> (295)
<223> Xaa equals stop translation

<400> 158
Met Arg Leu Asp Lys Pro Ile Gly Thr Trp Leu Leu Tyr Leu Pro Cys
1               5                   10                  15

Thr Trp Ser Ile Gly Leu Ala Ala Glu Pro Gly Cys Phe Pro Asp Trp
            20                  25                  30

Tyr Met Leu Ser Leu Phe Gly Thr Gly Ala Ile Leu Met Arg Gly Ala
        35                  40                  45

Gly Cys Thr Ile Asn Asp Met Trp Asp Gln Asp Tyr Asp Lys Lys Val
    50                  55                  60

Thr Arg Thr Ala Asn Arg Pro Ile Ala Ala Gly Asp Ile Ser Thr Phe
65                  70                  75                  80

Gln Ser Phe Val Phe Leu Gly Gly Gln Leu Thr Leu Ala Leu Gly Val
                85                  90                  95

Leu Leu Cys Leu Asn Tyr Tyr Ser Ile Ala Leu Gly Ala Gly Ser Leu
            100                 105                 110

Leu Leu Val Ile Thr Tyr Pro Leu Met Lys Arg Ile Ser Tyr Trp Pro
        115                 120                 125

Gln Leu Ala Leu Gly Leu Thr Phe Asn Trp Gly Ala Leu Leu Gly Trp
    130                 135                 140

Ser Ala Ile Lys Gly Ser Cys Asp Pro Ser Val Cys Leu Pro Leu Tyr
145                 150                 155                 160

Phe Ser Gly Val Met Trp Thr Leu Ile Tyr Asp Thr Ile Tyr Ala His
                165                 170                 175

Gln Asp Lys Arg Asp Asp Val Leu Ile Gly Leu Lys Ser Thr Ala Leu
            180                 185                 190

Arg Phe Gly Glu Asn Thr Lys Pro Trp Leu Ser Gly Phe Ser Val Ala
        195                 200                 205

Met Leu Gly Ala Leu Ser Leu Val Gly Val Asn Ser Gly Gln Thr Ala
    210                 215                 220

Pro Tyr Tyr Ala Ala Leu Gly Ala Val Gly Ala His Leu Thr His Gln
225                 230                 235                 240

Ile Tyr Thr Leu Asp Ile His Arg Pro Glu Asp Cys Trp Asn Lys Phe
                245                 250                 255

Ile Ser Asn Arg Thr Leu Gly Leu Ile Val Phe Leu Gly Ile Val Leu
            260                 265                 270

Gly Asn Leu Trp Lys Glu Lys Lys Thr Asp Lys Thr Lys Lys Gly Ile
        275                 280                 285

Glu Asn Lys Ile Glu Asn Xaa
    290                 295

<210> 159
<211> 60
<212> PRT
<213> Homo sapiens

<220>
<221> SITE
<222> (60)
<223> Xaa equals stop translation

<400> 159
Met Gly Pro Phe Leu Leu Val Phe Leu Phe Pro Ile Leu Arg Val Cys
1               5                   10                  15

Gly Ile Ile Arg Glu Pro Thr Gln Asp Trp Ser Val Leu Leu Glu Arg
            20                  25                  30

Ala Arg Leu Thr Ala Pro Gly Gln Pro Pro Ala Leu Phe Pro Leu Glu
        35                  40                  45

Ser Gly Pro Met Ala Thr Ala Gln Asn Thr Ser Xaa
    50                  55                  60


<210> 160
<211> 122
<212> PRT
<213> Homo sapiens

<220>
<221> SITE
<222> (30)
<223> Xaa equals any of the naturally occurring L-amino acids

<220>
<221> SITE
<222> (32)
<223> Xaa equals any of the naturally occurring L-amino acids

<220>
<221> SITE
<222> (87)
<223> Xaa equals any of the naturally occurring L-amino acids

<220>
<221> SITE
<222> (101)
<223> Xaa equals any of the naturally occurring L-amino acids

<220>
<221> SITE
<222> (115)
<223> Xaa equals any of the naturally occurring L-amino acids

<220>
<221> SITE
<222> (122)
<223> Xaa equals stop translation

<400> 160
Met Cys Ser His Ser Thr Leu Ile His Leu Tyr Leu Val Leu Pro Phe
1               5                   10                  15

Phe Phe Leu Phe Leu Pro Ser Ser Phe Pro Phe Pro Ser Xaa Ser Xaa
            20                  25                  30

Ser Ser Ile Leu Pro Ser Leu Arg Leu Pro Pro Phe Phe Pro Pro Ser

35                  40                  45

Leu Phe Leu His Ser Ser Leu Pro Pro Ser Leu Ser His Pro Leu Gly
    50                  55                  60

Leu Ser Ile Thr Ser Ser Arg Gln Ser Phe Leu Asp Tyr His His Leu
65                  70                  75                  80

Cys Thr Lys His Leu Ser Xaa Thr Leu Cys Gly Leu Ile Tyr His Cys
                85                  90                  95

Leu Asn Ile Phe Xaa Thr Arg Ala Val Met Trp His Met Gln Val Ser
            100                 105                 110

Phe Leu Xaa Ile His Trp Leu Leu Pro Xaa
        115                 120


<210> 161
<211> 73
<212> PRT
<213> Homo sapiens

<220>
<221> SITE
<222> (73)
<223> Xaa equals stop translation

<400> 161
Met Ser Ile Tyr His Val Cys Leu Ile Leu Leu Leu Tyr Ile Thr Ser
1               5                   10                  15

His Ser His Gln Asn Met Ser Ser Cys Leu Gln Val Pro Leu Ser Leu
            20                  25                  30

Leu Ser Cys Pro Leu Lys Gly Glu His Leu Ser Gln Phe Ala Gly Asp
        35                  40                  45

His Ser Leu Pro Glu Val Arg Asp Arg Asn His His Cys Ile Leu Phe
    50                  55                  60

Lys Glu Ser His Gln Lys Arg Lys Xaa
65                  70


<210> 162
<211> 123
<212> PRT
<213> Homo sapiens

<220>
<221> SITE
<222> (123)
<223> Xaa equals stop translation

<400> 162
Met Leu Ala Asn Phe Thr Leu Phe Ile Leu Thr Leu Ile Ser Phe Leu
1               5                   10                  15

Leu Leu Val Cys Ser Pro Cys Lys His Leu Lys Met Met Gln Leu His
            20                  25                  30

Gly Lys Gly Ser Gln Asp Leu Ser Thr Lys Val His Ile Lys Pro Leu
        35                  40                  45

Gln Thr Val Ile Ser Phe Leu Met Leu Phe Ala Ile Tyr Phe Leu Cys
    50                  55                  60

Ile Ile Thr Ser Thr Trp Asn Pro Arg Thr Gln Gln Ser Asn Leu Val
65                  70                  75                  80

Phe Leu Leu Tyr Gln Thr Leu Ala Ile Met Tyr Pro Ser Phe His Ser
                85                  90                  95

Phe Ile Leu Ile Met Arg Ser Arg Lys Leu Lys Gln Thr Ser Leu Ser
            100                 105                 110

Val Leu Cys Gln Val Thr Cys Trp Val Lys Xaa
        115                 120


<210> 163
<211> 143
<212> PRT
<213> Homo sapiens

<220>
<221> SITE
<222> (143)
<223> Xaa equals stop translation

<400> 163
Met Pro Gly Pro Cys Leu Ser Gln Gln His Pro Phe Leu Ser Leu Ser
 1               5                  10                  15

Leu Phe Pro Phe Cys Leu Trp Ile Cys Leu Ala Arg Val Pro Gly Val
            20                  25                  30

Arg Asn Ile Cys Lys Thr Gln Pro Ala Pro Ser Gln Pro Ser Leu Leu
        35                  40                  45

Gly Leu Gly Leu Ser His Pro Ala Ala Gly Thr Thr Asp Ala Gly Thr
    50                  55                  60

Gln Ser Leu Pro Arg Ser Gln His Lys Cys Thr Ser Ala Leu Trp Gly
65                  70                  75                  80

Leu Cys Pro Ala Gln Arg Pro Leu Leu Leu Pro Ala His Ile His Ser
                85                  90                  95

Ser Gly His Gly Ala Pro Gln Glu Leu Gln Ser His Leu Ser His Arg
            100                 105                 110

Leu Pro Ala Ser Ala Ser Leu Ser Met Met Ser Pro Phe Ser Glu Ala
        115                 120                 125

Trp Thr His Pro Ser Leu Ser Leu Gly Pro Ala Pro Ser His Xaa
    130                 135                 140

<210> 164
<211> 117
<212> PRT
<213> Homo sapiens

<220>
<221> SITE
<222> (46)
<223> Xaa equals any of the naturally occurring L-amino acids

<220>
<221> SITE
<222> (117)
<223> Xaa equals stop translation

<400> 164
Met Pro Gly Gly Thr Arg Cys Arg Val Leu Leu Leu Ser Leu Thr Phe
  1               5                  10                  15

Gly Thr Ser Met Ala Cys Gly Asn Val Gly Leu Arg Leu Cys Pro Trp
             20                  25                  30

Thr Trp His Asn Trp Leu Leu Pro Pro His Leu Cys Ser Xaa Trp Pro
         35                  40                  45

Cys Arg Arg Cys Cys Trp Ala Ala Ala Thr Thr His Phe Ser Trp Pro
     50                  55                  60

Pro Trp Val Arg Ser Ala Trp Gly Pro Pro Ala Ala Trp Leu Glu Ser
 65                  70                  75                  80

Ser Gly His Pro Leu Pro Ala Val Ala Ser Cys Ser Gln Pro Pro Ala
                 85                  90                  95

Ser Ala Asp Ser Ser Arg Phe Ser Lys Val Pro Cys Cys Arg Arg Arg
            100                 105                 110

Gly Trp Thr Arg Xaa
        115

<210> 165
<211> 59
<212> PRT
<213> Homo sapiens

<220>
<221> SITE
<222> (59)
<223> Xaa equals stop translation

<400> 165
Met Ser Val Cys Leu Pro Leu His Leu Pro Phe Leu Met Leu Ala Lys
  1               5                  10                  15

Val Ala Thr Ser Phe Cys Arg Trp Gln Leu Thr Leu Phe Val Ser Thr
            20                  25                  30

Phe Tyr Lys Asp Ala Leu Val His Thr Val Asn Asp Arg Asn Gln Glu
        35                  40                  45

Ala Glu Leu Glu Ala Leu Lys Lys Ser Cys Xaa
    50                  55


<210> 166
<211> 126
<212> PRT
<213> Homo sapiens

<220>
<221> SITE
<222> (126)
<223> Xaa equals stop translation

<400> 166
Met Lys Ala Leu Met Leu Leu Thr Leu Ser Val Leu Leu Cys Trp Val
  1               5                  10                  15

Ser Ala Asp Ile Arg Cys His Ser Cys Tyr Lys Val Pro Val Leu Gly
            20                  25                  30

Cys Val Asp Arg Gln Ser Cys Arg Leu Glu Pro Gly Gln Gln Cys Leu
        35                  40                  45

Thr Thr His Ala Tyr Leu Gly Lys Met Trp Val Phe Ser Asn Leu Arg
    50                  55                  60

Cys Gly Thr Pro Glu Glu Pro Cys Gln Glu Ala Phe Asn Gln Thr Asn
 65                  70                  75                  80

Arg Lys Leu Gly Leu Thr Tyr Asn Thr Thr Cys Cys Asn Lys Asp Asn
                 85                  90                  95

Cys Asn Ser Ala Gly Pro Arg Pro Thr Pro Ala Leu Gly Leu Val Phe
            100                 105                 110

Leu Thr Ser Leu Ala Gly Leu Gly Leu Trp Leu Leu His Xaa
        115                 120                 125


<210> 167
<211> 87
<212> PRT
<213> Homo sapiens

<220>
<221> SITE
<222> (87)
<223> Xaa equals stop translation

<400> 167

Met Phe Leu Val Ala Val Trp Trp Arg Phe Gly Ile Leu Ser Ile Cys
1               5                   10                  15

Met Leu Cys Val Gly Leu Val Leu Gly Phe Leu Ile Ser Ser Val Thr
            20                  25                  30

Phe Phe Thr Pro Leu Gly Asn Leu Lys Ile Phe His Asp Asp Gly Val
        35                  40                  45

Phe Trp Val Thr Phe Ser Cys Ile Ala Ile Leu Ile Pro Val Val Phe
    50                  55                  60

Met Gly Cys Leu Arg Ile Leu Asn Ile Leu Thr Cys Gly Ser His Trp
65                  70                  75                  80

Ala Pro Ile Arg Trp Phe Xaa
                85

<210> 168
<211> 63
<212> PRT
<213> Homo sapiens

<220>
<221> SITE
<222> (16)
<223> Xaa equals any of the naturally occurring L-amino acids

<220>
<221> SITE
<222> (54)
<223> Xaa equals any of the naturally occurring L-amino acids

<220>
<221> SITE
<222> (63)
<223> Xaa equals stop translation

<400> 168
Met Val Thr Gly Phe Phe Phe Ile Leu Met Thr Val Leu Trp Phe Xaa
1               5                   10                  15

Arg Glu Pro Gly Phe Val Pro Gly Trp Asp Ser Phe Phe Glu Lys Lys
            20                  25                  30

Gly Tyr Arg Thr Asp Ala Thr Val Ser Val Phe Leu Gly Phe Leu Leu
        35                  40                  45

Phe Leu Ile Pro Ala Xaa Glu Ala Leu Leu Trp Glu Lys Glu Xaa
    50                  55                  60

<210> 169
<211> 48
<212> PRT
<213> Homo sapiens

<220>
<221> SITE
<222> (48)
<223> Xaa equals stop translation

<400> 169
Met Ser Gln Leu Cys Phe Ser Leu Leu Leu Ser Ser Thr Cys His Gly
1               5                   10                  15

Gly Val Ala Ser Leu Leu Thr Ser Asp Leu Ser Ser Gln Ser His Arg
            20                  25                  30

Phe Ser Ile Cys Thr Asn Val Asn His Ser Lys Tyr Ser Ser Leu Xaa
        35                  40                  45

<210> 170
<211> 137
<212> PRT
<213> Homo sapiens

<220>
<221> SITE
<222> (84)
<223> Xaa equals any of the naturally occurring L-amino acids

<220>
<221> SITE
<222> (137)
<223> Xaa equals stop translation

<400> 170
Met Leu Phe Ser Leu Arg Glu Leu Val Gln Trp Leu Gly Phe Ala Thr
1               5                   10                  15

Phe Glu Ile Phe Val His Leu Leu Ala Leu Leu Val Phe Ser Val Leu
            20                  25                  30

Leu Ala Leu Arg Val Asp Gly Leu Val Pro Gly Leu Ser Trp Trp Asn
        35                  40                  45

Val Phe Val Pro Phe Phe Ala Ala Asp Gly Leu Ser Thr Tyr Phe Thr
    50                  55                  60

Thr Ile Val Ser Val Arg Leu Phe Gln Asp Gly Glu Lys Arg Leu Ala
65                  70                  75                  80

Val Leu Arg Xaa Phe Trp Val Leu Thr Val Leu Ser Leu Lys Phe Val
                85                  90                  95

Phe Glu Met Leu Leu Cys Gln Lys Leu Ala Glu Gln Thr Arg Glu Leu
            100                 105                 110

Trp Phe Gly Leu Ile Thr Ser Pro Leu Phe Ile Leu Leu Gln Leu Leu
        115                 120                 125

Met Ile Arg Ala Cys Arg Val Asn Xaa
    130                 135

<210> 171
<211> 89
<212> PRT
<213> Homo sapiens

<220>
<221> SITE
<222> (40)
<223> Xaa equals any of the naturally occurring L-amino acids

<220>
<221> SITE
<222> (89)
<223> Xaa equals stop translation

<400> 171
Met Glu Leu Ser Phe Val Arg Arg Leu Leu Leu Phe Thr Phe Phe Phe
  1               5                  10                  15

Ser Thr Phe Ser Pro Pro Pro Pro Thr Pro Cys Leu Glu Gly Leu Met
             20                  25                  30

Ser Cys Leu Pro Ser Pro Leu Xaa Lys Asn Thr Ala Gly Ser Gln Thr
         35                  40                  45

Lys Ser Leu Arg Glu Ile Gly Thr Gly Ile Ser Asp Thr His Val Ser
     50                  55                  60

Pro Ser Pro Ala Gln Ala Pro Leu Cys Ser Arg Ser Pro Thr Trp Asp
 65                  70                  75                  80

Ser Ser Asp Pro Asn Ser Met Asp Xaa
                 85

<210> 172
<211> 58
<212> PRT
<213> Homo sapiens

<220>
<221> SITE
<222> (58)
<223> Xaa equals stop translation

<400> 172
Met Thr Met Val Met Glu Gln Val Tyr Leu Met Ser Phe Leu Leu Leu
  1               5                  10                  15

Leu Leu Arg Thr Met Met Lys Ala His Trp Thr Tyr Thr Leu Gly Trp
             20                  25                  30

Thr Val Leu Phe Leu Thr Ala Leu Pro Asn Pro Val Tyr His Gln Glu

35                  40                  45

Ile Val Trp Thr Tyr Met Lys Arg Ser Xaa
     50                  55

<210> 173
<211> 64
<212> PRT
<213> Homo sapiens

<220>
<221> SITE
<222> (64)
<223> Xaa equals stop translation

<400> 173
Met Asp Thr Asp Asn Gly Gly Arg His Phe Lys Pro Phe Lys Leu Val
  1               5                  10                  15

Leu Phe Val Val Leu Leu Ile Lys Ile Leu Leu Ile Leu Ala Lys Thr
             20                  25                  30

Asn Cys Cys Asp Lys Leu Val Phe Phe Gly Cys Phe Lys His Thr Leu
         35                  40                  45

Thr Asn Phe Leu Ile Pro Leu Leu Val Pro Pro Ile Val Leu Lys Xaa
     50                  55                  60

<210> 174
<211> 61
<212> PRT
<213> Homo sapiens

<220>
<221> SITE
<222> (61)
<223> Xaa equals stop translation

<400> 174
Met Cys Leu Trp Gly Gln Ala Asn Leu Gly Leu Ile Leu Phe Gln His
  1               5                  10                  15

Cys Leu Thr Lys Phe Met Gly Gly Tyr Cys Phe Gly Leu Gly Ser Cys
             20                  25                  30

Thr Arg Pro Leu Arg Asp Gln Thr Lys Met Glu Ser Leu Ile Leu Lys
         35                  40                  45

Leu Gln Val Thr Glu Pro Lys Leu Ser Cys Phe Ile Xaa
     50                  55                  60

<210> 175

<211> 104
<212> PRT
<213> Homo sapiens

<220>
<221> SITE
<222> (104)
<223> Xaa equals stop translation

<400> 175
Met Gly Met Ala Gly Ala Leu Ser Ile Leu Leu Phe Ser Leu Pro Ser
1               5                   10                  15

His Gly Trp Pro Ser Pro Pro Lys Pro Pro Phe Pro Cys Cys Gln Pro
            20                  25                  30

Leu Cys His Ser Leu Ile Leu Gly Arg Arg Lys Gly Arg Phe Glu Gly
        35                  40                  45

Glu Gly Glu Lys Ala Tyr Gly Trp Val Trp Phe Leu Pro Phe Pro Glu
    50                  55                  60

Gly Leu Thr Val Pro Gly Trp Pro Gln Gly Arg Gln Gly Pro His Tyr
65                  70                  75                  80

Ala Cys Ala Leu Val Lys Val Thr Pro Ala Ile Tyr Gln Gln Pro Trp
                85                  90                  95

His Val Pro Ala Pro Gln Glu Xaa
            100


<210> 176
<211> 293
<212> PRT
<213> Homo sapiens

<220>
<221> SITE
<222> (293)
<223> Xaa equals stop translation

<400> 176
Met Leu Arg Val Leu Cys Leu Leu Arg Pro Trp Arg Pro Leu Arg Ala
1               5                   10                  15

Arg Gly Cys Ala Ser Asp Gly Ala Ala Gly Gly Ser Glu Ile Gln Val
            20                  25                  30

Arg Ala Leu Ala Gly Pro Asp Gln Gly Ile Thr Glu Ile Leu Met Asn
        35                  40                  45

Arg Pro Ser Ala Arg Asn Ala Leu Gly Asn Val Phe Val Ser Glu Leu
    50                  55                  60

Leu Glu Thr Leu Ala Gln Leu Arg Glu Asp Arg Gln Val Arg Val Leu
65                  70                  75                  80

Leu Phe Arg Ser Gly Val Lys Gly Val Phe Cys Ala Gly Ala Asp Leu
                85                  90                  95

Lys Glu Arg Glu Gln Met Ser Glu Ala Glu Val Gly Val Phe Val Gln
            100                 105                 110

Arg Leu Arg Gly Leu Met Asn Asp Ile Ala Ala Phe Pro Ala Pro Thr
        115                 120                 125

Ile Ala Ala Met Asp Gly Phe Ala Leu Gly Gly Gly Leu Glu Leu Ala
    130                 135                 140

Leu Ala Cys Asp Leu Arg Val Ala Ala Ser Ser Ala Val Met Gly Leu
145                 150                 155                 160

Ile Glu Thr Thr Arg Gly Leu Leu Pro Gly Ala Gly Gly Thr Gln Arg
                165                 170                 175

Leu Pro Arg Cys Leu Gly Val Ala Leu Ala Lys Glu Leu Ile Phe Thr
            180                 185                 190

Gly Arg Arg Leu Ser Gly Thr Glu Ala His Val Leu Gly Leu Val Asn
        195                 200                 205

His Ala Val Ala Gln Asn Glu Glu Gly Asp Ala Ala Tyr Gln Arg Ala
    210                 215                 220

Arg Ala Leu Ala Gln Glu Ile Leu Pro Gln Ala Pro Ile Ala Val Arg
225                 230                 235                 240

Leu Gly Lys Val Ala Ile Asp Arg Gly Thr Glu Val Asp Ile Ala Ser
                245                 250                 255

Gly Met Ala Ile Glu Gly Met Cys Tyr Ala Gln Asn Ile Pro Thr Arg
            260                 265                 270

Asp Arg Leu Glu Gly Met Ala Ala Phe Arg Glu Lys Arg Thr Pro Lys
        275                 280                 285

Phe Val Gly Lys Xaa
    290

<210> 177
<211> 46
<212> PRT
<213> Homo sapiens

<220>
<221> SITE
<222> (46)
<223> Xaa equals stop translation

<400> 177
Met Leu Ser Ser Leu Tyr Leu Leu Leu Met Pro Pro Tyr Lys Phe Thr
  1               5                  10                  15

Gly Glu Leu His Pro Pro Val Ala Ala Thr Cys Leu Leu Thr Val Leu

20                  25                  30

Leu Gly Cys Leu Ile Gly Val Ser Ser Asp Gly Trp Ile Xaa
        35                  40                  45

<210> 178
<211> 47
<212> PRT
<213> Homo sapiens

<220>
<221> SITE
<222> (47)
<223> Xaa equals stop translation

<400> 178
Met Cys Ile Pro Glu Ala Leu Gly Lys Asn Ser Leu Phe Leu Ser Ser
  1               5                  10                  15

Thr Phe Leu Trp Leu Leu Ala Phe Phe Gly Leu Trp Ser His His Ser
             20                  25                  30

Tyr Leu Glu Gly Gln His Leu Gln Ile Cys Phe Phe Phe Thr Xaa
        35                  40                  45

<210> 179
<211> 55
<212> PRT
<213> Homo sapiens

<220>
<221> SITE
<222> (55)
<223> Xaa equals stop translation

<400> 179
Met Thr Thr Ser Leu Phe Gly Leu Val Cys Val Val Cys Gln Gly Ala
  1               5                  10                  15

Gly Val Ser Ala Phe Thr Gln Val Asn Leu Phe Ser Phe Ser Leu Val
             20                  25                  30

Ile Val Lys Lys Gln Asn Lys Thr Ser Cys Glu Pro Phe Gly Thr Ser
        35                  40                  45

Gly Lys Val Pro Leu Leu Xaa
    50                  55

<210> 180
<211> 67
<212> PRT
<213> Homo sapiens

<220>
<221> SITE

<222> (67)
<223> Xaa equals stop translation

<400> 180
Met Leu Ile Tyr Trp Leu Gln Ser Ser Phe Ile Leu Ser Ala Phe Val
1               5                   10                  15

Leu Ile Asn Ser Pro Val Thr Thr Gly Ile Gln Lys Ser Cys Cys Lys
            20                  25                  30

Phe Phe Pro Val Ser Ile Asn Leu Cys Phe Ala Ser Leu His Arg Met
        35                  40                  45

Lys Val Val Thr Leu Val Ala Leu Gln Trp Leu Asn Ile Ala Leu Arg
    50                  55                  60

Ser Ser Xaa
65


<210> 181
<211> 51
<212> PRT
<213> Homo sapiens

<220>
<221> SITE
<222> (51)
<223> Xaa equals stop translation

<400> 181
Met Val Cys Cys Gly Phe Phe Leu Leu Trp Ser Arg Val Arg Ser Tyr
1               5                   10                  15

Met Lys Leu Ser Gly His Arg Trp Ser Ser Ser Cys Pro His His Cys
            20                  25                  30

Tyr Ser Lys Cys Gly Leu His Thr Ser Asn Gly Lys Ser Ser Val His
        35                  40                  45

Thr Val Xaa
    50


<210> 182
<211> 91
<212> PRT
<213> Homo sapiens

<220>
<221> SITE
<222> (29)
<223> Xaa equals any of the naturally occurring L-amino acids

<220>
<221> SITE
<222> (30)
<223> Xaa equals any of the naturally occurring L-amino acids

<220>
<221> SITE
<222> (65)
<223> Xaa equals any of the naturally occurring L-amino acids

<220>
<221> SITE
<222> (91)
<223> Xaa equals stop translation

<400> 182
Met Leu Arg Cys Ser Phe Ser Ser Phe Leu Leu Cys His Thr Ile Leu
1               5                   10                  15

Leu Phe Leu Gly Ser Ser Ala His Leu Leu Val Glu Xaa Xaa Val Trp
            20                  25                  30

Gly Leu Tyr Glu Tyr Arg Ile Gly Asp Met Val Asp Gln Lys Ala Thr
        35                  40                  45

Phe Cys Val Gln Lys Gln Glu Cys Leu Phe Pro Leu Gly Ser Trp Val
    50                  55                  60

Xaa Arg Val Glu Gly Gly Ala Phe Ala Arg Glu Pro Pro Ser Ser Thr
65                  70                  75                  80

Gln Tyr Phe Pro Val Ser Cys Leu Tyr Gln Xaa
                85                  90


<210> 183
<211> 55
<212> PRT
<213> Homo sapiens

<220>
<221> SITE
<222> (55)
<223> Xaa equals stop translation

<400> 183
Met Ser Ala Leu Leu Ser His His Val Pro Leu Phe Tyr Leu Thr Gly
1               5                   10                  15

Cys Leu Phe Ser Leu Leu Ala Ser Trp Asp Cys Asn Gly Lys Glu Gly
            20                  25                  30

Ala Gly Arg Ala Ile Lys Gly Lys Asn Asn Thr Trp Asn Cys Met Ile
        35                  40                  45

Leu Ser Lys Val Lys Phe Xaa
    50                  55


<210> 184
<211> 64
<212> PRT

<213> Homo sapiens

<220>
<221> SITE
<222> (26)
<223> Xaa equals any of the naturally occurring L-amino acids

<220>
<221> SITE
<222> (41)
<223> Xaa equals any of the naturally occurring L-amino acids

<220>
<221> SITE
<222> (64)
<223> Xaa equals stop translation

<400> 184
Met Val His Lys Ala Ile Leu Ala Leu Leu Pro Trp Gly Phe Ser Ala
1               5                   10                  15

Asp Glu Leu Leu Ala Ser Leu Met Met Xaa Leu Thr Glu Lys Tyr Gln
            20                  25                  30

Asn Cys Ser Ser Thr Thr Asp Ile Xaa Asn Gln Gln Leu Arg Ser Leu
        35                  40                  45

Gly Gln Asn Phe Met Phe Gln Gln Asn Leu Gln Leu Ile Leu Met Xaa
    50                  55                  60

<210> 185
<211> 113
<212> PRT
<213> Homo sapiens

<220>
<221> SITE
<222> (113)
<223> Xaa equals stop translation

<400> 185
Met Met Thr Ser Ser Leu Gly Leu Ser Phe Leu Leu Asn Leu Ile Leu
1               5                   10                  15

Gly Met Lys Phe Thr Tyr Leu Ile Pro Gln Asn Lys Tyr Ile Gln Leu
            20                  25                  30

Phe Thr Thr Ile Leu Ser Phe Phe Ser Gly Val Leu Ser Leu Leu Glu
        35                  40                  45

Cys Lys Leu Ser Thr Ser Ser Cys Thr Cys Leu Asn Ile His Lys Ser
    50                  55                  60

Asp Asn Glu Cys Lys Glu Ser Glu Asn Ser Ile Glu Asp Ile Ser Leu

65                  70                  75                  80

Pro Glu Arg Thr Ala Met Pro Arg Ser Ile Val Arg Ala His Thr Val
                85                  90                  95

Asn Ser Leu Asn Lys Lys Val Gln Thr Arg His Val Thr Trp Ala Leu
            100                 105                 110

Xaa

<210> 186
<211> 60
<212> PRT
<213> Homo sapiens

<220>
<221> SITE
<222> (60)
<223> Xaa equals stop translation

<400> 186
Met Leu His Leu Thr Leu Tyr Leu His Phe Ile Leu Phe Val Phe Pro
 1               5                  10                  15

Ile Thr Ser Asn Phe Ser Ser Leu His Pro Phe Leu Phe Ile Ser Ser
            20                  25                  30

Gln Phe Thr Ser Cys Cys Gln Ile Asn Phe Pro Asn Ala Gln Ala Leu
        35                  40                  45

Ser Tyr His Glu Phe Leu Ile Ala Thr Tyr Asp Xaa
    50                  55                  60

<210> 187
<211> 64
<212> PRT
<213> Homo sapiens

<220>
<221> SITE
<222> (64)
<223> Xaa equals stop translation

<400> 187
Met Pro Cys Ile Arg Gly Val Phe His Cys Phe Ile Leu Ile Ile Leu
 1               5                  10                  15

Ile Leu Leu Ala Ser His Ala Phe Ser Gly Ser Gly Asn Gln Arg Leu
            20                  25                  30

Lys Glu Ala Leu Thr Leu Ile Val Ser Val Asn Val Asp Ile Ala Arg
        35                  40                  45

His Arg Pro Phe Leu Glu Arg Ile His Val Lys Lys Gly Asn Thr Xaa
    50                  55                  60

<210> 188
<211> 71
<212> PRT
<213> Homo sapiens

<220>
<221> SITE
<222> (71)
<223> Xaa equals stop translation

<400> 188
Met Phe Ser Arg Leu His Phe Leu Thr His Ser Leu Ser Leu Leu His
1               5                   10                  15

Leu Pro Ser Gln Val Phe Gly Glu Val His Ser Ser Cys Val Ser Ser
            20                  25                  30

Leu Pro Cys Pro Asp Thr Pro Ala Leu Pro Tyr Cys Pro Ser Phe Leu
        35                  40                  45

Arg Tyr Asp Asp His Ile Glu Ala Gln Pro Leu Lys His Ile Asn Thr
    50                  55                  60

Asn Asp His Ile Ser Ile Xaa
65                  70


<210> 189
<211> 63
<212> PRT
<213> Homo sapiens

<220>
<221> SITE
<222> (63)
<223> Xaa equals stop translation

<400> 189
Met Cys Val Phe Ser Ser Asp Ser Ile Pro Ser Leu Leu Ile Leu Leu
1               5                   10                  15

Val Leu Cys His Ser Val Cys Cys Leu Lys Leu Phe Phe Lys Leu Ile
            20                  25                  30

Phe Pro Asn Ser Phe Ile Ile Tyr Glu Lys Leu Gly Leu Asn His Phe
        35                  40                  45

Ala Tyr His Leu Ser Gly Trp Phe Glu Leu Ser Leu Asp Thr Xaa
    50                  55                  60


<210> 190
<211> 193

<212> PRT
<213> Homo sapiens

<220>
<221> SITE
<222> (193)
<223> Xaa equals stop translation

<400> 190
Met Glu Ala Leu Leu Gln Ser Leu Val Ile Val Leu Leu Gly Phe Lys
1               5                   10                  15

Ser Phe Leu Ser Glu Glu Leu Gly Ser Glu Val Leu Asn Leu Leu Thr
            20                  25                  30

Asn Lys Gln Tyr Glu Leu Leu Ser Lys Asn Leu Arg Lys Thr Arg Glu
        35                  40                  45

Leu Phe Val His Gly Leu Pro Gly Ser Gly Lys Thr Ile Leu Ala Leu
    50                  55                  60

Arg Ile Met Glu Lys Ile Arg Asn Val Phe His Cys Glu Pro Ala Asn
65                  70                  75                  80

Ile Leu Tyr Ile Cys Glu Asn Gln Pro Leu Lys Lys Leu Val Ser Phe
                85                  90                  95

Ser Lys Lys Asn Ile Cys Gln Pro Val Thr Arg Lys Thr Phe Met Lys
            100                 105                 110

Asn Asn Phe Glu His Ile Gln His Ile Ile Ile Asp Asp Ala Gln Asn
        115                 120                 125

Phe Arg Thr Glu Asp Gly Asp Trp Tyr Gly Lys Ala Lys Phe Ile Thr
    130                 135                 140

Gln Thr Ala Arg Asp Gly Pro Gly Val Leu Trp Ile Phe Leu Asp Tyr
145                 150                 155                 160

Phe Gln Thr Tyr His Leu Ser Cys Ser Ala Ser Pro Leu Pro Gln Thr
                165                 170                 175

Ser Ile Gln Glu Lys Arg Ser Thr Glu Trp Ser Ala Met Gln Val Gln
            180                 185                 190

Xaa

<210> 191
<211> 112
<212> PRT
<213> Homo sapiens

<220>
<221> SITE
<222> (112)
<223> Xaa equals stop translation

<400> 191
Met Gln Phe Ser Leu Cys Leu Thr Ala Val Phe Leu Leu Gln Leu Ala
1               5                   10                  15

Ala Gly Ile Leu Gly Phe Val Phe Ser Asp Lys Ala Arg Gly Lys Val
            20                  25                  30

Ser Glu Ile Ile Asn Asn Ala Ile Val His Tyr Arg Asp Asp Leu Asp
        35                  40                  45

Leu Gln Asn Leu Ile Asp Phe Gly Gln Lys Lys Val Trp Val Ser Gln
    50                  55                  60

Trp Ser Gly Gly Leu Trp Val Lys Val Asn Val Ile Pro Arg Asp Ala
65                  70                  75                  80

Ser Pro Ser Met Pro Val Gly Leu Phe Ile Thr Cys Gln Val Met Ala
                85                  90                  95

Ser Gly Lys Gly Phe Gly Lys Lys Ser Thr Arg Ser Arg Val Leu Xaa
            100                 105                 110

<210> 192
<211> 80
<212> PRT
<213> Homo sapiens

<220>
<221> SITE
<222> (80)
<223> Xaa equals stop translation

<400> 192
Met Cys Arg Pro Leu Leu Pro Leu Leu Phe Pro Trp Gly His Cys Leu
1               5                   10                  15

Ser Ile Pro Leu Cys Lys Trp Pro Gln Ile Met Ser Gln Pro Pro Arg
            20                  25                  30

Leu His Arg Leu Leu Ala Ser Gly Pro Ser Thr Lys Lys His Ser Lys
        35                  40                  45

Leu Gln Thr His Ser Trp Glu Asn Ser Asn Gly Leu Thr Leu Pro Phe
    50                  55                  60

Glu Pro Ala Arg Ser His Gly Leu Trp Arg Ala Ala Phe Glu Ser Xaa
65                  70                  75                  80

<210> 193

<211> 88
<212> PRT
<213> Homo sapiens

<220>
<221> SITE
<222> (88)
<223> Xaa equals stop translation

<400> 193
Met Leu Ser Ile Ile Asp Leu Leu Phe Leu Leu Ser Pro Thr Phe Gly
1               5                   10                  15

Leu Ile Thr Glu Leu Leu Phe Ser Pro Glu Val Pro Lys Ala Leu Ser
            20                  25                  30

Cys Pro Leu Lys Ala Leu Gly Gly Gly Ser His Ser His Glu Pro Leu
        35                  40                  45

Gly Met Phe Ala Pro Val Pro Pro Gly Cys Glu Ser Ser Thr Pro Phe
    50                  55                  60

Pro Lys Gly Leu Gly Ala Ser Lys Ile Leu Thr Leu Gly Ala Gln Ala
65                  70                  75                  80

Glu Phe Arg Arg Arg Ser His Xaa
                85


<210> 194
<211> 42
<212> PRT
<213> Homo sapiens

<220>
<221> SITE
<222> (42)
<223> Xaa equals stop translation

<400> 194
Met Glu Asp His Phe Leu Ile Gly His Phe Pro Phe Phe Phe Leu Phe
1               5                   10                  15

Ser Phe Pro Cys Phe Cys Thr Lys Pro Leu Cys Arg Glu Tyr Phe Leu
            20                  25                  30

Ile Cys Ser Ile Gln Asp Glu Ser Lys Xaa
        35                  40


<210> 195
<211> 69
<212> PRT
<213> Homo sapiens

<220>
<221> SITE
<222> (69)

<223> Xaa equals stop translation

<400> 195
Met Phe Asn Leu Pro Lys Pro Val Phe Leu Ser Trp Trp Arg Trp Lys
1               5                   10                  15

Thr Ile Val Ile Phe Leu Ala Cys Leu Ala Ser Ala Ala Ile Lys Glu
            20                  25                  30

Thr Ala Val Ser Met Lys Thr Val Phe Pro Ile Phe Val Gln Ile Thr
        35                  40                  45

Leu Ile Leu Leu Leu Glu Ser Arg Val Leu Lys Ile Gly Asp Phe Ser
    50                  55                  60

Asn Phe Phe Cys Xaa
65


<210> 196
<211> 153
<212> PRT
<213> Homo sapiens

<220>
<221> SITE
<222> (66)
<223> Xaa equals any of the naturally occurring L-amino acids

<220>
<221> SITE
<222> (77)
<223> Xaa equals any of the naturally occurring L-amino acids

<220>
<221> SITE
<222> (81)
<223> Xaa equals any of the naturally occurring L-amino acids

<220>
<221> SITE
<222> (84)
<223> Xaa equals any of the naturally occurring L-amino acids

<220>
<221> SITE
<222> (86)
<223> Xaa equals any of the naturally occurring L-amino acids

<220>
<221> SITE
<222> (87)
<223> Xaa equals any of the naturally occurring L-amino acids

<220>
<221> SITE
<222> (93)
<223> Xaa equals any of the naturally occurring L-amino acids

<220>
<221> SITE
<222> (103)
<223> Xaa equals any of the naturally occurring L-amino acids

<220>
<221> SITE
<222> (110)
<223> Xaa equals any of the naturally occurring L-amino acids

<220>
<221> SITE
<222> (153)
<223> Xaa equals stop translation

<400> 196
Met Asp His Ser Pro Thr Thr Gly Val Val Thr Val Ile Val Ile Leu
1               5                   10                  15

Ile Ala Ile Ala Ala Leu Gly Ala Phe Asp Pro Gly Leu Leu Val Leu
            20                  25                  30

Pro Ala Ala Ala Ala His Gln Pro Val Arg Gly Arg Gly Glu His Arg
        35                  40                  45

Gly Gly Trp Gly Asp Gln Gly Thr Leu Pro Ala Gly Ala Val Phe Gly
    50                  55                  60

Gln Xaa Thr Val Arg Gly Glu Lys Gly Gln Ala Asp Xaa Ser Gln Thr
65                  70                  75                  80

Xaa Arg Lys Xaa Thr Xaa Xaa Pro Gly Cys Lys Gly Xaa Leu Val Pro
                85                  90                  95

Val Cys Lys Pro Ala Lys Xaa Gly Leu Gly Gly Ala Lys Xaa Ile Arg
            100                 105                 110

Met Arg Cys Cys Leu Arg Gly Arg Ala Asp Thr Cys Trp His Gly Leu
        115                 120                 125

Cys Gly Phe Arg Pro Ser His Ala Leu Met Pro Gly Asp Leu Ala Val
    130                 135                 140

Leu Gly Phe Pro Ser Ala Ser Arg Xaa
145                 150


<210> 197
<211> 63
<212> PRT
<213> Homo sapiens

<220>
<221> SITE
<222> (63)
<223> Xaa equals stop translation

<400> 197
Met Lys Asn Ser Thr Ser Leu Leu Tyr Lys Leu Phe Ser Ser Leu Ser
1               5                   10                  15

Val Phe Ile Phe Lys Phe Leu Leu Leu Phe Tyr Thr Leu His Ile Ala
            20                  25                  30

Leu Gly Val Lys Ile Gln Tyr Lys Pro Leu Ala His Phe Ile Asp His
        35                  40                  45

Ser Cys Ile Gln Gln Val Ser Gln Val Gln Trp Ser Ile Pro Xaa
    50                  55                  60

<210> 198
<211> 64
<212> PRT
<213> Homo sapiens

<220>
<221> SITE
<222> (64)
<223> Xaa equals stop translation

<400> 198
Met Gln Glu Pro His Gly Lys Phe Leu Ser Trp Gly Arg Trp Leu Trp
1               5                   10                  15

Trp Trp Ser Leu Ala Ala Pro Ala Leu Val Gln Ala Val Asn Met Pro
            20                  25                  30

Pro Ala Tyr Ile Gln Ile Glu Asn Trp Tyr Met Met Leu Leu Met Gly
        35                  40                  45

Trp Glu Thr Lys Cys Cys His Val Arg Ser Leu Trp Val Gly Thr Xaa
    50                  55                  60

<210> 199
<211> 43
<212> PRT
<213> Homo sapiens

<220>
<221> SITE
<222> (43)
<223> Xaa equals stop translation

<400> 199
Met Leu Ile Asn Cys Ile Phe Ser Leu Leu Leu Leu Leu Ser His Ala
1               5                   10                  15

Asp Gly Met His Leu Phe Ile Ser Ser Gly Asp Arg Ile Leu Phe Cys
            20                  25                  30

Leu Tyr Phe Leu His Ser Arg Val Cys Ala Xaa
        35                  40

<210> 200
<211> 41
<212> PRT
<213> Homo sapiens

<220>
<221> SITE
<222> (41)
<223> Xaa equals stop translation

<400> 200
Met Ser Val Tyr Val Asn Ile Met His Ile Val Ile Tyr Ile Tyr Leu
  1               5                  10                  15

Cys Val Tyr Met Cys Val Ala Gln Ser His Thr His Thr Gln Ile Cys
             20                  25                  30

Ile Gln Met Leu Pro Gly Leu Gln Xaa
        35                  40

<210> 201
<211> 44
<212> PRT
<213> Homo sapiens

<220>
<221> SITE
<222> (44)
<223> Xaa equals stop translation

<400> 201
Met Ile Leu Ser Phe Leu Met Leu Phe Leu Ile Val Lys Thr Ile Pro
  1               5                  10                  15

Leu Ile Leu Ala Tyr Cys Tyr Asn Ser Ile Ser Phe Phe Ser Asn Asn
             20                  25                  30

Leu Val Leu Val Lys Met Gly Tyr Asn Asn Lys Xaa
        35                  40

<210> 202
<211> 42
<212> PRT
<213> Homo sapiens

<220>
<221> SITE
<222> (42)
<223> Xaa equals stop translation

<400> 202
Met Arg Leu Leu Ser Thr Leu Leu Ser Phe Tyr Pro Phe Ser Asn Cys

1                   5                   10                  15

Phe Leu Leu Ser Phe Cys Asp Ser His Pro Pro Val Trp Leu Arg Asn
            20                  25                  30

Ser Gln Val Phe Pro Glu Glu Val Val Xaa
        35                  40


<210> 203
<211> 42
<212> PRT
<213> Homo sapiens

<220>
<221> SITE
<222> (42)
<223> Xaa equals stop translation

<400> 203
Met Thr Gly Lys Leu Trp Leu Leu Leu Pro Arg Leu Gly His Ala Ala
 1               5                  10                  15

Ala Ala Pro Thr Thr Ala Leu Ser Gly Ser Glu Leu Glu Gly Thr Ser
            20                  25                  30

Ile Ser Leu Leu Ile Ala Leu Asp Arg Xaa
        35                  40


<210> 204
<211> 113
<212> PRT
<213> Homo sapiens

<220>
<221> SITE
<222> (17)
<223> Xaa equals any of the naturally occurring L-amino acids

<220>
<221> SITE
<222> (57)
<223> Xaa equals any of the naturally occurring L-amino acids

<220>
<221> SITE
<222> (90)
<223> Xaa equals any of the naturally occurring L-amino acids

<220>
<221> SITE
<222> (91)
<223> Xaa equals any of the naturally occurring L-amino acids

<220>
<221> SITE
<222> (113)

<223> Xaa equals stop translation

<400> 204
Met Ala Pro Trp Leu Pro Leu Leu Ser Leu Leu Gly Leu Leu Leu Gly
1               5                   10                  15

Xaa Ala Pro Ala Pro Pro Arg Arg Ala Ala Asp Ala Gln Ala Arg Glu
            20                  25                  30

Ala Ala Tyr Pro Glu Leu Leu Gly Pro Ala Arg Phe Ala Leu Glu Met
        35                  40                  45

Tyr Asn Arg Gly Arg Ala Ala Gly Xaa Arg Ala Thr Leu Gly Ala Val
    50                  55                  60

Arg Gly Arg Val Arg Arg Ala Gly Glu Gly Ser Leu Tyr Ser Leu Arg
65                  70                  75                  80

Ala Thr Leu Glu Glu Pro Pro Cys Asn Xaa Xaa Thr Val Cys Gln Leu
                85                  90                  95

Pro Val Ser Lys Arg Pro Cys Ser Ala Ala Leu Lys Ser Trp Thr Ser
            100                 105                 110

Xaa

<210> 205
<211> 45
<212> PRT
<213> Homo sapiens

<220>
<221> SITE
<222> (45)
<223> Xaa equals stop translation

<400> 205
Met Pro Thr Trp Pro Leu Leu Gln Leu Leu Ser Cys Ser Phe Pro Ser
1               5                   10                  15

Leu Leu Cys Glu Thr Phe Thr Phe Cys Ser Lys Asp Glu Val Ser Arg
            20                  25                  30

Trp Lys Ala Gly Cys Phe Val Pro Leu Pro Ala Ser Xaa
        35                  40                  45

<210> 206
<211> 71
<212> PRT
<213> Homo sapiens

<220>
<221> SITE
<222> (71)
<223> Xaa equals stop translation

<400> 206
Met Thr His Trp Ser Gly Cys Ala Ala Leu Tyr Leu Ile Phe Leu Ser
1               5                   10                  15

Leu Lys Leu Ala Phe Gln Ala Gly Ala Gly Arg Gly Ala Gln Val Gly
            20                  25                  30

Ser Val Leu Pro Pro Ser Gly Gly Ala Val Val Val Asp Gln Tyr Cys
        35                  40                  45

Cys Arg Leu Ser Ala Gln Thr Tyr Phe Ser Leu Pro Ala Leu Gln Lys
    50                  55                  60

Cys Ile Gly Ile Cys Arg Xaa
65                  70


<210> 207
<211> 42
<212> PRT
<213> Homo sapiens

<220>
<221> SITE
<222> (42)
<223> Xaa equals stop translation

<400> 207
Met Ile Asn Phe Trp Pro Val Thr His Val Cys Ile Trp Leu Leu Trp
1               5                   10                  15

Leu Gln Ala Leu Glu Ala Arg Gly Gln Gly Ser Asn Ile Asp Cys Thr
            20                  25                  30

Arg Asn Ser Lys Thr Val Phe Thr Ser Xaa
        35                  40


<210> 208
<211> 51
<212> PRT
<213> Homo sapiens

<220>
<221> SITE
<222> (51)
<223> Xaa equals stop translation

<400> 208
Met Tyr Ile Tyr Leu Ile His Leu Cys Met Cys Val Tyr Ile Tyr Ile
1               5                   10                  15

Tyr Ile Leu Leu Ile Ile Tyr Thr Leu Asp Pro Glu Pro Pro Ser Trp
            20                  25                  30

Ser Pro Lys Leu Asp Ser His Leu Ser Leu Arg Gln Pro Ser Asn Asp
        35                  40                  45

Arg Phe Xaa
    50

<210> 209
<211> 65
<212> PRT
<213> Homo sapiens

<220>
<221> SITE
<222> (65)
<223> Xaa equals stop translation

<400> 209
Met Phe Val Leu Cys Thr Arg Ala Val Arg Thr Arg Leu Phe Ser Leu
  1               5                  10                  15

Cys Cys Cys Cys Cys Ser Ser Gln Pro Pro Thr Lys Ser Pro Ala Gly
             20                  25                  30

Thr Pro Lys Ala Pro Ala Pro Ser Lys Pro Gly Glu Ser Gln Glu Ser
         35                  40                  45

Gln Gly Thr Pro Gly Glu Leu Pro Ser Thr Trp Ser Phe Cys Pro Phe
     50                  55                  60

Xaa
65

<210> 210
<211> 77
<212> PRT
<213> Homo sapiens

<220>
<221> SITE
<222> (77)
<223> Xaa equals stop translation

<400> 210
Met Leu Ala Leu Leu Val Gly Gly Leu Val Ala Ala Leu Ala Cys His
  1               5                  10                  15

Gly Ile Leu Ala Ala Ile Leu Ala Val Cys Gly Glu Leu Val Ser Gly
             20                  25                  30

Lys Gly Thr Arg Ser Ser Asp Glu Asp Asp Gly Gly Asp Gly Asp Arg
         35                  40                  45

Gly His Arg Gly Leu Ser Leu Leu Asn Ser Ala Phe Gly His Met Gly
     50                  55                  60

Asp Gly Asp Arg Lys Asp Asp Asn Ser Gly Thr Leu Xaa
 65                  70                  75

<210> 211
<211> 45
<212> PRT
<213> Homo sapiens

<220>
<221> SITE
<222> (45)
<223> Xaa equals stop translation

<400> 211
Met Phe Val Gly Thr Arg Val Leu Leu Val Pro Leu Pro Phe Phe Ser
 1               5                  10                  15

Ile Ser Gly Met Leu Ala Ile Asp Lys Tyr Leu His Lys Lys Leu Leu
            20                  25                  30

Leu Asn Glu Ile Ile Thr Thr Ser Thr Trp Ala Leu Xaa
        35                  40                  45


<210> 212
<211> 66
<212> PRT
<213> Homo sapiens

<220>
<221> SITE
<222> (27)
<223> Xaa equals any of the naturally occurring L-amino acids

<220>
<221> SITE
<222> (66)
<223> Xaa equals stop translation

<400> 212
Met Gly Lys Gly His Gln Arg Pro Trp Trp Lys Val Leu Pro Leu Ser
 1               5                  10                  15

Cys Phe Leu Val Ala Leu Ile Ile Trp Cys Xaa Leu Arg Glu Glu Ser
            20                  25                  30

Glu Ala Asp Gln Trp Leu Arg Gln Val Trp Gly Glu Val Pro Glu Pro
        35                  40                  45

Ser Asp Arg Ser Glu Glu Pro Glu Thr Pro Ala Ala Tyr Arg Ala Arg
    50                  55                  60

Thr Xaa
65


<210> 213
<211> 62
<212> PRT
<213> Homo sapiens

<220>
<221> SITE
<222> (62)
<223> Xaa equals stop translation

<400> 213
Met Arg Leu Cys Thr Thr Trp Met Ala Val Lys Phe Leu Trp Trp Gly
1               5                   10                  15

Met Thr Trp Ile Pro Ser Gly Lys Ala Cys Ser Trp Thr Gln Pro Leu
            20                  25                  30

Cys Ser Ser Gly Gly Trp Ser Ser Pro Thr His Leu Pro Thr Ser Leu
        35                  40                  45

Leu Leu Gly Trp Arg Ala Ser Leu Cys Met Lys Arg Ser Xaa
    50                  55                  60


<210> 214
<211> 56
<212> PRT
<213> Homo sapiens

<220>
<221> SITE
<222> (56)
<223> Xaa equals stop translation

<400> 214
Met Phe Ala Ser Tyr His Ile Gln Phe Phe Thr Trp Leu Ile Gln Lys
1               5                   10                  15

Leu Ser Leu Val Trp Lys Ser Val Val Ala Ile Arg Glu Gln Gly Lys
            20                  25                  30

Glu Leu Val Trp Lys Gln His Leu Pro Leu Arg Ser Tyr Ser Pro Asn
        35                  40                  45

Asn Ala Lys Ser Leu Gly Leu Xaa
    50                  55


<210> 215
<211> 213
<212> PRT
<213> Homo sapiens

<220>
<221> SITE
<222> (88)
<223> Xaa equals any of the naturally occurring L-amino acids

<220>
<221> SITE
<222> (213)
<223> Xaa equals stop translation

<400> 215
Met Leu Ser Phe Asn Phe Thr Trp Met Val Trp Val Ser Leu Val Leu
1               5                   10                  15

Lys Ser Gln Arg Ala Lys Leu Ala Leu His Ser Leu His Leu His Gln
            20                  25                  30

Glu Val Arg Leu Arg Met Ser Arg Arg Glu Ser Pro Gly Arg Pro Leu
        35                  40                  45

Arg Cys Gly Val Arg Gly Asn Met Gly Ala Arg Thr Pro Val Pro Thr
    50                  55                  60

Ala Asp Tyr Pro Ser Pro Tyr Arg Thr Leu Pro Arg Met Ala Ala Pro
65                  70                  75                  80

Pro Pro Gln Lys Ser Ser Cys Xaa Arg Leu His Arg Pro His Trp Trp
                85                  90                  95

Arg Pro Arg Thr Pro Ser Ser Glu Lys Thr Gly Gly Gln Ser Gln Ser
            100                 105                 110

Thr Leu Asp Arg Cys Ala His Leu Val His Met Leu Leu Arg Asp Gln
        115                 120                 125

Arg Ala Thr Ser Gln Trp Lys Ala Gly Gly Arg Leu Cys Arg Ala Leu
    130                 135                 140

Ser Lys Thr Pro Leu Gln His Gln Leu His Ser Thr Ser Tyr Arg Lys
145                 150                 155                 160

Ala Leu Pro Ile Leu Arg Pro Ser Ser Arg Arg Glu Ala Gly Pro Leu
                165                 170                 175

His His Ile Asp Leu Arg Arg Cys Phe Ser Arg Leu Gly Arg Gly Ala
            180                 185                 190

Asp Phe Ala Val Cys Ala Lys Glu Pro Val Ser Asp Asn Pro Ile Phe
        195                 200                 205

Leu Leu Ile Thr Xaa
    210


<210> 216
<211> 41
<212> PRT
<213> Homo sapiens

<220>
<221> SITE
<222> (41)
<223> Xaa equals stop translation

<400> 216
Met Asn Met Phe Gln Thr Ile Leu Val Cys Val Leu Phe Val Phe Val
1               5                   10                  15

Arg Trp Phe Phe Leu Leu Leu Gln Ile Glu Ser Ile Gln Thr Lys Phe
            20                  25                  30

His Cys Ile Ser Ser Gln Phe Trp Xaa
        35                  40

<210> 217
<211> 60
<212> PRT
<213> Homo sapiens

<220>
<221> SITE
<222> (60)
<223> Xaa equals stop translation

<400> 217
Met Glu Leu Val Trp Phe Arg Phe Leu His Leu Asn Leu Leu Pro Arg
  1               5                  10                  15

Gly Val Cys Cys Gly Ile Cys Val Cys Val Arg Arg Gly Met Val Leu
            20                  25                  30

Ser Glu Pro Thr Ser Cys Gly Gln Arg Ala Leu Ser Cys Glu Gly Gly
        35                  40                  45

Cys His Ser Gly Arg Val Gln Phe Arg Arg Pro Xaa
    50                  55                  60

<210> 218
<211> 59
<212> PRT
<213> Homo sapiens

<220>
<221> SITE
<222> (59)
<223> Xaa equals stop translation

<400> 218
Met Arg Arg Met Arg Met Lys Ser Leu Ser Pro Arg Arg Ser Trp Trp
  1               5                  10                  15

Thr Leu Trp Leu Gly Gln Gly Val Leu Gly Ala Ala Leu Lys Ala Asn
            20                  25                  30

Thr Leu Trp Ile Ala Met Arg Arg Arg Met Met Met Met Gly Gly Pro
        35                  40                  45

Ala Asn Met Thr Ser Trp Pro Gln Arg Met Xaa
    50                  55

<210> 219
<211> 46

<212> PRT
<213> Homo sapiens

<220>
<221> SITE
<222> (46)
<223> Xaa equals stop translation

<400> 219
Met Pro Phe Phe Leu Leu Thr Phe Pro Leu Val Leu Tyr Pro His Leu
1               5                   10                  15

Ser Arg Gly Ser Asp Pro Val Leu Pro Cys Val Met Gly Ile His Val
            20                  25                  30

Phe Gly Leu Ser His His Ser Arg Lys Val Ala Pro Pro Xaa
        35                  40                  45


<210> 220
<211> 62
<212> PRT
<213> Homo sapiens

<220>
<221> SITE
<222> (62)
<223> Xaa equals stop translation

<400> 220
Met Asp Arg Val Arg Phe Arg Ser Trp Leu Leu Tyr Pro Cys Cys Val
1               5                   10                  15

Ala Leu Gly Gln Glu Leu Gly Leu Ser Ala Pro Gln Trp Leu Ile Thr
            20                  25                  30

Glu Asn Gly Met Pro Ala Leu Ala Leu Val Gly Cys Phe Glu Pro Thr
        35                  40                  45

Ala Gly Ser Gly Ser Ser Trp His Asp Val Phe Leu Pro Xaa
    50                  55                  60


<210> 221
<211> 52
<212> PRT
<213> Homo sapiens

<220>
<221> SITE
<222> (52)
<223> Xaa equals stop translation

<400> 221
Met Lys Leu Asn Val His Phe Leu Trp Cys Thr Phe Ile Phe Gln Thr
1               5                   10                  15

Ser Gly Ser His Ile Glu Leu Leu Ile Ser Gly Gln Val Ser Ser Tyr

            20                  25                  30

Ile Pro Ser Leu Asp Phe Cys Thr His Lys Val Val Ser Arg Glu Lys
        35                  40                  45

Phe Glu Glu Xaa
    50

<210> 222
<211> 51
<212> PRT
<213> Homo sapiens

<220>
<221> SITE
<222> (51)
<223> Xaa equals stop translation

<400> 222
Met Ala Ser Pro Val Phe Lys Thr Phe Trp Arg Leu Glu Leu Ser Val
1               5                   10                  15

Pro Leu Ser Leu Leu Phe Ile Leu Gln Ile Val Thr Ser Leu Ser Ser
            20                  25                  30

Asp Glu Ile Cys Tyr Ser Thr Arg Lys Val Phe Ile Ile Arg Arg Gln
        35                  40                  45

Leu Tyr Xaa
    50

<210> 223
<211> 47
<212> PRT
<213> Homo sapiens

<220>
<221> SITE
<222> (47)
<223> Xaa equals stop translation

<400> 223
Met Cys Met Cys Val Gly Val Cys Leu Ile Thr Leu Leu Asp Arg Phe
1               5                   10                  15

Leu Trp Phe Gly Thr Ala Gly Ala Lys Phe Ile Gln Lys Ser Thr Phe
            20                  25                  30

Leu Ser Lys Leu Pro Met Thr Leu Val Ser Phe His Ser Ile Xaa
        35                  40                  45

<210> 224
<211> 52
<212> PRT
<213> Homo sapiens

<220>
<221> SITE
<222> (52)
<223> Xaa equals stop translation

<400> 224
Met Cys Pro Phe His Lys Ala Tyr Leu Asp Cys Phe Phe Gln Ile Ser
1               5                   10                  15

Leu Leu Leu Leu Ile Phe Leu Thr Tyr Leu Asp Ile Gly Lys Cys Gly
            20                  25                  30

Leu Trp Ser His Glu Trp Arg Ile Arg Glu Leu Gly Lys His Glu Arg
        35                  40                  45

Trp Trp Asn Xaa
    50


<210> 225
<211> 66
<212> PRT
<213> Homo sapiens

<220>
<221> SITE
<222> (61)
<223> Xaa equals any of the naturally occurring L-amino acids.

<220>
<221> SITE
<222> (66)
<223> Xaa equals stop translation

<400> 225
Met Asn Gln Pro Ile Leu Arg Ser Gln Ala Leu Leu Trp Pro Trp Arg
1               5                   10                  15

Trp Val Val Lys Ala Lys Pro Cys Val Cys Val Ser Met Asp Ala Trp
            20                  25                  30

Ile Pro Asp Arg Ser Gln His Cys Pro Ser Ile Pro Gly Gln Lys Lys
        35                  40                  45

Glu Arg Ala Gly Ser His Gly His Gln Ala Leu Ala Xaa Leu Leu Phe
    50                  55                  60

Leu Xaa
65


<210> 226
<211> 47
<212> PRT
<213> Homo sapiens

<220>

<221> SITE
<222> (47)
<223> Xaa equals stop translation

<400> 226
Met Ala Ser Arg Gly Thr Ala Ala Pro Gly Arg Thr Phe Leu Ala Met
1               5                   10                  15

Met Val Thr Ser Phe Phe Phe Cys Met Arg Trp Gly Ser Trp Ala Glu
            20                  25                  30

Gln Met Pro Gln Arg Cys Leu Pro Cys Cys Met Gln Glu Cys Xaa
        35                  40                  45


<210> 227
<211> 222
<212> PRT
<213> Homo sapiens

<220>
<221> SITE
<222> (184)
<223> Xaa equals any of the naturally occurring L-amino acids

<220>
<221> SITE
<222> (222)
<223> Xaa equals stop translation

<400> 227
Met Ala Gly Gly Val Arg Pro Leu Arg Gly Leu Arg Ala Leu Cys Arg
1               5                   10                  15

Val Leu Leu Phe Leu Ser Gln Phe Cys Ile Leu Ser Gly Gly Glu Ser
            20                  25                  30

Thr Glu Ile Pro Pro Tyr Val Met Lys Cys Pro Ser Asn Gly Leu Cys
        35                  40                  45

Ser Arg Leu Pro Ala Asp Cys Ile Asp Cys Thr Thr Asn Phe Ser Cys
    50                  55                  60

Thr Tyr Gly Lys Pro Val Thr Phe Asp Cys Ala Val Lys Pro Ser Val
65                  70                  75                  80

Thr Cys Val Asp Gln Asp Phe Lys Ser Gln Lys Asn Phe Ile Ile Asn
                85                  90                  95

Met Thr Cys Arg Phe Cys Trp Gln Leu Pro Glu Thr Asp Tyr Glu Cys
            100                 105                 110

Thr Asn Ser Thr Ser Cys Met Thr Val Ser Cys Pro Arg Gln Arg Tyr
        115                 120                 125

Pro Ala Asn Cys Thr Val Arg Asp His Val His Cys Leu Gly Asn Arg
    130                 135                 140

Thr Phe Pro Lys Met Leu Tyr Cys Asn Trp Thr Gly Gly Tyr Lys Trp
145                 150                 155                 160

Ser Thr Ala Leu Ala Leu Ser Ile Thr Leu Gly Gly Phe Gly Ala Asp
                165                 170                 175

Arg Phe Tyr Leu Gly Gln Trp Xaa Glu Gly Leu Gly Lys Leu Phe Ser
            180                 185                 190

Phe Gly Gly Leu Gly Ile Trp Thr Leu Ile Asp Val Leu Leu Ile Gly
        195                 200                 205

Val Gly Tyr Val Gly Pro Ala Asp Gly Ser Leu Tyr Ile Xaa
    210                 215                 220


<210> 228
<211> 49
<212> PRT
<213> Homo sapiens

<220>
<221> SITE
<222> (49)
<223> Xaa equals stop translation

<400> 228
Met Cys Ile His Tyr Ser Arg Val Ile Phe Ser Phe Leu Lys Leu Arg
  1               5                  10                  15

Ile Lys Ser Ile Ser Trp Tyr Ala Met Trp Leu Tyr Phe Phe Cys Tyr
             20                  25                  30

Leu Asn Cys Leu Ala Lys Val Arg Ser Ala Thr Thr Tyr Leu Tyr Val
         35                  40                  45

Xaa



<210> 229
<211> 41
<212> PRT
<213> Homo sapiens

<220>
<221> SITE
<222> (41)
<223> Xaa equals stop translation

<400> 229
Met Leu Pro Val Cys Val Phe Lys Leu Leu Leu Tyr Leu Tyr Val Leu
  1               5                  10                  15

Ile Arg Ile Cys Thr Ile Ile Trp Cys Phe Lys Val Tyr Ile Asn Ala
             20                  25                  30

Val Ile Leu Asn Lys Ser Ser Arg Xaa

35                  40

<210> 230
<211> 53
<212> PRT
<213> Homo sapiens

<220>
<221> SITE
<222> (53)
<223> Xaa equals stop translation

<400> 230
Met Asn Cys Gly Gly Ser Thr Leu Cys Val Leu Ser Phe Cys Ser Val
1               5                   10                  15

Val Cys Ser Val Glu Ala Ser Cys Gln Ser Thr Val Gln Trp Gly Gly
            20                  25                  30

Ala Ala Ala Arg Val Gly Val Pro Phe Asp Trp Ser Arg Asn Glu Gln
        35                  40                  45

Gly Lys Gly His Xaa
    50

<210> 231
<211> 50
<212> PRT
<213> Homo sapiens

<220>
<221> SITE
<222> (45)
<223> Xaa equals any of the naturally occurring L-amino acids

<220>
<221> SITE
<222> (50)
<223> Xaa equals stop translation

<400> 231
Met Leu Gly Ser Ile Pro Lys Leu Trp Ser Val Leu Ser Phe Ser Ile
1               5                   10                  15

Asn Phe Cys Phe Cys Cys Phe Ile Leu Ser Leu Leu Cys Leu Ser Val
            20                  25                  30

Leu Ser Asn Tyr Leu Phe Lys Thr Pro Arg Thr Trp Xaa Thr Leu His
        35                  40                  45

Arg Xaa
    50

<210> 232
<211> 45

<212> PRT
<213> Homo sapiens

<220>
<221> SITE
<222> (16)
<223> Xaa equals any of the naturally occurring L-amino acids

<220>
<221> SITE
<222> (45)
<223> Xaa equals stop translation

<400> 232
Met Cys Leu Pro Leu Leu His Cys Thr Gly Ala Leu Trp Gly Lys Xaa
1               5                   10                  15

Val Leu Leu Phe Leu Tyr Cys Leu Ala Gln Ser Phe Ala Tyr Ser Arg
            20                  25                  30

His Gln Thr Val Gly Leu Val Val His Asp Tyr Trp Xaa
        35                  40                  45


<210> 233
<211> 55
<212> PRT
<213> Homo sapiens

<220>
<221> SITE
<222> (55)
<223> Xaa equals stop translation

<400> 233
Met Cys Trp Ile Cys Val Trp Leu Phe Phe Ser Pro Thr Lys Thr Ser
1               5                   10                  15

Cys Phe Pro Trp Leu Ile Arg Pro Gly Pro Arg Ser Phe Thr Asp Ser
            20                  25                  30

His Gly Thr Pro Pro Trp Gln Cys Leu Glu Pro Ser Ser Phe Thr Tyr
        35                  40                  45

Pro Gly Lys Gln Val Trp Xaa
    50                  55


<210> 234
<211> 69
<212> PRT
<213> Homo sapiens

<220>
<221> SITE
<222> (69)
<223> Xaa equals stop translation

<400> 234
Met Lys Arg Leu Arg Phe Val Leu Arg Val Phe Gln Met Thr Ala Phe
1               5                   10                  15

Ile Thr Gly Ala His Thr Ile Thr Asn Tyr Ser Asp Arg Arg Leu Tyr
            20                  25                  30

Ile Ser Pro Leu Ser His Phe Phe Met Asn Ser Gly Ser Ser Ala Gln
        35                  40                  45

Ser Val Leu Ser His Ser Tyr Val Ser Gln Ile Phe Phe Lys Asn Val
    50                  55                  60

Ser Lys Tyr Phe Xaa
65


<210> 235
<211> 41
<212> PRT
<213> Homo sapiens

<220>
<221> SITE
<222> (41)
<223> Xaa equals stop translation

<400> 235
Met Val Ala Met Val Phe Leu Lys Ile Ser Val Leu Pro Leu Met Cys
1               5                   10                  15

Arg Gly Gln Thr Lys His Lys Val Leu Arg Asp His Ala Tyr Pro Arg
            20                  25                  30

Val Ser Gln Lys Arg Gly His Ile Xaa
        35                  40


<210> 236
<211> 45
<212> PRT
<213> Homo sapiens

<220>
<221> SITE
<222> (34)
<223> Xaa equals any of the naturally occurring L-amino acids

<220>
<221> SITE
<222> (45)
<223> Xaa equals stop translation

<400> 236
Met Thr Lys Leu Leu Ser Leu Ser His Leu Leu Val Thr Phe Phe Asn
1               5                   10                  15

Ile Ile Ala Ile Lys Cys Lys Lys Gln His Leu Arg His Ser Lys Cys

                20                  25                  30

Asn Xaa Asp Thr Thr Phe Lys Asn Lys Met Leu Asn Xaa
        35                  40                  45


<210> 237
<211> 78
<212> PRT
<213> Homo sapiens

<220>
<221> SITE
<222> (78)
<223> Xaa equals stop translation

<400> 237
Met Gln Leu Cys Val Ile Trp Phe Thr Val Ile Phe Leu Ser Gln Ser
 1               5                  10                  15

Ser Arg Leu Val Lys Glu Lys Ile Ser Asn Thr Ser Gly Glu Lys Gly
            20                  25                  30

Arg Trp Pro Ala Ile Asp Val Val Ala Leu Cys Pro Ser Arg Thr Ala
        35                  40                  45

Gly Ile Ser Phe Pro Arg His Phe Leu Tyr Val Ser Cys Ile Val Gly
    50                  55                  60

Cys Thr Asn Ile Ile Cys Ser Phe Gly Phe Pro Gly Gln Xaa
65                  70                  75


<210> 238
<211> 53
<212> PRT
<213> Homo sapiens

<220>
<221> SITE
<222> (53)
<223> Xaa equals stop translation

<400> 238
Met Glu Val Val Leu Pro Lys His Ile Leu Asp Ile Trp Val Ile Val
 1               5                  10                  15

Leu Ile Ile Leu Ala Thr Ile Val Ile Met Thr Ser Leu Leu Leu Cys
            20                  25                  30

Pro Ala Thr Ala Val Ile Ile Tyr Arg Met Arg Thr His Pro Ile Leu
        35                  40                  45

Ser Gly Ala Val Xaa
    50


<210> 239

<211> 53
<212> PRT
<213> Homo sapiens

<220>
<221> SITE
<222> (53)
<223> Xaa equals stop translation

<400> 239
Met Tyr Tyr Leu Gly Lys Trp Asp Ile Trp Gln Pro Val Ser Leu Leu
1               5                   10                  15

Tyr Ile Ile Leu Phe Ala Ala Cys Pro Ser Leu Leu Ile Ser Ile Pro
            20                  25                  30

Ala Lys Ala Ser Gly Glu Gly Trp Arg Cys Gly Asp Ile Gln Leu Thr
        35                  40                  45

Val Val Thr Asp Xaa
    50


<210> 240
<211> 43
<212> PRT
<213> Homo sapiens

<220>
<221> SITE
<222> (43)
<223> Xaa equals stop translation

<400> 240
Met Pro Val Ala Phe His Leu Pro Phe Leu Leu Ile Leu Pro Tyr Arg
1               5                   10                  15

Val Leu Pro Val Gly Gln Val Thr Gln Leu Thr Pro Arg Ala Val Glu
            20                  25                  30

Val Lys Ile His Asn His Gly Arg Leu Pro Xaa
        35                  40


<210> 241
<211> 49
<212> PRT
<213> Homo sapiens

<220>
<221> SITE
<222> (49)
<223> Xaa equals stop translation

<400> 241
Met Ser Trp Pro Leu Cys Thr Leu Leu Phe Ser Trp Asp Cys Ile Leu
1               5                   10                  15

Ala Val Lys Thr Ser Arg Leu Lys Phe Asp Ser Gln Gly Tyr Ile Leu
            20                  25                  30

Gly Thr Phe Lys Val Ser Phe Gln Arg Asp Phe Ile Asn Arg Leu Asp
        35                  40                  45

Xaa

<210> 242
<211> 75
<212> PRT
<213> Homo sapiens

<220>
<221> SITE
<222> (75)
<223> Xaa equals stop translation

<400> 242
Met Ser Ile Ile Ile Tyr Trp Leu Leu Phe Phe Lys His Leu Leu Trp
  1               5                  10                  15

Val Leu Ile Ile Gly Met Val Lys Ala Leu His Pro His Tyr Leu Asn
            20                  25                  30

Leu Arg Ile Tyr Glu Phe Gly Glu Ile Thr Ala Val Leu Gln Arg Lys
        35                  40                  45

Lys Gln Gly Arg Glu Asn Gly Asn Phe Leu Lys Phe Ser Leu Leu Ser
    50                  55                  60

Leu Asn Arg Ser Arg Ile Pro Thr Gln Ile Xaa
 65                  70                  75

<210> 243
<211> 44
<212> PRT
<213> Homo sapiens

<220>
<221> SITE
<222> (44)
<223> Xaa equals stop translation

<400> 243
Met Ala Ile His Phe His Ile Ile Gln Trp Leu Leu Leu Cys Tyr Asn
  1               5                  10                  15

Cys His His Ala Gln Trp Gly Leu Trp His Thr Thr Ala Glu Val Ser
            20                  25                  30

Gly Cys Gly Arg Asn His Leu Ala Phe Lys Ala Xaa
        35                  40

<210> 244
<211> 65
<212> PRT
<213> Homo sapiens

<220>
<221> SITE
<222> (65)
<223> Xaa equals stop translation

<400> 244
Met Tyr Leu Ser Leu Phe Phe Phe Cys Phe Ser Leu Gln Ala Ser Ala
1               5                   10                  15

Val Glu Glu Arg Ser Ala Glu Ser Ser Arg Glu Gly Pro Val Arg Thr
            20                  25                  30

Asp Asn Trp Gln Arg Cys Phe Gly Asp Ile Pro Gly Thr Pro Thr His
        35                  40                  45

Leu Val Gln Arg Ser Leu Val Leu Thr Cys Phe Gly Arg Val Leu Ser
    50                  55                  60

Xaa
65


<210> 245
<211> 84
<212> PRT
<213> Homo sapiens

<220>
<221> SITE
<222> (84)
<223> Xaa equals stop translation

<400> 245
Met Lys Lys Val Cys Trp Val Trp Ala Leu Ala His Leu Val Leu Cys
1               5                   10                  15

Glu Arg Trp Leu Thr Ala Gly Cys Leu Leu Tyr Val Gly Val Ile Gln
            20                  25                  30

Pro Cys Lys Gly Ser Pro Ser Ser Val Cys Lys Ala Arg Arg Cys Leu
        35                  40                  45

His Pro Lys Tyr Arg Ile Lys Arg Tyr Gly Tyr Tyr Lys Tyr Ser Val
    50                  55                  60

Arg Leu Ile Ile Cys His His His Pro His Ala Leu Lys Ala Glu Leu
65                  70                  75                  80

Thr Asp Asp Xaa


<210> 246

<211> 72
<212> PRT
<213> Homo sapiens

<220>
<221> SITE
<222> (72)
<223> Xaa equals stop translation

<400> 246
Met Val Gln Gly Pro Leu Thr His Leu Met Leu Val Leu Leu Ile Ser
1               5                   10                  15

Leu Ile Phe Leu Ser Arg Gly Ser Gly Arg Ala Trp Ala Phe Ser His
            20                  25                  30

Ser Cys Phe Lys Thr Ser Asp Leu Leu Pro Cys Arg Asn Arg Trp Glu
        35                  40                  45

Val Ile Glu Phe Leu His Tyr Ser Asn Leu His Ser His Ile Ser Leu
    50                  55                  60

Ser Val Thr Lys Thr Phe Leu Xaa
65                  70


<210> 247
<211> 57
<212> PRT
<213> Homo sapiens

<220>
<221> SITE
<222> (57)
<223> Xaa equals stop translation

<400> 247
Met Arg Ser Tyr Phe Pro Phe Ser Val Cys Pro Phe Pro Phe Cys Ser
1               5                   10                  15

Pro Val Phe Phe Phe Val Phe Thr Asp Val Tyr Leu Cys Phe Phe Phe
            20                  25                  30

Val Phe Ala Val Gly Arg His Leu Ser Asp Pro Phe Pro Ile Leu Phe
        35                  40                  45

Phe Thr His Lys Cys Pro Asp Val Xaa
    50                  55


<210> 248
<211> 67
<212> PRT
<213> Homo sapiens

<220>
<221> SITE
<222> (67)

<223> Xaa equals stop translation

<400> 248
Met Arg Ala Cys Gly Trp Asp Leu Ser Ile Leu Leu Val Gly Leu Val
1               5                   10                  15

Met Gly Arg Glu Gly Cys Tyr Ser Arg Leu Pro Pro Thr Glu Tyr Gln
            20                  25                  30

Lys Gln Ala Gly Ser Ser Gly Val Cys Lys Asp Val Arg Pro Arg Asn
        35                  40                  45

Gln Pro Ser Pro Ser Tyr Pro Cys Lys Ser Leu Ser Pro His Ala Pro
    50                  55                  60

Leu Leu Xaa
65


<210> 249
<211> 46
<212> PRT
<213> Homo sapiens

<220>
<221> SITE
<222> (46)
<223> Xaa equals stop translation

<400> 249
Met Tyr Leu Ile Leu Ser Trp Leu Phe Leu Cys Lys Leu Val Lys Cys
1               5                   10                  15

Tyr Phe Glu Ile Leu Leu Phe Ser Thr Ser Pro Gln Leu Leu Gln Trp
            20                  25                  30

Thr Val Ile Val Thr Tyr Cys Gly Pro Leu Leu Arg Phe Xaa
        35                  40                  45


<210> 250
<211> 54
<212> PRT
<213> Homo sapiens

<220>
<221> SITE
<222> (54)
<223> Xaa equals stop translation

<400> 250
Met Leu Val Phe Leu Leu Leu Phe Ser Thr Val Thr Val Leu Cys Leu
1               5                   10                  15

Lys Val Val Phe Ser Leu Lys Ala Val Ala Tyr Ile Val Lys Asn Glu
            20                  25                  30

Gly Leu Cys Leu Lys Phe Ile Ala Leu Gln Arg Val Val Ser Leu Lys

35                  40                  45

Ser Cys Thr Ile Lys Xaa
    50


<210> 251
<211> 57
<212> PRT
<213> Homo sapiens

<220>
<221> SITE
<222> (57)
<223> Xaa equals stop translation

<400> 251
Met Thr Phe Leu Leu Gln Trp Phe Pro Leu Gly Arg Ala Arg Val Val
 1               5                  10                  15

Gly Asp Leu Cys Gly Phe Ser Thr Gln Ile His Pro Gly Val Ser Arg
            20                  25                  30

Ala Gly Met Ala Asp Leu Glu Ser Pro Pro Phe Pro Arg Thr Cys Ser
        35                  40                  45

Val Pro Arg Ala Ala Asn Lys Gly Xaa
    50                  55


<210> 252
<211> 42
<212> PRT
<213> Homo sapiens

<220>
<221> SITE
<222> (42)
<223> Xaa equals stop translation

<400> 252
Met Phe Val Lys Tyr His Val Ile Met Val Ile Ile Phe Ile Phe Ile
 1               5                  10                  15

Leu Ile Thr Ser Asp Lys His Gly Glu Ile Ile Tyr Ile Lys Tyr Ile
            20                  25                  30

Asp Arg Val Ile Ile Thr Glu Arg Ile Xaa
        35                  40


<210> 253
<211> 161
<212> PRT
<213> Homo sapiens

<220>
<221> SITE

<222> (161)
<223> Xaa equals stop translation

<400> 253
Met Gln Arg Val Ser Gly Leu Leu Ser Trp Thr Leu Ser Arg Val Leu
1               5                   10                  15

Trp Leu Ser Gly Leu Ser Glu Pro Gly Ala Ala Arg Gln Pro Arg Ile
            20                  25                  30

Met Glu Glu Lys Ala Leu Glu Val Tyr Asp Leu Ile Arg Thr Ile Arg
        35                  40                  45

Asp Pro Glu Lys Pro Asn Thr Leu Glu Glu Leu Glu Val Val Ser Glu
    50                  55                  60

Ser Cys Val Glu Val Gln Glu Ile Asn Glu Glu Glu Tyr Leu Val Ile
65                  70                  75                  80

Ile Arg Phe Thr Pro Thr Val Pro His Cys Ser Leu Ala Thr Leu Ile
                85                  90                  95

Gly Leu Cys Leu Arg Val Lys Leu Gln Arg Cys Leu Pro Phe Lys His
            100                 105                 110

Lys Leu Glu Ile Tyr Ile Ser Glu Gly Thr His Ser Thr Glu Glu Asp
        115                 120                 125

Ile Asn Lys Gln Ile Asn Asp Lys Glu Arg Val Ala Ala Ala Met Glu
    130                 135                 140

Asn Pro Asn Leu Arg Glu Ile Val Glu Gln Cys Val Leu Glu Pro Asp
145                 150                 155                 160

Xaa

<210> 254
<211> 51
<212> PRT
<213> Homo sapiens

<220>
<221> SITE
<222> (51)
<223> Xaa equals stop translation

<400> 254
Met Leu Phe Phe Ser Leu Lys Glu Ser Leu Tyr Ile Phe His Thr Ala
1               5                   10                  15

Ile Leu Leu Val Val Cys Phe Ala Cys Ala Val Val Cys Gln Tyr Val
            20                  25                  30

Ile Val Arg Val Cys Ala Val Val Phe Cys Phe Ser Lys Ser Gln Ser
        35                  40                  45

Leu Ile Xaa
     50

<210> 255
<211> 279
<212> PRT
<213> Homo sapiens

<220>
<221> SITE
<222> (279)
<223> Xaa equals stop translation

<400> 255
Met Leu Ile Phe Gly Ala Ile Phe Gly Cys Leu Asp Pro Val Ala Thr
  1               5                  10                  15

Leu Ala Ala Val Met Thr Glu Lys Ser Pro Phe Thr Thr Pro Ile Gly
             20                  25                  30

Arg Lys Asp Glu Ala Asp Leu Ala Lys Ser Ala Leu Ala Met Ala Asp
         35                  40                  45

Ser Asp His Leu Thr Ile Tyr Asn Ala Tyr Leu Gly Trp Lys Lys Ala
     50                  55                  60

Arg Gln Glu Gly Gly Tyr Arg Ser Glu Ile Thr Tyr Cys Arg Arg Asn
 65                  70                  75                  80

Phe Leu Asn Arg Thr Ser Leu Leu Thr Leu Glu Asp Val Lys Gln Glu
                 85                  90                  95

Leu Ile Lys Leu Val Lys Ala Ala Gly Phe Ser Ser Ser Thr Thr Ser
            100                 105                 110

Thr Ser Trp Glu Gly Asn Arg Ala Ser Gln Thr Leu Ser Phe Gln Glu
        115                 120                 125

Ile Ala Leu Leu Lys Ala Val Leu Val Ala Gly Leu Tyr Asp Asn Val
    130                 135                 140

Gly Lys Ile Ile Tyr Thr Lys Ser Val Asp Val Thr Glu Lys Leu Ala
145                 150                 155                 160

Cys Ile Val Glu Thr Ala Gln Gly Lys Ala Gln Val His Pro Ser Ser
                165                 170                 175

Val Asn Arg Asp Leu Gln Thr His Gly Trp Leu Leu Tyr Gln Glu Lys
            180                 185                 190

Ile Arg Tyr Ala Arg Val Tyr Leu Arg Glu Thr Thr Leu Ile Thr Pro
        195                 200                 205

Phe Pro Val Leu Leu Phe Gly Gly Asp Ile Glu Val Gln His Arg Glu
    210                 215                 220

Arg Leu Leu Ser Ile Asp Gly Trp Ile Tyr Phe Gln Ala Pro Val Lys

225                 230                 235                 240

Ile Ala Val Ile Phe Lys Gln Leu Arg Val Leu Ile Asp Ser Val Leu
                245                 250                 255

Arg Lys Lys Leu Glu Asn Pro Lys Met Ser Leu Glu Met Thr Arg Phe
            260                 265                 270

Cys Arg Ser Leu Arg Asn Xaa
        275


<210> 256
<211> 69
<212> PRT
<213> Homo sapiens

<220>
<221> SITE
<222> (69)
<223> Xaa equals stop translation

<400> 256
Met Lys Val Leu Ser Trp Ile His Phe Ile Leu Ile Ser Leu His Phe
  1               5                  10                  15

Thr Ser Ser Leu Asp Pro Ser Ser Arg Gly Leu Gly Thr Phe Thr Asp
             20                  25                  30

Ala Leu Pro Asp Ser Arg Ala Lys Val Trp Glu Gly Glu Met Glu Glu
         35                  40                  45

Cys Pro Pro Val Cys Val Val Leu Cys Ala Thr Ala Thr Asp Ala Glu
     50                  55                  60

Gly Phe Ser Gly Xaa
65


<210> 257
<211> 122
<212> PRT
<213> Homo sapiens

<220>
<221> SITE
<222> (122)
<223> Xaa equals stop translation

<400> 257
Met Ile Met Ala Gln Lys Ile Gly Gly Leu Thr Trp Trp Ala Ile Met
  1               5                  10                  15

Phe Ile Ile Leu Phe Glu Ile Thr Gly Thr Ser Ser Ser Phe Leu Arg
             20                  25                  30

Ile Asn Ala Leu Pro His Phe Ser Met Asn Arg Cys Gly Glu Ala Tyr
         35                  40                  45

Phe Pro Phe Ser Tyr Leu Tyr Thr Ser Leu Gln Lys Gln Phe Leu Met
     50                  55                  60

Lys Val Ser Gly Ile Val Lys Asn Leu Arg Gly Asn Asp Asp Trp Arg
65                  70                  75                  80

Cys Phe Gly Val Phe Phe Cys Ile His Phe Leu Met Arg Lys Val Leu
                85                  90                  95

Asn Val Val Gln Val Arg Pro Asn Tyr Tyr Leu Thr Ile Ile Gly Arg
            100                 105                 110

Phe Tyr Val Ser Val Lys Val Phe Lys Xaa
        115                 120

<210> 258
<211> 59
<212> PRT
<213> Homo sapiens

<220>
<221> SITE
<222> (59)
<223> Xaa equals stop translation

<400> 258
Met Gly Lys Ile Cys Lys Asn Trp Val Ser Phe Leu Asp Asn Val Leu
  1               5                  10                  15

Leu Leu Ile Leu Phe Leu Tyr Gly Leu Cys Leu Gly Trp Leu Cys Ile
             20                  25                  30

Tyr His Gln Ser Tyr Ser Thr Ala Cys Ile Cys Val Val Thr Asp Ala
         35                  40                  45

Glu Ile Gln Gln Lys Ser Leu His Ser Ile Xaa
     50                  55

<210> 259
<211> 68
<212> PRT
<213> Homo sapiens

<220>
<221> SITE
<222> (68)
<223> Xaa equals stop translation

<400> 259
Met Leu Val Leu Leu Trp Leu Gly Trp Ile Ser Ser Lys Ser Met Leu
  1               5                  10                  15

Ala Ala Tyr Phe Val Ala Pro Lys Tyr Pro Leu Lys Leu Ala Leu Val
             20                  25                  30

Ser Glu Pro Glu Ser Ser Ser Leu Ile Leu Lys Phe Leu Ser Leu Lys
        35                  40                  45

Asp Phe Leu Cys Cys Tyr Thr Thr Lys Leu Ser Val Asn Pro Pro Leu
    50                  55                  60

Leu Asn Asp Xaa
65

<210> 260
<211> 46
<212> PRT
<213> Homo sapiens

<220>
<221> SITE
<222> (46)
<223> Xaa equals stop translation

<400> 260
Met Val Ser Phe His Phe Gln Cys Thr Ser Tyr Phe Val Arg Leu Phe
 1               5                  10                  15

Phe Gln Leu Gln Leu Phe Val Gly Leu Val Ile Val Leu Ala Leu Leu
            20                  25                  30

Ile Ser His Ser Leu Thr Tyr Ser Phe His Lys His Leu Xaa
        35                  40                  45

<210> 261
<211> 110
<212> PRT
<213> Homo sapiens

<400> 261
Phe Tyr Ile Ala Asp His Ser Phe Thr Ala Arg Pro Thr Leu Arg Met
 1               5                  10                  15

Phe Arg Ile Ser Ala Val Val Ala Thr Asp Lys Met Thr Phe Thr Ser
            20                  25                  30

Gly Gly Thr Leu Phe Gly Asp Gly Cys Ala Ser Ser Val Ala Gly Glu
        35                  40                  45

Val Met Asn Cys Gln Thr Val Leu Cys Ile Leu Trp Thr Pro Phe Val
    50                  55                  60

Phe Cys Pro Ser Ile Ala Val Ile Ile Ile Pro Cys Val Phe Thr Ser
65                  70                  75                  80

Lys Ala Leu Glu Ala Ile Trp Lys Trp Cys Arg Val Glu Arg Arg Pro
                85                  90                  95

His Ile Ile Glu Val Asp Val Leu Gly Lys Cys Pro Ala Phe
            100                 105                 110

<210> 262
<211> 25
<212> PRT
<213> Homo sapiens

<400> 262
Arg Pro Thr Leu Arg Met Phe Arg Ile Ser Ala Val Val Ala Thr Asp
1               5                   10                  15

Lys Met Thr Phe Thr Ser Gly Gly Thr
            20                  25


<210> 263
<211> 28
<212> PRT
<213> Homo sapiens

<400> 263
Pro Ser Ile Ala Val Ile Ile Ile Pro Cys Val Phe Thr Ser Lys Ala
1               5                   10                  15

Leu Glu Ala Ile Trp Lys Trp Cys Arg Val Glu Arg
            20                  25


<210> 264
<211> 20
<212> PRT
<213> Homo sapiens

<400> 264
Thr Ser Val Ser Phe His His Arg Tyr Lys Ser Ser Asp Arg Pro Ala
1               5                   10                  15

His Lys Val Ser
            20


<210> 265
<211> 115
<212> PRT
<213> Homo sapiens

<400> 265
Arg His Asn Asp Phe Asn Lys Leu Ser Tyr Thr Glu Cys Asn Asn Met
1               5                   10                  15

Asn Lys Arg Met Ala Lys Pro Glu Lys Lys Lys Gly Ser Val Lys Ser
            20                  25                  30

Ser Leu Gly Ile Phe Leu Gly Pro Asn Cys His Leu Ile Ser Ser Leu
        35                  40                  45

Phe Leu Phe Ser Val Ser Leu Tyr Pro Phe Ala Thr Gln Phe Pro Phe
    50                  55                  60

His Tyr Val Leu Ile Phe Ile Ile Gln Ala Phe Gly Leu Cys Leu Pro
65                  70                  75                  80

Leu Thr Glu Arg Gln Glu Ala Lys Ser Gly Leu Gly Gly Leu Cys Pro
                85                  90                  95

Asp Tyr Thr Trp Pro Cys Pro Cys Leu Leu Val Ser Cys Leu Ser Leu
            100                 105                 110

Leu Arg Leu
        115

<210> 266
<211> 114
<212> PRT
<213> Homo sapiens

<400> 266
Cys Glu Val Phe Ser Trp His Phe Pro Trp Ser Lys Leu Ser Pro His
1               5                   10                  15

Leu Phe Leu Val Ser Phe Leu Cys Ile Pro Leu Ser Leu Cys His Thr
            20                  25                  30

Val Ser Phe Ser Leu Cys Ser Asn Ile Tyr Asn Pro Gly Leu Arg Thr
        35                  40                  45

Met Leu Ala Pro His Arg Glu Thr Gly Gly Gln Val Trp Ala Gly Trp
    50                  55                  60

Ala Leu Ser Arg Leu His Val Ala Leu Pro Met Ser Leu Gly Val Leu
65                  70                  75                  80

Ser Leu Pro Ala Pro Thr Val Thr Val Val Arg Met Glu Gly Gly Asp
                85                  90                  95

Trp Lys Val Cys Glu Gln Leu Gly Gln Cys Thr Tyr Ser His Arg Met
            100                 105                 110

Thr Lys

<210> 267
<211> 23
<212> PRT
<213> Homo sapiens

<400> 267
Lys Arg Met Ala Lys Pro Glu Lys Lys Lys Gly Ser Val Lys Ser Ser
1               5                   10                  15

Leu Gly Ile Phe Leu Gly Pro
            20

<210> 268

<211> 31
<212> PRT
<213> Homo sapiens

<400> 268
Tyr Asn Pro Gly Leu Arg Thr Met Leu Ala Pro His Arg Glu Thr Gly
1               5                   10                  15

Gly Gln Val Trp Ala Gly Trp Ala Leu Ser Arg Leu His Val Ala
            20                  25                  30


<210> 269
<211> 251
<212> PRT
<213> Homo sapiens

<400> 269

<210> 270
<211> 23
<212> PRT
<213> Homo sapiens

<400> 270
Leu Pro Pro Tyr Pro Pro Gln Glu Ala Asn Arg Ser Ile Thr Ser Leu
1               5                   10                  15

Ser Val Ala Asp Thr Val Ser
            20

<210> 271
<211> 27
<212> PRT
<213> Homo sapiens

<400> 271
Thr Ala Lys Pro Ala Ala Pro Ser Phe Gly Val Leu Ser Asn Leu Pro
1               5                   10                  15

Leu Pro Ile Pro Thr Val Asp Ala Ser Ile Pro
            20                  25

<210> 272
<211> 25
<212> PRT
<213> Homo sapiens

<400> 272
Pro Asp Val Pro Asp Ala Phe Pro Glu Leu Ser Glu Leu Ser Val Ser
1               5                   10                  15

Gln Leu Thr Asp Met Asn Glu Gln Glu
            20                  25

<210> 273
<211> 29
<212> PRT
<213> Homo sapiens

<400> 273
Gln Phe Leu Thr Leu Pro Gln Leu Lys Gln Ile Ile Thr Asp Lys Asp
1               5                   10                  15

Asp Leu Val Lys Ser Ile Glu Glu Leu Ala Arg Lys Asn

20                  25

<210> 274
<211> 25
<212> PRT
<213> Homo sapiens

<400> 274
Arg Gln Thr Val Leu Asp Lys Tyr Glu Leu Leu Thr Gln Met Lys Ser
1               5                   10                  15

Thr Phe Glu Lys Lys Met Gln Arg Gln
            20                  25

<210> 275
<211> 28
<212> PRT
<213> Homo sapiens

<400> 275
Ala Ser Ala Leu Gln Ala Arg Leu Lys Val Ala Ala His Glu Ala Glu
1               5                   10                  15

Glu Glu Ser Asp Asn Ile Ala Glu Asp Phe Leu Glu
            20                  25

<210> 276
<211> 27
<212> PRT
<213> Homo sapiens

<400> 276
Met Glu Lys Arg Thr Ile Cys His Cys Arg Arg Ala Lys Glu Glu Lys
1               5                   10                  15

Leu Gln Gln Ala Ile Ala Met His Ser Gln Phe
            20                  25

<210> 277
<211> 69
<212> PRT
<213> Homo sapiens

<400> 277
Thr Arg Pro Val Phe Leu Ser Met Thr Pro Leu Lys Gly Ile Lys Ser
1               5                   10                  15

Val Ile Leu Pro Gln Val Phe Leu Cys Ala Tyr Met Ala Ala Phe Asn
            20                  25                  30

Ser Ile Asn Gly Asn Arg Ser Tyr Thr Cys Lys Pro Leu Glu Arg Ser
        35                  40                  45

Leu Leu Met Ala Gly Ala Val Ala Ser Ser Thr Phe Leu Gly Val Ile

50                  55                  60

Pro Gln Phe Val Gln
65

<210> 278
<211> 21
<212> PRT
<213> Homo sapiens

<400> 278
Pro Leu Lys Gly Ile Lys Ser Val Ile Leu Pro Gln Val Phe Leu Cys
1               5                   10                  15

Ala Tyr Met Ala Ala
            20

<210> 279
<211> 21
<212> PRT
<213> Homo sapiens

<400> 279
Ala Phe Asn Ser Ile Asn Gly Asn Arg Ser Tyr Thr Cys Lys Pro Leu
1               5                   10                  15

Glu Arg Ser Leu Leu
            20

<210> 280
<211> 129
<212> PRT
<213> Homo sapiens

<400> 280

<210> 281
<211> 30
<212> PRT
<213> Homo sapiens

<400> 281
Val Gly Pro Leu Leu Leu Met Thr Thr Glu Ser Trp Gly Pro Ser Cys
1               5                   10                  15

Ala Pro Ser Pro Ala Leu Leu Ser Gly His Thr Ala Ala Ser
            20                  25                  30

<210> 282
<211> 27
<212> PRT
<213> Homo sapiens

<400> 282
Glu Thr Asn Lys Val Glu Glu Gly Arg Trp Val Asp Val Thr Arg Ser
1               5                   10                  15

Leu Gly Asn Phe Asn Phe Arg Arg Lys Phe Phe
            20                  25

<210> 283
<211> 140
<212> PRT
<213> Homo sapiens

<400> 283

<210> 284
<211> 146
<212> PRT
<213> Homo sapiens

<400> 284

<210> 285
<211> 30
<212> PRT
<213> Homo sapiens

<400> 285
Ser Leu Arg Leu Lys Gly Pro Pro Ser Cys Gln Ala Pro Ala Ser His
1               5                   10                  15

Ser Ser Thr Leu Ser Ser His Cys Pro Cys Ser Leu Phe Ala
            20                  25                  30

<210> 286
<211> 30
<212> PRT
<213> Homo sapiens

<400> 286
Gln Gln Met Leu Gly Pro Ser Leu Cys Pro Gly His Ser Thr Ser Ala
1               5                   10                  15

His Gln His Tyr Gly Ala Tyr Val Leu Pro Arg Asp Leu Cys
            20                  25                  30


<210> 287
<211> 31
<212> PRT
<213> Homo sapiens

<400> 287
Asp Leu Gln Val Phe Val Ser Arg Asp Leu Pro His Ala Arg Pro Leu
1               5                   10                  15

Pro Leu Thr Ala Ala Pro Phe Pro Leu Ile Val Pro Val Pro Phe
            20                  25                  30


<210> 288
<211> 39
<212> PRT
<213> Homo sapiens

<400> 288
Ala Gln Val His Ile Ser Thr Met Gly Pro Met Ser Cys Pro Glu Thr
1               5                   10                  15

Ser Ala Pro Ser Cys Ser His Pro Gln Phe Arg Ala Arg Arg Pro Ser
            20                  25                  30

Arg Thr Pro Glu Ser Pro Val
        35


<210> 289
<211> 17
<212> PRT
<213> Homo sapiens

<400> 289
Gln Ala Pro Pro Arg Gln Thr Cys Lys Ser Ser Ser Gln Gly Thr Ser
1               5                   10                  15

Leu


<210> 290

<211> 314
<212> PRT
<213> Homo sapiens

<220>
<221> SITE
<222> (27)
<223> Xaa equals any of the naturally occurring L-amino acids

<220>
<221> SITE
<222> (111)
<223> Xaa equals any of the naturally occurring L-amino acids

<400> 290
Ala Ala Leu Arg Pro Ser Gly Ser Leu Ala Gly Pro Glu Trp Pro Trp
1               5                   10                  15

Gln His Trp Cys Gly Cys Trp Arg Glu His Xaa Val Lys Pro Gln Gln
            20                  25                  30

Val Asp Leu His Ser Ala Arg Leu Trp Ala Ala Pro Ala Ala Val Gly
        35                  40                  45

Pro Ala His Ala Gly Gly Ser Pro Gly Met Pro Pro Gly Gly Thr Ala
    50                  55                  60

Pro His Ala Arg Arg His Ser Leu Pro Ser Pro Thr Ala Gln Ser His
65                  70                  75                  80

Leu Trp His Val His Gly Leu Arg Gln Arg Gly Pro Lys Ala Val Pro
                85                  90                  95

Leu Asp Leu Ala Gln Leu Val Thr Thr Thr Thr Pro Leu Phe Xaa Leu
            100                 105                 110

Ala Leu Ser Ala Leu Leu Leu Gly Arg Arg His His Pro Leu Gln Leu
        115                 120                 125

Ala Ala Met Gly Pro Leu Cys Leu Gly Ala Ala Cys Ser Leu Ala Gly
    130                 135                 140

Glu Phe Arg Thr Pro Pro Thr Gly Cys Gly Phe Leu Leu Ala Ala Thr
145                 150                 155                 160

Cys Leu Arg Gly Leu Lys Ser Val Gln Gln Ser Ala Leu Leu Gln Glu
                165                 170                 175

Glu Arg Leu Asp Ala Val Thr Leu Leu Tyr Ala Thr Ser Leu Pro Ser
            180                 185                 190

Phe Cys Leu Leu Ala Gly Ala Ala Leu Val Leu Glu Ala Gly Val Ala
        195                 200                 205

Pro Pro Pro Thr Ala Gly Asp Ser Arg Leu Trp Ala Cys Ile Leu Leu
    210                 215                 220

Ser Cys Leu Leu Ser Val Leu Tyr Asn Leu Ala Ser Phe Ser Leu Leu

225                 230                 235                 240

Ala Leu Thr Ser Ala Leu Thr Val His Val Leu Gly Asn Leu Thr Val
                245                 250                 255

Val Gly Asn Leu Ile Leu Ser Arg Leu Leu Phe Gly Ser Arg Leu Ser
            260                 265                 270

Ala Leu Ser Tyr Val Gly Ile Ala Leu Thr Leu Ser Gly Met Phe Leu
        275                 280                 285

Tyr His Asn Cys Glu Phe Val Ala Ser Trp Ala Ala Arg Arg Gly Leu
    290                 295                 300

Trp Arg Arg Asp Gln Pro Ser Lys Gly Leu
305                 310


<210> 291
<211> 66
<212> PRT
<213> Homo sapiens

<220>
<221> SITE
<222> (28)
<223> Xaa equals any of the naturally occurring L-amino acids

<400> 291
Gly Gln Pro Ser Gly Pro Pro Ala Ala Trp Pro Gly Pro Ser Gly His
 1               5                  10                  15

Gly Ser Thr Gly Val Ala Ala Gly Gly Ser Thr Xaa Ser Ser Leu Asn
            20                  25                  30

Lys Trp Ile Phe Thr Val His Gly Phe Gly Arg Pro Leu Leu Leu Ser
        35                  40                  45

Ala Leu His Met Leu Val Ala Ala Leu Ala Cys His Arg Gly Ala Arg
    50                  55                  60

Arg Pro
65


<210> 292
<211> 21
<212> PRT
<213> Homo sapiens

<220>
<221> SITE
<222> (19)
<223> Xaa equals any of the naturally occurring L-amino acids

<400> 292
Trp Pro Gly Pro Ser Gly His Gly Ser Thr Gly Val Ala Ala Gly Gly
 1               5                  10                  15

Ser Thr Xaa Ser Ser
            20

<210> 293
<211> 26
<212> PRT
<213> Homo sapiens

<220>
<221> SITE
<222> (15)
<223> Xaa equals any of the naturally occurring L-amino acids

<400> 293
Glu Trp Pro Trp Gln His Trp Cys Gly Cys Trp Arg Glu His Xaa Val
  1               5                  10                  15

Lys Pro Gln Gln Val Asp Leu His Ser Ala
            20                  25

<210> 294
<211> 28
<212> PRT
<213> Homo sapiens

<400> 294
Gln Gln Ser Ala Leu Leu Gln Glu Glu Arg Leu Asp Ala Val Thr Leu
  1               5                  10                  15

Leu Tyr Ala Thr Ser Leu Pro Ser Phe Cys Leu Leu
            20                  25

<210> 295
<211> 27
<212> PRT
<213> Homo sapiens

<400> 295
Ala Cys Ile Leu Leu Ser Cys Leu Leu Ser Val Leu Tyr Asn Leu Ala
  1               5                  10                  15

Ser Phe Ser Leu Leu Ala Leu Thr Ser Ala Leu
            20                  25

<210> 296
<211> 21
<212> PRT
<213> Homo sapiens

<400> 296
Ser Leu Asn Lys Trp Ile Phe Thr Val His Gly Phe Gly Arg Pro Leu
  1               5                  10                  15

Leu Leu Ser Ala Leu
            20

<210> 297
<211> 17
<212> PRT
<213> Homo sapiens

<400> 297
Lys Ser Thr Leu Ser Ala Ala Val Val Ala Thr Ile Leu Arg Thr Leu
1               5                   10                  15

Ala

<210> 298
<211> 100
<212> PRT
<213> Homo sapiens

<400> 298
Gly Asp His Ser Glu Gln Cys Leu Ile Lys Glu Met Gly Ala Arg Glu
1               5                   10                  15

Arg Arg Phe Cys Lys Ala Arg Gly Tyr Arg Asp Thr Gly Arg Glu Ala
            20                  25                  30

Gln Ala Lys Ala Gly Gly Arg Arg Gly Ser Gln Trp Asn Glu Ser Gln
        35                  40                  45

Cys Ser Ser Gln Arg Pro Arg Pro Ala Lys Glu Val Arg Lys Thr Arg
    50                  55                  60

Pro Arg Ala Gly Val Gly Arg Gly Pro Ala Leu Leu Gln Leu Ser Leu
65                  70                  75                  80

Leu Gln Gln Val Val Leu Tyr Val Arg Pro Ser Leu Arg Leu Val Trp
                85                  90                  95

Leu Lys Ala Ser
            100

<210> 299
<211> 84
<212> PRT
<213> Homo sapiens

<400> 299
Met Glu Arg Gly Glu Tyr Gly Gly Trp Gly Thr Tyr Gly Ser Leu Asp
1               5                   10                  15

Leu Gly Ser Gln Leu Cys Thr Val Arg Ser Ser Gly Pro Cys Gly Ser
            20                  25                  30

Leu His Trp Gly Gln His Arg Ser Pro Ile Ser Gly Pro Asp Pro Asn

35                    40                    45

Pro Ser Ser Ser Arg Gly Gln Gln Ser Ile Gly Ser Lys Val Gly Ser
    50                    55                    60

Pro Ser Arg Ser Gln Trp Arg Ser Trp Lys Glu Val Gly Arg Asp Pro
65                    70                    75                    80

Glu Lys Gly Glu

<210> 300
<211> 23
<212> PRT
<213> Homo sapiens

<400> 300
Gln Ala Lys Ala Gly Gly Arg Arg Gly Ser Gln Trp Asn Glu Ser Gln
1               5                   10                  15

Cys Ser Ser Gln Arg Pro Arg
            20

<210> 301
<211> 26
<212> PRT
<213> Homo sapiens

<400> 301
Val Gly Arg Gly Pro Ala Leu Leu Gln Leu Ser Leu Leu Gln Gln Val
1               5                   10                  15

Val Leu Tyr Val Arg Pro Ser Leu Arg Leu
            20                  25

<210> 302
<211> 22
<212> PRT
<213> Homo sapiens

<400> 302
Tyr Gly Ser Leu Asp Leu Gly Ser Gln Leu Cys Thr Val Arg Ser Ser
1               5                   10                  15

Gly Pro Cys Gly Ser Leu
            20

<210> 303
<211> 20
<212> PRT
<213> Homo sapiens

<400> 303
Lys Val Gly Ser Pro Ser Arg Ser Gln Trp Arg Ser Trp Lys Glu Val

1                 5                   10                  15

Gly Arg Asp Pro
            20


<210> 304
<211> 214
<212> PRT
<213> Homo sapiens

<220>
<221> SITE
<222> (18)
<223> Xaa equals any of the naturally occurring L-amino acids

<400> 304
Met Pro Gln Ser Leu Ser Ser Leu Ala Ser Ser Ser Ser Ser Phe Gln
1               5                   10                  15

Arg Xaa Lys Pro Cys Phe Gly Lys Lys Asn Asp Gly Glu Asn Gln Glu
            20                  25                  30

His Ser Leu Gly Thr Glu Pro Ile Ile Thr Trp Lys Asp Phe Gln Lys
        35                  40                  45

Thr Met Pro Trp Glu Ile Val Ile Leu Val Gly Gly Gly Tyr Ala Leu
    50                  55                  60

Ala Ser Gly Ser Lys Ser Ser Gly Leu Ser Thr Trp Ile Gly Asn Gln
65                  70                  75                  80

Met Leu Ser Leu Ser Ser Leu Pro Pro Trp Ala Val Thr Leu Leu Ala
                85                  90                  95

Cys Ile Leu Val Ser Ile Val Thr Glu Phe Val Ser Asn Pro Ala Thr
            100                 105                 110

Ile Thr Ile Phe Leu Pro Ile Leu Cys Ser Leu Ser Glu Thr Leu His
        115                 120                 125

Ile Asn Pro Leu Tyr Thr Leu Ile Pro Val Thr Met Cys Ile Ser Phe
    130                 135                 140

Ala Val Met Leu Pro Val Gly Asn Pro Pro Asn Ala Ile Val Phe Ser
145                 150                 155                 160

Tyr Gly His Cys Gln Ile Lys Asp Met Val Lys Ala Gly Leu Gly Val
                165                 170                 175

Asn Val Ile Gly Leu Val Ile Val Met Val Ala Ile Asn Thr Trp Gly
            180                 185                 190

Val Ser Leu Phe His Leu Asp Thr Tyr Pro Ala Trp Ala Arg Val Ser
        195                 200                 205

Asn Ile Thr Asp Gln Ala
    210

<210> 305
<211> 23
<212> PRT
<213> Homo sapiens

<400> 305
Asn Asp Gly Glu Asn Gln Glu His Ser Leu Gly Thr Glu Pro Ile Ile
1               5                   10                  15

Thr Trp Lys Asp Phe Gln Lys
            20


<210> 306
<211> 24
<212> PRT
<213> Homo sapiens

<400> 306
Ile Gly Asn Gln Met Leu Ser Leu Ser Ser Leu Pro Pro Trp Ala Val
1               5                   10                  15

Thr Leu Leu Ala Cys Ile Leu Val
            20


<210> 307
<211> 27
<212> PRT
<213> Homo sapiens

<400> 307
Ala Thr Ile Thr Ile Phe Leu Pro Ile Leu Cys Ser Leu Ser Glu Thr
1               5                   10                  15

Leu His Ile Asn Pro Leu Tyr Thr Leu Ile Pro
            20                  25


<210> 308
<211> 26
<212> PRT
<213> Homo sapiens

<400> 308
Leu Pro Val Gly Asn Pro Pro Asn Ala Ile Val Phe Ser Tyr Gly His
1               5                   10                  15

Cys Gln Ile Lys Asp Met Val Lys Ala Gly
            20                  25


<210> 309
<211> 29
<212> PRT
<213> Homo sapiens

<400> 309
Leu Val Ile Val Met Val Ala Ile Asn Thr Trp Gly Val Ser Leu Phe
1               5                   10                  15

His Leu Asp Thr Tyr Pro Ala Trp Ala Arg Val Ser Asn
            20                  25


<210> 310
<211> 133
<212> PRT
<213> Homo sapiens

<220>
<221> SITE
<222> (44)
<223> Xaa equals any of the naturally occurring L-amino acids

<220>
<221> SITE
<222> (46)
<223> Xaa equals any of the naturally occurring L-amino acids

<400> 310
Glu Thr Cys Pro Ser Asn Gly Ile Glu Leu Arg Gln Ala Pro Thr Ser
1               5                   10                  15

Leu Tyr Ile Leu Leu Leu His Ile Gln Pro Thr Pro Thr His Pro Met
            20                  25                  30

Leu Gly Arg Ser Tyr Val Leu Pro Ala Phe Ser Xaa Asn Xaa Glu His
        35                  40                  45

Gly Gly Leu Pro Asn Gln Ile Pro Lys Gly Asp Arg Asn Gly Asn Ile
    50                  55                  60

Arg His Ser Arg Ile Thr Phe Pro Cys Ser Ser Ser Thr Leu Gln Pro
65                  70                  75                  80

Glu Ser His Leu Gly Phe Ile Arg Ser Lys Leu His Gly Leu Val Arg
                85                  90                  95

Pro Gly Lys Asp Leu Arg Gly Arg Arg Ser Leu Gln Leu Ser Lys His
            100                 105                 110

Ser Leu Ser Thr Cys Tyr Met Leu Arg Trp Glu Thr Tyr Lys Gln Val
        115                 120                 125

Ser Tyr Thr Ala Val
    130


<210> 311
<211> 106
<212> PRT
<213> Homo sapiens

<400> 311
Gln Arg His Gln Glu Asn Asp Lys Arg Asn Val His Arg Phe Leu His
1               5                   10                  15

Thr Cys Val His Met Pro Met Cys Thr His Thr His Thr Gln Ala Val
            20                  25                  30

Leu Ser Thr Trp Glu Gly Gln Phe Ser Asn Val Ala Ser Phe Thr Ser
        35                  40                  45

Leu Lys Arg Ile Pro Leu Ser Ile Ile Tyr Ile His Ser Ser His Ser
    50                  55                  60

Pro Arg Arg Phe Val Lys Val Cys Gln Leu Arg Gln Glu Lys Ala Leu
65                  70                  75                  80

Glu Leu Thr Glu Val Tyr Val Ser Ala Ser Leu Lys Leu Gln Leu Tyr
                85                  90                  95

His Leu His Cys His Phe His Thr Ala Val
            100                 105


<210> 312
<211> 24
<212> PRT
<213> Homo sapiens

<400> 312
Arg Gln Ala Pro Thr Ser Leu Tyr Ile Leu Leu Leu His Ile Gln Pro
1               5                   10                  15

Thr Pro Thr His Pro Met Leu Gly
            20


<210> 313
<211> 25
<212> PRT
<213> Homo sapiens

<400> 313
Ser His Leu Gly Phe Ile Arg Ser Lys Leu His Gly Leu Val Arg Pro
1               5                   10                  15

Gly Lys Asp Leu Arg Gly Arg Arg Ser
            20                  25


<210> 314
<211> 22
<212> PRT
<213> Homo sapiens

<400> 314
Arg Asn Val His Arg Phe Leu His Thr Cys Val His Met Pro Met Cys
1               5                   10                  15

Thr His Thr His Thr Gln
            20

<210> 315
<211> 25
<212> PRT
<213> Homo sapiens

<400> 315
Gln Leu Arg Gln Glu Lys Ala Leu Glu Leu Thr Glu Val Tyr Val Ser
1               5                   10                  15

Ala Ser Leu Lys Leu Gln Leu Tyr His
            20                  25

<210> 316
<211> 31
<212> PRT
<213> Homo sapiens

<400> 316

<210> 317
<211> 296
<212> PRT
<213> Homo sapiens

<400> 317
Leu Ser Lys Gly Asn Arg Ile Met Ala Ala Asp Asp Asp Asn Gly Asp
1               5                   10                  15

Gly Thr Ser Leu Phe Asp Val Phe Ser Ala Ser Pro Leu Lys Asn Asn
            20                  25                  30

Asp Glu Gly Ser Leu Asp Ile Tyr Ala Gly Leu Asp Ser Ala Val Ser
        35                  40                  45

Asp Ser Ala Ser Lys Ser Cys Val Pro Ser Arg Asn Cys Leu Asp Leu
    50                  55                  60

Tyr Glu Glu Ile Leu Thr Glu Glu Gly Thr Ala Lys Glu Ala Thr Tyr
65                  70                  75                  80

Asn Asp Leu Gln Val Glu Tyr Gly Lys Cys Gln Leu Gln Met Lys Glu
                85                  90                  95

Leu Met Lys Lys Phe Lys Glu Ile Gln Thr Gln Asn Phe Ser Leu Ile
            100                 105                 110

Asn Glu Asn Gln Ser Leu Lys Lys Asn Ile Ser Ala Leu Ile Lys Thr

115                 120                 125

Ala Arg Val Glu Ile Asn Arg Lys Asp Glu Glu Ile Ser Asn Leu His
    130                 135                 140

Gln Lys Ile Val Leu Ser Phe His Ile Phe Glu Ile Ile Ile Lys Leu
145                 150                 155                 160

Gln Gly His Leu Ile Gln Leu Lys Gln Lys Ile Leu Asn Leu Asp Leu
                165                 170                 175

His Ile Trp Met Ile Val Gln Arg Leu Ile Thr Arg Ala Lys Ser Asp
            180                 185                 190

Val Ser Lys Asp Val His His Ser Thr Ser Leu Pro Asn Leu Glu Lys
        195                 200                 205

Glu Gly Lys Pro His Ser Asp Lys Arg Ser Thr Ser His Leu Pro Thr
    210                 215                 220

Ser Val Glu Lys His Cys Thr Asn Gly Val Trp Ser Arg Ser His Tyr
225                 230                 235                 240

Gln Val Gly Glu Gly Ser Ser Asn Glu Asp Ser Arg Arg Gly Arg Lys
                245                 250                 255

Asp Ile Arg His Ser Gln Phe Asn Arg Gly Thr Glu Arg Val Arg Lys
            260                 265                 270

Asp Leu Ser Thr Gly Cys Gly Asp Gly Glu Pro Arg Ile Leu Glu Ala
        275                 280                 285

Ser Gln Arg Leu Gln Gly Thr Ser
    290                 295


<210> 318
<211> 27
<212> PRT
<213> Homo sapiens

<400> 318
Asn Arg Ile Met Ala Ala Asp Asp Asp Asn Gly Asp Gly Thr Ser Leu
1               5                   10                  15

Phe Asp Val Phe Ser Ala Ser Pro Leu Lys Asn
            20                  25


<210> 319
<211> 23
<212> PRT
<213> Homo sapiens

<400> 319
Cys Leu Asp Leu Tyr Glu Glu Ile Leu Thr Glu Glu Gly Thr Ala Lys
1               5                   10                  15

Glu Ala Thr Tyr Asn Asp Leu
            20

<210> 320
<211> 26
<212> PRT
<213> Homo sapiens

<400> 320
Asp Glu Glu Ile Ser Asn Leu His Gln Lys Ile Val Leu Ser Phe His
  1               5                  10                  15

Ile Phe Glu Ile Ile Ile Lys Leu Gln Gly
            20                  25

<210> 321
<211> 22
<212> PRT
<213> Homo sapiens

<400> 321
Glu Lys Glu Gly Lys Pro His Ser Asp Lys Arg Ser Thr Ser His Leu
  1               5                  10                  15

Pro Thr Ser Val Glu Lys
            20

<210> 322
<211> 26
<212> PRT
<213> Homo sapiens

<400> 322
Thr Glu Arg Val Arg Lys Asp Leu Ser Thr Gly Cys Gly Asp Gly Glu
  1               5                  10                  15

Pro Arg Ile Leu Glu Ala Ser Gln Arg Leu
            20                  25

<210> 323
<211> 115
<212> PRT
<213> Homo sapiens

<400> 323
Lys Ser Tyr Phe Arg Thr Met Gly Gly Thr Lys Arg Gly Ile Lys Lys
  1               5                  10                  15

Leu Val Asn Val Cys Leu Lys His Pro Lys Asn Thr Ser Leu Ser Gln
            20                  25                  30

Gln Leu Val Phe Ala Lys Ile Asn Lys Ile Leu Ile Ser Lys Thr Thr
        35                  40                  45

Lys Ser Thr Asn Leu Lys Gly Leu Lys Cys Leu Pro Pro Leu Ser Val
    50                  55                  60

Ser Ile His Pro Thr Phe Ile Tyr Tyr Lys His Asn Thr Thr Leu Arg
65                  70                  75                  80

Ile Val Phe Gly Thr Tyr Phe Asp Phe Phe Pro Tyr Arg Lys Asn Lys
                85                  90                  95

Asp Gln Ala Phe Glu Gly Glu Asp Trp Glu Ser Ser Leu Asn Val Ser
            100                 105                 110

Asp Ala Trp
        115


<210> 324
<211> 22
<212> PRT
<213> Homo sapiens

<400> 324
Thr Lys Arg Gly Ile Lys Lys Leu Val Asn Val Cys Leu Lys His Pro
 1               5                  10                  15

Lys Asn Thr Ser Leu Ser
            20


<210> 325
<211> 26
<212> PRT
<213> Homo sapiens

<400> 325
Ser Ile His Pro Thr Phe Ile Tyr Tyr Lys His Asn Thr Thr Leu Arg
 1               5                  10                  15

Ile Val Phe Gly Thr Tyr Phe Asp Phe Phe
            20                  25


<210> 326
<211> 70
<212> PRT
<213> Homo sapiens

<220>
<221> SITE
<222> (9)
<223> Xaa equals any of the naturally occurring L-amino acids

<220>
<221> SITE
<222> (44)
<223> Xaa equals any of the naturally occurring L-amino acids

<220>

<221> SITE
<222> (45)
<223> Xaa equals any of the naturally occurring L-amino acids

<400> 326
Gln Arg Pro His Pro Gln Pro Trp Xaa Pro Met Thr Leu Met Gly Thr
1               5                   10                  15

Gly Ile Pro Val Phe Ala His Lys Met Leu Pro Phe Asp Pro Pro Cys
            20                  25                  30

His Leu Ser Cys Thr His Ile Asn Pro Lys Pro Xaa Xaa Pro Gln Gly
        35                  40                  45

Asp Glu Gln Lys Ser Gln Gly Thr Glu Glu Trp Cys Asp Arg Glu Gly
    50                  55                  60

Lys Lys Arg Arg Ser Ile
65                  70


<210> 327
<211> 21
<212> PRT
<213> Homo sapiens

<400> 327
Pro Met Thr Leu Met Gly Thr Gly Ile Pro Val Phe Ala His Lys Met
1               5                   10                  15

Leu Pro Phe Asp Pro
            20


<210> 328
<211> 21
<212> PRT
<213> Homo sapiens

<220>
<221> SITE
<222> (15)
<223> Xaa equals any of the naturally occurring L-amino acids

<220>
<221> SITE
<222> (16)
<223> Xaa equals any of the naturally occurring L-amino acids

<400> 328
Pro Pro Cys His Leu Ser Cys Thr His Ile Asn Pro Lys Pro Xaa Xaa
1               5                   10                  15

Pro Gln Gly Asp Glu
            20


<210> 329

<211> 21
<212> PRT
<213> Homo sapiens

<400> 329
Glu Gln Lys Ser Gln Gly Thr Glu Glu Trp Cys Asp Arg Glu Gly Lys
1               5                   10                  15

Lys Arg Arg Ser Ile
            20


<210> 330
<211> 70
<212> PRT
<213> Homo sapiens

<220>
<221> SITE
<222> (64)
<223> Xaa equals any of the naturally occurring L-amino acids

<220>
<221> SITE
<222> (65)
<223> Xaa equals any of the naturally occurring L-amino acids

<400> 330
Asp Glu Trp Gly Ala Gly Arg Arg Met Glu Trp Glu Asp Asn Leu Pro
1               5                   10                  15

Leu Glu Phe Ser Cys Pro Val Thr Lys Leu Leu Ser Val Pro Ser Trp
            20                  25                  30

Thr Pro Leu Asp Ala Gln Met Leu Leu Leu Phe Phe Pro Ser Leu Ser
        35                  40                  45

His His Ser Ser Val Pro Trp Leu Phe Cys Ser Ser Pro Cys Gly Xaa
    50                  55                  60

Xaa Gly Leu Gly Phe Ile
65                  70


<210> 331
<211> 21
<212> PRT
<213> Homo sapiens

<400> 331
Glu Trp Glu Asp Asn Leu Pro Leu Glu Phe Ser Cys Pro Val Thr Lys
1               5                   10                  15

Leu Leu Ser Val Pro
            20


<210> 332

<211> 21
<212> PRT
<213> Homo sapiens

<400> 332
Pro Ser Trp Thr Pro Leu Asp Ala Gln Met Leu Leu Leu Phe Phe Pro
1               5                   10                  15

Ser Leu Ser His His
            20


<210> 333
<211> 21
<212> PRT
<213> Homo sapiens

<220>
<221> SITE
<222> (15)
<223> Xaa equals any of the naturally occurring L-amino acids

<220>
<221> SITE
<222> (16)
<223> Xaa equals any of the naturally occurring L-amino acids

<400> 333
His Ser Ser Val Pro Trp Leu Phe Cys Ser Ser Pro Cys Gly Xaa Xaa
1               5                   10                  15

Gly Leu Gly Phe Ile
            20


<210> 334
<211> 15
<212> PRT
<213> Homo sapiens

<400> 334
Gln Gly Leu Ser His Ile Phe Trp Met Asn Glu Gln Thr Leu Lys
1               5                   10                  15


<210> 335
<211> 32
<212> PRT
<213> Homo sapiens

<400> 335
Thr Leu Val Cys Leu Gly Val Ser Ser Glu Glu Gly Ser Cys Pro Arg
1               5                   10                  15

Asp Val Thr Gly Pro Gly Cys Cys Phe Ser Leu Thr Leu Thr Gly Phe
            20                  25                  30

<210> 336
<211> 233
<212> PRT
<213> Homo sapiens

<220>
<221> SITE
<222> (57)
<223> Xaa equals any of the naturally occurring L-amino acids

<220>
<221> SITE
<222> (62)
<223> Xaa equals any of the naturally occurring L-amino acids

<220>
<221> SITE
<222> (78)
<223> Xaa equals any of the naturally occurring L-amino acids

<220>
<221> SITE
<222> (79)
<223> Xaa equals any of the naturally occurring L-amino acids

<220>
<221> SITE
<222> (80)
<223> Xaa equals any of the naturally occurring L-amino acids

<220>
<221> SITE
<222> (231)
<223> Xaa equals any of the naturally occurring L-amino acids

<400> 336
Ala Asp Leu Ile Val Leu Trp His His His Pro Leu Trp Pro Gln His
1               5                   10                  15

Leu Ala Leu Pro Ser Ser Gly Ala Ser His Asp His Val Glu Leu Thr
            20                  25                  30

Val Tyr Pro Lys Thr Val Ala Ala Ser Trp Leu Leu Glu Leu Ser Arg
        35                  40                  45

Pro Pro Ile Phe Cys Leu Phe Thr Xaa Pro Ala Leu Thr Xaa His Gly
    50                  55                  60

Leu Asp Arg Val Ala Ala Leu Val Glu Cys Thr Ile Trp Xaa Xaa Xaa
65                  70                  75                  80

Gly Met Trp Tyr Arg Arg Arg Tyr Ser Cys Cys Gln Phe Arg Asp Arg
                85                  90                  95

Ser Ile Arg Asp Val Phe Pro Glu Ala Val Met Leu Gln Gln His Leu

            100                 105                 110

Arg His Leu Ala Val Ala Thr Tyr Arg Cys Arg Arg Arg Ser Pro Cys
        115                 120                 125

Lys Ala Pro Thr Val Glu Glu Ala Glu Gly Gly Lys Pro Arg Ala Val
    130                 135                 140

Pro Ser Gly Thr Gly Phe Gln Lys His Gly Gln Glu Pro Gly Gly Ser
145                 150                 155                 160

Thr Ser Pro His Trp Phe Trp Gly His Leu Gln Leu Leu Val Leu Ser
                165                 170                 175

Val Asn Asn Arg Gln Leu Phe Val Gln Gly Arg Ala Gly Tyr Leu Glu
            180                 185                 190

Met Thr Gly Leu Pro Cys Pro Lys Leu Leu Leu Thr Leu Leu Arg Gly
        195                 200                 205

Leu Thr Pro Gly Val Gly His Gly Leu Cys Ala Tyr Arg Arg Gly Cys
    210                 215                 220

Leu Ala Trp Arg Leu Asp Xaa Ala Ser
225                 230

<210> 337
<211> 176
<212> PRT
<213> Homo sapiens

<220>
<221> SITE
<222> (70)
<223> Xaa equals any of the naturally occurring L-amino acids

<220>
<221> SITE
<222> (71)
<223> Xaa equals any of the naturally occurring L-amino acids

<220>
<221> SITE
<222> (92)
<223> Xaa equals any of the naturally occurring L-amino acids

<400> 337
Ile Leu Trp Arg Gln Ala Pro Glu Ala Pro His Cys Ser Gln Asp Ser
1               5                   10                  15

Val Ser Ser Ser Pro Arg Leu Gln Glu Asp Leu Ala His Val Thr Gln
            20                  25                  30

Val Thr Arg His Pro His Phe Arg Ser Leu Pro Ser Ala Trp Cys Ser
        35                  40                  45

His Ser Ser Leu Leu Pro Val Ser Leu Pro Arg His Ala Leu Ala Thr

50                  55                  60

Lys Ser Pro Asn Met Xaa Xaa Ser Ser Pro Ile Leu His Leu Ile Gln
65                  70                  75                  80

Phe Thr Gly Gln Ile Ser Ser Pro Leu Gly Gly Xaa Val Gln Pro Pro
                85                  90                  95

Gly Gln Thr Ala Ser Pro Ile Cys Thr Gln Pro Met Ser His Pro Arg
            100                 105                 110

Arg Gln Ala Ser Gln Gln Cys Glu Gln Gln Leu Trp Thr Gly Gln Thr
        115                 120                 125

Ser His Leu Gln Ile Pro Cys Pro Ala Leu Asn Lys Glu Leu Pro Val
    130                 135                 140

Val Asp Thr Gln Asp Lys Glu Leu Gln Met Ser Pro Glu Pro Met Trp
145                 150                 155                 160

Gly Cys Gly Pro Ser Arg Leu Leu Pro Met Leu Leu Glu Ser Cys Ala
                165                 170                 175

<210> 338
<211> 34
<212> PRT
<213> Homo sapiens

<400> 338
Met Leu Gln Gln His Leu Arg His Leu Ala Val Ala Thr Tyr Arg Cys
1               5                   10                  15

Arg Arg Arg Ser Pro Cys Lys Ala Pro Thr Val Glu Glu Ala Glu Gly
            20                  25                  30

Gly Lys

<210> 339
<211> 29
<212> PRT
<213> Homo sapiens

<400> 339
Val Thr Gln Val Thr Arg His Pro His Phe Arg Ser Leu Pro Ser Ala
1               5                   10                  15

Trp Cys Ser His Ser Ser Leu Leu Pro Val Ser Leu Pro
            20                  25

<210> 340
<211> 28

<212> PRT
<213> Homo sapiens

<400> 340
Gly Gln Thr Ala Ser Pro Ile Cys Thr Gln Pro Met Ser His Pro Arg
1               5                   10                  15

Arg Gln Ala Ser Gln Gln Cys Glu Gln Gln Leu Trp
            20                  25


<210> 341
<211> 79
<212> PRT
<213> Homo sapiens

<400> 341
Phe Ile Thr Leu Arg Leu Gly Pro Lys Asn Met Ala Gly Val Leu Trp
1               5                   10                  15

Arg His Ser Asn Leu Gln Thr Pro His Tyr Ile Ser Trp Cys Pro Leu
            20                  25                  30

Leu Asn Tyr Arg Glu Thr Gly Asn Cys Leu Leu His Val Ser Gly Phe
        35                  40                  45

Leu Asn Ser Arg Leu Leu Ala Asn Cys Ser Gly Glu Ala Ser Gly Lys
    50                  55                  60

Val Ile Gln Thr Leu Leu Trp Pro Gly Glu Ile Ser Ala Val Ala
65                  70                  75


<210> 342
<211> 82
<212> PRT
<213> Homo sapiens

<400> 342
Lys Ile Arg Thr Phe Leu Phe Ser Gly His Arg Leu Phe Ser Thr Gln
1               5                   10                  15

Gly Gln Ser Leu Thr Val Lys Ala His Thr Ala Phe Met Leu Ile Val
            20                  25                  30

Lys Asn Leu Arg Tyr Phe Ile Ala Phe Lys Phe Leu Met Gly Ile Ser
        35                  40                  45

Asp Ser Ser Glu Ile Gly Leu Val Met Gln Pro Leu Gln Lys Pro His
    50                  55                  60

Thr Val Ile Leu Ile Arg Gly Ile Glu Phe Leu Ser Pro Gly Gly Val
65                  70                  75                  80

Leu Pro

<210> 343
<211> 26
<212> PRT
<213> Homo sapiens

<400> 343
Met Ala Gly Val Leu Trp Arg His Ser Asn Leu Gln Thr Pro His Tyr
1               5                   10                  15

Ile Ser Trp Cys Pro Leu Leu Asn Tyr Arg
            20                  25


<210> 344
<211> 29
<212> PRT
<213> Homo sapiens

<400> 344
Tyr Phe Ile Ala Phe Lys Phe Leu Met Gly Ile Ser Asp Ser Ser Glu
1               5                   10                  15

Ile Gly Leu Val Met Gln Pro Leu Gln Lys Pro His Thr
            20                  25


<210> 345
<211> 31
<212> PRT
<213> Homo sapiens

<400> 345
Asp Val Leu Leu Pro Leu Leu Tyr Leu Leu Val Arg Lys His Ile Asn
1               5                   10                  15

Arg Ala Gly Ile Gly Asn Thr Phe Gln Gly Gly Ala Asn Cys Ile
            20                  25                  30


<210> 346
<211> 99
<212> PRT
<213> Homo sapiens

<400> 346
Met Cys Cys Cys Leu Cys Cys Thr Ser Trp Ser Gly Ser Thr Ser Thr
1               5                   10                  15

Glu Arg Val Ser Gly Thr Arg Phe Arg Glu Val Pro Thr Ala Ser Cys
            20                  25                  30

Ser Ser Ser Ala Pro Ala Pro Ser Glu Leu Gly Ser Ser Leu Ser Val
        35                  40                  45

Ala Ala Ala Ala Leu Leu Ser Leu Pro Pro Arg Ala Arg Leu Ala Leu
    50                  55                  60

Pro Arg Leu Pro Arg Leu Pro Ser Gln Glu Asn Leu Arg Asn Pro Lys

65                  70                  75                  80

Gly Pro Gln Gly Asn Phe Gln Ala Pro Gly Ala Phe Val Leu Ser Ser
                85                  90                  95

Ser Val Ala

<210> 347
<211> 216
<212> PRT
<213> Homo sapiens

<220>
<221> SITE
<222> (108)
<223> Xaa equals any of the naturally occurring L-amino acids

<220>
<221> SITE
<222> (114)
<223> Xaa equals any of the naturally occurring L-amino acids

<220>
<221> SITE
<222> (155)
<223> Xaa equals any of the naturally occurring L-amino acids

<400> 347
Cys Ala Ala Ala Ser Ala Val Pro Pro Gly Pro Glu Ala His Gln Gln
  1               5                  10                  15

Ser Gly Tyr Arg Glu His Val Ser Gly Arg Cys Gln Leu His His Val
             20                  25                  30

Arg Pro Leu His Pro Arg Arg Pro Asn Ser Ala Leu Leu Ser Leu Leu
         35                  40                  45

Leu Leu Leu Leu Phe Ser Ala Ser His Gln Glu Pro Gly Trp His Ser
     50                  55                  60

Gln Gly Ser Arg Ala Phe Gln Ala Arg Arg Ile Ser Gly Ile Pro Arg
 65                  70                  75                  80

Asp Pro Arg Gly Thr Ser Lys His Leu Glu Leu Leu Ser Phe Leu Val
                85                  90                  95

Leu Trp His Arg Cys Cys Leu Pro Gly Gly Arg Xaa Phe Cys Glu Ser
            100                 105                 110

Leu Xaa Gln Gly Arg Ser Ala Cys Leu Leu His Gln Lys Pro Pro Leu
        115                 120                 125

Leu Met Leu Ser Ala Pro Leu Gly Glu Gln Leu Pro Thr Gln Leu Leu
    130                 135                 140

Leu Pro Pro Arg Ser Ser Gly Ser Lys Phe Xaa Arg Tyr Gln Arg Pro

145                 150                 155                 160

Gly Pro Arg Val Gly Val His Leu His Lys Gly Ser Ser Glu Ile Arg
                165                 170                 175

Glu Ala Gly Gly Pro Gln Leu Trp Pro Gln Cys Pro His Pro Val Asp
            180                 185                 190

Leu Asp Val Leu Arg Thr Thr Gln His Cys Leu Gln Ser Glu Gly Pro
        195                 200                 205

Thr Ser Val His Leu Ser Ser Val
    210                 215


<210> 348
<211> 147
<212> PRT
<213> Homo sapiens

<220>
<221> SITE
<222> (34)
<223> Xaa equals any of the naturally occurring L-amino acids

<220>
<221> SITE
<222> (39)
<223> Xaa equals any of the naturally occurring L-amino acids

<400> 348
Glu Val Glu Glu Ala Glu Leu Ala Ala Ala Leu Pro Met Glu Pro Arg
  1               5                  10                  15

Ala Ser Ile Ala Gly Ala Ser Gly Ala Ala Asp Met His Phe Cys Pro
             20                  25                  30

Ala Xaa Gly Thr His Arg Xaa Ala Tyr Pro Gln Glu Gly Ser Thr Tyr
         35                  40                  45

Ala Thr Glu Leu Glu Arg Thr Lys Ala Pro Gly Ala Trp Lys Phe Pro
     50                  55                  60

Trp Gly Pro Leu Gly Phe Leu Arg Phe Ser Trp Leu Gly Arg Arg Gly
 65                  70                  75                  80

Ser Leu Gly Ser Ala Ser Arg Ala Leu Gly Gly Arg Leu Arg Arg Ala
                 85                  90                  95

Ala Ala Ala Thr Glu Arg Glu Glu Pro Ser Ser Asp Gly Ala Gly Ala
            100                 105                 110

Glu Asp Glu His Asp Ala Val Gly Thr Ser Leu Lys Arg Val Pro Asp
        115                 120                 125

Thr Arg Ser Val Asp Val Leu Pro Asp Gln Glu Val Gln Gln Arg Gln
    130                 135                 140

Gln His Ile
145

<210> 349
<211> 31
<212> PRT
<213> Homo sapiens

<400> 349
Arg Arg Ile Ser Gly Ile Pro Arg Asp Pro Arg Gly Thr Ser Lys His
1               5                   10                  15

Leu Glu Leu Leu Ser Phe Leu Val Leu Trp His Arg Cys Cys Leu
            20                  25                  30

<210> 350
<211> 29
<212> PRT
<213> Homo sapiens

<400> 350
Arg Thr Lys Ala Pro Gly Ala Trp Lys Phe Pro Trp Gly Pro Leu Gly
1               5                   10                  15

Phe Leu Arg Phe Ser Trp Leu Gly Arg Arg Gly Ser Leu
            20                  25

<210> 351
<211> 11
<212> PRT
<213> Homo sapiens

<400> 351
Pro Arg Leu Ala Gln Leu Arg Leu Leu Ser Leu
1               5                   10

<210> 352
<211> 178
<212> PRT
<213> Homo sapiens

<400> 352
Gln Ser Asp Phe Arg Glu Met Asn Gln Thr Asn Ser Thr Ser Asn Ala
1               5                   10                  15

Ala Lys Ala Arg Glu Ala Gln Gln Gly Arg Gly Arg Asp Arg Glu Ala
            20                  25                  30

Ile Phe Ser Ser Ser Ala Leu Glu His Leu Val Cys Tyr Leu Gln Ala
        35                  40                  45

Tyr Lys His Thr Leu Leu Phe Ile Arg Ser Leu Asn Glu His Gly Leu
    50                  55                  60

Gln Gln Leu Leu Phe Gln Trp Arg Asp Gly Leu Phe Gly Asn Trp Tyr
65                  70                  75                  80

Phe Arg Ile Pro Ile Leu Leu Phe Phe Thr Gly Phe His Cys Tyr His
                85                  90                  95

Leu Ser Cys Pro His Leu Pro Cys Ala Gln Arg Gln Ser Ser Arg Gly
            100                 105                 110

Thr Val Pro Tyr Val Leu Cys Pro His Pro His His His Leu His His
        115                 120                 125

Tyr Ser Trp Phe Pro Phe Leu Ile Pro Val Leu His Thr Leu Pro Lys
    130                 135                 140

Leu Gln Pro Lys Phe His Gly Arg Pro Glu Gln Pro Leu Asn Leu Leu
145                 150                 155                 160

Gln Val Lys Pro Thr Ser Gly Thr Ile Ala Ser Ala Glu Gln Val Trp
                165                 170                 175

Val Lys

<210> 353
<211> 29
<212> PRT
<213> Homo sapiens

<400> 353
Val Cys Tyr Leu Gln Ala Tyr Lys His Thr Leu Leu Phe Ile Arg Ser
1               5                   10                  15

Leu Asn Glu His Gly Leu Gln Gln Leu Leu Phe Gln Trp
            20                  25

<210> 354
<211> 32
<212> PRT
<213> Homo sapiens

<400> 354
Val Pro Tyr Val Leu Cys Pro His Pro His His His Leu His His Tyr
1               5                   10                  15

Ser Trp Phe Pro Phe Leu Ile Pro Val Leu His Thr Leu Pro Lys Leu
            20                  25                  30

<210> 355
<211> 31
<212> PRT
<213> Homo sapiens

<400> 355
Glu Ser Glu Arg Ala Val Val Tyr Leu Ile Thr Gly Ala Leu Phe Ile
1               5                   10                  15

Val Ser Ser Cys Val Leu Cys Phe Leu Pro Ser Ser Arg Arg Glu
            20                  25                  30


<210> 356
<211> 249
<212> PRT
<213> Homo sapiens

<220>
<221> SITE
<222> (4)
<223> Xaa equals any of the naturally occurring L-amino acids

<220>
<221> SITE
<222> (221)
<223> Xaa equals any of the naturally occurring L-amino acids

<400> 356
Met Trp Val Xaa Gly Glu Glu Val Leu Gly Ser His Ala Ala Ser Pro
1               5                   10                  15

Ala Phe Leu His Arg Cys Phe Ser Glu Glu Ser Cys Val Ser Ile Pro
            20                  25                  30

Glu Val Glu Gly Tyr Val Val Val Leu Gln Pro Asp Ala Pro Gln Ile
        35                  40                  45

Leu Leu Ser Gly Thr Ala His Phe Ala Arg Pro Ala Val Asp Phe Glu
    50                  55                  60

Gly Thr Asn Gly Val Pro Leu Phe Pro Asp Leu Gln Ile Thr Cys Ser
65                  70                  75                  80

Ile Ser His Gln Val Glu Ala Lys Lys Asp Glu Ser Trp Gln Gly Thr
                85                  90                  95

Val Thr Asp Thr Arg Met Ser Asp Glu Ile Val His Asn Leu Asp Gly
            100                 105                 110

Cys Glu Ile Ser Leu Val Gly Asp Asp Leu Asp Pro Glu Arg Glu Ser
        115                 120                 125

Leu Leu Leu Asp Thr Thr Ser Leu Gln Gln Arg Gly Leu Glu Leu Thr
    130                 135                 140

Asn Thr Ser Ala Tyr Leu Thr Ile Ala Gly Val Glu Ser Ile Thr Val
145                 150                 155                 160

Tyr Glu Glu Ile Leu Arg Gln Ala Arg Tyr Arg Leu Arg His Gly Ala
                165                 170                 175

Ala Leu Tyr Thr Arg Lys Phe Arg Leu Ser Cys Ser Glu Met Asn Gly
            180                 185                 190

Arg Tyr Ser Ser Asn Glu Phe Ile Val Glu Val Asn Val Leu His Ser
        195                 200                 205

Met Asn Arg Val Ala His Pro Ser His Val Leu Ser Xaa Gln Gln Phe
    210                 215                 220

Leu His Arg Gly His Gln Pro Pro Pro Glu Met Ala Gly His Ser Leu
225                 230                 235                 240

Ala Ser Ser His Arg Asn Ser Ser Thr
                245


<210> 357
<211> 23
<212> PRT
<213> Homo sapiens

<400> 357
Leu Gly Ser His Ala Ala Ser Pro Ala Phe Leu His Arg Cys Phe Ser
1               5                   10                  15

Glu Glu Ser Cys Val Ser Ile
            20


<210> 358
<211> 29
<212> PRT
<213> Homo sapiens

<400> 358
Gly Tyr Val Val Val Leu Gln Pro Asp Ala Pro Gln Ile Leu Leu Ser
1               5                   10                  15

Gly Thr Ala His Phe Ala Arg Pro Ala Val Asp Phe Glu
            20                  25


<210> 359
<211> 26
<212> PRT
<213> Homo sapiens

<400> 359
Ile Thr Cys Ser Ile Ser His Gln Val Glu Ala Lys Lys Asp Glu Ser
1               5                   10                  15

Trp Gln Gly Thr Val Thr Asp Thr Arg Met
            20                  25


<210> 360
<211> 29
<212> PRT

<213> Homo sapiens

<400> 360
Asn Leu Asp Gly Cys Glu Ile Ser Leu Val Gly Asp Asp Leu Asp Pro
1               5                   10                  15

Glu Arg Glu Ser Leu Leu Leu Asp Thr Thr Ser Leu Gln
            20                  25


<210> 361
<211> 23
<212> PRT
<213> Homo sapiens

<400> 361
Ser Ala Tyr Leu Thr Ile Ala Gly Val Glu Ser Ile Thr Val Tyr Glu
1               5                   10                  15

Glu Ile Leu Arg Gln Ala Arg
            20


<210> 362
<211> 26
<212> PRT
<213> Homo sapiens

<400> 362
Arg Leu Ser Cys Ser Glu Met Asn Gly Arg Tyr Ser Ser Asn Glu Phe
1               5                   10                  15

Ile Val Glu Val Asn Val Leu His Ser Met
            20                  25


<210> 363
<211> 25
<212> PRT
<213> Homo sapiens

<400> 363
Gln Gln Phe Leu His Arg Gly His Gln Pro Pro Pro Glu Met Ala Gly
1               5                   10                  15

His Ser Leu Ala Ser Ser His Arg Asn
            20                  25


<210> 364
<211> 299
<212> PRT
<213> Homo sapiens

<220>
<221> SITE
<222> (52)
<223> Xaa equals any of the naturally occurring L-amino acids

<400> 364
Met Ala Asp Ser Glu Thr Phe Ile Ser Leu Glu Glu Cys Arg Gly His
1               5                   10                  15

Lys Arg Ala Arg Lys Arg Thr Ser Met Glu Thr Ala Leu Ala Leu Glu
            20                  25                  30

Lys Leu Phe Pro Lys Gln Cys Gln Val Leu Gly Ile Val Thr Pro Gly
        35                  40                  45

Ile Val Val Xaa Pro Met Gly Ser Gly Ser Asn Arg Pro Gln Glu Ile
    50                  55                  60

Glu Ile Gly Glu Ser Gly Phe Ala Leu Leu Phe Pro Gln Ile Glu Gly
65                  70                  75                  80

Ile Lys Ile Gln Pro Phe His Phe Ile Lys Asp Pro Lys Asn Leu Thr
                85                  90                  95

Leu Glu Arg His Gln Leu Thr Glu Val Gly Leu Leu Asp Asn Pro Glu
            100                 105                 110

Leu Arg Val Val Leu Val Phe Gly Tyr Asn Cys Cys Lys Val Gly Ala
        115                 120                 125

Ser Asn Tyr Leu Gln Gln Val Val Ser Thr Phe Ser Asp Met Asn Ile
    130                 135                 140

Ile Leu Ala Gly Gly Gln Val Asp Asn Leu Ser Ser Leu Thr Ser Glu
145                 150                 155                 160

Lys Asn Pro Leu Asp Ile Asp Ala Ser Gly Val Val Gly Leu Ser Phe
                165                 170                 175

Ser Gly His Arg Ile Gln Ser Ala Thr Val Leu Leu Asn Glu Asp Val
            180                 185                 190

Ser Asp Glu Lys Thr Ala Glu Ala Ala Met Gln Arg Leu Lys Ala Ala
        195                 200                 205

Asn Ile Pro Glu His Asn Thr Ile Gly Phe Met Phe Ala Cys Val Gly
    210                 215                 220

Arg Gly Phe Gln Tyr Tyr Arg Ala Lys Gly Asn Val Glu Ala Asp Ala
225                 230                 235                 240

Phe Arg Lys Phe Phe Pro Ser Val Pro Leu Phe Gly Phe Phe Gly Asn
                245                 250                 255

Gly Glu Ile Gly Cys Asp Arg Ile Val Thr Gly Asn Phe Ile Leu Arg
            260                 265                 270

Lys Cys Asn Glu Val Lys Asp Asp Asp Leu Phe His Ser Tyr Thr Thr
        275                 280                 285

Ile Met Ala Leu Ile His Leu Gly Ser Ser Lys
    290                 295

<210> 365
<211> 21
<212> PRT
<213> Homo sapiens

<400> 365
His Lys Arg Ala Arg Lys Arg Thr Ser Met Glu Thr Ala Leu Ala Leu
1               5                   10                  15

Glu Lys Leu Phe Pro
            20


<210> 366
<211> 24
<212> PRT
<213> Homo sapiens

<400> 366
Met Gly Ser Gly Ser Asn Arg Pro Gln Glu Ile Glu Ile Gly Glu Ser
1               5                   10                  15

Gly Phe Ala Leu Leu Phe Pro Gln
            20


<210> 367
<211> 22
<212> PRT
<213> Homo sapiens

<400> 367
Phe His Phe Ile Lys Asp Pro Lys Asn Leu Thr Leu Glu Arg His Gln
1               5                   10                  15

Leu Thr Glu Val Gly Leu
            20


<210> 368
<211> 23
<212> PRT
<213> Homo sapiens

<400> 368
Phe Gly Tyr Asn Cys Cys Lys Val Gly Ala Ser Asn Tyr Leu Gln Gln
1               5                   10                  15

Val Val Ser Thr Phe Ser Asp
            20


<210> 369
<211> 20
<212> PRT
<213> Homo sapiens

<400> 369
Thr Ser Glu Lys Asn Pro Leu Asp Ile Asp Ala Ser Gly Val Val Gly
1               5                   10                  15

Leu Ser Phe Ser
            20


<210> 370
<211> 26
<212> PRT
<213> Homo sapiens

<400> 370
Asn Glu Asp Val Ser Asp Glu Lys Thr Ala Glu Ala Ala Met Gln Arg
1               5                   10                  15

Leu Lys Ala Ala Asn Ile Pro Glu His Asn
            20                  25


<210> 371
<211> 25
<212> PRT
<213> Homo sapiens

<400> 371
Tyr Tyr Arg Ala Lys Gly Asn Val Glu Ala Asp Ala Phe Arg Lys Phe
1               5                   10                  15

Phe Pro Ser Val Pro Leu Phe Gly Phe
            20                  25


<210> 372
<211> 26
<212> PRT
<213> Homo sapiens

<400> 372
Ile Gly Cys Asp Arg Ile Val Thr Gly Asn Phe Ile Leu Arg Lys Cys
1               5                   10                  15

Asn Glu Val Lys Asp Asp Asp Leu Phe His
            20                  25


<210> 373
<211> 341
<212> PRT
<213> Homo sapiens

<400> 373
Met Pro Lys Arg Lys Val Thr Phe Gln Gly Val Gly Asp Glu Glu Asp
1               5                   10                  15

Glu Asp Glu Ile Ile Val Pro Lys Lys Lys Leu Val Asp Pro Val Ala

            20                  25                  30

Gly Ser Gly Gly Pro Gly Ser Arg Phe Lys Gly Lys His Ser Leu Asp
        35                  40                  45

Ser Asp Glu Glu Glu Asp Asp Asp Asp Gly Gly Ser Ser Lys Tyr Asp
    50                  55                  60

Ile Leu Ala Ser Glu Asp Val Glu Gly Gln Glu Ala Ala Thr Leu Pro
65                  70                  75                  80

Ser Glu Gly Gly Val Arg Ile Thr Pro Phe Asn Leu Gln Glu Glu Met
                85                  90                  95

Glu Glu Gly His Phe Asp Ala Asp Gly Asn Tyr Phe Leu Asn Arg Asp
            100                 105                 110

Ala Gln Ile Arg Asp Ser Trp Leu Asp Asn Ile Asp Trp Val Lys Ile
        115                 120                 125

Arg Glu Arg Pro Pro Gly Gln Arg Gln Ala Ser Asp Ser Glu Glu Glu
    130                 135                 140

Asp Ser Leu Gly Gln Thr Ser Met Ser Ala Gln Ala Leu Leu Glu Gly
145                 150                 155                 160

Leu Leu Glu Leu Leu Leu Pro Arg Glu Thr Val Ala Gly Ala Leu Arg
                165                 170                 175

Arg Leu Gly Ala Arg Gly Gly Gly Lys Gly Arg Lys Gly Pro Gly Gln
            180                 185                 190

Pro Ser Ser Pro Gln Arg Leu Asp Arg Leu Ser Gly Leu Ala Asp Gln
        195                 200                 205

Met Val Ala Arg Gly Asn Leu Gly Val Tyr Gln Glu Thr Arg Glu Arg
    210                 215                 220

Leu Ala Met Arg Leu Lys Gly Leu Gly Cys Gln Thr Leu Gly Pro His
225                 230                 235                 240

Asn Pro Thr Pro Pro Pro Ser Leu Asp Met Phe Ala Glu Glu Leu Ala
                245                 250                 255

Glu Glu Glu Leu Glu Thr Pro Thr Pro Thr Gln Arg Gly Glu Ala Glu
            260                 265                 270

Ser Arg Gly Asp Gly Leu Val Asp Val Met Trp Glu Tyr Lys Trp Glu
        275                 280                 285

Asn Thr Gly Asp Ala Glu Leu Tyr Gly Pro Phe Thr Ser Ala Gln Met
    290                 295                 300

Gln Thr Trp Val Ser Glu Gly Tyr Phe Pro Asp Gly Val Tyr Cys Arg
305                 310                 315                 320

Lys Leu Asp Pro Pro Gly Gly Gln Phe Tyr Asn Ser Lys Arg Ile Asp
                325                 330                 335

Phe Asp Leu Tyr Thr
            340

<210> 374
<211> 24
<212> PRT
<213> Homo sapiens

<400> 374
Thr Phe Gln Gly Val Gly Asp Glu Glu Asp Glu Asp Glu Ile Ile Val
1               5                   10                  15

Pro Lys Lys Lys Leu Val Asp Pro
            20

<210> 375
<211> 27
<212> PRT
<213> Homo sapiens

<400> 375
Pro Gly Ser Arg Phe Lys Gly Lys His Ser Leu Asp Ser Asp Glu Glu
1               5                   10                  15

Glu Asp Asp Asp Asp Gly Gly Ser Ser Lys Tyr
            20                  25

<210> 376
<211> 25
<212> PRT
<213> Homo sapiens

<400> 376
Glu Ala Ala Thr Leu Pro Ser Glu Gly Gly Val Arg Ile Thr Pro Phe
1               5                   10                  15

Asn Leu Gln Glu Glu Met Glu Glu Gly
            20                  25

<210> 377
<211> 29
<212> PRT
<213> Homo sapiens

<400> 377
Phe Leu Asn Arg Asp Ala Gln Ile Arg Asp Ser Trp Leu Asp Asn Ile
1               5                   10                  15

Asp Trp Val Lys Ile Arg Glu Arg Pro Pro Gly Gln Arg
            20                  25

<210> 378

<211> 26
<212> PRT
<213> Homo sapiens

<400> 378
Ser Leu Gly Gln Thr Ser Met Ser Ala Gln Ala Leu Leu Glu Gly Leu
1               5                   10                  15

Leu Glu Leu Leu Leu Pro Arg Glu Thr Val
            20                  25


<210> 379
<211> 28
<212> PRT
<213> Homo sapiens

<400> 379
Arg Gly Gly Gly Lys Gly Arg Lys Gly Pro Gly Gln Pro Ser Ser Pro
1               5                   10                  15

Gln Arg Leu Asp Arg Leu Ser Gly Leu Ala Asp Gln
            20                  25


<210> 380
<211> 24
<212> PRT
<213> Homo sapiens

<400> 380
Gln Glu Thr Arg Glu Arg Leu Ala Met Arg Leu Lys Gly Leu Gly Cys
1               5                   10                  15

Gln Thr Leu Gly Pro His Asn Pro
            20


<210> 381
<211> 28
<212> PRT
<213> Homo sapiens

<400> 381
Asp Met Phe Ala Glu Glu Leu Ala Glu Glu Glu Leu Glu Thr Pro Thr
1               5                   10                  15

Pro Thr Gln Arg Gly Glu Ala Glu Ser Arg Gly Asp
            20                  25


<210> 382
<211> 30
<212> PRT
<213> Homo sapiens

<400> 382
Glu Leu Tyr Gly Pro Phe Thr Ser Ala Gln Met Gln Thr Trp Val Ser

1               5                   10                  15

Glu Gly Tyr Phe Pro Asp Gly Val Tyr Cys Arg Lys Leu Asp
            20                  25                  30

<210> 383
<211> 14
<212> PRT
<213> Homo sapiens

<400> 383
Pro His Ser Ser Arg Val Ser Phe Leu Gln Ser Leu Ser Phe
1               5                   10

<210> 384
<211> 141
<212> PRT
<213> Homo sapiens

<400> 384

<210> 385
<211> 133
<212> PRT
<213> Homo sapiens

<400> 385


<210> 386
<211> 25
<212> PRT
<213> Homo sapiens

<400> 386
Val Cys Leu Ser Pro His Ser Arg Phe Trp Glu Cys Cys Ser Phe Tyr
1               5                   10                  15

Leu Gln Gly Leu Pro Ala Leu Arg Cys
            20                  25


<210> 387
<211> 27
<212> PRT
<213> Homo sapiens

<400> 387
Gln Phe Ser Arg Ala Leu Trp Val Ser Thr Cys Leu Val Leu Ala Ile
1               5                   10                  15

Thr Pro Gly Lys Trp Leu Leu Pro Glu Asp Arg
            20                  25


<210> 388
<211> 27
<212> PRT
<213> Homo sapiens

<400> 388

Ser Leu Ser Leu Leu Arg Ala Gln Thr Gly Thr Asp Cys Ala Val Ser
1               5                   10                  15

Pro Gly Leu Ala Gly Pro Cys His Gln Arg Gly
            20                  25

<210> 389
<211> 28
<212> PRT
<213> Homo sapiens

<400> 389
Ser Gly Arg Ser His Phe Pro Gly Val Met Ala Lys Thr Lys His Val
1               5                   10                  15

Asp Thr His Asn Ala Arg Glu Asn Trp Ile Arg Thr
            20                  25

<210> 390
<211> 20
<212> PRT
<213> Homo sapiens

<400> 390
Ala Arg Val Glu Val Gln Gly Gln Gly Pro Gly Ala Lys Val Asp Ala
1               5                   10                  15

Gly Glu Gly Gln
            20

<210> 391
<211> 121
<212> PRT
<213> Homo sapiens

<220>
<221> SITE
<222> (46)
<223> Xaa equals any of the naturally occurring L-amino acids

<220>
<221> SITE
<222> (66)
<223> Xaa equals any of the naturally occurring L-amino acids

<220>
<221> SITE
<222> (98)
<223> Xaa equals any of the naturally occurring L-amino acids

<220>
<221> SITE
<222> (121)
<223> Xaa equals any of the naturally occurring L-amino acids

<400> 391
Trp Val Val Leu Ser Gln Leu Gln Ala Gln Gly Val Ala Gly Met Met
1               5                   10                  15

Cys Ser Tyr Pro Glu Gly Gln Lys Lys Gly Lys Glu Ala Thr Arg Ser
            20                  25                  30

His Arg Trp Val Pro Arg Ser Leu Pro Gly Met Gly Ser Xaa Leu Ala
        35                  40                  45

Ala Pro His Ser Asn Pro Trp Leu Ala Pro Leu Ala Leu Leu Glu Ile
    50                  55                  60

Pro Xaa Pro Val Leu Cys Glu Trp Lys Arg Lys Leu Ile Ala Leu Glu
65                  70                  75                  80

Glu Val Ser Glu Cys Arg Pro Gly Val Gly Gly Gly Gly Gly Phe Leu
                85                  90                  95

Ser Xaa Cys Arg Arg Gly His Leu Ser Phe Leu Ser Gly Ala Pro Tyr
            100                 105                 110

Pro Leu Phe Pro Ile Ser Pro Leu Xaa
        115                 120


<210> 392
<211> 206
<212> PRT
<213> Homo sapiens

<220>
<221> SITE
<222> (105)
<223> Xaa equals any of the naturally occurring L-amino acids

<220>
<221> SITE
<222> (127)
<223> Xaa equals any of the naturally occurring L-amino acids

<220>
<221> SITE
<222> (131)
<223> Xaa equals any of the naturally occurring L-amino acids

<220>
<221> SITE
<222> (180)
<223> Xaa equals any of the naturally occurring L-amino acids

<400> 392
Glu Leu Arg His Gly Gly Pro Arg Gln Val Lys Asp Ser Phe Leu Asp
1               5                   10                  15

Tyr Met Gly Tyr Pro Asp Glu Asp Arg Ala Gly Pro Pro Ser Arg Trp
            20                  25                  30

Phe Pro Arg Glu Arg Phe Leu Ser Pro Pro Thr Val Val Pro Leu Cys
            35                  40                  45

Val Glu Leu Arg Leu Gly Phe Glu Ser Gly Met Gly Trp Gly Val Pro
    50                  55                  60

Gly Ser Ser His Ser Glu Gly Gly Pro Glu Ala Arg Trp Pro Leu Ile
65                  70                  75                  80

Ala Pro Met Tyr Thr Val Thr Gln Trp Phe Gln Arg Pro Asn Ser Gly
                85                  90                  95

Arg Gly Pro Gln Pro Pro Pro Gln Xaa Arg Gly Glu Ile Gly Lys Arg
            100                 105                 110

Gly Tyr Gly Ala Pro Glu Arg Lys Leu Arg Trp Pro Leu Leu Xaa Trp
        115                 120                 125

Glu Arg Xaa Pro Pro Pro Pro Pro Thr Pro Gly Arg His Ser Glu Thr
    130                 135                 140

Ser Ser Ser Ala Ile Ser Phe Leu Phe His Ser Gln Arg Thr Gly Trp
145                 150                 155                 160

Gly Ile Ser Ser Ser Ala Asn Gly Ala Ser Gln Gly Leu Leu Trp Gly
                165                 170                 175

Ala Ala Arg Xaa Leu Pro Ile Pro Gly Arg Asp Leu Gly Thr His Leu
            180                 185                 190

Trp Asp Leu Val Ala Ser Phe Pro Phe Phe Cys Pro Ser Gly
        195                 200                 205


<210> 393
<211> 24
<212> PRT
<213> Homo sapiens

<400> 393
Pro Glu Gly Gln Lys Lys Gly Lys Glu Ala Thr Arg Ser His Arg Trp
 1               5                  10                  15

Val Pro Arg Ser Leu Pro Gly Met
            20


<210> 394
<211> 26
<212> PRT
<213> Homo sapiens

<400> 394
Leu Arg Leu Gly Phe Glu Ser Gly Met Gly Trp Gly Val Pro Gly Ser
 1               5                  10                  15

Ser His Ser Glu Gly Gly Pro Glu Ala Arg
            20                  25

<210> 395
<211> 24
<212> PRT
<213> Homo sapiens

<400> 395
His Ser Gln Arg Thr Gly Trp Gly Ile Ser Ser Ser Ala Asn Gly Ala
1               5                   10                  15

Ser Gln Gly Leu Leu Trp Gly Ala
            20


<210> 396
<211> 54
<212> PRT
<213> Homo sapiens

<400> 396
Phe Ile Met Lys Leu Leu Tyr Gln Leu Leu Met Leu Thr Thr Ser Ser
1               5                   10                  15

Ser Tyr Ser Leu Ile Thr His Leu Cys Tyr Ser Ile Phe Leu Cys Ser
            20                  25                  30

Phe Tyr Phe His Phe Pro Cys Asn Val Ser Leu Phe Val Leu Ile Ser
        35                  40                  45

Glu Glu Phe Ile Tyr Asp
    50


<210> 397
<211> 21
<212> PRT
<213> Homo sapiens

<400> 397
Leu Met Leu Thr Thr Ser Ser Ser Tyr Ser Leu Ile Thr His Leu Cys
1               5                   10                  15

Tyr Ser Ile Phe Leu
            20


<210> 398
<211> 21
<212> PRT
<213> Homo sapiens

<400> 398
Leu Cys Ser Phe Tyr Phe His Phe Pro Cys Asn Val Ser Leu Phe Val
1               5                   10                  15

Leu Ile Ser Glu Glu
            20

<210> 399
<211> 53
<212> PRT
<213> Homo sapiens

<400> 399


<210> 400
<211> 21
<212> PRT
<213> Homo sapiens

<400> 400
Asp Lys Met Leu Thr Cys Leu Ile Ile Asn Glu Leu Phe Arg Asn Gln
1               5                   10                  15

Tyr Lys Glu Thr Asn
            20


<210> 401
<211> 21
<212> PRT
<213> Homo sapiens

<400> 401
Asn Ile Thr Arg Glu Val Lys Ile Lys Gly Thr Glu Glu Asn Gly Ile
1               5                   10                  15

Ala Gln Met Ser Tyr
            20


<210> 402
<211> 7
<212> PRT
<213> Homo sapiens

<400> 402
Gly Ile Ser Glu Arg Lys Pro
1               5


<210> 403

<211> 25
<212> PRT
<213> Homo sapiens

<400> 403


<210> 404
<211> 100
<212> PRT
<213> Homo sapiens

<220>
<221> SITE
<222> (96)
<223> Xaa equals any of the naturally occurring L-amino acids

<400> 404
Leu Pro Ser His Pro Leu Arg Pro Leu Thr Phe Ser Ser Ala Met Cys
1               5                   10                  15

Met His Leu Pro Pro Pro Leu Cys Arg Arg Ala Ala Leu Ser Ala Pro
            20                  25                  30

Phe Ala Thr Gln His Arg Pro Trp Ser Val Ala Ala Ala Cys Leu Pro
        35                  40                  45

Arg Ile His Gln Asn Pro Leu Asp Ala Glu Tyr Pro Ser Gly Cys Cys
    50                  55                  60

Arg Met Ser Phe Leu Pro Ala Ala Cys Ser Asn Ile Tyr Ser Gln Glu
65                  70                  75                  80

Cys His Tyr Thr Leu Met Ser His Ser Glu Ala Ser Thr Leu Gln Xaa
                85                  90                  95

Ala Gln Leu Leu
            100


<210> 405
<211> 76
<212> PRT
<213> Homo sapiens

<400> 405

<210> 406
<211> 25
<212> PRT
<213> Homo sapiens

<400> 406
Leu Cys Arg Arg Ala Ala Leu Ser Ala Pro Phe Ala Thr Gln His Arg
1               5                   10                  15

Pro Trp Ser Val Ala Ala Ala Cys Leu
            20                  25

<210> 407
<211> 24
<212> PRT
<213> Homo sapiens

<400> 407
Arg Gly Phe Trp Trp Met Arg Gly Arg Gln Ala Ala Ala Thr Leu His
1               5                   10                  15

Gly Arg Cys Trp Val Ala Lys Gly
            20

<210> 408
<211> 23
<212> PRT
<213> Homo sapiens

<400> 408
Gln Arg Gly Gly Gly Arg Cys Met His Ile Ala Asp Glu Lys Val Arg
1               5                   10                  15

Gly Leu Ser Gly Cys Asp Gly
            20

<210> 409
<211> 106
<212> PRT
<213> Homo sapiens

<400> 409
Thr His Pro Ser His Pro Ser Ile Val Ile Gln Ser Thr Val Ser Leu
1               5                   10                  15

Cys Leu Thr Ala Ser Ser Arg Arg Lys Lys Ser Asp Cys Leu Ser Leu

            20                  25                  30

Cys Gln Val Ser Cys Ser Gln Arg Pro Gly Ser His Lys Thr Asn Val
        35                  40                  45

Ala Trp Gly Phe Leu Met Ser Arg Val His Phe Ser Val Arg Trp Val
    50                  55                  60

Ser Gly Gly Arg Gly Ile Thr Gly Ala Ile Cys Lys Glu Ser Ser Leu
65                  70                  75                  80

Pro Cys Lys Glu Ile Gln Gly Lys Ala Cys Tyr Phe Cys His His Pro
                85                  90                  95

Ala Gln Gln Ser Thr Pro Phe Ser His Ile
            100                 105

<210> 410
<211> 27
<212> PRT
<213> Homo sapiens

<400> 410
Val Ile Gln Ser Thr Val Ser Leu Cys Leu Thr Ala Ser Ser Arg Arg
1               5                   10                  15

Lys Lys Ser Asp Cys Leu Ser Leu Cys Gln Val
            20                  25

<210> 411
<211> 26
<212> PRT
<213> Homo sapiens

<400> 411
Ile Cys Lys Glu Ser Ser Leu Pro Cys Lys Glu Ile Gln Gly Lys Ala
1               5                   10                  15

Cys Tyr Phe Cys His His Pro Ala Gln Gln
            20                  25

<210> 412
<211> 188
<212> PRT
<213> Homo sapiens

<220>
<221> SITE
<222> (140)
<223> Xaa equals any of the naturally occurring L-amino acids

<220>
<221> SITE
<222> (149)
<223> Xaa equals any of the naturally occurring L-amino acids

<400> 412
Ser Leu Gln Val Leu Arg Thr Leu Gly Ser Lys Cys Gly Asp Phe Leu
1               5                   10                  15

Arg Ser Arg Phe Cys Lys Asp Val Leu Pro Lys Leu Ala Gly Ser Leu
            20                  25                  30

Val Thr Gln Ala Pro Ile Ser Ala Arg Ala Gly Pro Val Tyr Ser His
        35                  40                  45

Thr Leu Ala Phe Lys Leu Gln Leu Ala Val Leu Gln Gly Leu Gly Pro
    50                  55                  60

Leu Cys Glu Arg Leu Asp Leu Gly Glu Gly Asp Leu Asn Lys Val Ala
65                  70                  75                  80

Asp Ala Cys Leu Ile Tyr Leu Ser Val Lys Gln Pro Val Lys Leu Gln
                85                  90                  95

Glu Ala Ala Arg Ser Val Phe Leu His Leu Met Lys Val Asp Pro Asp
            100                 105                 110

Ser Thr Trp Phe Leu Leu Asn Glu Leu Tyr Cys Pro Val Gln Phe Thr
        115                 120                 125

Pro Pro His Pro Ser Leu His Pro Val Gln Leu Xaa Gly Ala Ser Gly
    130                 135                 140

Gln Gln Asn Pro Xaa His Asp Gln Arg Ala Pro Ala Ala Gln Gly Ala
145                 150                 155                 160

Ala Val Thr Leu Leu Pro His His Arg Gly His Arg Ser Leu Pro Tyr
                165                 170                 175

Cys Gln Pro Glu Ala Gly Leu Thr Pro Pro Arg Pro
            180                 185


<210> 413
<211> 138
<212> PRT
<213> Homo sapiens

<400> 413
Gly Ala Asp Gly Asn Val Ser Asp Phe Asp Asn Glu Glu Glu Glu Gln
1               5                   10                  15

Ser Val Pro Pro Lys Val Asp Glu Asn Asp Thr Arg Pro Asp Val Glu
            20                  25                  30

Pro Pro Leu Pro Leu Gln Ile Gln Ile Ala Met Asp Val Met Glu Arg
        35                  40                  45

Cys Ile His Leu Leu Ser Asp Lys Asn Leu Gln Ile Arg Leu Lys Val
    50                  55                  60

Leu Asp Val Leu Asp Leu Cys Val Val Val Leu Gln Ser His Lys Asn

65                  70                  75                  80

Gln Leu Leu Pro Leu Ala His Gln Ala Trp Pro Ser Leu Val His Arg
                85                  90                  95

Leu Thr Arg Asp Ala Pro Leu Ala Val Leu Arg Ala Phe Lys Phe Tyr
            100                 105                 110

Val Pro Trp Glu Ala Ser Val Val Thr Phe Phe Ala Ala Gly Ser Ala
        115                 120                 125

Lys Met Ser Cys Gln Ser Trp Leu Ala Pro
    130                 135


<210> 414
<211> 26
<212> PRT
<213> Homo sapiens

<400> 414
Thr Leu Gly Ser Lys Cys Gly Asp Phe Leu Arg Ser Arg Phe Cys Lys
1               5                   10                  15

Asp Val Leu Pro Lys Leu Ala Gly Ser Leu
            20                  25


<210> 415
<211> 29
<212> PRT
<213> Homo sapiens

<400> 415
Pro Val Tyr Ser His Thr Leu Ala Phe Lys Leu Gln Leu Ala Val Leu
1               5                   10                  15

Gln Gly Leu Gly Pro Leu Cys Glu Arg Leu Asp Leu Gly
            20                  25


<210> 416
<211> 27
<212> PRT
<213> Homo sapiens

<400> 416
Ser Val Pro Pro Lys Val Asp Glu Asn Asp Thr Arg Pro Asp Val Glu
1               5                   10                  15

Pro Pro Leu Pro Leu Gln Ile Gln Ile Ala Met
            20                  25


<210> 417
<211> 26
<212> PRT
<213> Homo sapiens

<400> 417
Trp Pro Ser Leu Val His Arg Leu Thr Arg Asp Ala Pro Leu Ala Val
1               5                   10                  15

Leu Arg Ala Phe Lys Phe Tyr Val Pro Trp
            20                  25


<210> 418
<211> 58
<212> PRT
<213> Homo sapiens

<400> 418
Ser Leu Gly Ile Ser Thr Phe Gly Ile Met Val Phe Ser Val Tyr Phe
1               5                   10                  15

Gly Gly Ile Met Ile Ser Ile Pro Tyr Ser Gly Ile Ser Phe Gly Asn
            20                  25                  30

Lys Lys Glu Leu Asn Ile Asp Ser Cys Tyr Asn Met Val Asn Leu Lys
        35                  40                  45

Asn Ile Met Phe Ser Glu Arg Ser Gln Thr
    50                  55


<210> 419
<211> 15
<212> PRT
<213> Homo sapiens

<400> 419
His Ala Ser Gly Asn Asn Asp Pro Leu Trp Phe Leu Thr Tyr Leu
1               5                   10                  15


<210> 420
<211> 21
<212> PRT
<213> Homo sapiens

<400> 420
Met Val Phe Ser Val Tyr Phe Gly Gly Ile Met Ile Ser Ile Pro Tyr
1               5                   10                  15

Ser Gly Ile Ser Phe
            20


<210> 421
<211> 20
<212> PRT
<213> Homo sapiens

<400> 421
Phe Gly Asn Lys Lys Glu Leu Asn Ile Asp Ser Cys Tyr Asn Met Val

1               5                   10                  15

Asn Leu Lys Asn
            20


<210> 422
<211> 75
<212> PRT
<213> Homo sapiens

<220>
<221> SITE
<222> (48)
<223> Xaa equals any of the naturally occurring L-amino acids

<220>
<221> SITE
<222> (49)
<223> Xaa equals any of the naturally occurring L-amino acids

<220>
<221> SITE
<222> (50)
<223> Xaa equals any of the naturally occurring L-amino acids

<220>
<221> SITE
<222> (72)
<223> Xaa equals any of the naturally occurring L-amino acids

<220>
<221> SITE
<222> (74)
<223> Xaa equals any of the naturally occurring L-amino acids

<400> 422
Met Asn Ser Phe Ser Val Ile Ala Ser Ile Val Val Leu Leu Pro Phe
 1               5                   10                  15

Pro Gly Leu Ser Val Ser Ala Cys Leu Pro Ser His Ser His Gln Cys
            20                  25                  30

Lys Thr Phe Ile Leu Leu Phe Leu Pro Ser Ser Glu Lys Thr Leu Xaa
        35                  40                  45

Xaa Xaa Pro Pro Ser His Ser Ser Thr Leu Gly Gly Gln Gly Gly Gln
    50                  55                  60

Ile Met Arg Ser Gly Asp Arg Xaa His Xaa Gly
 65                  70                  75


<210> 423
<211> 81
<212> PRT
<213> Homo sapiens

<220>
<221> SITE
<222> (5)
<223> Xaa equals any of the naturally occurring L-amino acids

<220>
<221> SITE
<222> (6)
<223> Xaa equals any of the naturally occurring L-amino acids

<220>
<221> SITE
<222> (60)
<223> Xaa equals any of the naturally occurring L-amino acids

<220>
<221> SITE
<222> (74)
<223> Xaa equals any of the naturally occurring L-amino acids

<400> 423
Val Val Phe Phe Xaa Xaa Phe Phe Glu Met Glu Ser His Ser Val Ala
1               5                   10                  15

Gln Ala Gly Val Gln Trp Arg Asn Leu Gly Ser Leu Gln Ala Leu Pro
            20                  25                  30

Pro Gly Phe Met Pro Phe Ser Cys Leu Ser Leu Pro Gly Ser Trp Asp
        35                  40                  45

Tyr Arg Arg Pro Pro Pro Ser Pro Ala Asn Leu Xaa Cys Ile Phe Ser
    50                  55                  60

Arg Asp Gly Gly His His Val Ser Gln Xaa Gly Leu Asp Leu Leu Thr
65                  70                  75                  80

Ser


<210> 424
<211> 28
<212> PRT
<213> Homo sapiens

<400> 424
Ile Val Val Leu Leu Pro Phe Pro Gly Leu Ser Val Ser Ala Cys Leu
1               5                   10                  15

Pro Ser His Ser His Gln Cys Lys Thr Phe Ile Leu
            20                  25


<210> 425
<211> 26
<212> PRT
<213> Homo sapiens

<400> 425
Pro Gly Phe Met Pro Phe Ser Cys Leu Ser Leu Pro Gly Ser Trp Asp
1               5                   10                  15

Tyr Arg Arg Pro Pro Pro Ser Pro Ala Asn
            20                  25


<210> 426
<211> 16
<212> PRT
<213> Homo sapiens

<400> 426
Tyr Arg Phe Lys Asn Pro Lys Cys Arg Leu Phe Ser Val Pro Cys Arg
1               5                   10                  15


<210> 427
<211> 128
<212> PRT
<213> Homo sapiens

<400> 427
Thr Gln Asn Arg Glu Leu Leu Ala Trp Lys Pro Lys Gly Thr Asp Asp
1               5                   10                  15

Ile Cys Thr Ser His Asn Thr Thr His Ile Gln Lys Met Pro Gly Glu
            20                  25                  30

Ala Asn Ser Cys Cys Pro Arg Gly Ala Lys Ser Tyr His Ile Asp Cys
        35                  40                  45

Trp Pro Pro Ala Leu Phe Pro Arg Cys Val Ala Tyr Leu Phe Leu Asn
    50                  55                  60

Lys Pro Ala Thr Leu Arg Lys Lys Tyr Tyr Cys Lys Pro Tyr His Thr
65                  70                  75                  80

Gln Leu His Pro Ala Trp His Arg Glu Lys Ser Ala Phe Trp Ile Phe
                85                  90                  95

Glu Thr Val Ser Gln Ser Lys Gln Ser Leu Thr Ser Leu Val Tyr Ser
            100                 105                 110

Val Asn Glu Leu Leu Val Leu Ser Asn Leu Ala Gln Trp Ala Leu Gly
        115                 120                 125


<210> 428
<211> 23
<212> PRT

<213> Homo sapiens

<400> 428
Ala Trp Lys Pro Lys Gly Thr Asp Asp Ile Cys Thr Ser His Asn Thr
1               5                   10                  15

Thr His Ile Gln Lys Met Pro
            20

<210> 429
<211> 25
<212> PRT
<213> Homo sapiens

<400> 429
Cys Pro Arg Gly Ala Lys Ser Tyr His Ile Asp Cys Trp Pro Pro Ala
1               5                   10                  15

Leu Phe Pro Arg Cys Val Ala Tyr Leu
            20                  25

<210> 430
<211> 26
<212> PRT
<213> Homo sapiens

<400> 430
Ser Tyr His Ile Asp Cys Trp Pro Pro Ala Leu Phe Pro Arg Cys Val
1               5                   10                  15

Ala Tyr Leu Phe Leu Asn Lys Pro Ala Thr
            20                  25

<210> 431
<211> 29
<212> PRT
<213> Homo sapiens

<400> 431
Arg Lys Lys Tyr Tyr Cys Lys Pro Tyr His Thr Gln Leu His Pro Ala
1               5                   10                  15

Trp His Arg Glu Lys Ser Ala Phe Trp Ile Phe Glu Thr
            20                  25

<210> 432
<211> 28
<212> PRT
<213> Homo sapiens

<400> 432
Ile Cys Leu Asp Ser Cys Ser Gln Val Ser Val Thr Ser Leu Trp Ser
1               5                   10                  15

Phe Leu Arg Val His Ser Leu Val Gln Thr Leu Trp
            20                  25

<210> 433
<211> 75
<212> PRT
<213> Homo sapiens

<400> 433
His Tyr Cys Cys Asp Phe Gly Thr Ser Leu Leu Gly Phe Tyr Val Pro
1               5                   10                  15

Phe His Tyr Tyr Val His Met Val Asn Ile Ile Leu Thr Thr Ile Asp
            20                  25                  30

Phe Tyr His Tyr Lys Phe Cys Cys Ser Gln Asn Ala Asn Lys His Cys
        35                  40                  45

Phe Lys His Phe Gln Ile Met Thr Thr Val Pro Tyr Leu Asn Ile Asn
    50                  55                  60

Lys Glu Asn Leu Arg Phe Lys Asn Ile Phe Lys
65                  70                  75

<210> 434
<211> 27
<212> PRT
<213> Homo sapiens

<400> 434
Thr Ser Leu Leu Gly Phe Tyr Val Pro Phe His Tyr Tyr Val His Met
1               5                   10                  15

Val Asn Ile Ile Leu Thr Thr Ile Asp Phe Tyr
            20                  25

<210> 435
<211> 22
<212> PRT
<213> Homo sapiens

<400> 435
Phe Gln Ile Met Thr Thr Val Pro Tyr Leu Asn Ile Asn Lys Glu Asn
1               5                   10                  15

Leu Arg Phe Lys Asn Ile
            20

<210> 436
<211> 106
<212> PRT
<213> Homo sapiens

<400> 436

Ile Ser Glu Ser Met Ser Leu Val Arg Ser Leu Gln Phe Tyr Arg Gly
1               5                   10                  15

Lys Asn Arg Ala Glu Arg Thr Val Ile Ser Ser Ser Ser His Ser Cys
            20                  25                  30

His Leu Ile Asp Leu Glu Phe Gln Pro Arg Ser Asp Gly Glu Val Ser
        35                  40                  45

Ile Ser Phe Leu Glu Lys Gly Val Glu Leu Arg Trp Gly Met Gly Leu
    50                  55                  60

Glu Asp Leu Ile Gly Leu Gly Leu Gly Val Ser Thr Arg Arg Ser Thr
65                  70                  75                  80

Val Arg Arg Lys Glu Pro Thr Lys Ala Gly Met His Thr Ala Cys Ser
                85                  90                  95

Glu Glu Met Glu Pro Glu Asn Arg Glu Asn
            100                 105

<210> 437
<211> 143
<212> PRT
<213> Homo sapiens

<400> 437

<210> 438
<211> 27

<212> PRT
<213> Homo sapiens

<400> 438
Arg Gly Lys Asn Arg Ala Glu Arg Thr Val Ile Ser Ser Ser Ser His
1               5                   10                  15

Ser Cys His Leu Ile Asp Leu Glu Phe Gln Pro
            20                  25


<210> 439
<211> 32
<212> PRT
<213> Homo sapiens

<400> 439
Leu Gly Leu Gly Val Ser Thr Arg Arg Ser Thr Val Arg Arg Lys Glu
1               5                   10                  15

Pro Thr Lys Ala Gly Met His Thr Ala Cys Ser Glu Glu Met Glu Pro
            20                  25                  30


<210> 440
<211> 24
<212> PRT
<213> Homo sapiens

<400> 440
Gly Asp Pro Pro Ala Leu Ala Ser Gln Ser Ala Gly Ile Thr Gly Val
1               5                   10                  15

Ser His Ser Thr Arg Pro Lys Leu
            20


<210> 441
<211> 25
<212> PRT
<213> Homo sapiens

<400> 441
Ala Leu Tyr Ser Ile Gln Trp Ala Leu Leu Ala Asn Ser Leu Tyr Phe
1               5                   10                  15

Gln Val Pro Ser Pro Leu Ser Met Leu
            20                  25


<210> 442
<211> 35
<212> PRT
<213> Homo sapiens

<400> 442
Asp Arg Ile Leu Leu Phe Tyr Ser Arg Asp Gly Gln Thr Thr Ser Lys
1               5                   10                  15

Gly Pro Asn Pro Ala Cys Cys Leu Phe Leu Leu Lys Lys Phe Tyr Trp
            20                  25                  30

Asn Thr Ala
        35


<210> 443
<211> 21
<212> PRT
<213> Homo sapiens

<400> 443
Asp Gly Gln Thr Thr Ser Lys Gly Pro Asn Pro Ala Cys Cys Leu Phe
1               5                   10                  15

Leu Leu Lys Lys Phe
            20


<210> 444
<211> 24
<212> PRT
<213> Homo sapiens

<400> 444
Asp Pro Arg Val Arg Arg Thr Leu Asp Leu Gly Ile Thr Leu Tyr Leu
1               5                   10                  15

Phe Leu Tyr Ile Phe Leu Ser Leu
            20


<210> 445
<211> 244
<212> PRT
<213> Homo sapiens

<400> 445
Pro Ala Leu Gly Glu Cys Cys Leu Asp Ala Phe Leu Phe Leu Leu Gly
1               5                   10                  15

Lys Gln Leu Lys Lys Ser Gly Glu Lys Pro Leu Leu Gly Gly Ser Leu
            20                  25                  30

Met Glu Tyr Ala Ile Leu Ser Ala Ile Ala Ala Met Asn Glu Pro Lys
        35                  40                  45

Thr Cys Ser Thr Thr Ala Leu Lys Lys Tyr Val Leu Glu Asn His Pro
    50                  55                  60

Gly Thr Asn Ser Asn Tyr Gln Met His Leu Leu Lys Lys Thr Leu Gln
65                  70                  75                  80

Lys Cys Glu Lys Asn Gly Trp Met Glu Gln Ile Ser Gly Lys Gly Phe
                85                  90                  95

Ser Gly Thr Phe Gln Leu Cys Phe Pro Tyr Tyr Pro Ser Pro Gly Val
            100                 105                 110

Leu Phe Pro Lys Lys Glu Pro Asp Asp Ser Arg Asp Glu Asp Glu Asp
        115                 120                 125

Glu Asp Glu Ser Ser Glu Glu Asp Ser Glu Asp Glu Glu Pro Pro Pro
    130                 135                 140

Lys Arg Arg Leu Gln Lys Lys Thr Pro Ala Lys Ser Pro Gly Lys Ala
145                 150                 155                 160

Ala Ser Val Lys Gln Arg Gly Ser Lys Pro Ala Pro Lys Val Ser Ala
                165                 170                 175

Ala Gln Arg Gly Lys Ala Arg Pro Leu Pro Lys Lys Ala Pro Pro Lys
            180                 185                 190

Ala Lys Thr Pro Ala Lys Lys Thr Arg Pro Ser Ser Thr Val Ile Lys
        195                 200                 205

Lys Pro Ser Gly Gly Ser Ser Lys Lys Pro Ala Thr Ser Ala Arg Lys
    210                 215                 220

Glu Val Lys Leu Pro Gly Lys Gly Lys Ser Thr Met Lys Lys Ser Phe
225                 230                 235                 240

Arg Val Lys Lys



<210> 446
<211> 152
<212> PRT
<213> Homo sapiens

<400> 446
Asp Phe Glu Phe His His Asp Thr Leu Phe Ser Tyr Lys Ile Tyr Phe
1               5                   10                  15

Phe Thr Leu Lys Asp Phe Phe Met Val Asp Leu Pro Leu Pro Gly Asn
            20                  25                  30

Phe Thr Ser Phe Leu Ala Leu Val Ala Gly Phe Phe Glu Glu Pro Pro
        35                  40                  45

Leu Gly Phe Leu Met Thr Val Asp Glu Gly Leu Val Phe Leu Ala Gly
    50                  55                  60

Val Leu Ala Leu Gly Gly Ala Phe Leu Gly Lys Gly Leu Ala Phe Pro
65                  70                  75                  80

Arg Trp Ala Ala Glu Thr Leu Gly Ala Gly Leu Asp Pro Leu Cys Phe
                85                  90                  95

Thr Asp Ala Ala Phe Pro Gly Asp Leu Ala Gly Val Phe Phe Cys Asn
            100                 105                 110

Leu Leu Leu Gly Gly Gly Ser Ser Ser Ser Glu Ser Ser Ser Asp Asp
        115                 120                 125

Ser Ser Ser Ser Ser Ser Ser Ser Leu Glu Ser Ser Gly Ser Phe Phe
    130                 135                 140

Gly Asn Arg Thr Pro Gly Leu Gly
145                 150


<210> 447
<211> 28
<212> PRT
<213> Homo sapiens

<400> 447
Cys Leu Asp Ala Phe Leu Phe Leu Leu Gly Lys Gln Leu Lys Lys Ser
 1               5                  10                  15

Gly Glu Lys Pro Leu Leu Gly Gly Ser Leu Met Glu
            20                  25


<210> 448
<211> 30
<212> PRT
<213> Homo sapiens

<400> 448
Tyr Gln Met His Leu Leu Lys Lys Thr Leu Gln Lys Cys Glu Lys Asn
 1               5                  10                  15

Gly Trp Met Glu Gln Ile Ser Gly Lys Gly Phe Ser Gly Thr
            20                  25                  30


<210> 449
<211> 28
<212> PRT
<213> Homo sapiens

<400> 449
Lys Thr Pro Ala Lys Ser Pro Gly Lys Ala Ala Ser Val Lys Gln Arg
 1               5                  10                  15

Gly Ser Lys Pro Ala Pro Lys Val Ser Ala Ala Gln
            20                  25


<210> 450
<211> 28
<212> PRT
<213> Homo sapiens

<400> 450

Ser Ser Lys Lys Pro Ala Thr Ser Ala Arg Lys Glu Val Lys Leu Pro
1               5                   10                  15

Gly Lys Gly Lys Ser Thr Met Lys Lys Ser Phe Arg
            20                  25


<210> 451
<211> 23
<212> PRT
<213> Homo sapiens

<400> 451
Val Asp Glu Gly Leu Val Phe Leu Ala Gly Val Leu Ala Leu Gly Gly
1               5                   10                  15

Ala Phe Leu Gly Lys Gly Leu
            20


<210> 452
<211> 25
<212> PRT
<213> Homo sapiens

<400> 452
Gly Leu Asp Pro Leu Cys Phe Thr Asp Ala Ala Phe Pro Gly Asp Leu
1               5                   10                  15

Ala Gly Val Phe Phe Cys Asn Leu Leu
            20                  25


<210> 453
<211> 59
<212> PRT
<213> Homo sapiens

<400> 453
Thr Met Leu Phe Tyr Leu Ser Ser Gln Pro Asp Trp Gln Leu Asp Phe
1               5                   10                  15

Phe Arg Val Ser Phe Asn Gly Pro Val Phe Phe Ile Ile Ile Phe Asn
            20                  25                  30

Asp Arg Ala Gly Phe Arg Met Gln Ala Leu Val Ser Gln Ala Ala Cys
        35                  40                  45

Arg Arg Ser Arg Tyr Lys Leu Ser Val Val Tyr
    50                  55


<210> 454
<211> 23
<212> PRT
<213> Homo sapiens

<400> 454

Asp Arg Ala Gly Phe Arg Met Gln Ala Leu Val Ser Gln Ala Ala Cys
1               5                   10                  15

Arg Arg Ser Arg Tyr Lys Leu
            20

<210> 455
<211> 22
<212> PRT
<213> Homo sapiens

<400> 455
Leu Ala Ala Gly Ile Leu Asn Ser Ser Leu Pro Ala Leu Tyr His Ser
1               5                   10                  15

Val Glu Glu Ile Ser Gln
            20

<210> 456
<211> 45
<212> PRT
<213> Homo sapiens

<400> 456
Xaa Tyr Arg Met Asn Thr Lys Phe Leu Glu Ser Tyr Lys Met Ser Thr
1               5                   10                  15

Thr Leu Ser Arg Arg His Gln Asn Val Ser Leu Cys Lys Asp Met Lys
            20                  25                  30

Thr Pro Ala Gly Thr Asp Thr Lys Ile Ala Phe Leu Glu
        35                  40                  45

<210> 457
<211> 21
<212> PRT
<213> Homo sapiens

<400> 457
Ser Tyr Lys Met Ser Thr Thr Leu Ser Arg Arg His Gln Asn Val Ser
1               5                   10                  15

Leu Cys Lys Asp Met
            20

<210> 458
<211> 57
<212> PRT
<213> Homo sapiens

<400> 458
Ile Cys Ile Glu Ser Leu Met Leu His Tyr Ile Ala Leu Val Phe Glu
1               5                   10                  15

Met Ala Phe Met Phe Pro Leu Val Tyr His Glu Met Gly Ser Asp Ser
            20                  25                  30

Ile Arg Phe His Leu Cys Gln Val Asp Ser Cys Leu Pro Ser Met Met
        35                  40                  45

Arg Phe Phe Phe Ser Phe Pro Phe Leu
    50                  55


<210> 459
<211> 21
<212> PRT
<213> Homo sapiens

<400> 459
Tyr Ile Ala Leu Val Phe Glu Met Ala Phe Met Phe Pro Leu Val Tyr
1               5                   10                  15

His Glu Met Gly Ser
            20


<210> 460
<211> 21
<212> PRT
<213> Homo sapiens

<400> 460
Ser Asp Ser Ile Arg Phe His Leu Cys Gln Val Asp Ser Cys Leu Pro
1               5                   10                  15

Ser Met Met Arg Phe
            20


<210> 461
<211> 115
<212> PRT
<213> Homo sapiens

<400> 461



<210> 462
<211> 111
<212> PRT
<213> Homo sapiens

<220>
<221> SITE
<222> (82)
<223> Xaa equals any of the naturally occurring L-amino acids

<400> 462
Phe Thr Asn Lys Ser Cys Ser Lys Met Ser Ser Thr His Leu Tyr Lys
  1               5                  10                  15

Gly Ser Asp Val Leu Cys Tyr Ala Arg Ser Ser Glu Ser Met Ser Leu
             20                  25                  30

Ser Cys Gly Asp Val Ala Asn Ala Gly Arg Leu Thr Pro Arg Leu His
         35                  40                  45

Leu Ala Arg Ser Ala Ser Gln Gly Pro Pro Thr Leu Pro Arg Val Pro
     50                  55                  60

Pro Arg Gly Ser Arg Pro Pro Thr Ala Gly Glu Ser Pro Ala Pro Arg
 65                  70                  75                  80

Thr Xaa Ser Leu Glu Asn His Lys Asn Ile Asp His Leu Ser Ser Asn
                 85                  90                  95

Ser His Gly Lys Phe Arg Ile Tyr Gly Gln Asn Asp Ile Lys Ile
            100                 105                 110



<210> 463
<211> 80
<212> PRT
<213> Homo sapiens

<400> 463
Gln Asp Val Ile Tyr Thr Phe Val Gln Arg Phe Arg Arg Pro Met Leu
  1               5                  10                  15

Cys Thr Ile Leu Arg Lys Tyr Glu Pro Val Val Arg Gly Arg Arg Lys
             20                  25                  30

Arg Trp Gln Ala His Pro Ser Ser Ala Phe Gly Lys Lys Arg Leu Pro
         35                  40                  45

Arg Pro Pro His Pro Ala Gln Gly Ala Pro Gln Arg Glu Gln Ala Ser

50                  55                  60

His Ser Trp Arg Glu Pro Gly Pro Gln Asn Thr Phe Pro Arg Lys Pro
65                  70                  75                  80

<210> 464
<211> 22
<212> PRT
<213> Homo sapiens

<400> 464
Arg Glu Glu Thr Asp Val Gly Glu Gly Gly Arg Pro Arg Gly Gly Gln
1               5                   10                  15

Ser Glu Gly Ala Arg Val
            20

<210> 465
<211> 27
<212> PRT
<213> Homo sapiens

<400> 465
Gly Pro Gly Leu Ala Pro Val Val Phe Val Ser Thr Leu Pro Ser Leu
1               5                   10                  15

Gln Pro Ser Ser Phe Cys Gly Trp Asp Leu Pro
            20                  25

<210> 466
<211> 24
<212> PRT
<213> Homo sapiens

<400> 466
Met Ser Ser Thr His Leu Tyr Lys Gly Ser Asp Val Leu Cys Tyr Ala
1               5                   10                  15

Arg Ser Ser Glu Ser Met Ser Leu
            20

<210> 467
<211> 28
<212> PRT
<213> Homo sapiens

<400> 467
Ser Gln Gly Pro Pro Thr Leu Pro Arg Val Pro Pro Arg Gly Ser Arg
1               5                   10                  15

Pro Pro Thr Ala Gly Glu Ser Pro Ala Pro Arg Thr

20                  25

<210> 468
<211> 25
<212> PRT
<213> Homo sapiens

<400> 468
Arg Phe Arg Arg Pro Met Leu Cys Thr Ile Leu Arg Lys Tyr Glu Pro
1               5                   10                  15

Val Val Arg Gly Arg Arg Lys Arg Trp
            20                  25

<210> 469
<211> 24
<212> PRT
<213> Homo sapiens

<400> 469
Arg Leu Pro Arg Pro Pro His Pro Ala Gln Gly Ala Pro Gln Arg Glu
1               5                   10                  15

Gln Ala Ser His Ser Trp Arg Glu
            20

<210> 470
<211> 81
<212> PRT
<213> Homo sapiens

<220>
<221> SITE
<222> (43)
<223> Xaa equals any of the naturally occurring L-amino acids

<400> 470
Arg Gly Met Arg Gly Arg Trp Leu Val Ser Ser Gly Ala Ala Phe Pro
1               5                   10                  15

Ile Pro Leu Asn Gly Phe Cys Glu Ser Arg Glu Phe Phe Pro Asp Ser
            20                  25                  30

Gly Ser Val Leu Leu His Trp Arg Pro Asn Xaa Val Leu Ile Glu Ile
        35                  40                  45

Lys Val Phe Gly Ser Arg Ser Gln Ser Leu Ile Ser Ser Lys Asn Leu
    50                  55                  60

Lys Thr Ser Leu Thr Phe Ile Tyr Gly Lys Val Glu Glu Val Leu Asn
65                  70                  75                  80

Asn

<210> 471
<211> 81
<212> PRT
<213> Homo sapiens

<220>
<221> SITE
<222> (62)
<223> Xaa equals any of the naturally occurring L-amino acids

<400> 471
Leu Lys Leu Ser Ser Ala Asp Ser Gln Ala Ile Met Asn Ile Phe Ser
1               5                   10                  15

Ala Asp Cys Met Pro Arg Leu His Ile Ala Leu Gln Thr Glu Met Ile
            20                  25                  30

Pro Asn Arg Ala Pro Gln Gly Gly Ala Ala Ala Asn Leu Trp His Glu
        35                  40                  45

Ala Gln Tyr Arg Arg Leu Pro Phe Ser Arg Ala Pro Glu Xaa Thr Asp
    50                  55                  60

Ala His Gln Ala Ser Ala Gln Arg Gly Ala Ala Gln Leu Pro Arg Glu
65                  70                  75                  80

Gln

<210> 472
<211> 28
<212> PRT
<213> Homo sapiens

<220>
<221> SITE
<222> (28)
<223> Xaa equals any of the naturally occurring L-amino acids

<400> 472
Pro Ile Pro Leu Asn Gly Phe Cys Glu Ser Arg Glu Phe Phe Pro Asp
1               5                   10                  15

Ser Gly Ser Val Leu Leu His Trp Arg Pro Asn Xaa
            20                  25

<210> 473
<211> 29
<212> PRT
<213> Homo sapiens

<400> 473
Asn Ile Phe Ser Ala Asp Cys Met Pro Arg Leu His Ile Ala Leu Gln
1               5                   10                  15

Thr Glu Met Ile Pro Asn Arg Ala Pro Gln Gly Gly Ala
            20                  25

<210> 474
<211> 37
<212> PRT
<213> Homo sapiens

<400> 474
Thr Phe Arg Leu Val Ser Ala His Leu Lys Thr Arg Lys Leu Ile Asn
1               5                   10                  15

Pro Glu Ala Ala Glu Arg Arg Trp Arg Asp Trp Asp Ser Arg Gln Gly
            20                  25                  30

Trp Leu Ser Val Lys
        35

<210> 475
<211> 21
<212> PRT
<213> Homo sapiens

<400> 475
Lys Thr Arg Lys Leu Ile Asn Pro Glu Ala Ala Glu Arg Arg Trp Arg
1               5                   10                  15

Asp Trp Asp Ser Arg
            20

<210> 476
<211> 83
<212> PRT
<213> Homo sapiens

<400> 476
Trp Asn Tyr Thr Val Asn Asn Leu Tyr Leu Phe Ser Phe Ser Ile Val
1               5                   10                  15

Ser Met Lys Phe Met His Val Leu Ser Ile Asn Ile Phe Phe Gly Arg
            20                  25                  30

Ala Arg Trp Leu Thr Pro Val Ile Pro Ala Leu Leu Glu Ala Glu Ala
        35                  40                  45

Gly Gly Ser Leu Gly Gln Glu Phe Lys Thr Ser Leu Gly Lys Asp Gly
    50                  55                  60

Glu Thr Pro Ser Leu Leu Lys Ile Gln Lys Leu Ala Gly His Gly Gly
65                  70                  75                  80

Arg Arg Leu

<210> 477
<211> 76
<212> PRT
<213> Homo sapiens

<400> 477


<210> 478
<211> 28
<212> PRT
<213> Homo sapiens

<400> 478
Val Ile Pro Ala Leu Leu Glu Ala Glu Ala Gly Gly Ser Leu Gly Gln
1               5                   10                  15

Glu Phe Lys Thr Ser Leu Gly Lys Asp Gly Glu Thr
            20                  25


<210> 479
<211> 29
<212> PRT
<213> Homo sapiens

<400> 479
Asn Arg Leu Asn Leu Gly Asp Arg Gly Cys Thr Glu Leu Leu His Ser
1               5                   10                  15

Ser Leu Gly Asn Arg Val Arg Leu Ser Lys Lys Lys Glu
            20                  25


<210> 480
<211> 17
<212> PRT
<213> Homo sapiens

<400> 480
His Ala Ser Glu His Leu Ala Ala Leu Pro Val Asn Val Lys Ile Gly
1               5                   10                  15

Lys

<210> 481
<211> 77
<212> PRT
<213> Homo sapiens

<400> 481
Leu Val Cys Ile Leu Leu Val His Trp Ile Pro Pro Leu Gly Ala Trp
1               5                   10                  15

Gly Leu Ser Leu Met Leu Phe Leu Ile Leu Glu Gln Arg Cys Gly Lys
            20                  25                  30

Gly Lys Trp Arg Asn Ala Leu Leu Ser Val Ser Phe Ser Val Pro Gln
        35                  40                  45

Leu Gln Met Gln Lys Val Ser Leu Asp Ser Thr Pro Leu Asn Val Asn
    50                  55                  60

His Asp Lys Met Asp Ile Trp Lys Leu Thr Pro Lys Leu
65                  70                  75


<210> 482
<211> 57
<212> PRT
<213> Homo sapiens

<400> 482
Ile Met Ile Lys Trp Ile Phe Gly Asn Leu Leu Leu Ser Cys Asp Leu
1               5                   10                  15

Gly Cys Ile Ser Thr Ser Gly Leu Pro Gln Tyr Gln Gly Leu Arg Leu
            20                  25                  30

Leu Asn Phe Glu Tyr Ser Leu Gly Phe Met Leu Arg Ser Leu Trp Ser
        35                  40                  45

Arg Ser Ala Ile Gln Cys Phe Phe Ser
    50                  55


<210> 483
<211> 21
<212> PRT
<213> Homo sapiens

<400> 483
Leu Leu Leu Ser Cys Asp Leu Gly Cys Ile Ser Thr Ser Gly Leu Pro
1               5                   10                  15

Gln Tyr Gln Gly Leu
            20


<210> 484

<211> 21
<212> PRT
<213> Homo sapiens

<400> 484
Leu Arg Leu Leu Asn Phe Glu Tyr Ser Leu Gly Phe Met Leu Arg Ser
1               5                   10                  15

Leu Trp Ser Arg Ser
            20


<210> 485
<211> 78
<212> PRT
<213> Homo sapiens

<400> 485
Ala Ser Pro His Leu Phe Ile Glu Lys Trp Gly Arg Ala Phe Ile Leu
1               5                   10                  15

Arg Lys Leu Leu Leu Val Pro Val Ile Ser Lys Arg Ile Ile Asn Ile
            20                  25                  30

Met Ala His Gln Val Lys Pro Pro Ile Phe Cys Ala Met Ile Met Cys
        35                  40                  45

Asn Leu Phe Cys Ser Gly Tyr Glu His Leu Leu Phe Thr Leu Met Arg
    50                  55                  60

Phe Phe Ser Phe Glu Gln Ile Phe Asp Glu Val Val Phe His
65                  70                  75


<210> 486
<211> 25
<212> PRT
<213> Homo sapiens

<400> 486
Lys Leu Leu Leu Val Pro Val Ile Ser Lys Arg Ile Ile Asn Ile Met
1               5                   10                  15

Ala His Gln Val Lys Pro Pro Ile Phe
            20                  25


<210> 487
<211> 358
<212> PRT
<213> Homo sapiens

<220>
<221> SITE
<222> (352)
<223> Xaa equals any of the naturally occurring L-amino acids

<220>

<221> SITE
<222> (356)
<223> Xaa equals any of the naturally occurring L-amino acids

<400> 487
Phe Ala Val Ile Arg Phe Glu Ser Ile Ile His Glu Phe Asp Pro Trp
1               5                   10                  15

Phe Asn Tyr Arg Ser Thr His His Leu Ala Ser His Gly Phe Tyr Glu
            20                  25                  30

Phe Leu Asn Trp Phe Asp Glu Arg Ala Trp Tyr Pro Leu Gly Arg Ile
        35                  40                  45

Val Gly Gly Thr Val Tyr Pro Gly Leu Met Ile Thr Ala Gly Leu Ile
    50                  55                  60

His Trp Ile Leu Asn Thr Leu Asn Ile Thr Val His Ile Arg Asp Val
65                  70                  75                  80

Cys Val Phe Leu Ala Pro Thr Phe Ser Gly Leu Thr Ser Ile Ser Thr
                85                  90                  95

Phe Leu Leu Thr Arg Glu Leu Trp Asn Gln Gly Ala Gly Leu Leu Ala
            100                 105                 110

Ala Cys Phe Ile Ala Ile Val Pro Gly Tyr Ile Ser Arg Ser Val Ala
        115                 120                 125

Gly Ser Phe Asp Asn Glu Gly Ile Ala Ile Phe Ala Leu Gln Phe Thr
    130                 135                 140

Tyr Tyr Leu Trp Val Lys Ser Val Lys Thr Gly Ser Val Phe Trp Thr
145                 150                 155                 160

Met Cys Cys Cys Leu Ser Tyr Phe Tyr Met Val Ser Ala Trp Gly Gly
                165                 170                 175

Tyr Val Phe Ile Ile Asn Leu Ile Pro Leu His Val Phe Val Leu Leu
            180                 185                 190

Leu Met Gln Arg Tyr Ser Lys Arg Val Tyr Ile Ala Tyr Ser Thr Phe
        195                 200                 205

Tyr Ile Val Gly Leu Ile Leu Ser Met Gln Ile Pro Phe Val Gly Phe
    210                 215                 220

Gln Pro Ile Arg Thr Ser Glu His Met Ala Ala Ala Gly Val Phe Ala
225                 230                 235                 240

Leu Leu Gln Ala Tyr Ala Phe Leu Gln Tyr Leu Arg Asp Arg Leu Thr
                245                 250                 255

Lys Gln Glu Phe Gln Thr Leu Phe Phe Leu Gly Val Ser Leu Ala Ala
            260                 265                 270

Gly Ala Val Phe Leu Ser Val Ile Tyr Leu Thr Tyr Thr Gly Tyr Ile
        275                 280                 285

Ala Pro Trp Ser Gly Arg Phe Tyr Ser Leu Trp Asp Thr Gly Tyr Ala
    290                 295                 300

Lys Ile His Ile Pro Ile Ile Ala Ser Val Ser Glu His Gln Pro Thr
305                 310                 315                 320

Thr Trp Val Ser Phe Phe Phe Asp Leu His Ile Leu Val Cys Thr Phe
                325                 330                 335

Pro Ala Gly Leu Trp Phe Cys Ile Lys Asn Ile Asn Asp Glu Arg Xaa
            340                 345                 350

Phe Gly Lys Xaa Gly Phe
        355


<210> 488
<211> 27
<212> PRT
<213> Homo sapiens

<400> 488
Glu Phe Asp Pro Trp Phe Asn Tyr Arg Ser Thr His His Leu Ala Ser
  1               5                  10                  15

His Gly Phe Tyr Glu Phe Leu Asn Trp Phe Asp
            20                  25


<210> 489
<211> 23
<212> PRT
<213> Homo sapiens

<400> 489
Thr Arg Glu Leu Trp Asn Gln Gly Ala Gly Leu Leu Ala Ala Cys Phe
  1               5                  10                  15

Ile Ala Ile Val Pro Gly Tyr
            20


<210> 490
<211> 22
<212> PRT
<213> Homo sapiens

<400> 490
Thr Tyr Tyr Leu Trp Val Lys Ser Val Lys Thr Gly Ser Val Phe Trp
  1               5                  10                  15

Thr Met Cys Cys Cys Leu
            20


<210> 491
<211> 25

<212> PRT
<213> Homo sapiens

<400> 491
Gly Val Phe Ala Leu Leu Gln Ala Tyr Ala Phe Leu Gln Tyr Leu Arg
1               5                   10                  15

Asp Arg Leu Thr Lys Gln Glu Phe Gln
            20                  25


<210> 492
<211> 27
<212> PRT
<213> Homo sapiens

<400> 492
Tyr Ser Leu Trp Asp Thr Gly Tyr Ala Lys Ile His Ile Pro Ile Ile
1               5                   10                  15

Ala Ser Val Ser Glu His Gln Pro Thr Thr Trp
            20                  25


<210> 493
<211> 408
<212> PRT
<213> Homo sapiens

<220>
<221> SITE
<222> (20)
<223> Xaa equals any of the naturally occurring L-amino acids

<400> 493
Met Gly His Met Leu Tyr Leu Leu Gly Asn Ile Asn Lys Arg Thr Met
1               5                   10                  15

His Lys Tyr Xaa Gln Glu Ser Lys Lys Ala Gly Lys Ala Ser Phe Ala
            20                  25                  30

Tyr Ala Trp Val Leu Asp Glu Thr Gly Glu Glu Arg Glu Arg Gly Val
        35                  40                  45

Thr Met Asp Val Gly Met Thr Lys Phe Glu Thr Thr Thr Lys Val Ile
    50                  55                  60

Thr Leu Met Asp Ala Pro Gly His Lys Asp Phe Ile Pro Asn Met Ile
65                  70                  75                  80

Thr Gly Ala Ala Gln Ala Asp Val Ala Val Leu Val Val Asp Ala Ser
                85                  90                  95

Arg Gly Glu Phe Glu Ala Gly Phe Glu Thr Gly Gly Gln Thr Arg Glu
            100                 105                 110

His Gly Leu Leu Val Arg Ser Leu Gly Val Thr Gln Leu Ala Val Ala
        115                 120                 125

Val Asn Lys Met Asp Gln Val Asn Trp Gln Gln Glu Arg Phe Gln Glu
    130                 135                 140

Ile Thr Gly Lys Leu Gly His Phe Leu Lys Gln Ala Gly Phe Lys Glu
145                 150                 155                 160

Ser Asp Val Gly Phe Ile Pro Thr Ser Gly Leu Ser Gly Glu Asn Leu
                165                 170                 175

Ile Thr Arg Ser Gln Ser Ser Glu Leu Thr Lys Trp Tyr Lys Gly Leu
            180                 185                 190

Cys Leu Leu Glu Gln Ile Asp Ser Phe Lys Pro Pro Gln Arg Ser Ile
        195                 200                 205

Asp Lys Pro Phe Arg Leu Cys Val Ser Asp Val Phe Lys Asp Gln Gly
    210                 215                 220

Ser Gly Phe Cys Ile Thr Gly Lys Ile Glu Ala Gly Tyr Ile Gln Thr
225                 230                 235                 240

Gly Asp Arg Leu Leu Ala Met Pro Pro Asn Glu Thr Cys Thr Val Lys
                245                 250                 255

Gly Ile Thr Leu His Asp Glu Pro Val Asp Trp Ala Ala Ala Gly Asp
            260                 265                 270

His Val Ser Leu Thr Leu Val Gly Met Asp Ile Ile Lys Ile Asn Val
        275                 280                 285

Gly Cys Ile Phe Cys Gly Pro Lys Val Pro Ile Lys Ala Cys Thr Arg
    290                 295                 300

Phe Arg Ala Arg Ile Leu Ile Phe Asn Ile Glu Ile Pro Ile Thr Lys
305                 310                 315                 320

Gly Phe Pro Val Leu Leu His Tyr Gln Thr Val Ser Glu Pro Ala Val
                325                 330                 335

Ile Lys Arg Leu Ile Ser Val Leu Asn Lys Ser Thr Gly Glu Val Thr
            340                 345                 350

Lys Lys Lys Pro Lys Phe Leu Thr Lys Gly Gln Asn Ala Leu Val Glu
        355                 360                 365

Leu Gln Thr Gln Arg Pro Ile Ala Leu Glu Leu Tyr Lys Asp Phe Lys
    370                 375                 380

Glu Leu Gly Arg Phe Met Leu Arg Tyr Gly Gly Ser Thr Ile Ala Ala
385                 390                 395                 400

Gly Val Val Thr Glu Ile Lys Glu
                405

<210> 494
<211> 21

<212> PRT
<213> Homo sapiens

<220>
<221> SITE
<222> (16)
<223> Xaa equals any of the naturally occurring L-amino acids

<400> 494
Leu Tyr Leu Leu Gly Asn Ile Asn Lys Arg Thr Met His Lys Tyr Xaa
1               5                   10                  15

Gln Glu Ser Lys Lys
            20


<210> 495
<211> 23
<212> PRT
<213> Homo sapiens

<400> 495
Leu Asp Glu Thr Gly Glu Glu Arg Glu Arg Gly Val Thr Met Asp Val
1               5                   10                  15

Gly Met Thr Lys Phe Glu Thr
            20


<210> 496
<211> 22
<212> PRT
<213> Homo sapiens

<400> 496
Gly His Lys Asp Phe Ile Pro Asn Met Ile Thr Gly Ala Ala Gln Ala
1               5                   10                  15

Asp Val Ala Val Leu Val
            20


<210> 497
<211> 23
<212> PRT
<213> Homo sapiens

<400> 497
Gly Phe Glu Thr Gly Gly Gln Thr Arg Glu His Gly Leu Leu Val Arg
1               5                   10                  15

Ser Leu Gly Val Thr Gln Leu
            20


<210> 498
<211> 23
<212> PRT

<213> Homo sapiens

<400> 498
Trp Gln Gln Glu Arg Phe Gln Glu Ile Thr Gly Lys Leu Gly His Phe
1               5                   10                  15

Leu Lys Gln Ala Gly Phe Lys
            20


<210> 499
<211> 22
<212> PRT
<213> Homo sapiens

<400> 499
Thr Ser Gly Leu Ser Gly Glu Asn Leu Ile Thr Arg Ser Gln Ser Ser
1               5                   10                  15

Glu Leu Thr Lys Trp Tyr
            20


<210> 500
<211> 23
<212> PRT
<213> Homo sapiens

<400> 500
Pro Gln Arg Ser Ile Asp Lys Pro Phe Arg Leu Cys Val Ser Asp Val
1               5                   10                  15

Phe Lys Asp Gln Gly Ser Gly
            20


<210> 501
<211> 22
<212> PRT
<213> Homo sapiens

<400> 501
Leu Ile Ser Val Leu Asn Lys Ser Thr Gly Glu Val Thr Lys Lys Lys
1               5                   10                  15

Pro Lys Phe Leu Thr Lys
            20


<210> 502
<211> 25
<212> PRT
<213> Homo sapiens

<400> 502
Gln Arg Pro Ile Ala Leu Glu Leu Tyr Lys Asp Phe Lys Glu Leu Gly
1               5                   10                  15

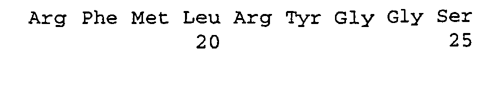

taccatctac ctgaatcccc tcctaagaga gtaccatggc aagaacatgc gcctagacaa 780
gtctgctggc gactctgccc catccgartg ggactggarg araaaggggg scgtcaccaa 840
agtcaagaac caggcatgtk tggctcctgc tgggctttct cartcactgg taacgtggag 900
ggccagtggt tcctgaaaca ggggcctgnt ctscctctcc gancargarc tcttggactg 960
tgacaaggtg gacaaggctg cctg 984

<210> 80
<211> 1247
<212> DNA
<213> Homo sapiens

<400> 80
gcgagttcat ctaactatgt gctccacgag ggatctctcc ttgagacttg acagttgatt 60
tgcagacaga agtatgccct catggaactc tcacctccag gggttcccct tagacctcac 120
atccattagg aggggtgtgg gaaaggatgc ccacgtggcc acttttacaa ctgctgtcct 180
gctcatttcc ttccctactt tgtgaaacgt tcactttctg ctccaaagat gaagtgtcac 240
gttggaaggc gggatgcttt gtgccccttc cagcaagcta acttccaaat aaattctcta 300
wttttatatc agaccttgtt cttgttaatt agactttaca tgaagtgagc aactaagctt 360
ttctgttaca agacttcatg cccacagata cattcaagtc ccaggtggaa ggatgatctg 420
gattaggcaa ggtgctgacc atggaacag gagacagtca agggaatctg aggaagcaca 480
tgtttgtgtc caatatagtc ccctccttgt gccactcaga tgtgctcatg ccctccaact 540
gtggctggtt ttataagcag atgccttgtg tagttgcacg gggtcttgtg cttgaggggc 600
tttttgcttg gattaatgtt ctgcacttgc atttttattt ttcagacagc ggatactcct 660
ccactgaaga gggaatagtt ctgcagtaat tccctagggg tttgctttct cctctcctac 720
gggcttgatg gagacaggca cagagtcctg tgatgcccac gtkgcatgcc tctagcagct 780
ttgaattctg ctggatcatc tggcacaatg ggccgagcag gttgggccag accctgtatc 840
tgctgtagag atattatatg ttctggattc cacttgaaac caggagtctt tgcattcatt 900
ctgtaaccca tctggttttg gcagaggcta ggtaggttaa ctgaacaggg atttaaaaag 960
gactttatta acacatcaga gcaatatcct ctactgtggt atttttggac tccaaaatcc 1020
ctagaggctc aataaacgcc gtgcttctct tttagtgata ggaagtatat aggaagccat 1080
ttaccttaaa caggatgttt ctgctcggca tggtggttca cgcctgtaat accagcactt 1140
tgggagacca acgcaggcag atcactggag gccaggagtt tgagactagc ctgagcaaca 1200
tggtgaaacc ccatctctac ttaaaaaaaa aaaaaaaaaa ctcgtag 1247

<210> 81
<211> 946
<212> DNA
<213> Homo sapiens

<400> 81
ggcacgagtg agattgcatc cagacagagt tttaaaagtt tcccggttga gtttaatgta 60
cagttgaagt tgagacatga atctctgcat gtaggggaaa ttttgtgtct ggttagtcaa 120
gaaactatgg aaaccaattc ttgatatttt gaaccattca cgaagatagt ttgagtcatg 180
agcatgctgt tgtctagagt gggcggggat gactcattgg agtggatgcg ctgctctgta 240
cttgattttt ttgagtctga aattagcttt ccaggctggg gcagggaggg gagcacaggt 300
gggatcagta ctgcccccaa gcggtggagc tgtggtggtg gatcaatact gctgccgcct 360
gtctgcacaa acatatttct ctcttccagc ccttcagaag tgtattggaa tatgtcgata 420
acaataatga tggtagtgaa gatgatgatg atgtgggtaa ttctggctac cttattgggt 480
ccaagctccc cacaattcgt tgcacaaagc actctacata cattctcttt agtcctgatc 540
aaaccacctt tcagagtagg atttagtgtc ctattttaaa gatgaaggag ctcgggctca 600
gagagagatc gtttagacac acacacaact ttggaatgaa acatttacag ccgggcgcgg 660
tggcgcgtgc ctgtagtccc agctacttgg gaggctgagg ctggaggatc gcttgagtcc 720
aggagttctg ggctgtagtg cgctatgccg atcgggtgtc cgcactaagt ttggcatcaa 780
tatggtgacc tcccgggagt ggaggaccac caggttgcct aaggaggggt gaaccggtcc 840
aggtcggaat gaaacattta caaaaattga catttcctta tgcatagata tttcactagg 900
tccttaaaac ccacgtgaat ctgtgattaa aaaaaaaaaa aaaaaa 946

## Claims

1. A nucleic acid molecule comprising a polynucleotide having a nucleotide sequence selected from the group consisting of:
(a) a polynucleotide having the entire nucleotide sequence of SEQ ID NO: 102;
(b) a polynucleotide having the entire cDNA sequence contained in clone HMEED18 included in ATCC Deposit No. 209368, which is hybridizable to SEQ ID NO: 102;
(c) a polynucleotide fragment of SEQ ID NO: 102;
(d) a polynucleotide fragment of the cDNA sequence contained in clone HMEED18 included in ATCC Deposit No. 209368, which is hybridizable to SEQ ID NO: 102;
(e) a polynucleotide encoding a polypeptide fragment of SEQ ID NO: 102;
(f) a polynucleotide encoding a polypeptide fragment encoded by the cDNA sequence contained in clone HMEED18 included in ATCC Deposit No. 209368, which is hybridizable to SEQ ID NO: 102;
(g) a polynucleotide encoding a polypeptide fragment of SEQ ID NO: 227 or a polypeptide fragment encoded by the cDNA sequence contained in clone HMEED18 included in ATCC Deposit No. 209368, having biological activity;
(h) a polynucleotide encoding a polypeptide fragment of SEQ ID NO: 227 or a polypeptide fragment encoded by the cDNA sequence contained in clone HMEED18 included in ATCC Deposit No. 209368, which is secreted;
(i) a polynucleotide encoding a full length polypeptide of SEQ ID NO: 227 or being encoded by the cDNA sequence contained in clone HMEED18 included in ATCC Deposit No. 209368;
(j) a polynucleotide encoding a polypeptide domain of SEQ ID NO: 227;
(k) a polynucleotide encoding a polypeptide domain encoded by the cDNA sequence contained in clone HMEED18 included in ATCC Deposit No. 209368, which is hybridizable to SEQ ID NO: 102;
(l) a polynucleotide encoding a polypeptide epitope of SEQ ID NO: 227;
(m) a polynucleotide encoding a polypeptide epitope encoded by the cDNA sequence contained in clone HMEED18 included in ATCC Deposit No. 209368, which is hybridizable to SEQ ID NO: 102;
(n) a polynucleotide which is at least 90% identical to a polynucleotide as defined in any one of (a) to (m);
(o) a polynucleotide encoding a polypeptide having an amino acid sequence at least 95% identical to the amino acid sequence of a polypeptide encoded by a polynucleotide of any one of (a) to (n);
(p) the polynucleotide of any one of (a) to (o), wherein the polynucleotide fragment comprises a nucleotide sequence encoding a secreted protein; and
(q) a polynucleotide capable of hybridizing under stringent conditions to any one of the polynucleotides specified in (a) to (p), wherein said polynucleotide does not hybridize under stringent conditions to a nucleic acid molecule having a nucleotide sequence of only A residues or of only T residues;
or the complementary strand of polynucleotide (a) to (q).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. A gene corresponding to the cDNA sequence of SEQ ID NO: 102 or encoding the polypeptide having the amino acid sequence of SEQ ID NO: 227.

5. A vector comprising the nucleic acid molecule of any one of claims 1 to 3 or the gene of claim 4.

6. A method of making a recombinant host cell comprising introducing the nucleic acid molecule of any one of claims 1 to 3, the gene of claim 4 or the vector of claim 5.

7. A recombinant host cell containing the nucleic acid molecule of claims 1 to 3, the gene of claim 4 or the vector of claim 5 or produced by the method of claim 6.

8. The recombinant host cell of claim 7 which expresses a polypeptide encoded by the nucleic acid molecule of any one of claims 1 to 3 or the gene of claim 4.

9. A method of making a polypeptide encoded by the nucleic acid molecule of any one of claims 1 to 3 or the gene of claim 4 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3, the gene of claim 4 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3, the gene of claim 4 or the antibody of claim 11.

23. A method for the production of a pharmaceutical composition comprising the steps of the method of claim 14, 17 or 18 and formulating the compound identified in a pharmaceutically acceptable form.
